# EUROPEAN PATENT APPLICATION

(11) **EP 1 382 611 A2**
(43) Date of publication of application: **21.01.2004**
(21) Application number: 03022998.3
(22) Date of filing: 27.09.1995
(51) Int. Cl.: C07K 14/32, C07K 14/325, C12N 15/62, C12Q 1/68, C12N 15/82, C12N 1/21, A01H 5/00

(54) **Bacillus strains and Pesticidal proteins**

(30) Priority: 28.09.1994 US 314594; 05.06.1995 US 463483
(62) Divisional of application: 95935394.7
(71) Applicant: Syngenta Participations AG, 4058 Basel (CH)
(72) Inventor: Warren, Gregory Wayne, Apex, NC 27502 (US); Koziel, Michael Gene, Raleigh, NC 27613 (US); Mullins, Martha Alice, Apex, NC 27502 (US); Nye, Gordon James, Morrisville, NC 27560 (US); Carr, Brian C., Raleigh, NC 27613 (US); Desai, Nalini Manoj, Chapel Hill, NC 27514 (US); Kostichka, Kristy, Wilmington, DE 19810 (US); Duck, Nicholas Brendan, Apex, NC 27502 (US); Estruch, Juan Jose, San Diego, CA 92130 (US)

(57) **Abstract**

The present invention is drawn to pesticidal strains and proteins. Bacillus strains which are capable of producing pesticidal proteins and auxiliary proteins during vegetative growth are provided. Also provided are the purified proteins, nucleotide sequences encoding the proteins and methods for using the strains, proteins and genes for controlling pests.

SEQUENCE LISTING

(1) GENERAL INFORMATION:

(A) NAME: CIBA-GEIGY AG

(B) STREET: Klybeckstr. 141

(C) CITY: Basel

(E) COUNTRY: Switzerland

(F) POSTAL CODE (ZIP): 4002

(G) TELEPHONE: +41 61 69 11 11

(H) TELEFAX: + 41 61 696 79 76

(I) TELEX: 962 991

(ii) TITLE OF INVENTION: Novel Pesticidal Proteins and Strains

(iii) NUMBER OF SEQUENCES: 52

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B

(2) INFORMATION FOR SEQ ID NO:1:

(i) SEQUENCE CHARACTERISTICS:
(A) LENGTH: 6049 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: CDS
(B) LOCATION: 1082..2467
(D) OTHER INFORMATION: /product= "VIP2A(a)"

(ix) FEATURE:
(A) NAME/KEY: misc_feature
(B) LOCATION: 2475..5126
(D) OTHER INFORMATION: /note= "Coding sequence for the 100
kd VIP1A(a) protein. This coding sequence is repeated in SEQ ID
NO:4 and translated separately."

(xi) SEQUENCE DESCRIPTION: SEQ ID NO:1:

ATCGATACAA TGTTGTTTTA CTTAGACCGG TAGTCTCTGT AATTTGTTTA ATGCTATATT 60

CTTTACTTTG ATACATTTTA ATAGCCATTT CAACCTTATC AGTATGTTTT TGTGGTCTTC 120

CTCCTTTTTT TCCACGAGCT CTAGCTGCGT TTAATCCTGT TTTGGTACGT TCGCTAATAA 180

TATCTCTTTC TAATTCTGCA ATACTTGCCA TCATTCGAAA GAAGAATTTC CCCATAGCAT 240

TAGAGGTATC AATGTTGTCA TGAATAGAAA TAAAATCTAC ACCTAGCTCT TTGAATTTTT 300

CACTTAACTC AATTAGGTGT TTTGTAGAGC GAGAAATTCG ATCAAGTTTG TAAACAACTA 360

TCTTATCGCC TTTACGTAAT ACTTTTAGCA ACTCTTCGAG TTGAGGGCGC TCTTTTTTTA 420

TTCCTGTTAT TTTCTCCTGA TATAGCCTTT CTACACCATA TTGTTGCAAA GCATCTATTT 480

GCATATCGAG ATTTTGTTCT TCTGTGCTGA CACGAGCATA ACCAAAAATC AAATTGGTTT 540

CACTTCCTAT CTAAATATAT CTATTAAAAT AGCACCAAAA ACCTTATTAA ATTAAAATAA 600

GGAACTTTGT TTTTGGATAT GGATTTTGGT ACTCAATATG GATGAGTTTT TAACGCTTTT 660

GTTAAAAAAC AAACAAGTGC CATAAACGGT CGTTTTTGGG ATGACATAAT AAATAATCTG 720

TTTGATTAAC CTAACCTTGT ATCCTTACAG CCCAGTTTTA TTTGTACTTC AACTGACTGA 780

ATATGAAAAC AACATGAAGG TTTCATAAAA TTTATATATT TTCCATAACG GATGCTCTAT 840

CTTTAGGTTA TAGTTAAATT ATAAGAAAAA AACAAACGGA GGGAGTGAAA AAAAGCATCT 900

TCTCTATAAT TTTACAGGCT CTTTAATAAG AAGGGGGGAG ATTAGATAAT AAATATGAAT 960

ATCTATCTAT AATTGTTTGC TTCTACAATA ACTTATCTAA CTTTCATATA CAACAACAAA 1020

ACAGACTAAA TCCAGATTGT ATATTCATTT TCAGTTGTTC CTTTATAAAA TAATTTCATA 1080

A ATG AAA AGA ATG GAG GGA AAG TTG TTT ATG GTG TCA AAA AAA TTA 1126
Met Lys Arg Met Glu Gly Lys Leu Phe Met Val Ser Lys Lys Leu
1               5                   10                  15

CAA GTA GTT ACT AAA ACT GTA TTG CTT AGT ACA GTT TTC TCT ATA TCT 1174
Gln Val Val Thr Lys Thr Val Leu Leu Ser Thr Val Phe Ser Ile Ser
                20                  25                  30

TTA TTA AAT AAT GAA GTG ATA AAA GCT GAA CAA TTA AAT ATA AAT TCT 1222
Leu Leu Asn Asn Glu Val Ile Lys Ala Glu Gln Leu Asn Ile Asn Ser
            35                  40                  45

CAA AGT AAA TAT ACT AAC TTG CAA AAT CTA AAA ATC ACT GAC AAG GTA 1270
Gln Ser Lys Tyr Thr Asn Leu Gln Asn Leu Lys Ile Thr Asp Lys Val
        50                  55                  60

GAG GAT TTT AAA GAA GAT AAG GAA AAA GCG AAA GAA TGG GGG AAA GAA 1318

Glu Asp Phe Lys Glu Asp Lys Glu Lys Ala Lys Glu Trp Gly Lys Glu
65                  70                  75

AAA GAA AAA GAG TGG AAA CTA ACT GCT ACT GAA AAA GGA AAA ATG AAT     1366
Lys Glu Lys Glu Trp Lys Leu Thr Ala Thr Glu Lys Gly Lys Met Asn
80                  85                  90                  95

AAT TTT TTA GAT AAT AAA AAT GAT ATA AAG ACA AAT TAT AAA GAA ATT     1414
Asn Phe Leu Asp Asn Lys Asn Asp Ile Lys Thr Asn Tyr Lys Glu Ile
                100                 105                 110

ACT TTT TCT ATG GCA GGC TCA TTT GAA GAT GAA ATA AAA GAT TTA AAA     1462
Thr Phe Ser Met Ala Gly Ser Phe Glu Asp Glu Ile Lys Asp Leu Lys
            115                 120                 125

GAA ATT GAT AAG ATG TTT GAT AAA ACC AAT CTA TCA AAT TCT ATT ATC     1510
Glu Ile Asp Lys Met Phe Asp Lys Thr Asn Leu Ser Asn Ser Ile Ile
        130                 135                 140

ACC TAT AAA AAT GTG GAA CCG ACA ACA ATT GGA TTT AAT AAA TCT TTA     1558
Thr Tyr Lys Asn Val Glu Pro Thr Thr Ile Gly Phe Asn Lys Ser Leu
    145                 150                 155

ACA GAA GGT AAT ACG ATT AAT TCT GAT GCA ATG GCA CAG TTT AAA GAA     1606
Thr Glu Gly Asn Thr Ile Asn Ser Asp Ala Met Ala Gln Phe Lys Glu
160                 165                 170                 175

CAA TTT TTA GAT AGG GAT ATT AAG TTT GAT AGT TAT CTA GAT ACG CAT     1654
Gln Phe Leu Asp Arg Asp Ile Lys Phe Asp Ser Tyr Leu Asp Thr His
                180                 185                 190

TTA ACT GCT CAA CAA GTT TCC AGT AAA GAA AGA GTT ATT TTG AAG GTT     1702
Leu Thr Ala Gln Gln Val Ser Ser Lys Glu Arg Val Ile Leu Lys Val
            195                 200                 205

ACG GTT CCG AGT GGG AAA GGT TCT ACT ACT CCA ACA AAA GCA GGT GTC     1750
Thr Val Pro Ser Gly Lys Gly Ser Thr Thr Pro Thr Lys Ala Gly Val
        210                 215                 220

ATT TTA AAT AAT AGT GAA TAC AAA ATG CTC ATT GAT AAT GGG TAT ATG     1798
Ile Leu Asn Asn Ser Glu Tyr Lys Met Leu Ile Asp Asn Gly Tyr Met
    225                 230                 235

GTC CAT GTA GAT AAG GTA TCA AAA GTG GTG AAA AAA GGG GTG GAG TGC     1846
Val His Val Asp Lys Val Ser Lys Val Val Lys Lys Gly Val Glu Cys
240                 245                 250                 255

TTA CAA ATT GAA GGG ACT TTA AAA AAG AGT CTT GAC TTT AAA AAT GAT     1894
Leu Gln Ile Glu Gly Thr Leu Lys Lys Ser Leu Asp Phe Lys Asn Asp
                260                 265                 270

ATA AAT GCT GAA GCG CAT AGC TGG GGT ATG AAG AAT TAT GAA GAG TGG     1942
Ile Asn Ala Glu Ala His Ser Trp Gly Met Lys Asn Tyr Glu Glu Trp
            275                 280                 285

GCT AAA GAT TTA ACC GAT TCG CAA AGG GAA GCT TTA GAT GGG TAT GCT 1990
Ala Lys Asp Leu Thr Asp Ser Gln Arg Glu Ala Leu Asp Gly Tyr Ala
        290                 295                 300

AGG CAA GAT TAT AAA GAA ATC AAT AAT TAT TTA AGA AAT CAA GGC GGA 2038
Arg Gln Asp Tyr Lys Glu Ile Asn Asn Tyr Leu Arg Asn Gln Gly Gly
    305                 310                 315

AGT GGA AAT GAA AAA CTA GAT GCT CAA ATA AAA AAT ATT TCT GAT GCT 2086
Ser Gly Asn Glu Lys Leu Asp Ala Gln Ile Lys Asn Ile Ser Asp Ala
320                 325                 330                 335

TTA GGG AAG AAA CCA ATA CCG GAA AAT ATT ACT GTG TAT AGA TGG TGT 2134
Leu Gly Lys Lys Pro Ile Pro Glu Asn Ile Thr Val Tyr Arg Trp Cys
                340                 345                 350

GGC ATG CCG GAA TTT GGT TAT CAA ATT AGT GAT CCG TTA CCT TCT TTA 2182
Gly Met Pro Glu Phe Gly Tyr Gln Ile Ser Asp Pro Leu Pro Ser Leu
            355                 360                 365

AAA GAT TTT GAA GAA CAA TTT TTA AAT ACA ATC AAA GAA GAC AAA GGA 2230
Lys Asp Phe Glu Glu Gln Phe Leu Asn Thr Ile Lys Glu Asp Lys Gly
        370                 375                 380

TAT ATG AGT ACA AGC TTA TCG AGT GAA CGT CTT GCA GCT TTT GGA TCT 2278
Tyr Met Ser Thr Ser Leu Ser Ser Glu Arg Leu Ala Ala Phe Gly Ser
    385                 390                 395

AGA AAA ATT ATA TTA CGA TTA CAA GTT CCG AAA GGA AGT ACG GGT GCG 2326
Arg Lys Ile Ile Leu Arg Leu Gln Val Pro Lys Gly Ser Thr Gly Ala
400                 405                 410                 415

TAT TTA AGT GCC ATT GGT GGA TTT GCA AGT GAA AAA GAG ATC CTA CTT 2374
Tyr Leu Ser Ala Ile Gly Gly Phe Ala Ser Glu Lys Glu Ile Leu Leu
                420                 425                 430

GAT AAA GAT AGT AAA TAT CAT ATT GAT AAA GTA ACA GAG GTA ATT ATT 2422
Asp Lys Asp Ser Lys Tyr His Ile Asp Lys Val Thr Glu Val Ile Ile
            435                 440                 445

AAA GGT GTT AAG CGA TAT GTA GTG GAT GCA ACA TTA TTA ACA AAT 2467
Lys Gly Val Lys Arg Tyr Val Val Asp Ala Thr Leu Leu Thr Asn
        450                 455                 460

TAAGGAGATG AAAAATATGA AGAAAAAGTT AGCAAGTGTT GTAACGTGTA CGTTATTAGC 2527

TCCTATGTTT TTGAATGGAA ATGTGAATGC TGTTTACGCA GACAGCAAAA CAAATCAAAT 2587

TTCTACAACA CAGAAAAATC AACAGAAAGA GATGGACCGA AAAGGATTAC TTGGGTATTA 2647

TTTCAAAGGA AAAGATTTTA GTAATCTTAC TATGTTTGCA CCGACACGTG ATAGTACTCT 2707

TATTTATGAT CAACAAACAG CAAATAAACT ATTAGATAAA AAACAACAAG AATATCAGTC 2767

TATTCGTTGG ATTGGTTTGA TTCAGAGTAA AGAAACGGGA GATTTCACAT TTAACTTATC 2827

TGAGGATGAA CAGGCAATTA TAGAAATCAA TGGGAAAATT ATTTCTAATA AAGGGAAAGA 2887

AAAGCAAGTT GTCCATTTAG AAAAAGGAAA ATTAGTTCCA ATCAAAATAG AGTATCAATC 2947

AGATACAAAA TTTAATATTG ACAGTAAAAC ATTTAAAGAA CTTAAATTAT TTAAAATAGA 3007

TAGTCAAAAC CAACCCCAGC AAGTCCAGCA AGATGAACTG AGAAATCCTG AATTTAACAA 3067

GAAAGAATCA CAGGAATTCT TAGCGAAACC ATCGAAAATA AATCTTTTCA CTCAAAAAAT 3127

GAAAAGGGAA ATTGATGAAG ACACGGATAC GGATGGGGAC TCTATTCCTG ACCTTTGGGA 3187

AGAAAATGGG TATACGATTC ACAATAGAAT CGCTGTAAAG TGGGACGATT CTCTAGCAAG 3247

TAAAGGGTAT ACGAAATTTG TTTCAAATCC ACTAGAAAGT CACACAGTTG GTGATCCTTA 3307

TACAGATTAT GAAAAGGCAG CAAGAGATCT AGATTTGTCA AATGCAAAGG AAACGTTTAA 3367

CCCATTGGTA GCTGCTTTTC CAAGTGTGAA TGTTAGTATG GAAAAGGTGA TATTATCACC 3427

AAATGAAAAT TTATCCAATA GTGTAGAGTC TCATTCATCC ACGAATTGGT CTTATACAAA 3487

TACAGAAGGT GCTTCTGTTG AAGCGGGGAT TGGACCAAAA GGTATTTCGT TCGGAGTTAG 3547

CGTAAACTAT CAACACTCTG AAACAGTTGC ACAAGAATGG GGAACATCTA CAGGAAATAC 3607

TTCGCAATTC AATACGGCTT CAGCGGGATA TTTAAATGCA AATGTTCGAT ATAACAATGT 3667

AGGAACTGGT GCCATCTACG ATGTAAAACC TACAACAAGT TTTGTATTAA ATAACGATAC 3727

TATCGCAACT ATTACGGCGA AATCTAATTC TACAGCCTTA AATATATCTC CTGGAGAAAG 3787

TTACCCGAAA AAAGGACAAA ATGGAATCGC AATAACATCA ATGGATGATT TTAATTCCCA 3847

TCCGATTACA TTAAATAAAA AACAAGTAGA TAATCTGCTA AATAATAAAC CTATGATGTT 3907

GGAAACAAAC CAAACAGATG GTGTTTATAA GATAAAAGAT ACACATGGAA ATATAGTAAC 3967

TGGCGGAGAA TGGAATGGTG TCATACAACA AATCAAGGCT AAAACAGCGT CTATTATTGT 4027

GGATGATGGG GAACGTGTAG CAGAAAAACG TGTAGCGGCA AAAGATTATG AAAATCCAGA 4087

AGATAAAACA CCGTCTTTAA CTTTAAAAGA TGCCCTGAAG CTTTCATATC CAGATGAAAT 4147

AAAAGAAATA GAGGGATTAT TATATTATAA AAACAAACCG ATATACGAAT CGAGCGTTAT 4207

GACTTACTTA GATGAAAATA CAGCAAAAGA AGTGACCAAA CAATTAAATG ATACCACTGG 4267

GAAATTTAAA GATGTAAGTC ATTTATATGA TGTAAAACTG ACTCCAAAAA TGAATGTTAC 4327

AATCAAATTG TCTATACTTT ATGATAATGC TGAGTCTAAT GATAACTCAA TTGGTAAATG 4387

GACAAACACA AATATTGTTT CAGGTGGAAA TAACGGAAAA AAACAATATT CTTCTAATAA 4447

TCCGGATGCT AATTTGACAT TAAATACAGA TGCTCAAGAA AAATTAAATA AAAATCGTGA 4507

CTATTATATA AGTTTATATA TGAAGTCAGA AAAAAACACA CAATGTGAGA TTACTATAGA 4567

TGGGGAGATT TATCCGATCA CTACAAAAAC AGTGAATGTG AATAAAGACA ATTACAAAAG 4627

ATTAGATATT ATAGCTCATA ATATAAAAAG TAATCCAATT TCTTCACTTC ATATTAAAAC 4687

GAATGATGAA ATAACTTTAT TTTGGGATGA TATTTCTATA ACAGATGTAG CATCAATAAA 4747

ACCGGAAAAT TTAACAGATT CAGAAATTAA ACAGATTTAT AGTAGGTATG GTATTAAGTT 4807

AGAAGATGGA ATCCTTATTG ATAAAAAAGG TGGGATTCAT TATGGTGAAT TTATTAATGA 4867

AGCTAGTTTT AATATTGAAC CATTGCAAAA TTATGTGACC AAATATGAAG TTACTTATAG 4927

TAGTGAGTTA GGACCAAACG TGAGTGACAC ACTTGAAAGT GATAAAATTT ACAAGGATGG 4987

GACAATTAAA TTTGATTTTA CCAAATATAG TAAAAATGAA CAAGGATTAT TTTATGACAG 5047

TGGATTAAAT TGGGACTTTA AAATTAATGC TATTACTTAT GATGGTAAAG AGATGAATGT 5107

TTTTCATAGA TATAATAAAT AGTTATTATA TCTATGAAGC TGGTGCTAAA GATAGTGTAA 5167

AAGTTAATAT ACTGTAGGAT TGTAATAAAA GTAATGGAAT TGATATCGTA CTTTGGAGTG 5227

GGGGATACTT TGTAAATAGT TCTATCAGAA ACATTAGACT AAGAAAAGTT ACTACCCCCA 5287

CTTGAAAATG AAGATTCAAC TGATTACAAA CAACCTGTTA AATATTATAA GGTTTTAACA 5347

AAATATTAAA CTCTTTATGT TAATACTGTA ATATAAAGAG TTTAATTGTA TTCAAATGAA 5407

GCTTTCCCAC AAAATTAGAC TGATTATCTA ATGAAATAAT CAGTCTAATT TTGTAGAACA 5467

GGTCTGGTAT TATTGTACGT GGTCACTAAA AGATATCTAA TATTATTGGG CAAGGCGTTC 5527

CATGATTGAA TCCTCGAATG TCTTGCCCTT TTCATTTATT TAAGAAGGAT TGTGGAGAAA 5587

TTATGGTTTA GATAATGAAG AAAGACTTCA CTTCTAATTT TTGATGTTAA ATAAATCAAA 5647

ATTTGGCGAT TCACATTGTT TAATCCACTG ATAAAACATA CTGGAGTGTT CTTAAAAAAT 5707

CAGCTTTTTT CTTTATAAAA TTTTGCTTAG CGTACGAAAT TCGTGTTTTG TTGGTGGGAC 5767

CCCATGCCCA TCAACTTAAG AGTAAATTAG TAATGAACTT TCGTTCATCT GGATTAAAAT 5827

AACCTCAAAT TAGGACATGT TTTTAAAAAT AAGCAGACCA AATAAGCCTA GAATAGGTAT 5887

CATTTTTAAA AATTATGCTG CTTTCTTTTG TTTTCCAAAT CCATTATACT CATAAGCAAC 5947

ACCCATAATG TCAAAGACTG TTTTTGTCTC ATATCGATAA GCTTGATATC GAATTCCTGC 6007

AGCCCGGGGG ATCCACTAGT TCTAGAGCGG CCGCCACCGC GG 6049

(2) INFORMATION FOR SEQ ID NO:2:

(i) SEQUENCE CHARACTERISTICS:
(A) LENGTH: 462 amino acids
(B) TYPE: amino acid
(D) TOPOLOGY: linear

(ii) MOLECULE TYPE: protein

(xi) SEQUENCE DESCRIPTION: SEQ ID NO:2:

Met Lys Arg Met Glu Gly Lys Leu Phe Met Val Ser Lys Lys Leu Gln
1               5                   10                  15

Val Val Thr Lys Thr Val Leu Leu Ser Thr Val Phe Ser Ile Ser Leu
            20                  25                  30

Leu Asn Asn Glu Val Ile Lys Ala Glu Gln Leu Asn Ile Asn Ser Gln
        35                  40                  45

Ser Lys Tyr Thr Asn Leu Gln Asn Leu Lys Ile Thr Asp Lys Val Glu
    50                  55                  60

Asp Phe Lys Glu Asp Lys Glu Lys Ala Lys Glu Trp Gly Lys Glu Lys
65                  70                  75                  80

Glu Lys Glu Trp Lys Leu Thr Ala Thr Glu Lys Gly Lys Met Asn Asn
                85                  90                  95

Phe Leu Asp Asn Lys Asn Asp Ile Lys Thr Asn Tyr Lys Glu Ile Thr
            100                 105                 110

Phe Ser Met Ala Gly Ser Phe Glu Asp Glu Ile Lys Asp Leu Lys Glu
        115                 120                 125

Ile Asp Lys Met Phe Asp Lys Thr Asn Leu Ser Asn Ser Ile Ile Thr
    130                 135                 140

Tyr Lys Asn Val Glu Pro Thr Thr Ile Gly Phe Asn Lys Ser Leu Thr
145                 150                 155                 160

Glu Gly Asn Thr Ile Asn Ser Asp Ala Met Ala Gln Phe Lys Glu Gln
                165                 170                 175

Phe Leu Asp Arg Asp Ile Lys Phe Asp Ser Tyr Leu Asp Thr His Leu
            180                 185                 190

Thr Ala Gln Gln Val Ser Ser Lys Glu Arg Val Ile Leu Lys Val Thr
        195                 200                 205

Val Pro Ser Gly Lys Gly Ser Thr Thr Pro Thr Lys Ala Gly Val Ile
    210                 215                 220

Leu Asn Asn Ser Glu Tyr Lys Met Leu Ile Asp Asn Gly Tyr Met Val
225                 230                 235                 240

His Val Asp Lys Val Ser Lys Val Val Lys Lys Gly Val Glu Cys Leu
                245                 250                 255

Gln Ile Glu Gly Thr Leu Lys Lys Ser Leu Asp Phe Lys Asn Asp Ile
            260                 265                 270

Asn Ala Glu Ala His Ser Trp Gly Met Lys Asn Tyr Glu Glu Trp Ala
        275                 280                 285

Lys Asp Leu Thr Asp Ser Gln Arg Glu Ala Leu Asp Gly Tyr Ala Arg
    290                 295                 300

Gln Asp Tyr Lys Glu Ile Asn Asn Tyr Leu Arg Asn Gln Gly Gly Ser
305                 310                 315                 320

Gly Asn Glu Lys Leu Asp Ala Gln Ile Lys Asn Ile Ser Asp Ala Leu
                325                 330                 335

Gly Lys Lys Pro Ile Pro Glu Asn Ile Thr Val Tyr Arg Trp Cys Gly
            340                 345                 350

Met Pro Glu Phe Gly Tyr Gln Ile Ser Asp Pro Leu Pro Ser Leu Lys
        355                 360                 365

Asp Phe Glu Glu Gln Phe Leu Asn Thr Ile Lys Glu Asp Lys Gly Tyr
    370                 375                 380

Met Ser Thr Ser Leu Ser Ser Glu Arg Leu Ala Ala Phe Gly Ser Arg
385                 390                 395                 400

Lys Ile Ile Leu Arg Leu Gln Val Pro Lys Gly Ser Thr Gly Ala Tyr
                405                 410                 415

Leu Ser Ala Ile Gly Gly Phe Ala Ser Glu Lys Glu Ile Leu Leu Asp
            420                 425                 430

Lys Asp Ser Lys Tyr His Ile Asp Lys Val Thr Glu Val Ile Ile Lys
        435                 440                 445

Gly Val Lys Arg Tyr Val Val Asp Ala Thr Leu Leu Thr Asn
    450                 455                 460

(2) INFORMATION FOR SEQ ID NO:3:

    (i) SEQUENCE CHARACTERISTICS:
        (A) LENGTH: 20 amino acids
        (B) TYPE: amino acid
        (C) STRANDEDNESS: single
        (D) TOPOLOGY: linear

    (ii) MOLECULE TYPE: peptide

    (ix) FEATURE:

(A) NAME/KEY: Peptide
(B) LOCATION: 1..20
(D) OTHER INFORMATION: /note= "Signal peptide for vacuolar
targetting"

(xi) SEQUENCE DESCRIPTION: SEQ ID NO:3:

Ser Ser Ser Ser Phe Ala Asp Ser Asn Pro Ile Arg Val Thr Asp Arg
1               5                   10                  15

Ala Ala Ser Thr
            20

(2) INFORMATION FOR SEQ ID NO:4:

(i) SEQUENCE CHARACTERISTICS:
(A) LENGTH: 2655 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: CDS
(B) LOCATION: 1..2652
(D) OTHER INFORMATION: /product= "100 kDa protein VIP1A(a)"
/note= "This sequence is identical to the portion of SEQ ID NO:1
between and including nucleotide 2475 to 5126."

(xi) SEQUENCE DESCRIPTION: SEQ ID NO:4:

ATG AAA AAT ATG AAG AAA AAG TTA GCA AGT GTT GTA ACG TGT ACG TTA      48
Met Lys Asn Met Lys Lys Lys Leu Ala Ser Val Val Thr Cys Thr Leu
        465                 470                 475

TTA GCT CCT ATG TTT TTG AAT GGA AAT GTG AAT GCT GTT TAC GCA GAC      96
Leu Ala Pro Met Phe Leu Asn Gly Asn Val Asn Ala Val Tyr Ala Asp
    480                 485                 490

AGC AAA ACA AAT CAA ATT TCT ACA ACA CAG AAA AAT CAA CAG AAA GAG     144
Ser Lys Thr Asn Gln Ile Ser Thr Thr Gln Lys Asn Gln Gln Lys Glu
495                 500                 505                 510

ATG GAC CGA AAA GGA TTA CTT GGG TAT TAT TTC AAA GGA AAA GAT TTT 192
Met Asp Arg Lys Gly Leu Leu Gly Tyr Tyr Phe Lys Gly Lys Asp Phe
515 520 525

AGT AAT CTT ACT ATG TTT GCA CCG ACA CGT GAT AGT ACT CTT ATT TAT 240
Ser Asn Leu Thr Met Phe Ala Pro Thr Arg Asp Ser Thr Leu Ile Tyr
530 535 540

GAT CAA CAA ACA GCA AAT AAA CTA TTA GAT AAA AAA CAA CAA GAA TAT 288
Asp Gln Gln Thr Ala Asn Lys Leu Leu Asp Lys Lys Gln Gln Glu Tyr
545 550 555

CAG TCT ATT CGT TGG ATT GGT TTG ATT CAG AGT AAA GAA ACG GGA GAT 336
Gln Ser Ile Arg Trp Ile Gly Leu Ile Gln Ser Lys Glu Thr Gly Asp
560 565 570

TTC ACA TTT AAC TTA TCT GAG GAT GAA CAG GCA ATT ATA GAA ATC AAT 384
Phe Thr Phe Asn Leu Ser Glu Asp Glu Gln Ala Ile Ile Glu Ile Asn
575 580 585 590

GGG AAA ATT ATT TCT AAT AAA GGG AAA GAA AAG CAA GTT GTC CAT TTA 432
Gly Lys Ile Ile Ser Asn Lys Gly Lys Glu Lys Gln Val Val His Leu
595 600 605

GAA AAA GGA AAA TTA GTT CCA ATC AAA ATA GAG TAT CAA TCA GAT ACA 480
Glu Lys Gly Lys Leu Val Pro Ile Lys Ile Glu Tyr Gln Ser Asp Thr
610 615 620

AAA TTT AAT ATT GAC AGT AAA ACA TTT AAA GAA CTT AAA TTA TTT AAA 528
Lys Phe Asn Ile Asp Ser Lys Thr Phe Lys Glu Leu Lys Leu Phe Lys
625 630 635

ATA GAT AGT CAA AAC CAA CCC CAG CAA GTC CAG CAA GAT GAA CTG AGA 576
Ile Asp Ser Gln Asn Gln Pro Gln Gln Val Gln Gln Asp Glu Leu Arg
640 645 650

AAT CCT GAA TTT AAC AAG AAA GAA TCA CAG GAA TTC TTA GCG AAA CCA 624
Asn Pro Glu Phe Asn Lys Lys Glu Ser Gln Glu Phe Leu Ala Lys Pro
655 660 665 670

TCG AAA ATA AAT CTT TTC ACT CAA AAA ATG AAA AGG GAA ATT GAT GAA 672
Ser Lys Ile Asn Leu Phe Thr Gln Lys Met Lys Arg Glu Ile Asp Glu
675 680 685

GAC ACG GAT ACG GAT GGG GAC TCT ATT CCT GAC CTT TGG GAA GAA AAT 720
Asp Thr Asp Thr Asp Gly Asp Ser Ile Pro Asp Leu Trp Glu Glu Asn
690 695 700

GGG TAT ACG ATT CAA AAT AGA ATC GCT GTA AAG TGG GAC GAT TCT CTA 768
Gly Tyr Thr Ile Gln Asn Arg Ile Ala Val Lys Trp Asp Asp Ser Leu
705 710 715

GCA AGT AAA GGG TAT ACG AAA TTT GTT TCA AAT CCA CTA GAA AGT CAC 816
Ala Ser Lys Gly Tyr Thr Lys Phe Val Ser Asn Pro Leu Glu Ser His
720 725 730

ACA GTT GGT GAT CCT TAT ACA GAT TAT GAA AAG GCA GCA AGA GAT CTA 864
Thr Val Gly Asp Pro Tyr Thr Asp Tyr Glu Lys Ala Ala Arg Asp Leu
735 740 745 750

GAT TTG TCA AAT GCA AAG GAA ACG TTT AAC CCA TTG GTA GCT GCT TTT 912
Asp Leu Ser Asn Ala Lys Glu Thr Phe Asn Pro Leu Val Ala Ala Phe
755 760 765

CCA AGT GTG AAT GTT AGT ATG GAA AAG GTG ATA TTA TCA CCA AAT GAA 960
Pro Ser Val Asn Val Ser Met Glu Lys Val Ile Leu Ser Pro Asn Glu
770 775 780

AAT TTA TCC AAT AGT GTA GAG TCT CAT TCA TCC ACG AAT TGG TCT TAT 1008
Asn Leu Ser Asn Ser Val Glu Ser His Ser Ser Thr Asn Trp Ser Tyr
785 790 795

ACA AAT ACA GAA GGT GCT TCT GTT GAA GCG GGG ATT GGA CCA AAA GGT 1056
Thr Asn Thr Glu Gly Ala Ser Val Glu Ala Gly Ile Gly Pro Lys Gly
800 805 810

ATT TCG TTC GGA GTT AGC GTA AAC TAT CAA CAC TCT GAA ACA GTT GCA 1104
Ile Ser Phe Gly Val Ser Val Asn Tyr Gln His Ser Glu Thr Val Ala
815 820 825 830

CAA GAA TGG GGA ACA TCT ACA GGA AAT ACT TCG CAA TTC AAT ACG GCT 1152
Gln Glu Trp Gly Thr Ser Thr Gly Asn Thr Ser Gln Phe Asn Thr Ala
835 840 845

TCA GCG GGA TAT TTA AAT GCA AAT GTT CGA TAT AAC AAT GTA GGA ACT 1200
Ser Ala Gly Tyr Leu Asn Ala Asn Val Arg Tyr Asn Asn Val Gly Thr
850 855 860

GGT GCC ATC TAC GAT GTA AAA CCT ACA ACA AGT TTT GTA TTA AAT AAC 1248
Gly Ala Ile Tyr Asp Val Lys Pro Thr Thr Ser Phe Val Leu Asn Asn
865 870 875

GAT ACT ATC GCA ACT ATT ACG GCG AAA TCT AAT TCT ACA GCC TTA AAT 1296
Asp Thr Ile Ala Thr Ile Thr Ala Lys Ser Asn Ser Thr Ala Leu Asn
880 885 890

ATA TCT CCT GGA GAA AGT TAC CCG AAA AAA GGA CAA AAT GGA ATC GCA 1344
Ile Ser Pro Gly Glu Ser Tyr Pro Lys Lys Gly Gln Asn Gly Ile Ala
895 900 905 910

ATA ACA TCA ATG GAT GAT TTT AAT TCC CAT CCG ATT ACA TTA AAT AAA 1392
Ile Thr Ser Met Asp Asp Phe Asn Ser His Pro Ile Thr Leu Asn Lys
915 920 925

AAA CAA GTA GAT AAT CTG CTA AAT AAT AAA CCT ATG ATG TTG GAA ACA 1440
Lys Gln Val Asp Asn Leu Leu Asn Asn Lys Pro Met Met Leu Glu Thr
930 935 940

AAC CAA ACA GAT GGT GTT TAT AAG ATA AAA GAT ACA CAT GGA AAT ATA 1488
Asn Gln Thr Asp Gly Val Tyr Lys Ile Lys Asp Thr His Gly Asn Ile

945 950 955

GTA ACT GGC GGA GAA TGG AAT GGT GTC ATA CAA CAA ATC AAG GCT AAA 1536
Val Thr Gly Gly Glu Trp Asn Gly Val Ile Gln Gln Ile Lys Ala Lys
960 965 970

ACA GCG TCT ATT ATT GTG GAT GAT GGG GAA CGT GTA GCA GAA AAA CGT 1584
Thr Ala Ser Ile Ile Val Asp Asp Gly Glu Arg Val Ala Glu Lys Arg
975 980 985 990

GTA GCG GCA AAA GAT TAT GAA AAT CCA GAA GAT AAA ACA CCG TCT TTA 1632
Val Ala Ala Lys Asp Tyr Glu Asn Pro Glu Asp Lys Thr Pro Ser Leu
995 1000 1005

ACT TTA AAA GAT GCC CTG AAG CTT TCA TAT CCA GAT GAA ATA AAA GAA 1680
Thr Leu Lys Asp Ala Leu Lys Leu Ser Tyr Pro Asp Glu Ile Lys Glu
1010 1015 1020

ATA GAG GGA TTA TTA TAT TAT AAA AAC AAA CCG ATA TAC GAA TCG AGC 1728
Ile Glu Gly Leu Leu Tyr Tyr Lys Asn Lys Pro Ile Tyr Glu Ser Ser
1025 1030 1035

GTT ATG ACT TAC TTA GAT GAA AAT ACA GCA AAA GAA GTG ACC AAA CAA 1776
Val Met Thr Tyr Leu Asp Glu Asn Thr Ala Lys Glu Val Thr Lys Gln
1040 1045 1050

TTA AAT GAT ACC ACT GGG AAA TTT AAA GAT GTA AGT CAT TTA TAT GAT 1824
Leu Asn Asp Thr Thr Gly Lys Phe Lys Asp Val Ser His Leu Tyr Asp
1055 1060 1065 1070

GTA AAA CTG ACT CCA AAA ATG AAT GTT ACA ATC AAA TTG TCT ATA CTT 1872
Val Lys Leu Thr Pro Lys Met Asn Val Thr Ile Lys Leu Ser Ile Leu
1075 1080 1085

TAT GAT AAT GCT GAG TCT AAT GAT AAC TCA ATT GGT AAA TGG ACA AAC 1920
Tyr Asp Asn Ala Glu Ser Asn Asp Asn Ser Ile Gly Lys Trp Thr Asn
1090 1095 1100

ACA AAT ATT GTT TCA GGT GGA AAT AAC GGA AAA AAA CAA TAT TCT TCT 1968
Thr Asn Ile Val Ser Gly Gly Asn Asn Gly Lys Lys Gln Tyr Ser Ser
1105 1110 1115

AAT AAT CCG GAT GCT AAT TTG ACA TTA AAT ACA GAT GCT CAA GAA AAA 2016
Asn Asn Pro Asp Ala Asn Leu Thr Leu Asn Thr Asp Ala Gln Glu Lys
1120 1125 1130

TTA AAT AAA AAT CGT GAC TAT TAT ATA AGT TTA TAT ATG AAG TCA GAA 2064
Leu Asn Lys Asn Arg Asp Tyr Tyr Ile Ser Leu Tyr Met Lys Ser Glu
1135 1140 1145 1150

AAA AAC ACA CAA TGT GAG ATT ACT ATA GAT GGG GAG ATT TAT CCG ATC 2112
Lys Asn Thr Gln Cys Glu Ile Thr Ile Asp Gly Glu Ile Tyr Pro Ile
1155 1160 1165

ACT ACA AAA ACA GTG AAT GTG AAT AAA GAC AAT TAC AAA AGA TTA GAT 2160

Thr Thr Lys Thr Val Asn Val Asn Lys Asp Asn Tyr Lys Arg Leu Asp
            1170                    1175                    1180

ATT ATA GCT CAT AAT ATA AAA AGT AAT CCA ATT TCT TCA CTT CAT ATT        2208
Ile Ile Ala His Asn Ile Lys Ser Asn Pro Ile Ser Ser Leu His Ile
        1185                    1190                    1195

AAA ACG AAT GAT GAA ATA ACT TTA TTT TGG GAT GAT ATT TCT ATA ACA        2256
Lys Thr Asn Asp Glu Ile Thr Leu Phe Trp Asp Asp Ile Ser Ile Thr
    1200                    1205                    1210

GAT GTA GCA TCA ATA AAA CCG GAA AAT TTA ACA GAT TCA GAA ATT AAA        2304
Asp Val Ala Ser Ile Lys Pro Glu Asn Leu Thr Asp Ser Glu Ile Lys
1215                    1220                    1225                    1230

CAG ATT TAT AGT AGG TAT GGT ATT AAG TTA GAA GAT GGA ATC CTT ATT        2352
Gln Ile Tyr Ser Arg Tyr Gly Ile Lys Leu Glu Asp Gly Ile Leu Ile
                1235                    1240                    1245

GAT AAA AAA GGT GGG ATT CAT TAT GGT GAA TTT ATT AAT GAA GCT AGT        2400
Asp Lys Lys Gly Gly Ile His Tyr Gly Glu Phe Ile Asn Glu Ala Ser
            1250                    1255                    1260

TTT AAT ATT GAA CCA TTG CAA AAT TAT GTG ACC AAA TAT GAA GTT ACT        2448
Phe Asn Ile Glu Pro Leu Gln Asn Tyr Val Thr Lys Tyr Glu Val Thr
        1265                    1270                    1275

TAT AGT AGT GAG TTA GGA CCA AAC GTG AGT GAC ACA CTT GAA AGT GAT        2496
Tyr Ser Ser Glu Leu Gly Pro Asn Val Ser Asp Thr Leu Glu Ser Asp
    1280                    1285                    1290

AAA ATT TAC AAG GAT GGG ACA ATT AAA TTT GAT TTT ACC AAA TAT AGT        2544
Lys Ile Tyr Lys Asp Gly Thr Ile Lys Phe Asp Phe Thr Lys Tyr Ser
1295                    1300                    1305                    1310

AAA AAT GAA CAA GGA TTA TTT TAT GAC AGT GGA TTA AAT TGG GAC TTT        2592
Lys Asn Glu Gln Gly Leu Phe Tyr Asp Ser Gly Leu Asn Trp Asp Phe
                1315                    1320                    1325

AAA ATT AAT GCT ATT ACT TAT GAT GGT AAA GAG ATG AAT GTT TTT CAT        2640
Lys Ile Asn Ala Ile Thr Tyr Asp Gly Lys Glu Met Asn Val Phe His
            1330                    1335                    1340

AGA TAT AAT AAA TAG                                                    2655
Arg Tyr Asn Lys
        1345

(2) INFORMATION FOR SEQ ID NO:5:

    (i) SEQUENCE CHARACTERISTICS:
        (A) LENGTH: 884 amino acids
        (B) TYPE: amino acid
        (D) TOPOLOGY: linear

(ii) MOLECULE TYPE: protein

(xi) SEQUENCE DESCRIPTION: SEQ ID NO:5:

Met Lys Asn Met Lys Lys Lys Leu Ala Ser Val Val Thr Cys Thr Leu
1               5                   10                  15

Leu Ala Pro Met Phe Leu Asn Gly Asn Val Asn Ala Val Tyr Ala Asp
            20                  25                  30

Ser Lys Thr Asn Gln Ile Ser Thr Thr Gln Lys Asn Gln Gln Lys Glu
        35                  40                  45

Met Asp Arg Lys Gly Leu Leu Gly Tyr Tyr Phe Lys Gly Lys Asp Phe
    50                  55                  60

Ser Asn Leu Thr Met Phe Ala Pro Thr Arg Asp Ser Thr Leu Ile Tyr
65                  70                  75                  80

Asp Gln Gln Thr Ala Asn Lys Leu Leu Asp Lys Lys Gln Gln Glu Tyr
                85                  90                  95

Gln Ser Ile Arg Trp Ile Gly Leu Ile Gln Ser Lys Glu Thr Gly Asp
            100                 105                 110

Phe Thr Phe Asn Leu Ser Glu Asp Glu Gln Ala Ile Ile Glu Ile Asn
        115                 120                 125

Gly Lys Ile Ile Ser Asn Lys Gly Lys Glu Lys Gln Val Val His Leu
    130                 135                 140

Glu Lys Gly Lys Leu Val Pro Ile Lys Ile Glu Tyr Gln Ser Asp Thr
145                 150                 155                 160

Lys Phe Asn Ile Asp Ser Lys Thr Phe Lys Glu Leu Lys Leu Phe Lys
                165                 170                 175

Ile Asp Ser Gln Asn Gln Pro Gln Gln Val Gln Gln Asp Glu Leu Arg
            180                 185                 190

Asn Pro Glu Phe Asn Lys Lys Glu Ser Gln Glu Phe Leu Ala Lys Pro
        195                 200                 205

Ser Lys Ile Asn Leu Phe Thr Gln Lys Met Lys Arg Glu Ile Asp Glu
    210                 215                 220

Asp Thr Asp Thr Asp Gly Asp Ser Ile Pro Asp Leu Trp Glu Glu Asn
225                 230                 235                 240

Gly Tyr Thr Ile Gln Asn Arg Ile Ala Val Lys Trp Asp Asp Ser Leu
                245                 250                 255

Ala Ser Lys Gly Tyr Thr Lys Phe Val Ser Asn Pro Leu Glu Ser His
            260                 265                 270

Thr Val Gly Asp Pro Tyr Thr Asp Tyr Glu Lys Ala Ala Arg Asp Leu
        275                 280                 285

Asp Leu Ser Asn Ala Lys Glu Thr Phe Asn Pro Leu Val Ala Ala Phe
    290                 295                 300

Pro Ser Val Asn Val Ser Met Glu Lys Val Ile Leu Ser Pro Asn Glu
305                 310                 315                 320

Asn Leu Ser Asn Ser Val Glu Ser His Ser Ser Thr Asn Trp Ser Tyr
                325                 330                 335

Thr Asn Thr Glu Gly Ala Ser Val Glu Ala Gly Ile Gly Pro Lys Gly
            340                 345                 350

Ile Ser Phe Gly Val Ser Val Asn Tyr Gln His Ser Glu Thr Val Ala
        355                 360                 365

Gln Glu Trp Gly Thr Ser Thr Gly Asn Thr Ser Gln Phe Asn Thr Ala
    370                 375                 380

Ser Ala Gly Tyr Leu Asn Ala Asn Val Arg Tyr Asn Asn Val Gly Thr
385                 390                 395                 400

Gly Ala Ile Tyr Asp Val Lys Pro Thr Thr Ser Phe Val Leu Asn Asn
                405                 410                 415

Asp Thr Ile Ala Thr Ile Thr Ala Lys Ser Asn Ser Thr Ala Leu Asn
            420                 425                 430

Ile Ser Pro Gly Glu Ser Tyr Pro Lys Lys Gly Gln Asn Gly Ile Ala
        435                 440                 445

Ile Thr Ser Met Asp Asp Phe Asn Ser His Pro Ile Thr Leu Asn Lys
    450                 455                 460

Lys Gln Val Asp Asn Leu Leu Asn Asn Lys Pro Met Met Leu Glu Thr
465                 470                 475                 480

Asn Gln Thr Asp Gly Val Tyr Lys Ile Lys Asp Thr His Gly Asn Ile
                485                 490                 495

Val Thr Gly Gly Glu Trp Asn Gly Val Ile Gln Gln Ile Lys Ala Lys
            500                 505                 510

Thr Ala Ser Ile Ile Val Asp Asp Gly Glu Arg Val Ala Glu Lys Arg
        515                 520                 525

Val Ala Ala Lys Asp Tyr Glu Asn Pro Glu Asp Lys Thr Pro Ser Leu
    530                 535                 540

Thr Leu Lys Asp Ala Leu Lys Leu Ser Tyr Pro Asp Glu Ile Lys Glu
545                 550                 555                 560

Ile Glu Gly Leu Leu Tyr Tyr Lys Asn Lys Pro Ile Tyr Glu Ser Ser

                565                 570                 575

Val Met Thr Tyr Leu Asp Glu Asn Thr Ala Lys Glu Val Thr Lys Gln
            580                 585                 590

Leu Asn Asp Thr Thr Gly Lys Phe Lys Asp Val Ser His Leu Tyr Asp
        595                 600                 605

Val Lys Leu Thr Pro Lys Met Asn Val Thr Ile Lys Leu Ser Ile Leu
    610                 615                 620

Tyr Asp Asn Ala Glu Ser Asn Asp Asn Ser Ile Gly Lys Trp Thr Asn
625                 630                 635                 640

Thr Asn Ile Val Ser Gly Gly Asn Asn Gly Lys Lys Gln Tyr Ser Ser
                645                 650                 655

Asn Asn Pro Asp Ala Asn Leu Thr Leu Asn Thr Asp Ala Gln Glu Lys
            660                 665                 670

Leu Asn Lys Asn Arg Asp Tyr Tyr Ile Ser Leu Tyr Met Lys Ser Glu
        675                 680                 685

Lys Asn Thr Gln Cys Glu Ile Thr Ile Asp Gly Glu Ile Tyr Pro Ile
    690                 695                 700

Thr Thr Lys Thr Val Asn Val Asn Lys Asp Asn Tyr Lys Arg Leu Asp
705                 710                 715                 720

Ile Ile Ala His Asn Ile Lys Ser Asn Pro Ile Ser Ser Leu His Ile
                725                 730                 735

Lys Thr Asn Asp Glu Ile Thr Leu Phe Trp Asp Asp Ile Ser Ile Thr
            740                 745                 750

Asp Val Ala Ser Ile Lys Pro Glu Asn Leu Thr Asp Ser Glu Ile Lys
        755                 760                 765

Gln Ile Tyr Ser Arg Tyr Gly Ile Lys Leu Glu Asp Gly Ile Leu Ile
    770                 775                 780

Asp Lys Lys Gly Gly Ile His Tyr Gly Glu Phe Ile Asn Glu Ala Ser
785                 790                 795                 800

Phe Asn Ile Glu Pro Leu Gln Asn Tyr Val Thr Lys Tyr Glu Val Thr
                805                 810                 815

Tyr Ser Ser Glu Leu Gly Pro Asn Val Ser Asp Thr Leu Glu Ser Asp
            820                 825                 830

Lys Ile Tyr Lys Asp Gly Thr Ile Lys Phe Asp Phe Thr Lys Tyr Ser
        835                 840                 845

Lys Asn Glu Gln Gly Leu Phe Tyr Asp Ser Gly Leu Asn Trp Asp Phe
    850                 855                 860

Lys Ile Asn Ala Ile Thr Tyr Asp Gly Lys Glu Met Asn Val Phe His
865                 870                 875                 880

Arg Tyr Asn Lys

(2) INFORMATION FOR SEQ ID NO:6:

(i) SEQUENCE CHARACTERISTICS:
(A) LENGTH: 2004 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: CDS
(B) LOCATION: 1..2001
(D) OTHER INFORMATION: /product= "80 kDa protein VIP1A(a)"
/note= "This sequence is identical to that found in SEQ ID NO:1
between and including nucleotide positions 3126 and 5126"

(xi) SEQUENCE DESCRIPTION: SEQ ID NO:6:

ATG AAA AGG GAA ATT GAT GAA GAC ACG GAT ACG GAT GGG GAC TCT ATT      48
Met Lys Arg Glu Ile Asp Glu Asp Thr Asp Thr Asp Gly Asp Ser Ile
885                 890                 895                 900

CCT GAC CTT TGG GAA GAA AAT GGG TAT ACG ATT CAA AAT AGA ATC GCT      96
Pro Asp Leu Trp Glu Glu Asn Gly Tyr Thr Ile Gln Asn Arg Ile Ala
            905                 910                 915

GTA AAG TGG GAC GAT TCT CTA GCA AGT AAA GGG TAT ACG AAA TTT GTT     144
Val Lys Trp Asp Asp Ser Leu Ala Ser Lys Gly Tyr Thr Lys Phe Val
        920                 925                 930

TCA AAT CCA CTA GAA AGT CAC ACA GTT GGT GAT CCT TAT ACA GAT TAT     192
Ser Asn Pro Leu Glu Ser His Thr Val Gly Asp Pro Tyr Thr Asp Tyr
    935                 940                 945

GAA AAG GCA GCA AGA GAT CTA GAT TTG TCA AAT GCA AAG GAA ACG TTT     240
Glu Lys Ala Ala Arg Asp Leu Asp Leu Ser Asn Ala Lys Glu Thr Phe
    950                 955                 960

AAC CCA TTG GTA GCT GCT TTT CCA AGT GTG AAT GTT AGT ATG GAA AAG 288
Asn Pro Leu Val Ala Ala Phe Pro Ser Val Asn Val Ser Met Glu Lys
965 970 975 980

GTG ATA TTA TCA CCA AAT GAA AAT TTA TCC AAT AGT GTA GAG TCT CAT 336
Val Ile Leu Ser Pro Asn Glu Asn Leu Ser Asn Ser Val Glu Ser His
985 990 995

TCA TCC ACG AAT TGG TCT TAT ACA AAT ACA GAA GGT GCT TCT GTT GAA 384
Ser Ser Thr Asn Trp Ser Tyr Thr Asn Thr Glu Gly Ala Ser Val Glu
1000 1005 1010

GCG GGG ATT GGA CCA AAA GGT ATT TCG TTC GGA GTT AGC GTA AAC TAT 432
Ala Gly Ile Gly Pro Lys Gly Ile Ser Phe Gly Val Ser Val Asn Tyr
1015 1020 1025

CAA CAC TCT GAA ACA GTT GCA CAA GAA TGG GGA ACA TCT ACA GGA AAT 480
Gln His Ser Glu Thr Val Ala Gln Glu Trp Gly Thr Ser Thr Gly Asn
1030 1035 1040

ACT TCG CAA TTC AAT ACG GCT TCA GCG GGA TAT TTA AAT GCA AAT GTT 528
Thr Ser Gln Phe Asn Thr Ala Ser Ala Gly Tyr Leu Asn Ala Asn Val
1045 1050 1055 1060

CGA TAT AAC AAT GTA GGA ACT GGT GCC ATC TAC GAT GTA AAA CCT ACA 576
Arg Tyr Asn Asn Val Gly Thr Gly Ala Ile Tyr Asp Val Lys Pro Thr
1065 1070 1075

ACA AGT TTT GTA TTA AAT AAC GAT ACT ATC GCA ACT ATT ACG GCG AAA 624
Thr Ser Phe Val Leu Asn Asn Asp Thr Ile Ala Thr Ile Thr Ala Lys
1080 1085 1090

TCT AAT TCT ACA GCC TTA AAT ATA TCT CCT GGA GAA AGT TAC CCG AAA 672
Ser Asn Ser Thr Ala Leu Asn Ile Ser Pro Gly Glu Ser Tyr Pro Lys
1095 1100 1105

AAA GGA CAA AAT GGA ATC GCA ATA ACA TCA ATG GAT GAT TTT AAT TCC 720
Lys Gly Gln Asn Gly Ile Ala Ile Thr Ser Met Asp Asp Phe Asn Ser
1110 1115 1120

CAT CCG ATT ACA TTA AAT AAA AAA CAA GTA GAT AAT CTG CTA AAT AAT 768
His Pro Ile Thr Leu Asn Lys Lys Gln Val Asp Asn Leu Leu Asn Asn
1125 1130 1135 1140

AAA CCT ATG ATG TTG GAA ACA AAC CAA ACA GAT GGT GTT TAT AAG ATA 816
Lys Pro Met Met Leu Glu Thr Asn Gln Thr Asp Gly Val Tyr Lys Ile
1145 1150 1155

AAA GAT ACA CAT GGA AAT ATA GTA ACT GGC GGA GAA TGG AAT GGT GTC 864
Lys Asp Thr His Gly Asn Ile Val Thr Gly Gly Glu Trp Asn Gly Val
1160 1165 1170

ATA CAA CAA ATC AAG GCT AAA ACA GCG TCT ATT ATT GTG GAT GAT GGG 912
Ile Gln Gln Ile Lys Ala Lys Thr Ala Ser Ile Ile Val Asp Asp Gly

1175                1180                1185

GAA CGT GTA GCA GAA AAA CGT GTA GCG GCA AAA GAT TAT GAA AAT CCA        960
Glu Arg Val Ala Glu Lys Arg Val Ala Ala Lys Asp Tyr Glu Asn Pro
    1190                1195                1200

GAA GAT AAA ACA CCG TCT TTA ACT TTA AAA GAT GCC CTG AAG CTT TCA        1008
Glu Asp Lys Thr Pro Ser Leu Thr Leu Lys Asp Ala Leu Lys Leu Ser
1205                1210                1215                1220

TAT CCA GAT GAA ATA AAA GAA ATA GAG GGA TTA TTA TAT TAT AAA AAC        1056
Tyr Pro Asp Glu Ile Lys Glu Ile Glu Gly Leu Leu Tyr Tyr Lys Asn
                1225                1230                1235

AAA CCG ATA TAC GAA TCG AGC GTT ATG ACT TAC TTA GAT GAA AAT ACA        1104
Lys Pro Ile Tyr Glu Ser Ser Val Met Thr Tyr Leu Asp Glu Asn Thr
            1240                1245                1250

GCA AAA GAA GTG ACC AAA CAA TTA AAT GAT ACC ACT GGG AAA TTT AAA        1152
Ala Lys Glu Val Thr Lys Gln Leu Asn Asp Thr Thr Gly Lys Phe Lys
        1255                1260                1265

GAT GTA AGT CAT TTA TAT GAT GTA AAA CTG ACT CCA AAA ATG AAT GTT        1200
Asp Val Ser His Leu Tyr Asp Val Lys Leu Thr Pro Lys Met Asn Val
    1270                1275                1280

ACA ATC AAA TTG TCT ATA CTT TAT GAT AAT GCT GAG TCT AAT GAT AAC        1248
Thr Ile Lys Leu Ser Ile Leu Tyr Asp Asn Ala Glu Ser Asn Asp Asn
1285                1290                1295                1300

TCA ATT GGT AAA TGG ACA AAC ACA AAT ATT GTT TCA GGT GGA AAT AAC        1296
Ser Ile Gly Lys Trp Thr Asn Thr Asn Ile Val Ser Gly Gly Asn Asn
                1305                1310                1315

GGA AAA AAA CAA TAT TCT TCT AAT AAT CCG GAT GCT AAT TTG ACA TTA        1344
Gly Lys Lys Gln Tyr Ser Ser Asn Asn Pro Asp Ala Asn Leu Thr Leu
            1320                1325                1330

AAT ACA GAT GCT CAA GAA AAA TTA AAT AAA AAT CGT GAC TAT TAT ATA        1392
Asn Thr Asp Ala Gln Glu Lys Leu Asn Lys Asn Arg Asp Tyr Tyr Ile
        1335                1340                1345

AGT TTA TAT ATG AAG TCA GAA AAA AAC ACA CAA TGT GAG ATT ACT ATA        1440
Ser Leu Tyr Met Lys Ser Glu Lys Asn Thr Gln Cys Glu Ile Thr Ile
    1350                1355                1360

GAT GGG GAG ATT TAT CCG ATC ACT ACA AAA ACA GTG AAT GTG AAT AAA        1488
Asp Gly Glu Ile Tyr Pro Ile Thr Thr Lys Thr Val Asn Val Asn Lys
1365                1370                1375                1380

GAC AAT TAC AAA AGA TTA GAT ATT ATA GCT CAT AAT ATA AAA AGT AAT        1536
Asp Asn Tyr Lys Arg Leu Asp Ile Ile Ala His Asn Ile Lys Ser Asn
                1385                1390                1395

CCA ATT TCT TCA CTT CAT ATT AAA ACG AAT GAT GAA ATA ACT TTA TTT        1584

Pro Ile Ser Ser Leu His Ile Lys Thr Asn Asp Glu Ile Thr Leu Phe
1400 1405 1410

TGG GAT GAT ATT TCT ATA ACA GAT GTA GCA TCA ATA AAA CCG GAA AAT 1632
Trp Asp Asp Ile Ser Ile Thr Asp Val Ala Ser Ile Lys Pro Glu Asn
1415 1420 1425

TTA ACA GAT TCA GAA ATT AAA CAG ATT TAT AGT AGG TAT GGT ATT AAG 1680
Leu Thr Asp Ser Glu Ile Lys Gln Ile Tyr Ser Arg Tyr Gly Ile Lys
1430 1435 1440

TTA GAA GAT GGA ATC CTT ATT GAT AAA AAA GGT GGG ATT CAT TAT GGT 1728
Leu Glu Asp Gly Ile Leu Ile Asp Lys Lys Gly Gly Ile His Tyr Gly
1445 1450 1455 1460

GAA TTT ATT AAT GAA GCT AGT TTT AAT ATT GAA CCA TTG CCA AAT TAT 1776
Glu Phe Ile Asn Glu Ala Ser Phe Asn Ile Glu Pro Leu Pro Asn Tyr
1465 1470 1475

GTG ACC AAA TAT GAA GTT ACT TAT AGT AGT GAG TTA GGA CCA AAC GTG 1824
Val Thr Lys Tyr Glu Val Thr Tyr Ser Ser Glu Leu Gly Pro Asn Val
1480 1485 1490

AGT GAC ACA CTT GAA AGT GAT AAA ATT TAC AAG GAT GGG ACA ATT AAA 1872
Ser Asp Thr Leu Glu Ser Asp Lys Ile Tyr Lys Asp Gly Thr Ile Lys
1495 1500 1505

TTT GAT TTT ACC AAA TAT AGT AAA AAT GAA CAA GGA TTA TTT TAT GAC 1920
Phe Asp Phe Thr Lys Tyr Ser Lys Asn Glu Gln Gly Leu Phe Tyr Asp
1510 1515 1520

AGT GGA TTA AAT TGG GAC TTT AAA ATT AAT GCT ATT ACT TAT GAT GGT 1968
Ser Gly Leu Asn Trp Asp Phe Lys Ile Asn Ala Ile Thr Tyr Asp Gly
1525 1530 1535 1540

AAA GAG ATG AAT GTT TTT CAT AGA TAT AAT AAA TAG 2004
Lys Glu Met Asn Val Phe His Arg Tyr Asn Lys
1545 1550

(2) INFORMATION FOR SEQ ID NO:7:

(i) SEQUENCE CHARACTERISTICS:
(A) LENGTH: 667 amino acids
(B) TYPE: amino acid
(D) TOPOLOGY: linear

(ii) MOLECULE TYPE: protein

(xi) SEQUENCE DESCRIPTION: SEQ ID NO:7:

Met Lys Arg Glu Ile Asp Glu Asp Thr Asp Thr Asp Gly Asp Ser Ile
1               5                   10                  15

Pro Asp Leu Trp Glu Glu Asn Gly Tyr Thr Ile Gln Asn Arg Ile Ala

20                  25                  30

Val Lys Trp Asp Asp Ser Leu Ala Ser Lys Gly Tyr Thr Lys Phe Val
        35                  40                  45

Ser Asn Pro Leu Glu Ser His Thr Val Gly Asp Pro Tyr Thr Asp Tyr
    50                  55                  60

Glu Lys Ala Ala Arg Asp Leu Asp Leu Ser Asn Ala Lys Glu Thr Phe
65                  70                  75                  80

Asn Pro Leu Val Ala Ala Phe Pro Ser Val Asn Val Ser Met Glu Lys
                85                  90                  95

Val Ile Leu Ser Pro Asn Glu Asn Leu Ser Asn Ser Val Glu Ser His
            100                 105                 110

Ser Ser Thr Asn Trp Ser Tyr Thr Asn Thr Glu Gly Ala Ser Val Glu
        115                 120                 125

Ala Gly Ile Gly Pro Lys Gly Ile Ser Phe Gly Val Ser Val Asn Tyr
    130                 135                 140

Gln His Ser Glu Thr Val Ala Gln Glu Trp Gly Thr Ser Thr Gly Asn
145                 150                 155                 160

Thr Ser Gln Phe Asn Thr Ala Ser Ala Gly Tyr Leu Asn Ala Asn Val
                165                 170                 175

Arg Tyr Asn Asn Val Gly Thr Gly Ala Ile Tyr Asp Val Lys Pro Thr
            180                 185                 190

Thr Ser Phe Val Leu Asn Asn Asp Thr Ile Ala Thr Ile Thr Ala Lys
        195                 200                 205

Ser Asn Ser Thr Ala Leu Asn Ile Ser Pro Gly Glu Ser Tyr Pro Lys
    210                 215                 220

Lys Gly Gln Asn Gly Ile Ala Ile Thr Ser Met Asp Asp Phe Asn Ser
225                 230                 235                 240

His Pro Ile Thr Leu Asn Lys Lys Gln Val Asp Asn Leu Leu Asn Asn
                245                 250                 255

Lys Pro Met Met Leu Glu Thr Asn Gln Thr Asp Gly Val Tyr Lys Ile
            260                 265                 270

Lys Asp Thr His Gly Asn Ile Val Thr Gly Gly Glu Trp Asn Gly Val
        275                 280                 285

Ile Gln Gln Ile Lys Ala Lys Thr Ala Ser Ile Ile Val Asp Asp Gly
    290                 295                 300

Glu Arg Val Ala Glu Lys Arg Val Ala Ala Lys Asp Tyr Glu Asn Pro
305                 310                 315                 320

Glu Asp Lys Thr Pro Ser Leu Thr Leu Lys Asp Ala Leu Lys Leu Ser
                325                 330                 335

Tyr Pro Asp Glu Ile Lys Glu Ile Glu Gly Leu Leu Tyr Tyr Lys Asn
            340                 345                 350

Lys Pro Ile Tyr Glu Ser Ser Val Met Thr Tyr Leu Asp Glu Asn Thr
        355                 360                 365

Ala Lys Glu Val Thr Lys Gln Leu Asn Asp Thr Thr Gly Lys Phe Lys
    370                 375                 380

Asp Val Ser His Leu Tyr Asp Val Lys Leu Thr Pro Lys Met Asn Val
385                 390                 395                 400

Thr Ile Lys Leu Ser Ile Leu Tyr Asp Asn Ala Glu Ser Asn Asp Asn
                405                 410                 415

Ser Ile Gly Lys Trp Thr Asn Thr Asn Ile Val Ser Gly Gly Asn Asn
            420                 425                 430

Gly Lys Lys Gln Tyr Ser Ser Asn Asn Pro Asp Ala Asn Leu Thr Leu
        435                 440                 445

Asn Thr Asp Ala Gln Glu Lys Leu Asn Lys Asn Arg Asp Tyr Tyr Ile
    450                 455                 460

Ser Leu Tyr Met Lys Ser Glu Lys Asn Thr Gln Cys Glu Ile Thr Ile
465                 470                 475                 480

Asp Gly Glu Ile Tyr Pro Ile Thr Thr Lys Thr Val Asn Val Asn Lys
                485                 490                 495

Asp Asn Tyr Lys Arg Leu Asp Ile Ile Ala His Asn Ile Lys Ser Asn
            500                 505                 510

Pro Ile Ser Ser Leu His Ile Lys Thr Asn Asp Glu Ile Thr Leu Phe
        515                 520                 525

Trp Asp Asp Ile Ser Ile Thr Asp Val Ala Ser Ile Lys Pro Glu Asn
    530                 535                 540

Leu Thr Asp Ser Glu Ile Lys Gln Ile Tyr Ser Arg Tyr Gly Ile Lys
545                 550                 555                 560

Leu Glu Asp Gly Ile Leu Ile Asp Lys Lys Gly Gly Ile His Tyr Gly
                565                 570                 575

Glu Phe Ile Asn Glu Ala Ser Phe Asn Ile Glu Pro Leu Pro Asn Tyr
            580                 585                 590

Val Thr Lys Tyr Glu Val Thr Tyr Ser Ser Glu Leu Gly Pro Asn Val
        595                 600                 605

Ser Asp Thr Leu Glu Ser Asp Lys Ile Tyr Lys Asp Gly Thr Ile Lys
    610                 615                 620

Phe Asp Phe Thr Lys Tyr Ser Lys Asn Glu Gln Gly Leu Phe Tyr Asp
625                 630                 635                 640

Ser Gly Leu Asn Trp Asp Phe Lys Ile Asn Ala Ile Thr Tyr Asp Gly
                645                 650                 655

Lys Glu Met Asn Val Phe His Arg Tyr Asn Lys
            660                 665

(2) INFORMATION FOR SEQ ID NO:8:

     (i) SEQUENCE CHARACTERISTICS:
          (A) LENGTH: 16 amino acids
          (B) TYPE: amino acid
          (C) STRANDEDNESS: single
          (D) TOPOLOGY: linear

    (ii) MOLECULE TYPE: peptide

   (iii) HYPOTHETICAL: NO

     (v) FRAGMENT TYPE: N-terminal

    (vi) ORIGINAL SOURCE:
          (A) ORGANISM: Bacillus cereus
          (B) STRAIN: AB78
          (C) INDIVIDUAL ISOLATE: NRRL B-21058

    (ix) FEATURE:
          (A) NAME/KEY: Peptide
          (B) LOCATION: 1..16
          (D) OTHER INFORMATION: /note= "N-terminal sequence of
  protein purified from strain AB78"

    (xi) SEQUENCE DESCRIPTION: SEQ ID NO:8:

     Lys Arg Glu Ile Asp Glu Asp Thr Asp Thr Asx Gly Asp Ser Ile Pro
     1               5                   10                  15

(2) INFORMATION FOR SEQ ID NO:9:

     (i) SEQUENCE CHARACTERISTICS:
          (A) LENGTH: 21 base pairs
          (B) TYPE: nucleic acid
          (C) STRANDEDNESS: single
          (D) TOPOLOGY: linear

    (ii) MOLECULE TYPE: DNA (genomic)

   (iii) HYPOTHETICAL: NO

(iv) ANTI-SENSE: NO

(ix) FEATURE:
(A) NAME/KEY: misc_feature
(B) LOCATION: 1..21
(D) OTHER INFORMATION: /note= "Oligonucleotide probe based
on amino acids 3 to 9 of SEQ ID NO:8, using codon usage of
Bacillus thuringiensis"

(xi) SEQUENCE DESCRIPTION: SEQ ID NO:9:

GAAATTGATC AAGATACNGA T 21

(2) INFORMATION FOR SEQ ID NO:10:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis
(B) STRAIN: AB88

(ix) FEATURE:
(A) NAME/KEY: Peptide
(B) LOCATION: 1..14
(D) OTHER INFORMATION: /note= "N-terminal amino acid
sequence of protein known as anion exchange fraction 23
(smaller)"

(xi) SEQUENCE DESCRIPTION: SEQ ID NO:10:

Xaa Glu Pro Phe Val Ser Ala Xaa Xaa Xaa Gln Xaa Xaa Xaa
1               5                   10

(2) INFORMATION FOR SEQ ID NO:11:

(i) SEQUENCE CHARACTERISTICS:
(A) LENGTH: 13 amino acids
(B) TYPE: amino acid
(C) STRANDEDNESS: single
(D) TOPOLOGY: N-terminal

(vi) ORIGINAL SOURCE:
(A) ORGANISM: Bacillus thuringiensis

(xi) SEQUENCE DESCRIPTION: SEQ ID NO:11:

Xaa Glu Tyr Glu Asn Val Glu Pro Phe Val Ser Ala Xaa
1               5                   10

(2) INFORMATION FOR SEQ ID NO:12:

(i) SEQUENCE CHARACTERISTICS:
(A) LENGTH: 14 amino acids
(B) TYPE: amino acid
(C) STRANDEDNESS: single
(D) TOPOLOGY: N-terminal

(vi) ORIGINAL SOURCE:
(A) ORGANISM: Bacillus thurigiensis

(xi) SEQUENCE DESCRIPTION: SEQ ID NO:12:

Met Asn Lys Asn Asn Thr Lys Leu Pro Thr Arg Ala Leu Pro
1               5                   10

(2) INFORMATION FOR SEQ ID NO:13:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis
(B) STRAIN: AB88

(ix) FEATURE:
(A) NAME/KEY: Peptide
(B) LOCATION: 1..15
(D) OTHER INFORMATION: /note= "N-terminal amino acid
sequence of 35 kDa VIP active against Agrotis ipsilon"

(xi) SEQUENCE DESCRIPTION: SEQ ID NO:13:

Ala Leu Ser Glu Asn Thr Gly Lys Asp Gly Gly Tyr Ile Val Pro
1               5                   10                  15

(2) INFORMATION FOR SEQ ID NO:14:

(i) SEQUENCE CHARACTERISTICS:
(A) LENGTH: 9 amino acids
(B) TYPE: amino acid
(C) STRANDEDNESS: single
(D) TOPOLOGY: N-terminal

(vi) ORIGINAL SOURCE:
(A) ORGANISM: Bacillus thuringiensis

(xi) SEQUENCE DESCRIPTION: SEQ ID NO:14:

Met Asp Asn Asn Pro Asn Ile Asn Glu
1               5

(2) INFORMATION FOR SEQ ID NO:15:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(ix) FEATURE:
(A) NAME/KEY: Peptide
(B) LOCATION: 1..9
(D) OTHER INFORMATION: /note= "N-terminal sequence of 80
kDa delta-endotoxin"

(xi) SEQUENCE DESCRIPTION: SEQ ID NO:15:

Met Asp Asn Asn Pro Asn Ile Asn Glu
1               5

(2) INFORMATION FOR SEQ ID NO:16:

(i) SEQUENCE CHARACTERISTICS:
(A) LENGTH: 11 amino acids

(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis

(ix) FEATURE:
(A) NAME/KEY: Peptide
(B) LOCATION: 1..11
(D) OTHER INFORMATION: /note= "N-terminal sequence from 60
kDa delta-endotoxin"

(xi) SEQUENCE DESCRIPTION: SEQ ID NO:16:

Met Asn Val Leu Asn Ser Gly Arg Thr Thr Ile
1               5                   10

(2) INFORMATION FOR SEQ ID NO:17:

(i) SEQUENCE CHARACTERISTICS:
(A) LENGTH: 2655 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: misc_feature
(B) LOCATION: 1..2652
(D) OTHER INFORMATION: /note= "Maize optimized DNA
sequence for 100 kd VIP1A(a) protein from AB78"

(xi) SEQUENCE DESCRIPTION: SEQ ID NO:17:

ATGAAGAACA TGAAGAAGAA GCTGGCCAGC GTGGTGACCT GCACCCTGCT GGCCCCCATG      60

TTCCTGAACG GCAACGTGAA CGCCGTGTAC GCCGACAGCA AGACCAACCA GATCAGCACC     120

ACCCAGAAGA ACCAGCAGAA GGAGATGGAC CGCAAGGGCC TGCTGGGCTA CTACTTCAAG     180

GGCAAGGACT TCAGCAACCT GACCATGTTC GCCCCCACGC GTGACAGCAC CCTGATCTAC 240
GACCAGCAGA CCGCCAACAA GCTGCTGGAC AAGAAGCAGC AGGAGTACCA GAGCATCCGC 300
TGGATCGGCC TGATCCAGAG CAAGGAGACC GGCGACTTCA CCTTCAACCT GAGCGAGGAC 360
GAGCAGGCCA TCATCGAGAT CAACGGCAAG ATCATCAGCA ACAAGGGCAA GGAGAAGCAG 420
GTGGTGCACC TGGAGAAGGG CAAGCTGGTG CCCATCAAGA TCGAGTACCA GAGCGACACC 480
AAGTTCAACA TCGACAGCAA GACCTTCAAG GAGCTGAAGC TTTTCAAGAT CGACAGCCAG 540
AACCAGCCCC AGCAGGTGCA GCAGGACGAG CTGCGCAACC CCGAGTTCAA CAAGAAGGAG 600
AGCCAGGAGT TCCTGGCCAA GCCCAGCAAG ATCAACCTGT TCACCCAGCA GATGAAGCGC 660
GAGATCGACG AGGACACCGA CACCGACGGC GACAGCATCC CCGACCTGTG GGAGGAGAAC 720
GGCTACACCA TCCAGAACCG CATCGCCGTG AAGTGGGACG ACAGCCTGGC TAGCAAGGGC 780
TACACCAAGT TCGTGAGCAA CCCCCTGGAG AGCCACACCG TGGGCGACCC CTACACCGAC 840
TACGAGAAGG CCGCCCGCGA CCTGGACCTG AGCAACGCCA AGGAGACCTT CAACCCCCTG 900
GTGGCCGCCT TCCCCAGCGT GAACGTGAGC ATGGAGAAGG TGATCCTGAG CCCCAACGAG 960
AACCTGAGCA ACAGCGTGGA GAGCCACTCG AGCACCAACT GGAGCTACAC CAACACCGAG 1020
GGCGCCAGCG TGGAGGCCGG CATCGGTCCC AAGGGCATCA GCTTCGGCGT GAGCGTGAAC 1080
TACCAGCACA GCGAGACCGT GGCCCAGGAG TGGGGCACCA GCACCGGCAA CACCAGCCAG 1140
TTCAACACCG CCAGCGCCGG CTACCTGAAC GCCAACGTGC GCTACAACAA CGTGGGCACC 1200
GGCGCCATCT ACGACGTGAA GCCCACCACC AGCTTCGTGC TGAACAACGA CACCATCGCC 1260
ACCATCACCG CCAAGTCGAA TTCCACCGCC CTGAACATCA GCCCCGGCGA GAGCTACCCC 1320
AAGAAGGGCC AGAACGGCAT CGCCATCACC AGCATGGACG ACTTCAACAG CCACCCCATC 1380
ACCCTGAACA AGAAGCAGGT GGACAACCTG CTGAACAACA AGCCCATGAT GCTGGAGACC 1440
AACCAGACCG ACGGCGTCTA CAAGATCAAG GACACCCACG GCAACATCGT GACCGGCGGC 1500
GAGTGGAACG GCGTGATCCA GCAGATCAAG GCCAAGACCG CCAGCATCAT CGTCGACGAC 1560
GGCGAGCGCG TGGCCGAGAA GCGCGTGGCC GCCAAGGACT ACGAGAACCC CGAGGACAAG 1620
ACCCCCAGCC TGACCCTGAA GGACGCCCTG AAGCTGAGCT ACCCCGACGA GATCAAGGAG 1680
ATCGAGGGCC TGCTGTACTA CAAGAACAAG CCCATCTACG AGAGCAGCGT GATGACCTAT 1740
CTAGACGAGA ACACCGCCAA GGAGGTGACC AAGCAGCTGA ACGACACCAC CGGCAAGTTC 1800
AAGGACGTGA GCCACCTGTA CGACGTGAAG CTGACCCCCA AGATGAACGT GACCATCAAG 1860

CTGAGCATCC TGTACGACAA CGCCGAGAGC AACGACAACA GCATCGGCAA GTGGACCAAC 1920

ACCAACATCG TGAGCGGCGG CAACAACGGC AAGAAGCAGT ACAGCAGCAA CAACCCCGAC 1980

GCCAACCTGA CCCTGAACAC CGACGCCCAG GAGAAGCTGA ACAAGAACCG CGACTACTAC 2040

ATCAGCCTGT ACATGAAGAG CGAGAAGAAC ACCCAGTGCG AGATCACCAT CGACGGCGAG 2100

ATATACCCCA TCACCACCAA GACCGTGAAC GTGAACAAGG ACAACTACAA GCGCCTGGAC 2160

ATCATCGCCC ACAACATCAA GAGCAACCCC ATCAGCAGCC TGCACATCAA GACCAACGAC 2220

GAGATCACCC TGTTCTGGGA CGACATATCG ATTACCGACG TCGCCAGCAT CAAGCCCGAG 2280

AACCTGACCG ACAGCGAGAT CAAGCAGATA TACAGTCGCT ACGGCATCAA GCTGGAGGAC 2340

GGCATCCTGA TCGACAAGAA GGGCGGCATC CACTACGGCG AGTTCATCAA CGAGGCCAGC 2400

TTCAACATCG AGCCCCTGCA GAACTACGTG ACCAAGTACG AGGTGACCTA CAGCAGCGAG 2460

CTGGGCCCCA ACGTGAGCGA CACCCTGGAG AGCGACAAGA TTTACAAGGA CGGCACCATC 2520

AAGTTCGACT TCACCAAGTA CAGCAAGAAC GAGCAGGGCC TGTTCTACGA CAGCGGCCTG 2580

AACTGGGACT TCAAGATCAA CGCCATCACC TACGACGGCA AGGAGATGAA CGTGTTCCAC 2640

CGCTACAACA AGTAG 2655

(2) INFORMATION FOR SEQ ID NO:18:

(i) SEQUENCE CHARACTERISTICS:
(A) LENGTH: 2004 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: misc_feature
(B) LOCATION: 1..2004
(D) OTHER INFORMATION: /note= "Maize optimized DNA
sequence for VIP1A(a) 80 kd protein from AB78"

(xi) SEQUENCE DESCRIPTION: SEQ ID NO:18:

ATGAAGCGCG AGATCGACGA GGACACCGAC ACCGACGGCG ACAGCATCCC CGACCTGTGG 60

GAGGAGAACG GCTACACCAT CCAGAACCGC ATCGCCGTGA AGTGGGACGA CAGCCTGGCT 120
AGCAAGGGCT ACACCAAGTT CGTGAGCAAC CCCCTGGAGA GCCACACCGT GGGCGACCCC 180
TACACCGACT ACGAGAAGGC CGCCCGCGAC CTGGACCTGA GCAACGCCAA GGAGACCTTC 240
AACCCCCTGG TGGCCGCCTT CCCCAGCGTG AACGTGAGCA TGGAGAAGGT GATCCTGAGC 300
CCCAACGAGA ACCTGAGCAA CAGCGTGGAG AGCCACTCGA GCACCAACTG GAGCTACACC 360
AACACCGAGG GCGCCAGCGT GGAGGCCGGC ATCGGTCCCA AGGGCATCAG CTTCGGCGTG 420
AGCGTGAACT ACCAGCACAG CGAGACCGTG GCCCAGGAGT GGGGCACCAG CACCGGCAAC 480
ACCAGCCAGT TCAACACCGC CAGCGCCGGC TACCTGAACG CCAACGTGCG CTACAACAAC 540
GTGGGCACCG GCGCCATCTA CGACGTGAAG CCCACCACCA GCTTCGTGCT GAACAACGAC 600
ACCATCGCCA CCATCACCGC CAAGTCGAAT TCCACCGCCC TGAACATCAG CCCCGGCGAG 660
AGCTACCCCA AGAAGGGCCA GAACGGCATC GCCATCACCA GCATGGACGA CTTCAACAGC 720
CACCCCATCA CCCTGAACAA GAAGCAGGTG GACAACCTGC TGAACAACAA GCCCATGATG 780
CTGGAGACCA ACCAGACCGA CGGCGTCTAC AAGATCAAGG ACACCCACGG CAACATCGTG 840
ACCGGCGGCG AGTGGAACGG CGTGATCCAG CAGATCAAGG CCAAGACCGC CAGCATCATC 900
GTCGACGACG GCGAGCGCGT GGCCGAGAAG CGCGTGGCCG CCAAGGACTA CGAGAACCCC 960
GAGGACAAGA CCCCCAGCCT GACCCTGAAG GACGCCCTGA AGCTGAGCTA CCCCGACGAG 1020
ATCAAGGAGA TCGAGGGCCT GCTGTACTAC AAGAACAAGC CCATCTACGA GAGCAGCGTG 1080
ATGACCTATC TAGACGAGAA CACCGCCAAG GAGGTGACCA AGCAGCTGAA CGACACCACC 1140
GGCAAGTTCA AGGACGTGAG CCACCTGTAC GACGTGAAGC TGACCCCCAA GATGAACGTG 1200
ACCATCAAGC TGAGCATCCT GTACGACAAC GCCGAGAGCA ACGACAACAG CATCGGCAAG 1260
TGGACCAACA CCAACATCGT GAGCGGCGGC AACAACGGCA AGAAGCAGTA CAGCAGCAAC 1320
AACCCCGACG CCAACCTGAC CCTGAACACC GACGCCCAGG AGAAGCTGAA CAAGAACCGC 1380
GACTACTACA TCAGCCTGTA CATGAAGAGC GAGAAGAACA CCCAGTGCGA GATCACCATC 1440
GACGGCGAGA TATACCCCAT CACCACCAAG ACCGTGAACG TGAACAAGGA CAACTACAAG 1500
CGCCTGGACA TCATCGCCCA CAACATCAAG AGCAACCCCA TCAGCAGCCT GCACATCAAG 1560
ACCAACGACG AGATCACCCT GTTCTGGGAC GACATATCGA TTACCGACGT CGCCAGCATC 1620
AAGCCCGAGA ACCTGACCGA CAGCGAGATC AAGCAGATAT ACAGTCGCTA CGGCATCAAG 1680
CTGGAGGACG GCATCCTGAT CGACAAGAAG GGCGGCATCC ACTACGGCGA GTTCATCAAC 1740

GAGGCCAGCT TCAACATCGA GCCCCTGCAG AACTACGTGA CCAAGTACGA GGTGACCTAC     1800

AGCAGCGAGC TGGGCCCCAA CGTGAGCGAC ACCCTGGAGA GCGACAAGAT TTACAAGGAC     1860

GGCACCATCA AGTTCGACTT CACCAAGTAC AGCAAGAACG AGCAGGGCCT GTTCTACGAC     1920

AGCGGCCTGA ACTGGGACTT CAAGATCAAC GCCATCACCT ACGACGGCAA GGAGATGAAC     1980

GTGTTCCACC GCTACAACAA GTAG                                           2004

(2) INFORMATION FOR SEQ ID NO:19:

(i) SEQUENCE CHARACTERISTICS:
(A) LENGTH: 4074 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 1..1386
(D) OTHER INFORMATION: /product= "VIP2A(b) from Btt"

(ix) FEATURE:
(A) NAME/KEY: CDS
(B) LOCATION: 1394..3895
(D) OTHER INFORMATION: /product= "VIP1A(b) from Btt"

(ix) FEATURE:
(A) NAME/KEY: misc_feature
(B) LOCATION: 1..4074
(D) OTHER INFORMATION: /note= "Cloned DNA sequence from
Btt which contains the genes for both VIP1A(b) and VIP2A(b)"

(xi) SEQUENCE DESCRIPTION: SEQ ID NO:19:

ATG CAA AGA ATG GAG GGA AAG TTG TTT GTG GTG TCA AAA ACA TTA CAA     48
Met Gln Arg Met Glu Gly Lys Leu Phe Val Val Ser Lys Thr Leu Gln
        670                 675                 680

GTA GTT ACT AGA ACT GTA TTG CTT AGT ACA GTT TAC TCT ATA ACT TTA     96
Val Val Thr Arg Thr Val Leu Leu Ser Thr Val Tyr Ser Ile Thr Leu
    685                 690                 695

TTA AAT AAT GTA GTG ATA AAA GCT GAC CAA TTA AAT ATA AAT TCT CAA     144
Leu Asn Asn Val Val Ile Lys Ala Asp Gln Leu Asn Ile Asn Ser Gln
700                 705                 710                 715

AGT AAA TAT ACT AAC TTG CAA AAT CTA AAA ATC CCT GAT AAT GCA GAG     192
Ser Lys Tyr Thr Asn Leu Gln Asn Leu Lys Ile Pro Asp Asn Ala Glu

720 725 730

GAT TTT AAA GAA GAT AAG GGG AAA GCG AAA GAA TGG GGG AAA GAG AAA 240
Asp Phe Lys Glu Asp Lys Gly Lys Ala Lys Glu Trp Gly Lys Glu Lys
735 740 745

GGG GAA GAG TGG AGG CCT CCT GCT ACT GAG AAA GGA GAA ATG AAT AAT 288
Gly Glu Glu Trp Arg Pro Pro Ala Thr Glu Lys Gly Glu Met Asn Asn
750 755 760

TTT TTA GAT AAT AAA AAT GAT ATA AAG ACC AAT TAT AAA GAA ATT ACT 336
Phe Leu Asp Asn Lys Asn Asp Ile Lys Thr Asn Tyr Lys Glu Ile Thr
765 770 775

TTT TCT ATG GCA GGT TCA TGT GAA GAT GAA ATA AAA GAT TTA GAA GAA 384
Phe Ser Met Ala Gly Ser Cys Glu Asp Glu Ile Lys Asp Leu Glu Glu
780 785 790 795

ATT GAT AAG ATC TTT GAT AAA GCC AAT CTC TCG AGT TCT ATT ATC ACC 432
Ile Asp Lys Ile Phe Asp Lys Ala Asn Leu Ser Ser Ser Ile Ile Thr
800 805 810

TAT AAA AAT GTG GAA CCA GCA ACA ATT GGA TTT AAT AAA TCT TTA ACA 480
Tyr Lys Asn Val Glu Pro Ala Thr Ile Gly Phe Asn Lys Ser Leu Thr
815 820 825

GAA GGT AAT ACG ATT AAT TCT GAT GCA ATG GCA CAG TTT AAA GAA CAA 528
Glu Gly Asn Thr Ile Asn Ser Asp Ala Met Ala Gln Phe Lys Glu Gln
830 835 840

TTT TTA GGT AAG GAT ATG AAG TTT GAT AGT TAT CTA GAT ACT CAT TTA 576
Phe Leu Gly Lys Asp Met Lys Phe Asp Ser Tyr Leu Asp Thr His Leu
845 850 855

ACT GCT CAA CAA GTT TCC AGT AAA AAA AGA GTT ATT TTG AAG GTT ACG 624
Thr Ala Gln Gln Val Ser Ser Lys Lys Arg Val Ile Leu Lys Val Thr
860 865 870 875

GTT CCG AGT GGG AAA GGT TCT ACT ACT CCA ACA AAA GCA GGT GTC ATT 672
Val Pro Ser Gly Lys Gly Ser Thr Thr Pro Thr Lys Ala Gly Val Ile
880 885 890

TTA AAC AAT AAT GAA TAC AAA ATG CTC ATT GAT AAT GGG TAT GTG CTC 720
Leu Asn Asn Asn Glu Tyr Lys Met Leu Ile Asp Asn Gly Tyr Val Leu
895 900 905

CAT GTA GAT AAG GTA TCA AAA GTA GTA AAA AAA GGG ATG GAG TGC TTA 768
His Val Asp Lys Val Ser Lys Val Val Lys Lys Gly Met Glu Cys Leu
910 915 920

CAA GTT GAA GGG ACT TTA AAA AAG AGT CTC GAC TTT AAA AAT GAT ATA 816
Gln Val Glu Gly Thr Leu Lys Lys Ser Leu Asp Phe Lys Asn Asp Ile
925 930 935

AAT GCT GAA GCG CAT AGC TGG GGG ATG AAA ATT TAT GAA GAC TGG GCT 864

Asn Ala Glu Ala His Ser Trp Gly Met Lys Ile Tyr Glu Asp Trp Ala
940                 945                 950                 955

AAA AAT TTA ACC GCT TCG CAA AGG GAA GCT TTA GAT GGG TAT GCT AGG     912
Lys Asn Leu Thr Ala Ser Gln Arg Glu Ala Leu Asp Gly Tyr Ala Arg
                960                 965                 970

CAA GAT TAT AAA GAA ATC AAT AAT TAT TTG CGC AAT CAA GGC GGG AGT     960
Gln Asp Tyr Lys Glu Ile Asn Asn Tyr Leu Arg Asn Gln Gly Gly Ser
            975                 980                 985

GGA AAT GAA AAG CTG GAT GCC CAA TTA AAA AAT ATT TCT GAT GCT TTA     1008
Gly Asn Glu Lys Leu Asp Ala Gln Leu Lys Asn Ile Ser Asp Ala Leu
        990                 995                 1000

GGG AAG AAA CCC ATA CCA GAA AAT ATT ACC GTG TAT AGA TGG TGT GGC     1056
Gly Lys Lys Pro Ile Pro Glu Asn Ile Thr Val Tyr Arg Trp Cys Gly
    1005                1010                1015

ATG CCG GAA TTT GGT TAT CAA ATT AGT GAT CCG TTA CCT TCT TTA AAA     1104
Met Pro Glu Phe Gly Tyr Gln Ile Ser Asp Pro Leu Pro Ser Leu Lys
1020                1025                1030                1035

GAT TTT GAA GAA CAA TTT TTA AAT ACA ATT AAA GAA GAC AAA GGG TAT     1152
Asp Phe Glu Glu Gln Phe Leu Asn Thr Ile Lys Glu Asp Lys Gly Tyr
                1040                1045                1050

ATG AGT ACA AGC TTA TCG AGT GAA CGT CTT GCA GCT TTT GGA TCT AGA     1200
Met Ser Thr Ser Leu Ser Ser Glu Arg Leu Ala Ala Phe Gly Ser Arg
            1055                1060                1065

AAA ATT ATA TTA CGC TTA CAA GTT CCG AAA GGA AGT ACG GGG GCG TAT     1248
Lys Ile Ile Leu Arg Leu Gln Val Pro Lys Gly Ser Thr Gly Ala Tyr
        1070                1075                1080

TTA AGT GCC ATT GGT GGA TTT GCA AGT GAA AAA GAG ATC CTA CTT GAT     1296
Leu Ser Ala Ile Gly Gly Phe Ala Ser Glu Lys Glu Ile Leu Leu Asp
    1085                1090                1095

AAA GAT AGT AAA TAT CAT ATT GAT AAA GCA ACA GAG GTA ATC ATT AAA     1344
Lys Asp Ser Lys Tyr His Ile Asp Lys Ala Thr Glu Val Ile Ile Lys
1100                1105                1110                1115

GGT GTT AAG CGA TAT GTA GTG GAT GCA ACA TTA TTA ACA AAT             1386
Gly Val Lys Arg Tyr Val Val Asp Ala Thr Leu Leu Thr Asn
                1120                1125

TAAGGAG ATG AAA AAT ATG AAG AAA AAG TTA GCA AGT GTT GTA ACC TGT     1435
        Met Lys Asn Met Lys Lys Lys Leu Ala Ser Val Val Thr Cys
          1                 5                   10

ATG TTA TTA GCT CCT ATG TTT TTG AAT GGA AAT GTG AAT GCT GTT AAC     1483
Met Leu Leu Ala Pro Met Phe Leu Asn Gly Asn Val Asn Ala Val Asn
 15                 20                  25                  30

GCG GAT AGT AAA ATA AAT CAG ATT TCT ACA ACG CAG GAA AAC CAA CAG 1531
Ala Asp Ser Lys Ile Asn Gln Ile Ser Thr Thr Gln Glu Asn Gln Gln
35 40 45

AAA GAG ATG GAC CGA AAG GGA TTA TTG GGA TAT TAT TTC AAA GGA AAA 1579
Lys Glu Met Asp Arg Lys Gly Leu Leu Gly Tyr Tyr Phe Lys Gly Lys
50 55 60

GAT TTT AAT AAT CTT ACT ATG TTT GCA CCG ACA CGT GAT AAT ACC CTT 1627
Asp Phe Asn Asn Leu Thr Met Phe Ala Pro Thr Arg Asp Asn Thr Leu
65 70 75

ATG TAT GAC CAA CAA ACA GCG AAT GCA TTA TTA GAT AAA AAA CAA CAA 1675
Met Tyr Asp Gln Gln Thr Ala Asn Ala Leu Leu Asp Lys Lys Gln Gln
80 85 90

GAA TAT CAG TCC ATT CGT TGG ATT GGT TTG ATT CAG CGT AAA GAA ACG 1723
Glu Tyr Gln Ser Ile Arg Trp Ile Gly Leu Ile Gln Arg Lys Glu Thr
95 100 105 110

GGC GAT TTC ACA TTT AAC TTA TCA AAG GAT GAA CAG GCA ATT ATA GAA 1771
Gly Asp Phe Thr Phe Asn Leu Ser Lys Asp Glu Gln Ala Ile Ile Glu
115 120 125

ATC GAT GGG AAA ATC ATT TCT AAT AAA GGG AAA GAA AAG CAA GTT GTC 1819
Ile Asp Gly Lys Ile Ile Ser Asn Lys Gly Lys Glu Lys Gln Val Val
130 135 140

CAT TTA GAA AAA GAA AAA TTA GTT CCA ATC AAA ATA GAG TAT CAA TCA 1867
His Leu Glu Lys Glu Lys Leu Val Pro Ile Lys Ile Glu Tyr Gln Ser
145 150 155

GAT ACG AAA TTT AAT ATT GAT AGT AAA ACA TTT AAA GAA CTT AAA TTA 1915
Asp Thr Lys Phe Asn Ile Asp Ser Lys Thr Phe Lys Glu Leu Lys Leu
160 165 170

TTT AAA ATA GAT AGT CAA AAC CAA TCT CAA CAA GTT CAA CTG AGA AAC 1963
Phe Lys Ile Asp Ser Gln Asn Gln Ser Gln Gln Val Gln Leu Arg Asn
175 180 185 190

CCT GAA TTT AAC AAA AAA GAA TCA CAG GAA TTT TTA GCA AAA GCA TCA 2011
Pro Glu Phe Asn Lys Lys Glu Ser Gln Glu Phe Leu Ala Lys Ala Ser
195 200 205

AAA ACA AAC CTT TTT AAG CAA AAA ATG AAA AGA GAT ATT GAT GAA GAT 2059
Lys Thr Asn Leu Phe Lys Gln Lys Met Lys Arg Asp Ile Asp Glu Asp
210 215 220

ACG GAT ACA GAT GGA GAC TCC ATT CCT GAT CTT TGG GAA GAA AAT GGG 2107
Thr Asp Thr Asp Gly Asp Ser Ile Pro Asp Leu Trp Glu Glu Asn Gly
225 230 235

TAC ACG ATT CAA AAT AAA GTT GCT GTC AAA TGG GAT GAT TCG CTA GCA 2155
Tyr Thr Ile Gln Asn Lys Val Ala Val Lys Trp Asp Asp Ser Leu Ala
240 245 250

AGT AAG GGA TAT ACA AAA TTT GTT TCG AAT CCA TTA GAC AGC CAC ACA     2203
Ser Lys Gly Tyr Thr Lys Phe Val Ser Asn Pro Leu Asp Ser His Thr
255                 260                 265                 270

GTT GGC GAT CCC TAT ACT GAT TAT GAA AAG GCC GCA AGG GAT TTA GAT     2251
Val Gly Asp Pro Tyr Thr Asp Tyr Glu Lys Ala Ala Arg Asp Leu Asp
                275                 280                 285

TTA TCA AAT GCA AAG GAA ACG TTC AAC CCA TTG GTA GCT GCT TTT CCA     2299
Leu Ser Asn Ala Lys Glu Thr Phe Asn Pro Leu Val Ala Ala Phe Pro
            290                 295                 300

AGT GTG AAT GTT AGT ATG GAA AAG GTG ATA TTA TCA CCA AAT GAA AAT     2347
Ser Val Asn Val Ser Met Glu Lys Val Ile Leu Ser Pro Asn Glu Asn
        305                 310                 315

TTA TCC AAT AGT GTA GAG TCT CAT TCA TCC ACG AAT TGG TCT TAT ACG     2395
Leu Ser Asn Ser Val Glu Ser His Ser Ser Thr Asn Trp Ser Tyr Thr
    320                 325                 330

AAT ACA GAA GGA GCT TCC ATT GAA GCT GGT GGC GGT CCA TTA GGC CTT     2443
Asn Thr Glu Gly Ala Ser Ile Glu Ala Gly Gly Gly Pro Leu Gly Leu
335                 340                 345                 350

TCT TTT GGC GTG AGT GTT ACT TAT CAA CAC TCT GAA ACA GTT GCA CAA     2491
Ser Phe Gly Val Ser Val Thr Tyr Gln His Ser Glu Thr Val Ala Gln
                355                 360                 365

GAA TGG GGA ACA TCT ACA GGA AAT ACT TCA CAA TTC AAT ACG GCT TCA     2539
Glu Trp Gly Thr Ser Thr Gly Asn Thr Ser Gln Phe Asn Thr Ala Ser
            370                 375                 380

GCG GGA TAT TTA AAT GCA AAT GTT CGG TAT AAC AAT GTA GGG ACT GGT     2587
Ala Gly Tyr Leu Asn Ala Asn Val Arg Tyr Asn Asn Val Gly Thr Gly
        385                 390                 395

GCC ATC TAT GAT GTA AAA CCT ACA ACA AGT TTT GTA TTA AAT AAC AAT     2635
Ala Ile Tyr Asp Val Lys Pro Thr Thr Ser Phe Val Leu Asn Asn Asn
    400                 405                 410

ACC ATC GCA ACG ATT ACA GCA AAA TCA AAT TCA ACA GCT TTA CGT ATA     2683
Thr Ile Ala Thr Ile Thr Ala Lys Ser Asn Ser Thr Ala Leu Arg Ile
415                 420                 425                 430

TCT CCG GGG GAT AGT TAT CCA GAA ATA GGA GAA AAC GCT ATT GCG ATT     2731
Ser Pro Gly Asp Ser Tyr Pro Glu Ile Gly Glu Asn Ala Ile Ala Ile
                435                 440                 445

ACA TCT ATG GAT GAT TTT AAT TCT CAT CCA ATT ACA TTA AAT AAA CAA     2779
Thr Ser Met Asp Asp Phe Asn Ser His Pro Ile Thr Leu Asn Lys Gln
            450                 455                 460

CAG GTA AAT CAA TTG ATA AAT AAT AAG CCA ATT ATG CTA GAG ACA GAC     2827
Gln Val Asn Gln Leu Ile Asn Asn Lys Pro Ile Met Leu Glu Thr Asp

465 470 475

CAA ACA GAT GGT GTT TAT AAA ATA AGA GAT ACA CAT GGA AAT ATT GTA 2875
Gln Thr Asp Gly Val Tyr Lys Ile Arg Asp Thr His Gly Asn Ile Val
480 485 490

ACT GGT GGA GAA TGG AAT GGT GTA ACA CAA CAA ATT AAA GCA AAA ACA 2923
Thr Gly Gly Glu Trp Asn Gly Val Thr Gln Gln Ile Lys Ala Lys Thr
495 500 505 510

GCG TCT ATT ATT GTG GAT GAC GGG AAA CAG GTA GCA GAA AAA CGT GTG 2971
Ala Ser Ile Ile Val Asp Asp Gly Lys Gln Val Ala Glu Lys Arg Val
515 520 525

GCG GCA AAA GAT TAT GGT CAT CCA GAA GAT AAA ACA CCA CCT TTA ACT 3019
Ala Ala Lys Asp Tyr Gly His Pro Glu Asp Lys Thr Pro Pro Leu Thr
530 535 540

TTA AAA GAT ACC CTG AAG CTT TCA TAC CCA GAT GAA ATA AAA GAA ACT 3067
Leu Lys Asp Thr Leu Lys Leu Ser Tyr Pro Asp Glu Ile Lys Glu Thr
545 550 555

AAT GGA TTG TTG TAC TAT GAT GAC AAA CCA ATC TAT GAA TCG AGT GTC 3115
Asn Gly Leu Leu Tyr Tyr Asp Asp Lys Pro Ile Tyr Glu Ser Ser Val
560 565 570

ATG ACT TAT CTG GAT GAA AAT ACG GCA AAA GAA GTC AAA AAA CAA ATA 3163
Met Thr Tyr Leu Asp Glu Asn Thr Ala Lys Glu Val Lys Lys Gln Ile
575 580 585 590

AAT GAT ACA ACC GGA AAA TTT AAG GAT GTA AAT CAC TTA TAT GAT GTA 3211
Asn Asp Thr Thr Gly Lys Phe Lys Asp Val Asn His Leu Tyr Asp Val
595 600 605

AAA CTG ACT CCA AAA ATG AAT TTT ACG ATT AAA ATG GCT TCC TTG TAT 3259
Lys Leu Thr Pro Lys Met Asn Phe Thr Ile Lys Met Ala Ser Leu Tyr
610 615 620

GAT GGG GCT GAA AAT AAT CAT AAC TCT TTA GGA ACC TGG TAT TTA ACA 3307
Asp Gly Ala Glu Asn Asn His Asn Ser Leu Gly Thr Trp Tyr Leu Thr
625 630 635

TAT AAT GTT GCT GGT GGA AAT ACT GGG AAG AGA CAA TAT CGT TCA GCT 3355
Tyr Asn Val Ala Gly Gly Asn Thr Gly Lys Arg Gln Tyr Arg Ser Ala
640 645 650

CAT TCT TGT GCA CAT GTA GCT CTA TCT TCA GAA GCG AAA AAG AAA CTA 3403
His Ser Cys Ala His Val Ala Leu Ser Ser Glu Ala Lys Lys Lys Leu
655 660 665 670

AAT CAA AAT GCG AAT TAC TAT CTT AGC ATG TAT ATG AAG GCT GAT TCT 3451
Asn Gln Asn Ala Asn Tyr Tyr Leu Ser Met Tyr Met Lys Ala Asp Ser
675 680 685

ACT ACG GAA CCT ACA ATA GAA GTA GCT GGG GAA AAA TCT GCA ATA ACA 3499

Thr Thr Glu Pro Thr Ile Glu Val Ala Gly Glu Lys Ser Ala Ile Thr
690 695 700

AGT AAA AAA GTA AAA TTA AAT AAT CAA AAT TAT CAA AGA GTT GAT ATT 3547
Ser Lys Lys Val Lys Leu Asn Asn Gln Asn Tyr Gln Arg Val Asp Ile
705 710 715

TTA GTG AAA AAT TCT GAA AGA AAT CCA ATG GAT AAA ATA TAT ATA AGA 3595
Leu Val Lys Asn Ser Glu Arg Asn Pro Met Asp Lys Ile Tyr Ile Arg
720 725 730

GGA AAT GGC ACG ACA AAT GTT TAT GGG GAT GAT GTT ACT ATC CCA GAG 3643
Gly Asn Gly Thr Thr Asn Val Tyr Gly Asp Asp Val Thr Ile Pro Glu
735 740 745 750

GTA TCA GCT ATA AAT CCG GCT AGT CTA TCA GAT GAA GAA ATT CAA GAA 3691
Val Ser Ala Ile Asn Pro Ala Ser Leu Ser Asp Glu Glu Ile Gln Glu
755 760 765

ATA TTT AAA GAC TCA ACT ATT GAA TAT GGA AAT CCT AGT TTC GTT GCT 3739
Ile Phe Lys Asp Ser Thr Ile Glu Tyr Gly Asn Pro Ser Phe Val Ala
770 775 780

GAT GCC GTA ACA TTT AAA AAT ATA AAA CCT TTA CAA AAT TAT GTA AAG 3787
Asp Ala Val Thr Phe Lys Asn Ile Lys Pro Leu Gln Asn Tyr Val Lys
785 790 795

GAA TAT GAA ATA TAT CAT AAA TCT CAT CGA TAT GAA AAG AAA ACG GTC 3835
Glu Tyr Glu Ile Tyr His Lys Ser His Arg Tyr Glu Lys Lys Thr Val
800 805 810

TTT GAT ATC ATG GGT GTT CAT TAT GAG TAT AGT ATA GCT AGG GAA CAA 3883
Phe Asp Ile Met Gly Val His Tyr Glu Tyr Ser Ile Ala Arg Glu Gln
815 820 825 830

AAG AAA GCC GCA TAATTTTAAA AATAAAACTC GTTAGAGTTT ATTTAGCATG 3935
Lys Lys Ala Ala

GTATTTTTAA GAATAATCAA TATGTTGAAC CGTTTGTAGC TGTTTTGGAA GGGAATTTCA 3995

TTTTATTTGG TCTCTTAAGT TGATGGGCAT GGGATATGTT CAGCATCCAA GCGTTTNGGG 4055

GGTTANAAAA TCCAATTTT 4074

(2) INFORMATION FOR SEQ ID NO:20:

(i) SEQUENCE CHARACTERISTICS:
(A) LENGTH: 462 amino acids
(B) TYPE: amino acid
(D) TOPOLOGY: linear

(ii) MOLECULE TYPE: protein

(xi) SEQUENCE DESCRIPTION: SEQ ID NO:20:

Met Gln Arg Met Glu Gly Lys Leu Phe Val Val Ser Lys Thr Leu Gln
1               5                   10                  15

Val Val Thr Arg Thr Val Leu Leu Ser Thr Val Tyr Ser Ile Thr Leu
            20                  25                  30

Leu Asn Asn Val Val Ile Lys Ala Asp Gln Leu Asn Ile Asn Ser Gln
        35                  40                  45

Ser Lys Tyr Thr Asn Leu Gln Asn Leu Lys Ile Pro Asp Asn Ala Glu
    50                  55                  60

Asp Phe Lys Glu Asp Lys Gly Lys Ala Lys Glu Trp Gly Lys Glu Lys
65                  70                  75                  80

Gly Glu Glu Trp Arg Pro Pro Ala Thr Glu Lys Gly Glu Met Asn Asn
                85                  90                  95

Phe Leu Asp Asn Lys Asn Asp Ile Lys Thr Asn Tyr Lys Glu Ile Thr
            100                 105                 110

Phe Ser Met Ala Gly Ser Cys Glu Asp Glu Ile Lys Asp Leu Glu Glu
        115                 120                 125

Ile Asp Lys Ile Phe Asp Lys Ala Asn Leu Ser Ser Ser Ile Ile Thr
    130                 135                 140

Tyr Lys Asn Val Glu Pro Ala Thr Ile Gly Phe Asn Lys Ser Leu Thr
145                 150                 155                 160

Glu Gly Asn Thr Ile Asn Ser Asp Ala Met Ala Gln Phe Lys Glu Gln
                165                 170                 175

Phe Leu Gly Lys Asp Met Lys Phe Asp Ser Tyr Leu Asp Thr His Leu
            180                 185                 190

Thr Ala Gln Gln Val Ser Ser Lys Lys Arg Val Ile Leu Lys Val Thr
        195                 200                 205

Val Pro Ser Gly Lys Gly Ser Thr Thr Pro Thr Lys Ala Gly Val Ile
    210                 215                 220

Leu Asn Asn Asn Glu Tyr Lys Met Leu Ile Asp Asn Gly Tyr Val Leu
225                 230                 235                 240

His Val Asp Lys Val Ser Lys Val Val Lys Lys Gly Met Glu Cys Leu
                245                 250                 255

Gln Val Glu Gly Thr Leu Lys Lys Ser Leu Asp Phe Lys Asn Asp Ile
            260                 265                 270

Asn Ala Glu Ala His Ser Trp Gly Met Lys Ile Tyr Glu Asp Trp Ala
        275                 280                 285

Lys Asn Leu Thr Ala Ser Gln Arg Glu Ala Leu Asp Gly Tyr Ala Arg
    290                 295                 300

Gln Asp Tyr Lys Glu Ile Asn Asn Tyr Leu Arg Asn Gln Gly Gly Ser
305                 310                 315                 320

Gly Asn Glu Lys Leu Asp Ala Gln Leu Lys Asn Ile Ser Asp Ala Leu
                325                 330                 335

Gly Lys Lys Pro Ile Pro Glu Asn Ile Thr Val Tyr Arg Trp Cys Gly
            340                 345                 350

Met Pro Glu Phe Gly Tyr Gln Ile Ser Asp Pro Leu Pro Ser Leu Lys
        355                 360                 365

Asp Phe Glu Glu Gln Phe Leu Asn Thr Ile Lys Glu Asp Lys Gly Tyr
    370                 375                 380

Met Ser Thr Ser Leu Ser Ser Glu Arg Leu Ala Ala Phe Gly Ser Arg
385                 390                 395                 400

Lys Ile Ile Leu Arg Leu Gln Val Pro Lys Gly Ser Thr Gly Ala Tyr
                405                 410                 415

Leu Ser Ala Ile Gly Gly Phe Ala Ser Glu Lys Glu Ile Leu Leu Asp
            420                 425                 430

Lys Asp Ser Lys Tyr His Ile Asp Lys Ala Thr Glu Val Ile Ile Lys
        435                 440                 445

Gly Val Lys Arg Tyr Val Val Asp Ala Thr Leu Leu Thr Asn
    450                 455                 460

(2) INFORMATION FOR SEQ ID NO:21:

        (i) SEQUENCE CHARACTERISTICS:
            (A) LENGTH: 834 amino acids
            (B) TYPE: amino acid
            (D) TOPOLOGY: linear

       (ii) MOLECULE TYPE: protein

       (xi) SEQUENCE DESCRIPTION: SEQ ID NO:21:

Met Lys Asn Met Lys Lys Lys Leu Ala Ser Val Val Thr Cys Met Leu
  1               5                  10                  15

Leu Ala Pro Met Phe Leu Asn Gly Asn Val Asn Ala Val Asn Ala Asp
             20                  25                  30

Ser Lys Ile Asn Gln Ile Ser Thr Thr Gln Glu Asn Gln Gln Lys Glu
         35                  40                  45

Met Asp Arg Lys Gly Leu Leu Gly Tyr Tyr Phe Lys Gly Lys Asp Phe
    50                  55                  60

Asn Asn Leu Thr Met Phe Ala Pro Thr Arg Asp Asn Thr Leu Met Tyr
65                  70                  75                  80

Asp Gln Gln Thr Ala Asn Ala Leu Leu Asp Lys Lys Gln Gln Glu Tyr
                85                  90                  95

Gln Ser Ile Arg Trp Ile Gly Leu Ile Gln Arg Lys Glu Thr Gly Asp
            100                 105                 110

Phe Thr Phe Asn Leu Ser Lys Asp Glu Gln Ala Ile Ile Glu Ile Asp
        115                 120                 125

Gly Lys Ile Ile Ser Asn Lys Gly Lys Glu Lys Gln Val Val His Leu
    130                 135                 140

Glu Lys Glu Lys Leu Val Pro Ile Lys Ile Glu Tyr Gln Ser Asp Thr
145                 150                 155                 160

Lys Phe Asn Ile Asp Ser Lys Thr Phe Lys Glu Leu Lys Leu Phe Lys
                165                 170                 175

Ile Asp Ser Gln Asn Gln Ser Gln Gln Val Gln Leu Arg Asn Pro Glu
            180                 185                 190

Phe Asn Lys Lys Glu Ser Gln Glu Phe Leu Ala Lys Ala Ser Lys Thr
        195                 200                 205

Asn Leu Phe Lys Gln Lys Met Lys Arg Asp Ile Asp Glu Asp Thr Asp
    210                 215                 220

Thr Asp Gly Asp Ser Ile Pro Asp Leu Trp Glu Glu Asn Gly Tyr Thr
225                 230                 235                 240

Ile Gln Asn Lys Val Ala Val Lys Trp Asp Asp Ser Leu Ala Ser Lys
                245                 250                 255

Gly Tyr Thr Lys Phe Val Ser Asn Pro Leu Asp Ser His Thr Val Gly
            260                 265                 270

Asp Pro Tyr Thr Asp Tyr Glu Lys Ala Ala Arg Asp Leu Asp Leu Ser
        275                 280                 285

Asn Ala Lys Glu Thr Phe Asn Pro Leu Val Ala Ala Phe Pro Ser Val
    290                 295                 300

Asn Val Ser Met Glu Lys Val Ile Leu Ser Pro Asn Glu Asn Leu Ser
305                 310                 315                 320

Asn Ser Val Glu Ser His Ser Ser Thr Asn Trp Ser Tyr Thr Asn Thr
                325                 330                 335

Glu Gly Ala Ser Ile Glu Ala Gly Gly Gly Pro Leu Gly Leu Ser Phe

            340                 345                 350

Gly Val Ser Val Thr Tyr Gln His Ser Glu Thr Val Ala Gln Glu Trp
        355                 360                 365

Gly Thr Ser Thr Gly Asn Thr Ser Gln Phe Asn Thr Ala Ser Ala Gly
    370                 375                 380

Tyr Leu Asn Ala Asn Val Arg Tyr Asn Asn Val Gly Thr Gly Ala Ile
385                 390                 395                 400

Tyr Asp Val Lys Pro Thr Thr Ser Phe Val Leu Asn Asn Asn Thr Ile
                405                 410                 415

Ala Thr Ile Thr Ala Lys Ser Asn Ser Thr Ala Leu Arg Ile Ser Pro
            420                 425                 430

Gly Asp Ser Tyr Pro Glu Ile Gly Glu Asn Ala Ile Ala Ile Thr Ser
        435                 440                 445

Met Asp Asp Phe Asn Ser His Pro Ile Thr Leu Asn Lys Gln Gln Val
    450                 455                 460

Asn Gln Leu Ile Asn Asn Lys Pro Ile Met Leu Glu Thr Asp Gln Thr
465                 470                 475                 480

Asp Gly Val Tyr Lys Ile Arg Asp Thr His Gly Asn Ile Val Thr Gly
                485                 490                 495

Gly Glu Trp Asn Gly Val Thr Gln Gln Ile Lys Ala Lys Thr Ala Ser
            500                 505                 510

Ile Ile Val Asp Asp Gly Lys Gln Val Ala Glu Lys Arg Val Ala Ala
        515                 520                 525

Lys Asp Tyr Gly His Pro Glu Asp Lys Thr Pro Pro Leu Thr Leu Lys
    530                 535                 540

Asp Thr Leu Lys Leu Ser Tyr Pro Asp Glu Ile Lys Glu Thr Asn Gly
545                 550                 555                 560

Leu Leu Tyr Tyr Asp Asp Lys Pro Ile Tyr Glu Ser Ser Val Met Thr
                565                 570                 575

Tyr Leu Asp Glu Asn Thr Ala Lys Glu Val Lys Lys Gln Ile Asn Asp
            580                 585                 590

Thr Thr Gly Lys Phe Lys Asp Val Asn His Leu Tyr Asp Val Lys Leu
        595                 600                 605

Thr Pro Lys Met Asn Phe Thr Ile Lys Met Ala Ser Leu Tyr Asp Gly
    610                 615                 620

Ala Glu Asn Asn His Asn Ser Leu Gly Thr Trp Tyr Leu Thr Tyr Asn
625                 630                 635                 640

Val Ala Gly Gly Asn Thr Gly Lys Arg Gln Tyr Arg Ser Ala His Ser
                645                 650                 655

Cys Ala His Val Ala Leu Ser Ser Glu Ala Lys Lys Lys Leu Asn Gln
            660                 665                 670

Asn Ala Asn Tyr Tyr Leu Ser Met Tyr Met Lys Ala Asp Ser Thr Thr
        675                 680                 685

Glu Pro Thr Ile Glu Val Ala Gly Glu Lys Ser Ala Ile Thr Ser Lys
    690                 695                 700

Lys Val Lys Leu Asn Asn Gln Asn Tyr Gln Arg Val Asp Ile Leu Val
705                 710                 715                 720

Lys Asn Ser Glu Arg Asn Pro Met Asp Lys Ile Tyr Ile Arg Gly Asn
                725                 730                 735

Gly Thr Thr Asn Val Tyr Gly Asp Asp Val Thr Ile Pro Glu Val Ser
            740                 745                 750

Ala Ile Asn Pro Ala Ser Leu Ser Asp Glu Glu Ile Gln Glu Ile Phe
        755                 760                 765

Lys Asp Ser Thr Ile Glu Tyr Gly Asn Pro Ser Phe Val Ala Asp Ala
    770                 775                 780

Val Thr Phe Lys Asn Ile Lys Pro Leu Gln Asn Tyr Val Lys Glu Tyr
785                 790                 795                 800

Glu Ile Tyr His Lys Ser His Arg Tyr Glu Lys Lys Thr Val Phe Asp
                805                 810                 815

Ile Met Gly Val His Tyr Glu Tyr Ser Ile Ala Arg Glu Gln Lys Lys
            820                 825                 830

Ala Ala

(2) INFORMATION FOR SEQ ID NO:22:

    (i) SEQUENCE CHARACTERISTICS:
        (A) LENGTH: 4041 base pairs
        (B) TYPE: nucleic acid
        (C) STRANDEDNESS: single
        (D) TOPOLOGY: linear

    (ii) MOLECULE TYPE: DNA (genomic)

    (ix) FEATURE:
        (A) NAME/KEY: CDS
        (B) LOCATION: 1..4038
        (D) OTHER INFORMATION: /product= "VIP1A(a)/VIP2A(a) fusion

product"

(xi) SEQUENCE DESCRIPTION: SEQ ID NO:22:

ATG AAA AGA ATG GAG GGA AAG TTG TTT ATG GTG TCA AAA AAA TTA CAA 48
Met Lys Arg Met Glu Gly Lys Leu Phe Met Val Ser Lys Lys Leu Gln
835 840 845 850

GTA GTT ACT AAA ACT GTA TTG CTT AGT ACA GTT TTC TCT ATA TCT TTA 96
Val Val Thr Lys Thr Val Leu Leu Ser Thr Val Phe Ser Ile Ser Leu
855 860 865

TTA AAT AAT GAA GTG ATA AAA GCT GAA CAA TTA AAT ATA AAT TCT CAA 144
Leu Asn Asn Glu Val Ile Lys Ala Glu Gln Leu Asn Ile Asn Ser Gln
870 875 880

AGT AAA TAT ACT AAC TTG CAA AAT CTA AAA ATC ACT GAC AAG GTA GAG 192
Ser Lys Tyr Thr Asn Leu Gln Asn Leu Lys Ile Thr Asp Lys Val Glu
885 890 895

GAT TTT AAA GAA GAT AAG GAA AAA GCG AAA GAA TGG GGG AAA GAA AAA 240
Asp Phe Lys Glu Asp Lys Glu Lys Ala Lys Glu Trp Gly Lys Glu Lys
900 905 910

GAA AAA GAG TGG AAA CTA ACT GCT ACT GAA AAA GGA AAA ATG AAT AAT 288
Glu Lys Glu Trp Lys Leu Thr Ala Thr Glu Lys Gly Lys Met Asn Asn
915 920 925 930

TTT TTA GAT AAT AAA AAT GAT ATA AAG ACA AAT TAT AAA GAA ATT ACT 336
Phe Leu Asp Asn Lys Asn Asp Ile Lys Thr Asn Tyr Lys Glu Ile Thr
935 940 945

TTT TCT ATG GCA GGC TCA TTT GAA GAT GAA ATA AAA GAT TTA AAA GAA 384
Phe Ser Met Ala Gly Ser Phe Glu Asp Glu Ile Lys Asp Leu Lys Glu
950 955 960

ATT GAT AAG ATG TTT GAT AAA ACC AAT CTA TCA AAT TCT ATT ATC ACC 432
Ile Asp Lys Met Phe Asp Lys Thr Asn Leu Ser Asn Ser Ile Ile Thr
965 970 975

TAT AAA AAT GTG GAA CCG ACA ACA ATT GGA TTT AAT AAA TCT TTA ACA 480
Tyr Lys Asn Val Glu Pro Thr Thr Ile Gly Phe Asn Lys Ser Leu Thr
980 985 990

GAA GGT AAT ACG ATT AAT TCT GAT GCA ATG GCA CAG TTT AAA GAA CAA 528
Glu Gly Asn Thr Ile Asn Ser Asp Ala Met Ala Gln Phe Lys Glu Gln
995 1000 1005 1010

TTT TTA GAT AGG GAT ATT AAG TTT GAT AGT TAT CTA GAT ACG CAT TTA 576
Phe Leu Asp Arg Asp Ile Lys Phe Asp Ser Tyr Leu Asp Thr His Leu
1015 1020 1025

ACT GCT CAA CAA GTT TCC AGT AAA GAA AGA GTT ATT TTG AAG GTT ACG 624
Thr Ala Gln Gln Val Ser Ser Lys Glu Arg Val Ile Leu Lys Val Thr

1030                1035                1040

GTT CCG AGT GGG AAA GGT TCT ACT ACT CCA ACA AAA GCA GGT GTC ATT        672
Val Pro Ser Gly Lys Gly Ser Thr Thr Pro Thr Lys Ala Gly Val Ile
        1045                1050                1055

TTA AAT AAT AGT GAA TAC AAA ATG CTC ATT GAT AAT GGG TAT ATG GTC        720
Leu Asn Asn Ser Glu Tyr Lys Met Leu Ile Asp Asn Gly Tyr Met Val
    1060                1065                1070

CAT GTA GAT AAG GTA TCA AAA GTG GTG AAA AAA GGG GTG GAG TGC TTA        768
His Val Asp Lys Val Ser Lys Val Val Lys Lys Gly Val Glu Cys Leu
1075                1080                1085                1090

CAA ATT GAA GGG ACT TTA AAA AAG AGT CTT GAC TTT AAA AAT GAT ATA        816
Gln Ile Glu Gly Thr Leu Lys Lys Ser Leu Asp Phe Lys Asn Asp Ile
                1095                1100                1105

AAT GCT GAA GCG CAT AGC TGG GGT ATG AAG AAT TAT GAA GAG TGG GCT        864
Asn Ala Glu Ala His Ser Trp Gly Met Lys Asn Tyr Glu Glu Trp Ala
            1110                1115                1120

AAA GAT TTA ACC GAT TCG CAA AGG GAA GCT TTA GAT GGG TAT GCT AGG        912
Lys Asp Leu Thr Asp Ser Gln Arg Glu Ala Leu Asp Gly Tyr Ala Arg
        1125                1130                1135

CAA GAT TAT AAA GAA ATC AAT AAT TAT TTA AGA AAT CAA GGC GGA AGT        960
Gln Asp Tyr Lys Glu Ile Asn Asn Tyr Leu Arg Asn Gln Gly Gly Ser
    1140                1145                1150

GGA AAT GAA AAA CTA GAT GCT CAA ATA AAA AAT ATT TCT GAT GCT TTA        1008
Gly Asn Glu Lys Leu Asp Ala Gln Ile Lys Asn Ile Ser Asp Ala Leu
1155                1160                1165                1170

GGG AAG AAA CCA ATA CCG GAA AAT ATT ACT GTG TAT AGA TGG TGT GGC        1056
Gly Lys Lys Pro Ile Pro Glu Asn Ile Thr Val Tyr Arg Trp Cys Gly
                1175                1180                1185

ATG CCG GAA TTT GGT TAT CAA ATT AGT GAT CCG TTA CCT TCT TTA AAA        1104
Met Pro Glu Phe Gly Tyr Gln Ile Ser Asp Pro Leu Pro Ser Leu Lys
            1190                1195                1200

GAT TTT GAA GAA CAA TTT TTA AAT ACA ATC AAA GAA GAC AAA GGA TAT        1152
Asp Phe Glu Glu Gln Phe Leu Asn Thr Ile Lys Glu Asp Lys Gly Tyr
        1205                1210                1215

ATG AGT ACA AGC TTA TCG AGT GAA CGT CTT GCA GCT TTT GGA TCT AGA        1200
Met Ser Thr Ser Leu Ser Ser Glu Arg Leu Ala Ala Phe Gly Ser Arg
    1220                1225                1230

AAA ATT ATA TTA CGA TTA CAA GTT CCG AAA GGA AGT ACG GGT GCG TAT        1248
Lys Ile Ile Leu Arg Leu Gln Val Pro Lys Gly Ser Thr Gly Ala Tyr
1235                1240                1245                1250

TTA AGT GCC ATT GGT GGA TTT GCA AGT GAA AAA GAG ATC CTA CTT GAT        1296

Leu Ser Ala Ile Gly Gly Phe Ala Ser Glu Lys Glu Ile Leu Leu Asp
                1255                    1260                    1265

AAA GAT AGT AAA TAT CAT ATT GAT AAA GTA ACA GAG GTA ATT ATT AAA        1344
Lys Asp Ser Lys Tyr His Ile Asp Lys Val Thr Glu Val Ile Ile Lys
            1270                    1275                    1280

GGT GTT AAG CGA TAT GTA GTG GAT GCA ACA TTA TTA ACA AAT ATG AAA        1392
Gly Val Lys Arg Tyr Val Val Asp Ala Thr Leu Leu Thr Asn Met Lys
        1285                    1290                    1295

AAT ATG AAG AAA AAG TTA GCA AGT GTT GTA ACG TGT ACG TTA TTA GCT        1440
Asn Met Lys Lys Lys Leu Ala Ser Val Val Thr Cys Thr Leu Leu Ala
    1300                    1305                    1310

CCT ATG TTT TTG AAT GGA AAT GTG AAT GCT GTT TAC GCA GAC AGC AAA        1488
Pro Met Phe Leu Asn Gly Asn Val Asn Ala Val Tyr Ala Asp Ser Lys
1315                    1320                    1325                    1330

ACA AAT CAA ATT TCT ACA ACA CAG AAA AAT CAA CAG AAA GAG ATG GAC        1536
Thr Asn Gln Ile Ser Thr Thr Gln Lys Asn Gln Gln Lys Glu Met Asp
                1335                    1340                    1345

CGA AAA GGA TTA CTT GGG TAT TAT TTC AAA GGA AAA GAT TTT AGT AAT        1584
Arg Lys Gly Leu Leu Gly Tyr Tyr Phe Lys Gly Lys Asp Phe Ser Asn
            1350                    1355                    1360

CTT ACT ATG TTT GCA CCG ACA CGT GAT AGT ACT CTT ATT TAT GAT CAA        1632
Leu Thr Met Phe Ala Pro Thr Arg Asp Ser Thr Leu Ile Tyr Asp Gln
        1365                    1370                    1375

CAA ACA GCA AAT AAA CTA TTA GAT AAA AAA CAA CAA GAA TAT CAG TCT        1680
Gln Thr Ala Asn Lys Leu Leu Asp Lys Lys Gln Gln Glu Tyr Gln Ser
    1380                    1385                    1390

ATT CGT TGG ATT GGT TTG ATT CAG AGT AAA GAA ACG GGA GAT TTC ACA        1728
Ile Arg Trp Ile Gly Leu Ile Gln Ser Lys Glu Thr Gly Asp Phe Thr
1395                    1400                    1405                    1410

TTT AAC TTA TCT GAG GAT GAA CAG GCA ATT ATA GAA ATC AAT GGG AAA        1776
Phe Asn Leu Ser Glu Asp Glu Gln Ala Ile Ile Glu Ile Asn Gly Lys
                1415                    1420                    1425

ATT ATT TCT AAT AAA GGG AAA GAA AAG CAA GTT GTC CAT TTA GAA AAA        1824
Ile Ile Ser Asn Lys Gly Lys Glu Lys Gln Val Val His Leu Glu Lys
            1430                    1435                    1440

GGA AAA TTA GTT CCA ATC AAA ATA GAG TAT CAA TCA GAT ACA AAA TTT        1872
Gly Lys Leu Val Pro Ile Lys Ile Glu Tyr Gln Ser Asp Thr Lys Phe
        1445                    1450                    1455

AAT ATT GAC AGT AAA ACA TTT AAA GAA CTT AAA TTA TTT AAA ATA GAT        1920
Asn Ile Asp Ser Lys Thr Phe Lys Glu Leu Lys Leu Phe Lys Ile Asp
    1460                    1465                    1470

AGT CAA AAC CAA CCC CAG CAA GTC CAG CAA GAT GAA CTG AGA AAT CCT 1968
Ser Gln Asn Gln Pro Gln Gln Val Gln Gln Asp Glu Leu Arg Asn Pro
1475 1480 1485 1490

GAA TTT AAC AAG AAA GAA TCA CAG GAA TTC TTA GCG AAA CCA TCG AAA 2016
Glu Phe Asn Lys Lys Glu Ser Gln Glu Phe Leu Ala Lys Pro Ser Lys
1495 1500 1505

ATA AAT CTT TTC ACT CAA AAA ATG AAA AGG GAA ATT GAT GAA GAC ACG 2064
Ile Asn Leu Phe Thr Gln Lys Met Lys Arg Glu Ile Asp Glu Asp Thr
1510 1515 1520

GAT ACG GAT GGG GAC TCT ATT CCT GAC CTT TGG GAA GAA AAT GGG TAT 2112
Asp Thr Asp Gly Asp Ser Ile Pro Asp Leu Trp Glu Glu Asn Gly Tyr
1525 1530 1535

ACG ATT CAA AAT AGA ATC GCT GTA AAG TGG GAC GAT TCT CTA GCA AGT 2160
Thr Ile Gln Asn Arg Ile Ala Val Lys Trp Asp Asp Ser Leu Ala Ser
1540 1545 1550

AAA GGG TAT ACG AAA TTT GTT TCA AAT CCA CTA GAA AGT CAC ACA GTT 2208
Lys Gly Tyr Thr Lys Phe Val Ser Asn Pro Leu Glu Ser His Thr Val
1555 1560 1565 1570

GGT GAT CCT TAT ACA GAT TAT GAA AAG GCA GCA AGA GAT CTA GAT TTG 2256
Gly Asp Pro Tyr Thr Asp Tyr Glu Lys Ala Ala Arg Asp Leu Asp Leu
1575 1580 1585

TCA AAT GCA AAG GAA ACG TTT AAC CCA TTG GTA GCT GCT TTT CCA AGT 2304
Ser Asn Ala Lys Glu Thr Phe Asn Pro Leu Val Ala Ala Phe Pro Ser
1590 1595 1600

GTG AAT GTT AGT ATG GAA AAG GTG ATA TTA TCA CCA AAT GAA AAT TTA 2352
Val Asn Val Ser Met Glu Lys Val Ile Leu Ser Pro Asn Glu Asn Leu
1605 1610 1615

TCC AAT AGT GTA GAG TCT CAT TCA TCC ACG AAT TGG TCT TAT ACA AAT 2400
Ser Asn Ser Val Glu Ser His Ser Ser Thr Asn Trp Ser Tyr Thr Asn
1620 1625 1630

ACA GAA GGT GCT TCT GTT GAA GCG GGG ATT GGA CCA AAA GGT ATT TCG 2448
Thr Glu Gly Ala Ser Val Glu Ala Gly Ile Gly Pro Lys Gly Ile Ser
1635 1640 1645 1650

TTC GGA GTT AGC GTA AAC TAT CAA CAC TCT GAA ACA GTT GCA CAA GAA 2496
Phe Gly Val Ser Val Asn Tyr Gln His Ser Glu Thr Val Ala Gln Glu
1655 1660 1665

TGG GGA ACA TCT ACA GGA AAT ACT TCG CAA TTC AAT ACG GCT TCA GCG 2544
Trp Gly Thr Ser Thr Gly Asn Thr Ser Gln Phe Asn Thr Ala Ser Ala
1670 1675 1680

GGA TAT TTA AAT GCA AAT GTT CGA TAT AAC AAT GTA GGA ACT GGT GCC 2592
Gly Tyr Leu Asn Ala Asn Val Arg Tyr Asn Asn Val Gly Thr Gly Ala
1685 1690 1695

ATC TAC GAT GTA AAA CCT ACA ACA AGT TTT GTA TTA AAT AAC GAT ACT 2640
Ile Tyr Asp Val Lys Pro Thr Thr Ser Phe Val Leu Asn Asn Asp Thr
1700 1705 1710

ATC GCA ACT ATT ACG GCG AAA TCT AAT TCT ACA GCC TTA AAT ATA TCT 2688
Ile Ala Thr Ile Thr Ala Lys Ser Asn Ser Thr Ala Leu Asn Ile Ser
1715 1720 1725 1730

CCT GGA GAA AGT TAC CCG AAA AAA GGA CAA AAT GGA ATC GCA ATA ACA 2736
Pro Gly Glu Ser Tyr Pro Lys Lys Gly Gln Asn Gly Ile Ala Ile Thr
1735 1740 1745

TCA ATG GAT GAT TTT AAT TCC CAT CCG ATT ACA TTA AAT AAA AAA CAA 2784
Ser Met Asp Asp Phe Asn Ser His Pro Ile Thr Leu Asn Lys Lys Gln
1750 1755 1760

GTA GAT AAT CTG CTA AAT AAT AAA CCT ATG ATG TTG GAA ACA AAC CAA 2832
Val Asp Asn Leu Leu Asn Asn Lys Pro Met Met Leu Glu Thr Asn Gln
1765 1770 1775

ACA GAT GGT GTT TAT AAG ATA AAA GAT ACA CAT GGA AAT ATA GTA ACT 2880
Thr Asp Gly Val Tyr Lys Ile Lys Asp Thr His Gly Asn Ile Val Thr
1780 1785 1790

GGC GGA GAA TGG AAT GGT GTC ATA CAA CAA ATC AAG GCT AAA ACA GCG 2928
Gly Gly Glu Trp Asn Gly Val Ile Gln Gln Ile Lys Ala Lys Thr Ala
1795 1800 1805 1810

TCT ATT ATT GTG GAT GAT GGG GAA CGT GTA GCA GAA AAA CGT GTA GCG 2976
Ser Ile Ile Val Asp Asp Gly Glu Arg Val Ala Glu Lys Arg Val Ala
1815 1820 1825

GCA AAA GAT TAT GAA AAT CCA GAA GAT AAA ACA CCG TCT TTA ACT TTA 3024
Ala Lys Asp Tyr Glu Asn Pro Glu Asp Lys Thr Pro Ser Leu Thr Leu
1830 1835 1840

AAA GAT GCC CTG AAG CTT TCA TAT CCA GAT GAA ATA AAA GAA ATA GAG 3072
Lys Asp Ala Leu Lys Leu Ser Tyr Pro Asp Glu Ile Lys Glu Ile Glu
1845 1850 1855

GGA TTA TTA TAT TAT AAA AAC AAA CCG ATA TAC GAA TCG AGC GTT ATG 3120
Gly Leu Leu Tyr Tyr Lys Asn Lys Pro Ile Tyr Glu Ser Ser Val Met
1860 1865 1870

ACT TAC TTA GAT GAA AAT ACA GCA AAA GAA GTG ACC AAA CAA TTA AAT 3168
Thr Tyr Leu Asp Glu Asn Thr Ala Lys Glu Val Thr Lys Gln Leu Asn
1875 1880 1885 1890

GAT ACC ACT GGG AAA TTT AAA GAT GTA AGT CAT TTA TAT GAT GTA AAA 3216
Asp Thr Thr Gly Lys Phe Lys Asp Val Ser His Leu Tyr Asp Val Lys
1895 1900 1905

CTG ACT CCA AAA ATG AAT GTT ACA ATC AAA TTG TCT ATA CTT TAT GAT 3264
Leu Thr Pro Lys Met Asn Val Thr Ile Lys Leu Ser Ile Leu Tyr Asp

1910                    1915                    1920

AAT GCT GAG TCT AAT GAT AAC TCA ATT GGT AAA TGG ACA AAC ACA AAT          3312
Asn Ala Glu Ser Asn Asp Asn Ser Ile Gly Lys Trp Thr Asn Thr Asn
        1925                    1930                    1935

ATT GTT TCA GGT GGA AAT AAC GGA AAA AAA CAA TAT TCT TCT AAT AAT          3360
Ile Val Ser Gly Gly Asn Asn Gly Lys Lys Gln Tyr Ser Ser Asn Asn
    1940                    1945                    1950

CCG GAT GCT AAT TTG ACA TTA AAT ACA GAT GCT CAA GAA AAA TTA AAT          3408
Pro Asp Ala Asn Leu Thr Leu Asn Thr Asp Ala Gln Glu Lys Leu Asn
1955                    1960                    1965                    1970

AAA AAT CGT GAC TAT TAT ATA AGT TTA TAT ATG AAG TCA GAA AAA AAC          3456
Lys Asn Arg Asp Tyr Tyr Ile Ser Leu Tyr Met Lys Ser Glu Lys Asn
                1975                    1980                    1985

ACA CAA TGT GAG ATT ACT ATA GAT GGG GAG ATT TAT CCG ATC ACT ACA          3504
Thr Gln Cys Glu Ile Thr Ile Asp Gly Glu Ile Tyr Pro Ile Thr Thr
            1990                    1995                    2000

AAA ACA GTG AAT GTG AAT AAA GAC AAT TAC AAA AGA TTA GAT ATT ATA          3552
Lys Thr Val Asn Val Asn Lys Asp Asn Tyr Lys Arg Leu Asp Ile Ile
        2005                    2010                    2015

GCT CAT AAT ATA AAA AGT AAT CCA ATT TCT TCA CTT CAT ATT AAA ACG          3600
Ala His Asn Ile Lys Ser Asn Pro Ile Ser Ser Leu His Ile Lys Thr
    2020                    2025                    2030

AAT GAT GAA ATA ACT TTA TTT TGG GAT GAT ATT TCT ATA ACA GAT GTA          3648
Asn Asp Glu Ile Thr Leu Phe Trp Asp Asp Ile Ser Ile Thr Asp Val
2035                    2040                    2045                    2050

GCA TCA ATA AAA CCG GAA AAT TTA ACA GAT TCA GAA ATT AAA CAG ATT          3696
Ala Ser Ile Lys Pro Glu Asn Leu Thr Asp Ser Glu Ile Lys Gln Ile
                2055                    2060                    2065

TAT AGT AGG TAT GGT ATT AAG TTA GAA GAT GGA ATC CTT ATT GAT AAA          3744
Tyr Ser Arg Tyr Gly Ile Lys Leu Glu Asp Gly Ile Leu Ile Asp Lys
            2070                    2075                    2080

AAA GGT GGG ATT CAT TAT GGT GAA TTT ATT AAT GAA GCT AGT TTT AAT          3792
Lys Gly Gly Ile His Tyr Gly Glu Phe Ile Asn Glu Ala Ser Phe Asn
        2085                    2090                    2095

ATT GAA CCA TTG CAA AAT TAT GTG ACC AAA TAT GAA GTT ACT TAT AGT          3840
Ile Glu Pro Leu Gln Asn Tyr Val Thr Lys Tyr Glu Val Thr Tyr Ser
    2100                    2105                    2110

AGT GAG TTA GGA CCA AAC GTG AGT GAC ACA CTT GAA AGT GAT AAA ATT          3888
Ser Glu Leu Gly Pro Asn Val Ser Asp Thr Leu Glu Ser Asp Lys Ile
2115                    2120                    2125                    2130

TAC AAG GAT GGG ACA ATT AAA TTT GAT TTT ACC AAA TAT AGT AAA AAT          3936

Tyr Lys Asp Gly Thr Ile Lys Phe Asp Phe Thr Lys Tyr Ser Lys Asn
2135 2140 2145

GAA CAA GGA TTA TTT TAT GAC AGT GGA TTA AAT TGG GAC TTT AAA ATT 3984
Glu Gln Gly Leu Phe Tyr Asp Ser Gly Leu Asn Trp Asp Phe Lys Ile
2150 2155 2160

AAT GCT ATT ACT TAT GAT GGT AAA GAG ATG AAT GTT TTT CAT AGA TAT 4032
Asn Ala Ile Thr Tyr Asp Gly Lys Glu Met Asn Val Phe His Arg Tyr
2165 2170 2175

AAT AAA TAG 4041
Asn Lys
2180

(2) INFORMATION FOR SEQ ID NO:23:

(i) SEQUENCE CHARACTERISTICS:
(A) LENGTH: 1346 amino acids
(B) TYPE: amino acid
(D) TOPOLOGY: linear

(ii) MOLECULE TYPE: protein

(xi) SEQUENCE DESCRIPTION: SEQ ID NO:23:

Met Lys Arg Met Glu Gly Lys Leu Phe Met Val Ser Lys Lys Leu Gln
1               5                   10                  15

Val Val Thr Lys Thr Val Leu Leu Ser Thr Val Phe Ser Ile Ser Leu
            20                  25                  30

Leu Asn Asn Glu Val Ile Lys Ala Glu Gln Leu Asn Ile Asn Ser Gln
        35                  40                  45

Ser Lys Tyr Thr Asn Leu Gln Asn Leu Lys Ile Thr Asp Lys Val Glu
    50                  55                  60

Asp Phe Lys Glu Asp Lys Glu Lys Ala Lys Glu Trp Gly Lys Glu Lys
65                  70                  75                  80

Glu Lys Glu Trp Lys Leu Thr Ala Thr Glu Lys Gly Lys Met Asn Asn
                85                  90                  95

Phe Leu Asp Asn Lys Asn Asp Ile Lys Thr Asn Tyr Lys Glu Ile Thr
            100                 105                 110

Phe Ser Met Ala Gly Ser Phe Glu Asp Glu Ile Lys Asp Leu Lys Glu
        115                 120                 125

Ile Asp Lys Met Phe Asp Lys Thr Asn Leu Ser Asn Ser Ile Ile Thr
    130                 135                 140

Tyr Lys Asn Val Glu Pro Thr Thr Ile Gly Phe Asn Lys Ser Leu Thr

145                 150                 155                 160

Glu Gly Asn Thr Ile Asn Ser Asp Ala Met Ala Gln Phe Lys Glu Gln
                165                 170                 175

Phe Leu Asp Arg Asp Ile Lys Phe Asp Ser Tyr Leu Asp Thr His Leu
            180                 185                 190

Thr Ala Gln Gln Val Ser Ser Lys Glu Arg Val Ile Leu Lys Val Thr
        195                 200                 205

Val Pro Ser Gly Lys Gly Ser Thr Thr Pro Thr Lys Ala Gly Val Ile
    210                 215                 220

Leu Asn Asn Ser Glu Tyr Lys Met Leu Ile Asp Asn Gly Tyr Met Val
225                 230                 235                 240

His Val Asp Lys Val Ser Lys Val Val Lys Lys Gly Val Glu Cys Leu
                245                 250                 255

Gln Ile Glu Gly Thr Leu Lys Lys Ser Leu Asp Phe Lys Asn Asp Ile
            260                 265                 270

Asn Ala Glu Ala His Ser Trp Gly Met Lys Asn Tyr Glu Glu Trp Ala
        275                 280                 285

Lys Asp Leu Thr Asp Ser Gln Arg Glu Ala Leu Asp Gly Tyr Ala Arg
    290                 295                 300

Gln Asp Tyr Lys Glu Ile Asn Asn Tyr Leu Arg Asn Gln Gly Gly Ser
305                 310                 315                 320

Gly Asn Glu Lys Leu Asp Ala Gln Ile Lys Asn Ile Ser Asp Ala Leu
                325                 330                 335

Gly Lys Lys Pro Ile Pro Glu Asn Ile Thr Val Tyr Arg Trp Cys Gly
            340                 345                 350

Met Pro Glu Phe Gly Tyr Gln Ile Ser Asp Pro Leu Pro Ser Leu Lys
        355                 360                 365

Asp Phe Glu Glu Gln Phe Leu Asn Thr Ile Lys Glu Asp Lys Gly Tyr
    370                 375                 380

Met Ser Thr Ser Leu Ser Ser Glu Arg Leu Ala Ala Phe Gly Ser Arg
385                 390                 395                 400

Lys Ile Ile Leu Arg Leu Gln Val Pro Lys Gly Ser Thr Gly Ala Tyr
                405                 410                 415

Leu Ser Ala Ile Gly Gly Phe Ala Ser Glu Lys Glu Ile Leu Leu Asp
            420                 425                 430

Lys Asp Ser Lys Tyr His Ile Asp Lys Val Thr Glu Val Ile Ile Lys
        435                 440                 445

Gly Val Lys Arg Tyr Val Val Asp Ala Thr Leu Leu Thr Asn Met Lys
450                 455                 460

Asn Met Lys Lys Lys Leu Ala Ser Val Val Thr Cys Thr Leu Leu Ala
465                 470                 475                 480

Pro Met Phe Leu Asn Gly Asn Val Asn Ala Val Tyr Ala Asp Ser Lys
                485                 490                 495

Thr Asn Gln Ile Ser Thr Thr Gln Lys Asn Gln Gln Lys Glu Met Asp
            500                 505                 510

Arg Lys Gly Leu Leu Gly Tyr Tyr Phe Lys Gly Lys Asp Phe Ser Asn
        515                 520                 525

Leu Thr Met Phe Ala Pro Thr Arg Asp Ser Thr Leu Ile Tyr Asp Gln
    530                 535                 540

Gln Thr Ala Asn Lys Leu Leu Asp Lys Lys Gln Gln Glu Tyr Gln Ser
545                 550                 555                 560

Ile Arg Trp Ile Gly Leu Ile Gln Ser Lys Glu Thr Gly Asp Phe Thr
                565                 570                 575

Phe Asn Leu Ser Glu Asp Glu Gln Ala Ile Ile Glu Ile Asn Gly Lys
            580                 585                 590

Ile Ile Ser Asn Lys Gly Lys Glu Lys Gln Val Val His Leu Glu Lys
        595                 600                 605

Gly Lys Leu Val Pro Ile Lys Ile Glu Tyr Gln Ser Asp Thr Lys Phe
    610                 615                 620

Asn Ile Asp Ser Lys Thr Phe Lys Glu Leu Lys Leu Phe Lys Ile Asp
625                 630                 635                 640

Ser Gln Asn Gln Pro Gln Gln Val Gln Gln Asp Glu Leu Arg Asn Pro
                645                 650                 655

Glu Phe Asn Lys Lys Glu Ser Gln Glu Phe Leu Ala Lys Pro Ser Lys
            660                 665                 670

Ile Asn Leu Phe Thr Gln Lys Met Lys Arg Glu Ile Asp Glu Asp Thr
        675                 680                 685

Asp Thr Asp Gly Asp Ser Ile Pro Asp Leu Trp Glu Glu Asn Gly Tyr
    690                 695                 700

Thr Ile Gln Asn Arg Ile Ala Val Lys Trp Asp Asp Ser Leu Ala Ser
705                 710                 715                 720

Lys Gly Tyr Thr Lys Phe Val Ser Asn Pro Leu Glu Ser His Thr Val
                725                 730                 735

Gly Asp Pro Tyr Thr Asp Tyr Glu Lys Ala Ala Arg Asp Leu Asp Leu
            740                 745                 750

Ser Asn Ala Lys Glu Thr Phe Asn Pro Leu Val Ala Ala Phe Pro Ser
        755                 760                 765

Val Asn Val Ser Met Glu Lys Val Ile Leu Ser Pro Asn Glu Asn Leu
    770                 775                 780

Ser Asn Ser Val Glu Ser His Ser Ser Thr Asn Trp Ser Tyr Thr Asn
785                 790                 795                 800

Thr Glu Gly Ala Ser Val Glu Ala Gly Ile Gly Pro Lys Gly Ile Ser
                805                 810                 815

Phe Gly Val Ser Val Asn Tyr Gln His Ser Glu Thr Val Ala Gln Glu
            820                 825                 830

Trp Gly Thr Ser Thr Gly Asn Thr Ser Gln Phe Asn Thr Ala Ser Ala
        835                 840                 845

Gly Tyr Leu Asn Ala Asn Val Arg Tyr Asn Asn Val Gly Thr Gly Ala
    850                 855                 860

Ile Tyr Asp Val Lys Pro Thr Thr Ser Phe Val Leu Asn Asn Asp Thr
865                 870                 875                 880

Ile Ala Thr Ile Thr Ala Lys Ser Asn Ser Thr Ala Leu Asn Ile Ser
                885                 890                 895

Pro Gly Glu Ser Tyr Pro Lys Lys Gly Gln Asn Gly Ile Ala Ile Thr
            900                 905                 910

Ser Met Asp Asp Phe Asn Ser His Pro Ile Thr Leu Asn Lys Lys Gln
        915                 920                 925

Val Asp Asn Leu Leu Asn Asn Lys Pro Met Met Leu Glu Thr Asn Gln
    930                 935                 940

Thr Asp Gly Val Tyr Lys Ile Lys Asp Thr His Gly Asn Ile Val Thr
945                 950                 955                 960

Gly Gly Glu Trp Asn Gly Val Ile Gln Gln Ile Lys Ala Lys Thr Ala
                965                 970                 975

Ser Ile Ile Val Asp Asp Gly Glu Arg Val Ala Glu Lys Arg Val Ala
            980                 985                 990

Ala Lys Asp Tyr Glu Asn Pro Glu Asp Lys Thr Pro Ser Leu Thr Leu
        995                 1000                1005

Lys Asp Ala Leu Lys Leu Ser Tyr Pro Asp Glu Ile Lys Glu Ile Glu
    1010                1015                1020

Gly Leu Leu Tyr Tyr Lys Asn Lys Pro Ile Tyr Glu Ser Ser Val Met

1025                    1030                    1035                    1040

Thr Tyr Leu Asp Glu Asn Thr Ala Lys Glu Val Thr Lys Gln Leu Asn
                1045                    1050                    1055

Asp Thr Thr Gly Lys Phe Lys Asp Val Ser His Leu Tyr Asp Val Lys
            1060                    1065                    1070

Leu Thr Pro Lys Met Asn Val Thr Ile Lys Leu Ser Ile Leu Tyr Asp
        1075                    1080                    1085

Asn Ala Glu Ser Asn Asp Asn Ser Ile Gly Lys Trp Thr Asn Thr Asn
    1090                    1095                    1100

Ile Val Ser Gly Gly Asn Asn Gly Lys Lys Gln Tyr Ser Ser Asn Asn
1105                    1110                    1115                    1120

Pro Asp Ala Asn Leu Thr Leu Asn Thr Asp Ala Gln Glu Lys Leu Asn
                1125                    1130                    1135

Lys Asn Arg Asp Tyr Tyr Ile Ser Leu Tyr Met Lys Ser Glu Lys Asn
            1140                    1145                    1150

Thr Gln Cys Glu Ile Thr Ile Asp Gly Glu Ile Tyr Pro Ile Thr Thr
        1155                    1160                    1165

Lys Thr Val Asn Val Asn Lys Asp Asn Tyr Lys Arg Leu Asp Ile Ile
    1170                    1175                    1180

Ala His Asn Ile Lys Ser Asn Pro Ile Ser Ser Leu His Ile Lys Thr
1185                    1190                    1195                    1200

Asn Asp Glu Ile Thr Leu Phe Trp Asp Asp Ile Ser Ile Thr Asp Val
                1205                    1210                    1215

Ala Ser Ile Lys Pro Glu Asn Leu Thr Asp Ser Glu Ile Lys Gln Ile
            1220                    1225                    1230

Tyr Ser Arg Tyr Gly Ile Lys Leu Glu Asp Gly Ile Leu Ile Asp Lys
        1235                    1240                    1245

Lys Gly Gly Ile His Tyr Gly Glu Phe Ile Asn Glu Ala Ser Phe Asn
    1250                    1255                    1260

Ile Glu Pro Leu Gln Asn Tyr Val Thr Lys Tyr Glu Val Thr Tyr Ser
1265                    1270                    1275                    1280

Ser Glu Leu Gly Pro Asn Val Ser Asp Thr Leu Glu Ser Asp Lys Ile
                1285                    1290                    1295

Tyr Lys Asp Gly Thr Ile Lys Phe Asp Phe Thr Lys Tyr Ser Lys Asn
            1300                    1305                    1310

Glu Gln Gly Leu Phe Tyr Asp Ser Gly Leu Asn Trp Asp Phe Lys Ile
        1315                    1320                    1325

Asn Ala Ile Thr Tyr Asp Gly Lys Glu Met Asn Val Phe His Arg Tyr
    1330                1335                1340

Asn Lys
1345

(2) INFORMATION FOR SEQ ID NO:24:

    (i) SEQUENCE CHARACTERISTICS:
        (A) LENGTH: 1399 base pairs
        (B) TYPE: nucleic acid
        (C) STRANDEDNESS: single
        (D) TOPOLOGY: linear

    (ii) MOLECULE TYPE: DNA (genomic)

    (ix) FEATURE:
        (A) NAME/KEY: misc_feature
        (B) LOCATION: 1..1386
        (D) OTHER INFORMATION: /note= "Maize optimized DNA
  sequence for VIP2A(a) protein from AB78"

    (xi) SEQUENCE DESCRIPTION: SEQ ID NO:24:

ATGAAGCGCA TGGAGGGCAA GCTGTTCATG GTGAGCAAGA AGCTCCAGGT GGTGACCAAG      60

ACCGTGCTGC TGAGCACCGT GTTCAGCATC AGCCTGCTGA ACAACGAGGT GATCAAGGCC     120

GAGCAGCTGA ACATCAACAG CCAGAGCAAG TACACCAACC TCCAGAACCT GAAGATCACC     180

GACAAGGTGG AGGACTTCAA GGAGGACAAG GAGAAGGCCA AGGAGTGGGG CAAGGAGAAG     240

GAGAAGGAGT GGAAGCTTAC CGCCACCGAG AAGGGCAAGA TGAACAACTT CCTGGACAAC     300

AAGAACGACA TCAAGACCAA CTACAAGGAG ATCACCTTCA GCATGGCCGG CAGCTTCGAG     360

GACGAGATCA AGGACCTGAA GGAGATCGAC AAGATGTTCG ACAAGACCAA CCTGAGCAAC     420

AGCATCATCA CCTACAAGAA CGTGGAGCCC ACCACCATCG GCTTCAACAA GAGCCTGACC     480

GAGGGCAACA CCATCAACAG CGACGCCATG GCCCAGTTCA AGGAGCAGTT CCTGGACCGC     540

GACATCAAGT TCGACAGCTA CCTGGACACC CACCTGACCG CCCAGCAGGT GAGCAGCAAG     600

GAGCGCGTGA TCCTGAAGGT GACCGTCCCC AGCGGCAAGG GCAGCACCAC CCCCACCAAG     660

GCCGGCGTGA TCCTGAACAA CAGCGAGTAC AAGATGCTGA TCGACAACGG CTACATGGTG     720

CACGTGGACA AGGTGAGCAA GGTGGTGAAG AAGGGCGTGG AGTGCCTCCA GATCGAGGGC     780

ACCCTGAAGA AGAGTCTAGA CTTCAAGAAC GACATCAACG CCGAGGCCCA CAGCTGGGGC     840

ATGAAGAACT ACGAGGAGTG GGCCAAGGAC CTGACCGACA GCCAGCGCGA GGCCCTGGAC 900

GGCTACGCCC GCCAGGACTA CAAGGAGATC AACAACTACC TGCGCAACCA GGGCGGCAGC 960

GGCAACGAGA AGCTGGACGC CCAGATCAAG AACATCAGCG ACGCCCTGGG CAAGAAGCCC 1020

ATCCCCGAGA ACATCACCGT GTACCGCTGG TGCGGCATGC CCGAGTTCGG CTACCAGATC 1080

AGCGACCCCC TGCCCAGCCT GAAGGACTTC GAGGAGCAGT TCCTGAACAC CATCAAGGAG 1140

GACAAGGGCT ACATGAGCAC CAGCCTGAGC AGCGAGCGCC TGGCCGCCTT CGGCAGCCGC 1200

AAGATCATCC TGCGCCTGCA GGTGCCCAAG GGCAGCACCG GCGCCTACCT GAGCGCCATC 1260

GGCGGCTTCG CCAGCGAGAA GGAGATCCTG CTGGACAAGG ACAGCAAGTA CCACATCGAC 1320

AAGGTGACCG AGGTGATCAT CAAGGGCGTG AAGCGCTACG TGGTGGACGC CACCCTGCTG 1380

ACCAACTAGA TCTGAGCTC 1399

(2) INFORMATION FOR SEQ ID NO:25:

(i) SEQUENCE CHARACTERISTICS:
(A) LENGTH: 19 amino acids
(B) TYPE: amino acid
(C) STRANDEDNESS: single
(D) TOPOLOGY: linear

(ii) MOLECULE TYPE: peptide

(ix) FEATURE:
(A) NAME/KEY: Peptide
(B) LOCATION: 1..19
(D) OTHER INFORMATION: /note= "Secretion signal peptide to
secrete VIP2 out of a cell"

(xi) SEQUENCE DESCRIPTION: SEQ ID NO:25:

Gly Trp Ser Trp Ile Phe Leu Phe Leu Leu Ser Gly Ala Ala Gly Val
1               5                   10                  15

His Cys Leu

(2) INFORMATION FOR SEQ ID NO:26:

(i) SEQUENCE CHARACTERISTICS:
(A) LENGTH: 2655 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Synthetic DNA"

(iii) HYPOTHETICAL: NO

(ix) FEATURE:
(A) NAME/KEY: misc_feature
(B) LOCATION: 1..2655
(D) OTHER INFORMATION: /note= "maize optimized DNA
sequence encoding VIP1A(a)"

(xi) SEQUENCE DESCRIPTION: SEQ ID NO:26:

ATGAAGAACA TGAAGAAGAA GCTGGCCAGC GTGGTGACCT GCACCCTGCT GGCCCCCATG 60

TTCCTGAACG GCAACGTGAA CGCCGTGTAC GCCGACAGCA AGACCAACCA GATCAGCACC 120

ACCCAGAAGA ACCAGCAGAA GGAGATGGAC CGCAAGGGCC TGCTGGGCTA CTACTTCAAG 180

GGCAAGGACT TCAGCAACCT GACCATGTTC GCCCCCACGC GTGACAGCAC CCTGATCTAC 240

GACCAGCAGA CCGCCAACAA GCTGCTGGAC AAGAAGCAGC AGGAGTACCA GAGCATCCGC 300

TGGATCGGCC TGATCCAGAG CAAGGAGACC GGCGACTTCA CCTTCAACCT GAGCGAGGAC 360

GAGCAGGCCA TCATCGAGAT CAACGGCAAG ATCATCAGCA ACAAGGGCAA GGAGAAGCAG 420

GTGGTGCACC TGGAGAAGGG CAAGCTGGTG CCCATCAAGA TCGAGTACCA GAGCGACACC 480

AAGTTCAACA TCGACAGCAA GACCTTCAAG GAGCTGAAGC TTTTCAAGAT CGACAGCCAG 540

AACCAGCCCC AGCAGGTGCA GCAGGACGAG CTGCGCAACC CCGAGTTCAA CAAGAAGGAG 600

AGCCAGGAGT TCCTGGCCAA GCCCAGCAAG ATCAACCTGT TCACCCAGCA GATGAAGCGC 660

GAGATCGACG AGGACACCGA CACCGACGGC GACAGCATCC CCGACCTGTG GGAGGAGAAC 720

GGCTACACCA TCCAGAACCG CATCGCCGTG AAGTGGGACG ACAGCCTGGC TAGCAAGGGC 780

TACACCAAGT TCGTGAGCAA CCCCCTGGAG AGCCACACCG TGGGCGACCC CTACACCGAC 840

TACGAGAAGG CCGCCCGCGA CCTGGACCTG AGCAACGCCA AGGAGACCTT CAACCCCCTG 900

GTGGCCGCCT TCCCCAGCGT GAACGTGAGC ATGGAGAAGG TGATCCTGAG CCCCAACGAG 960

AACCTGAGCA ACAGCGTGGA GAGCCACTCG AGCACCAACT GGAGCTACAC CAACACCGAG 1020

GGCGCCAGCG TGGAGGCCGG CATCGGTCCC AAGGGCATCA GCTTCGGCGT GAGCGTGAAC 1080

TACCAGCACA GCGAGACCGT GGCCCAGGAG TGGGGCACCA GCACCGGCAA CACCAGCCAG 1140

TTCAACACCG CCAGCGCCGG CTACCTGAAC GCCAACGTGC GCTACAACAA CGTGGGCACC 1200

GGCGCCATCT ACGACGTGAA GCCCACCACC AGCTTCGTGC TGAACAACGA CACCATCGCC 1260

ACCATCACCG CCAAGTCGAA TTCCACCGCC CTGAACATCA GCCCCGGCGA GAGCTACCCC 1320

AAGAAGGGCC AGAACGGCAT CGCCATCACC AGCATGGACG ACTTCAACAG CCACCCCATC 1380

ACCCTGAACA AGAAGCAGGT GGACAACCTG CTGAACAACA AGCCCATGAT GCTGGAGACC 1440

AACCAGACCG ACGGCGTCTA CAAGATCAAG GACACCCACG GCAACATCGT GACGGGCGGC 1500

GAGTGGAACG GCGTGATCCA GCAGATCAAG GCCAAGACCG CCAGCATCAT CGTCGACGAC 1560

GGCGAGCGCG TGGCCGAGAA GCGCGTGGCC GCCAAGGACT ACGAGAACCC CGAGGACAAG 1620

ACCCCCAGCC TGACCCTGAA GGACGCCCTG AAGCTGAGCT ACCCCGACGA GATCAAGGAG 1680

ATCGAGGGCT TGCTGTACTA CAAGAACAAG CCCATCTACG AGAGCAGCGT GATGACCTAT 1740

CTAGACGAGA ACACCGCCAA GGAGGTGACC AAGCAGCTGA ACGACACCAC CGGCAAGTTC 1800

AAGGACGTGA GCCACCTGTA CGACGTGAAG CTGACCCCCA AGATGAACGT GACCATCAAG 1860

CTGAGCATCC TGTACGACAA CGCCGAGAGC AACGACAACA GCATCGGCAA GTGGACCAAC 1920

ACCAACATCG TGAGCGGCGG CAACAACGGC AAGAAGCAGT ACAGCAGCAA CAACCCCGAC 1980

GCCAACCTGA CCCTGAACAC CGACGCCCAG GAGAAGCTGA ACAAGAACCG CGACTACTAC 2040

ATCAGCCTGT ACATGAAGAG CGAGAAGAAC ACCCAGTGCG AGATCACCAT CGACGGCGAG 2100

ATATACCCCA TCACCACCAA GACCGTGAAC GTGAACAAGG ACAACTACAA GCGCCTGGAC 2160

ATCATCGCCC ACAACATCAA GAGCAACCCC ATCAGCAGCC TGCACATCAA GACCAACGAC 2220

GAGATCACCC TGTTCTGGGA CGACATATCG ATTACCGACG TCGCCAGCAT CAAGCCCGAG 2280

AACCTGACCG ACAGCGAGAT CAAGCAGATA TACAGTCGCT ACGGCATCAA GCTGGAGGAC 2340

GGCATCCTGA TCGACAAGAA AGGCGGCATC CACTACGGCG AGTTCATCAA CGAGGCCAGC 2400

TTCAACATCG AGCCCCTGCA GAACTACGTG ACCAAGTACG AGGTGACCTA CAGCAGCGAG 2460

CTGGGCCCCA ACGTGAGCGA CACCCTGGAG AGCGACAAGA TTTACAAGGA CGGCACCATC 2520

AAGTTCGACT TCACCAAGTA CAGCAAGAAC GAGCAGGGCC TGTTCTACGA CAGCGGCCTG 2580

AACTGGGACT TCAAGATCAA CGCCATCACC TACGACGGCA AGGAGATGAA CGTGTTCCAC 2640

CGCTACAACA AGTAG 2655

(2) INFORMATION FOR SEQ ID NO:27:

(i) SEQUENCE CHARACTERISTICS:
(A) LENGTH: 1389 base pairs
(B) TYPE: nucleic acid
(C) STRANDEDNESS: single

(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: misc_feature
(B) LOCATION: 1..1389
(D) OTHER INFORMATION: /note= "maize optimized DNA
sequence encoding VIP2A(a)"

(xi) SEQUENCE DESCRIPTION: SEQ ID NO:27:

ATGAAGCGCA TGGAGGGCAA GCTGTTCATG GTGAGCAAGA AGCTCCAGGT GGTGACCAAG 60

ACCGTGCTGC TGAGCACCGT GTTCAGCATC AGCCTGCTGA ACAACGAGGT GATCAAGGCC 120

GAGCAGCTGA ACATCAACAG CCAGAGCAAG TACACCAACC TCCAGAACCT GAAGATCACC 180

GACAAGGTGG AGGACTTCAA GGAGGACAAG GAGAAGGCCA AGGAGTGGGG CAAGGAGAAG 240

GAGAAGGAGT GGAAGCTTAC CGCCACCGAG AAGGGCAAGA TGAACAACTT CCTGGACAAC 300

AAGAACGACA TCAAGACCAA CTACAAGGAG ATCACCTTCA GCATAGCCGG CAGCTTCGAG 360

GACGAGATCA AGGACCTGAA GGAGATCGAC AAGATGTTCG ACAAGACCAA CCTGAGCAAC 420

AGCATCATCA CCTACAAGAA CGTGGAGCCC ACCACCATCG GCTTCAACAA GAGCCTGACC 480

GAGGGCAACA CCATCAACAG CGACGCCATG GCCCAGTTCA AGGAGCAGTT CCTGGACCGC 540

GACATCAAGT TCGACAGCTA CCTGGACACC CACCTGACCG CCCAGCAGGT GAGCAGCAAG 600

GAGCGCGTGA TCCTGAAGGT GACCGTCCCC AGCGGCAAGG GCAGCACCAC CCCCACCAAG 660

GCCGGCGTGA TCCTGAACAA CAGCGAGTAC AAGATGCTGA TCGACAACGG CTACATGGTG 720

CACGTGGACA AGGTGAGCAA GGTGGTGAAG AAGGGCGTGG AGTGCCTCCA GATCGAGGGC 780

ACCCTGAAGA AGAGTCTAGA CTTCAAGAAC GACATCAACG CCGAGGCCCA CAGCTGGGGC 840

ATGAAGAACT ACGAGGAGTG GGCCAAGGAC CTGACCGACA GCCAGCGCGA GGCCCTGGAC 900

GGCTACGCCC GCCAGGACTA CAAGGAGATC AACAACTACC TGCGCAACCA GGGCGGCAGC 960

GGCAACGAGA AGCTGGACGC CCAGATCAAG AACATCAGCG ACGCCCTGGG CAAGAAGCCC 1020

ATCCCCGAGA ACATCACCGT GTACCGCTGG TGCGGCATGC CCGAGTTCGG CTACCAGATC 1080

AGCGACCCCC TGCCCAGCCT GAAGGACTTC GAGGAGCAGT TCCTGAACAC CATCAAGGAG 1140

GACAAGGGCT ACATGAGCAC CAGCCTGAGC AGCGAGCGCC TGGCCGCCTT CGGCAGCCGC 1200

AAGATCATCC TGCGCCTGCA GGTGCCCAAG GGCAGCACTG GTGCCTACCT GAGCGCCATC 1260

GGCGGCTTCG CCAGCGAGAA GGAGATCCTG CTGGATAAGG ACAGCAAGTA CCACATCGAC 1320

AAGGTGACCG AGGTGATCAT CAAGGGCGTG AAGCGCTACG TGGTGGACGC CACCCTGCTG 1380

ACCAACTAG 1389

(2) INFORMATION FOR SEQ ID NO:28:

(i) SEQUENCE CHARACTERISTICS:
(A) LENGTH: 2378 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(ix) FEATURE:
(A) NAME/KEY: CDS
(B) LOCATION: 9..2375
(D) OTHER INFORMATION: /note= "Native DNA sequence
encoding VIP3A(a) protein from AB88 as contained in pCIB7104"

(xi) SEQUENCE DESCRIPTION: SEQ ID NO:28:

AGATGAAC ATG AAC AAG AAT AAT ACT AAA TTA AGC ACA AGA GCC TTA CCA 50
Met Asn Lys Asn Asn Thr Lys Leu Ser Thr Arg Ala Leu Pro
1               5                   10

AGT TTT ATT GAT TAT TTT AAT GGC ATT TAT GGA TTT GCC ACT GGT ATC 98
Ser Phe Ile Asp Tyr Phe Asn Gly Ile Tyr Gly Phe Ala Thr Gly Ile
15                  20                  25                  30

AAA GAC ATT ATG AAC ATG ATT TTT AAA ACG GAT ACA GGT GGT GAT CTA 146
Lys Asp Ile Met Asn Met Ile Phe Lys Thr Asp Thr Gly Gly Asp Leu
                35                  40                  45

ACC CTA GAC GAA ATT TTA AAG AAT CAG CAG TTA CTA AAT GAT ATT TCT 194
Thr Leu Asp Glu Ile Leu Lys Asn Gln Gln Leu Leu Asn Asp Ile Ser
            50                  55                  60

GGT AAA TTG GAT GGG GTG AAT GGA AGC TTA AAT GAT CTT ATC GCA CAG 242
Gly Lys Leu Asp Gly Val Asn Gly Ser Leu Asn Asp Leu Ile Ala Gln
        65                  70                  75

GGA AAC TTA AAT ACA GAA TTA TCT AAG GAA ATA TTA AAA ATT GCA AAT 290
Gly Asn Leu Asn Thr Glu Leu Ser Lys Glu Ile Leu Lys Ile Ala Asn
    80                  85                  90

GAA CAA AAT CAA GTT TTA AAT GAT GTT AAT AAC AAA CTC GAT GCG ATA 338
Glu Gln Asn Gln Val Leu Asn Asp Val Asn Asn Lys Leu Asp Ala Ile
95                  100                 105                 110

AAT ACG ATG CTT CGG GTA TAT CTA CCT AAA ATT ACC TCT ATG TTG AGT 386
Asn Thr Met Leu Arg Val Tyr Leu Pro Lys Ile Thr Ser Met Leu Ser
                115                 120                 125

GAT GTA ATG AAA CAA AAT TAT GCG CTA AGT CTG CAA ATA GAA TAC TTA 434
Asp Val Met Lys Gln Asn Tyr Ala Leu Ser Leu Gln Ile Glu Tyr Leu
            130                 135                 140

AGT AAA CAA TTG CAA GAG ATT TCT GAT AAG TTG GAT ATT ATT AAT GTA 482
Ser Lys Gln Leu Gln Glu Ile Ser Asp Lys Leu Asp Ile Ile Asn Val
        145                 150                 155

AAT GTA CTT ATT AAC TCT ACA CTT ACT GAA ATT ACA CCT GCG TAT CAA 530
Asn Val Leu Ile Asn Ser Thr Leu Thr Glu Ile Thr Pro Ala Tyr Gln
    160                 165                 170

AGG ATT AAA TAT GTG AAC GAA AAA TTT GAG GAA TTA ACT TTT GCT ACA 578
Arg Ile Lys Tyr Val Asn Glu Lys Phe Glu Glu Leu Thr Phe Ala Thr
175                 180                 185                 190

GAA ACT AGT TCA AAA GTA AAA AAG GAT GGC TCT CCT GCA GAT ATT CTT 626
Glu Thr Ser Ser Lys Val Lys Lys Asp Gly Ser Pro Ala Asp Ile Leu
                195                 200                 205

GAT GAG TTA ACT GAG TTA ACT GAA CTA GCG AAA AGT GTA ACA AAA AAT 674
Asp Glu Leu Thr Glu Leu Thr Glu Leu Ala Lys Ser Val Thr Lys Asn
            210                 215                 220

GAT GTG GAT GGT TTT GAA TTT TAC CTT AAT ACA TTC CAC GAT GTA ATG 722
Asp Val Asp Gly Phe Glu Phe Tyr Leu Asn Thr Phe His Asp Val Met
        225                 230                 235

GTA GGA AAT AAT TTA TTC GGG CGT TCA GCT TTA AAA ACT GCA TCG GAA 770
Val Gly Asn Asn Leu Phe Gly Arg Ser Ala Leu Lys Thr Ala Ser Glu
    240                 245                 250

TTA ATT ACT AAA GAA AAT GTG AAA ACA AGT GGC AGT GAG GTC GGA AAT 818
Leu Ile Thr Lys Glu Asn Val Lys Thr Ser Gly Ser Glu Val Gly Asn
255                 260                 265                 270

GTT TAT AAC TTC TTA ATT GTA TTA ACA GCT CTG CAA GCC CAA GCT TTT 866
Val Tyr Asn Phe Leu Ile Val Leu Thr Ala Leu Gln Ala Gln Ala Phe
                275                 280                 285

CTT ACT TTA ACA ACA TGC CGA AAA TTA TTA GGC TTA GCA GAT ATT GAT 914
Leu Thr Leu Thr Thr Cys Arg Lys Leu Leu Gly Leu Ala Asp Ile Asp
            290                 295                 300

TAT ACT TCT ATT ATG AAT GAA CAT TTA AAT AAG GAA AAA GAG GAA TTT 962
Tyr Thr Ser Ile Met Asn Glu His Leu Asn Lys Glu Lys Glu Glu Phe

        305                 310                 315

AGA GTA AAC ATC CTC CCT ACA CTT TCT AAT ACT TTT TCT AAT CCT AAT 1010
Arg Val Asn Ile Leu Pro Thr Leu Ser Asn Thr Phe Ser Asn Pro Asn
    320                 325                 330

TAT GCA AAA GTT AAA GGA AGT GAT GAA GAT GCA AAG ATG ATT GTG GAA 1058
Tyr Ala Lys Val Lys Gly Ser Asp Glu Asp Ala Lys Met Ile Val Glu
335                 340                 345                 350

GCT AAA CCA GGA CAT GCA TTG ATT GGG TTT GAA ATT AGT AAT GAT TCA 1106
Ala Lys Pro Gly His Ala Leu Ile Gly Phe Glu Ile Ser Asn Asp Ser
                355                 360                 365

ATT ACA GTA TTA AAA GTA TAT GAG GCT AAG CTA AAA CAA AAT TAT CAA 1154
Ile Thr Val Leu Lys Val Tyr Glu Ala Lys Leu Lys Gln Asn Tyr Gln
            370                 375                 380

GTC GAT AAG GAT TCC TTA TCG GAA GTT ATT TAT GGT GAT ATG GAT AAA 1202
Val Asp Lys Asp Ser Leu Ser Glu Val Ile Tyr Gly Asp Met Asp Lys
        385                 390                 395

TTA TTG TGC CCA GAT CAA TCT GAA CAA ATC TAT TAT ACA AAT AAC ATA 1250
Leu Leu Cys Pro Asp Gln Ser Glu Gln Ile Tyr Tyr Thr Asn Asn Ile
    400                 405                 410

GTA TTT CCA AAT GAA TAT GTA ATT ACT AAA ATT GAT TTC ACT AAA AAA 1298
Val Phe Pro Asn Glu Tyr Val Ile Thr Lys Ile Asp Phe Thr Lys Lys
415                 420                 425                 430

ATG AAA ACT TTA AGA TAT GAG GTA ACA GCG AAT TTT TAT GAT TCT TCT 1346
Met Lys Thr Leu Arg Tyr Glu Val Thr Ala Asn Phe Tyr Asp Ser Ser
                435                 440                 445

ACA GGA GAA ATT GAC TTA AAT AAG AAA AAA GTA GAA TCA AGT GAA GCG 1394
Thr Gly Glu Ile Asp Leu Asn Lys Lys Lys Val Glu Ser Ser Glu Ala
            450                 455                 460

GAG TAT AGA ACG TTA AGT GCT AAT GAT GAT GGG GTG TAT ATG CCG TTA 1442
Glu Tyr Arg Thr Leu Ser Ala Asn Asp Asp Gly Val Tyr Met Pro Leu
        465                 470                 475

GGT GTC ATC AGT GAA ACA TTT TTG ACT CCG ATT AAT GGG TTT GGC CTC 1490
Gly Val Ile Ser Glu Thr Phe Leu Thr Pro Ile Asn Gly Phe Gly Leu
    480                 485                 490

CAA GCT GAT GAA AAT TCA AGA TTA ATT ACT TTA ACA TGT AAA TCA TAT 1538
Gln Ala Asp Glu Asn Ser Arg Leu Ile Thr Leu Thr Cys Lys Ser Tyr
495                 500                 505                 510

TTA AGA GAA CTA CTG CTA GCA ACA GAC TTA AGC AAT AAA GAA ACT AAA 1586
Leu Arg Glu Leu Leu Leu Ala Thr Asp Leu Ser Asn Lys Glu Thr Lys
                515                 520                 525

TTG ATC GTC CCG CCA AGT GGT TTT ATT AGC AAT ATT GTA GAG AAC GGG 1634

Leu Ile Val Pro Pro Ser Gly Phe Ile Ser Asn Ile Val Glu Asn Gly
            530                 535                 540

TCC ATA GAA GAG GAC AAT TTA GAG CCG TGG AAA GCA AAT AAT AAG AAT 1682
Ser Ile Glu Glu Asp Asn Leu Glu Pro Trp Lys Ala Asn Asn Lys Asn
        545                 550                 555

GCG TAT GTA GAT CAT ACA GGC GGA GTG AAT GGA ACT AAA GCT TTA TAT 1730
Ala Tyr Val Asp His Thr Gly Gly Val Asn Gly Thr Lys Ala Leu Tyr
    560                 565                 570

GTT CAT AAG GAC GGA GGA ATT TCA CAA TTT ATT GGA GAT AAG TTA AAA 1778
Val His Lys Asp Gly Gly Ile Ser Gln Phe Ile Gly Asp Lys Leu Lys
575                 580                 585                 590

CCG AAA ACT GAG TAT GTA ATC CAA TAT ACT GTT AAA GGA AAA CCT TCT 1826
Pro Lys Thr Glu Tyr Val Ile Gln Tyr Thr Val Lys Gly Lys Pro Ser
                595                 600                 605

ATT CAT TTA AAA GAT GAA AAT ACT GGA TAT ATT CAT TAT GAA GAT ACA 1874
Ile His Leu Lys Asp Glu Asn Thr Gly Tyr Ile His Tyr Glu Asp Thr
            610                 615                 620

AAT AAT AAT TTA GAA GAT TAT CAA ACT ATT AAT AAA CGT TTT ACT ACA 1922
Asn Asn Asn Leu Glu Asp Tyr Gln Thr Ile Asn Lys Arg Phe Thr Thr
        625                 630                 635

GGA ACT GAT TTA AAG GGA GTG TAT TTA ATT TTA AAA AGT CAA AAT GGA 1970
Gly Thr Asp Leu Lys Gly Val Tyr Leu Ile Leu Lys Ser Gln Asn Gly
    640                 645                 650

GAT GAA GCT TGG GGA GAT AAC TTT ATT ATT TTG GAA ATT AGT CCT TCT 2018
Asp Glu Ala Trp Gly Asp Asn Phe Ile Ile Leu Glu Ile Ser Pro Ser
655                 660                 665                 670

GAA AAG TTA TTA AGT CCA GAA TTA ATT AAT ACA AAT AAT TGG ACG AGT 2066
Glu Lys Leu Leu Ser Pro Glu Leu Ile Asn Thr Asn Asn Trp Thr Ser
                675                 680                 685

ACG GGA TCA ACT AAT ATT AGC GGT AAT ACA CTC ACT CTT TAT CAG GGA 2114
Thr Gly Ser Thr Asn Ile Ser Gly Asn Thr Leu Thr Leu Tyr Gln Gly
            690                 695                 700

GGA CGA GGG ATT CTA AAA CAA AAC CTT CAA TTA GAT AGT TTT TCA ACT 2162
Gly Arg Gly Ile Leu Lys Gln Asn Leu Gln Leu Asp Ser Phe Ser Thr
        705                 710                 715

TAT AGA GTG TAT TTT TCT GTG TCC GGA GAT GCT AAT GTA AGG ATT AGA 2210
Tyr Arg Val Tyr Phe Ser Val Ser Gly Asp Ala Asn Val Arg Ile Arg
    720                 725                 730

AAT TCT AGG GAA GTG TTA TTT GAA AAA AGA TAT ATG AGC GGT GCT AAA 2258
Asn Ser Arg Glu Val Leu Phe Glu Lys Arg Tyr Met Ser Gly Ala Lys
735                 740                 745                 750

GAT GTT TCT GAA ATG TTC ACT ACA AAA TTT GAG AAA GAT AAC TTT TAT 2306
Asp Val Ser Glu Met Phe Thr Thr Lys Phe Glu Lys Asp Asn Phe Tyr
                755                 760                 765

ATA GAG CTT TCT CAA GGG AAT AAT TTA TAT GGT GGT CCT ATT GTA CAT 2354
Ile Glu Leu Ser Gln Gly Asn Asn Leu Tyr Gly Gly Pro Ile Val His
            770                 775                 780

TTT TAC GAT GTC TCT ATT AAG TAA 2378
Phe Tyr Asp Val Ser Ile Lys
        785

(2) INFORMATION FOR SEQ ID NO:29:

(i) SEQUENCE CHARACTERISTICS:
(A) LENGTH: 789 amino acids
(B) TYPE: amino acid
(D) TOPOLOGY: linear

(ii) MOLECULE TYPE: protein

(xi) SEQUENCE DESCRIPTION: SEQ ID NO:29:

Met Asn Lys Asn Asn Thr Lys Leu Ser Thr Arg Ala Leu Pro Ser Phe
1               5                   10                  15

Ile Asp Tyr Phe Asn Gly Ile Tyr Gly Phe Ala Thr Gly Ile Lys Asp
            20                  25                  30

Ile Met Asn Met Ile Phe Lys Thr Asp Thr Gly Gly Asp Leu Thr Leu
        35                  40                  45

Asp Glu Ile Leu Lys Asn Gln Gln Leu Leu Asn Asp Ile Ser Gly Lys
    50                  55                  60

Leu Asp Gly Val Asn Gly Ser Leu Asn Asp Leu Ile Ala Gln Gly Asn
65                  70                  75                  80

Leu Asn Thr Glu Leu Ser Lys Glu Ile Leu Lys Ile Ala Asn Glu Gln
                85                  90                  95

Asn Gln Val Leu Asn Asp Val Asn Asn Lys Leu Asp Ala Ile Asn Thr
            100                 105                 110

Met Leu Arg Val Tyr Leu Pro Lys Ile Thr Ser Met Leu Ser Asp Val
        115                 120                 125

Met Lys Gln Asn Tyr Ala Leu Ser Leu Gln Ile Glu Tyr Leu Ser Lys
    130                 135                 140

Gln Leu Gln Glu Ile Ser Asp Lys Leu Asp Ile Ile Asn Val Asn Val
145                 150                 155                 160

Leu Ile Asn Ser Thr Leu Thr Glu Ile Thr Pro Ala Tyr Gln Arg Ile

                165                 170                 175

Lys Tyr Val Asn Glu Lys Phe Glu Glu Leu Thr Phe Ala Thr Glu Thr
            180                 185                 190

Ser Ser Lys Val Lys Lys Asp Gly Ser Pro Ala Asp Ile Leu Asp Glu
        195                 200                 205

Leu Thr Glu Leu Thr Glu Leu Ala Lys Ser Val Thr Lys Asn Asp Val
    210                 215                 220

Asp Gly Phe Glu Phe Tyr Leu Asn Thr Phe His Asp Val Met Val Gly
225                 230                 235                 240

Asn Asn Leu Phe Gly Arg Ser Ala Leu Lys Thr Ala Ser Glu Leu Ile
                245                 250                 255

Thr Lys Glu Asn Val Lys Thr Ser Gly Ser Glu Val Gly Asn Val Tyr
            260                 265                 270

Asn Phe Leu Ile Val Leu Thr Ala Leu Gln Ala Gln Ala Phe Leu Thr
        275                 280                 285

Leu Thr Thr Cys Arg Lys Leu Leu Gly Leu Ala Asp Ile Asp Tyr Thr
    290                 295                 300

Ser Ile Met Asn Glu His Leu Asn Lys Glu Lys Glu Glu Phe Arg Val
305                 310                 315                 320

Asn Ile Leu Pro Thr Leu Ser Asn Thr Phe Ser Asn Pro Asn Tyr Ala
                325                 330                 335

Lys Val Lys Gly Ser Asp Glu Asp Ala Lys Met Ile Val Glu Ala Lys
            340                 345                 350

Pro Gly His Ala Leu Ile Gly Phe Glu Ile Ser Asn Asp Ser Ile Thr
        355                 360                 365

Val Leu Lys Val Tyr Glu Ala Lys Leu Lys Gln Asn Tyr Gln Val Asp
    370                 375                 380

Lys Asp Ser Leu Ser Glu Val Ile Tyr Gly Asp Met Asp Lys Leu Leu
385                 390                 395                 400

Cys Pro Asp Gln Ser Glu Gln Ile Tyr Tyr Thr Asn Asn Ile Val Phe
                405                 410                 415

Pro Asn Glu Tyr Val Ile Thr Lys Ile Asp Phe Thr Lys Lys Met Lys
            420                 425                 430

Thr Leu Arg Tyr Glu Val Thr Ala Asn Phe Tyr Asp Ser Ser Thr Gly
        435                 440                 445

Glu Ile Asp Leu Asn Lys Lys Lys Val Glu Ser Ser Glu Ala Glu Tyr
    450                 455                 460

Arg Thr Leu Ser Ala Asn Asp Asp Gly Val Tyr Met Pro Leu Gly Val
465                 470                 475                 480

Ile Ser Glu Thr Phe Leu Thr Pro Ile Asn Gly Phe Gly Leu Gln Ala
                485                 490                 495

Asp Glu Asn Ser Arg Leu Ile Thr Leu Thr Cys Lys Ser Tyr Leu Arg
            500                 505                 510

Glu Leu Leu Leu Ala Thr Asp Leu Ser Asn Lys Glu Thr Lys Leu Ile
        515                 520                 525

Val Pro Pro Ser Gly Phe Ile Ser Asn Ile Val Glu Asn Gly Ser Ile
    530                 535                 540

Glu Glu Asp Asn Leu Glu Pro Trp Lys Ala Asn Asn Lys Asn Ala Tyr
545                 550                 555                 560

Val Asp His Thr Gly Gly Val Asn Gly Thr Lys Ala Leu Tyr Val His
                565                 570                 575

Lys Asp Gly Gly Ile Ser Gln Phe Ile Gly Asp Lys Leu Lys Pro Lys
            580                 585                 590

Thr Glu Tyr Val Ile Gln Tyr Thr Val Lys Gly Lys Pro Ser Ile His
        595                 600                 605

Leu Lys Asp Glu Asn Thr Gly Tyr Ile His Tyr Glu Asp Thr Asn Asn
    610                 615                 620

Asn Leu Glu Asp Tyr Gln Thr Ile Asn Lys Arg Phe Thr Thr Gly Thr
625                 630                 635                 640

Asp Leu Lys Gly Val Tyr Leu Ile Leu Lys Ser Gln Asn Gly Asp Glu
                645                 650                 655

Ala Trp Gly Asp Asn Phe Ile Ile Leu Glu Ile Ser Pro Ser Glu Lys
            660                 665                 670

Leu Leu Ser Pro Glu Leu Ile Asn Thr Asn Asn Trp Thr Ser Thr Gly
        675                 680                 685

Ser Thr Asn Ile Ser Gly Asn Thr Leu Thr Leu Tyr Gln Gly Gly Arg
    690                 695                 700

Gly Ile Leu Lys Gln Asn Leu Gln Leu Asp Ser Phe Ser Thr Tyr Arg
705                 710                 715                 720

Val Tyr Phe Ser Val Ser Gly Asp Ala Asn Val Arg Ile Arg Asn Ser
                725                 730                 735

Arg Glu Val Leu Phe Glu Lys Arg Tyr Met Ser Gly Ala Lys Asp Val
            740                 745                 750

Ser Glu Met Phe Thr Thr Lys Phe Glu Lys Asp Asn Phe Tyr Ile Glu
        755                 760                 765

Leu Ser Gln Gly Asn Asn Leu Tyr Gly Gly Pro Ile Val His Phe Tyr
    770                 775                 780

Asp Val Ser Ile Lys
785

(2) INFORMATION FOR SEQ ID NO:30:

(i) SEQUENCE CHARACTERISTICS:
(A) LENGTH: 2403 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: misc_feature
(B) LOCATION: 11..2389
(D) OTHER INFORMATION: /note= "maize optimized DNA
sequence encoding VIP3A(a)"

(xi) SEQUENCE DESCRIPTION: SEQ ID NO:30:

GGATCCACCA ATGAACATGA ACAAGAACAA CACCAAGCTG AGCACCCGCG CCCTGCCGAG 60

CTTCATCGAC TACTTCAACG GCATCTACGG CTTCGCCACC GGCATCAAGG ACATCATGAA 120

CATGATCTTC AAGACCGACA CCGGCGGCGA CCTGACCCTG GACGAGATCC TGAAGAACCA 180

GCAGCTGCTG AACGACATCA GCGGCAAGCT GGACGGCGTG AACGGCAGCC TGAACGACCT 240

GATCGCCCAG GGCAACCTGA ACACCGAGCT GAGCAAGGAG ATCCTTAAGA TCGCCAACGA 300

GCAGAACCAG GTGCTGAACG ACGTGAACAA CAAGCTGGAC GCCATCAACA CCATGCTGCG 360

CGTGTACCTG CCGAAGATCA CCAGCATGCT GAGCGACGTG ATGAAGCAGA ACTACGCCCT 420

GAGCCTGCAG ATCGAGTACC TGAGCAAGCA GCTGCAGGAG ATCAGCGACA AGCTGGACAT 480

CATCAACGTG AACGTCCTGA TCAACAGCAC CCTGACCGAG ATCACCCCGG CCTACCAGCG 540

CATCAAGTAC GTGAACGAGA AGTTCGAAGA GCTGACCTTC GCCACCGAGA CCAGCAGCAA 600

GGTGAAGAAG GACGGCAGCC CGGCCGACAT CCTGGACGAG CTGACCGAGC TGACCGAGCT 660

GGCCAAGAGC GTGACCAAGA ACGACGTGGA CGGCTTCGAG TTCTACCTGA ACACCTTCCA 720

CGACGTGATG GTGGGCAACA ACCTGTTCGG CCGCAGCGCC CTGAAGACCG CCAGCGAGCT 780

GATCACCAAG GAGAACGTGA AGACCAGCGG CAGCGAGGTG GGCAACGTGT ACAACTTCCT 840

GATCGTGCTG ACCGCCCTGC AGGCCCAGGC CTTCCTGACC CTGACCACCT GTCGCAAGCT 900

GCTGGGCCTG GCCGACATCG ACTACACCAG CATCATGAAC GAGCACTTGA ACAAGGAGAA 960

GGAGGAGTTC CGCGTGAACA TCCTGCCGAC CCTGAGCAAC ACCTTCAGCA ACCCGAACTA 1020

CGCCAAGGTG AAGGGCAGCG ACGAGGACGC CAAGATGATC GTGGAGGCTA AGCCGGGCCA 1080

CGCGTTGATC GGCTTCGAGA TCAGCAACGA CAGCATCACC GTGCTGAAGG TGTACGAGGC 1140

CAAGCTGAAG CAGAACTACC AGGTGGACAA GGACAGCTTG AGCGAGGTGA TCTACGGCGA 1200

CATGGACAAG CTGCTGTGTC CGGACCAGAG CGAGCAAATC TACTACACCA ACAACATCGT 1260

GTTCCCGAAC GAGTACGTGA TCACCAAGAT CGACTTCACC AAGAAGATGA AGACCCTGCG 1320

CTACGAGGTG ACCGCCAACT TCTACGACAG CAGCACCGGC GAGATCGACC TGAACAAGAA 1380

GAAGGTGGAG AGCAGCGAGG CCGAGTACCG CACCCTGAGC GCGAACGACG ACGGCGTCTA 1440

CATGCCACTG GGCGTGATCA GCGAGACCTT CCTGACCCCG ATCAACGGCT TTGGCCTGCA 1500

GGCCGACGAG AACAGCCGCC TGATCACCCT GACCTGTAAG AGCTACCTGC GCGAGCTGCT 1560

GCTAGCCACC GACCTGAGCA ACAAGGAGAC CAAGCTGATC GTGCCACCGA GCGGCTTCAT 1620

CAGCAACATC GTGGAGAACG GCAGCATCGA GGAGGACAAC CTGGAGCCGT GGAAGGCCAA 1680

CAACAAGAAC GCCTACGTGG ACCACACCGG CGGCGTGAAC GGCACCAAGG CCCTGTACGT 1740

GCACAAGGAC GGCGGCATCA GCCAGTTCAT CGGCGACAAG CTGAAGCCGA AGACCGAGTA 1800

CGTGATCCAG TACACCGTGA AGGGCAAGCC ATCGATTCAC CTGAAGGACG AGAACACCGG 1860

CTACATCCAC TACGAGGACA CCAACAACAA CCTGGAGGAC TACCAGACCA TCAACAAGCG 1920

CTTCACCACC GGCACCGACC TGAAGGGCGT GTACCTGATC CTGAAGAGCC AGAACGGCGA 1980

CGAGGCCTGG GGCGACAACT TCATCATCCT GGAGATCAGC CCGAGCGAGA AGCTGCTGAG 2040

CCCGGAGCTG ATCAACACCA ACAACTGGAC CAGCACCGGC AGCACCAACA TCAGCGGCAA 2100

CACCCTGACC CTGTACCAGG GCGGCCGCGG CATCCTGAAG CAGAACCTGC AGCTGGACAG 2160

CTTCAGCACC TACCGCGTGT ACTTCAGCGT GAGCGGCGAC GCCAACGTGC GCATCCGCAA 2220

CAGCCGCGAG GTGCTGTTCG AGAAGAGGTA CATGAGCGGC GCCAAGGACG TGAGCGAGAT 2280

GTTCACCACC AAGTTCGAGA AGGACAACTT CTACATCGAG CTGAGCCAGG GCAACAACCT 2340

GTACGGCGGC CCGATCGTGC ACTTCTACGA CGTGAGCATC AAGTTAACGT AGAGCTCAGA 2400

TCT 2403

(2) INFORMATION FOR SEQ ID NO:31:

(i) SEQUENCE CHARACTERISTICS:
(A) LENGTH: 2612 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(ix) FEATURE:
(A) NAME/KEY: CDS
(B) LOCATION: 118..2484
(D) OTHER INFORMATION: /note= "Native DNA sequence
encoding VIP3A(b) from AB424"

(xi) SEQUENCE DESCRIPTION: SEQ ID NO:31:

ATTGAAATTG ATAAAAAGTT ATGAGTGTTT AATAATCAGT AATTACCAAT AAAGAATTAA 60

GAATACAAGT TTACAAGAAA TAAGTGTTAC AAAAAATAGC TGAAAAGGAA GATGAAC 117

ATG AAC AAG AAT AAT ACT AAA TTA AGC ACA AGA GCC TTA CCA AGT TTT 165
Met Asn Lys Asn Asn Thr Lys Leu Ser Thr Arg Ala Leu Pro Ser Phe
790 795 800 805

ATT GAT TAT TTC AAT GGC ATT TAT GGA TTT GCC ACT GGT ATC AAA GAC 213
Ile Asp Tyr Phe Asn Gly Ile Tyr Gly Phe Ala Thr Gly Ile Lys Asp
810 815 820

ATT ATG AAC ATG ATT TTT AAA ACG GAT ACA GGT GGT GAT CTA ACC CTA 261
Ile Met Asn Met Ile Phe Lys Thr Asp Thr Gly Gly Asp Leu Thr Leu
825 830 835

GAC GAA ATT TTA AAG AAT CAG CAG CTA CTA AAT GAT ATT TCT GGT AAA 309
Asp Glu Ile Leu Lys Asn Gln Gln Leu Leu Asn Asp Ile Ser Gly Lys
840 845 850

TTG GAT GGG GTG AAT GGA AGC TTA AAT GAT CTT ATC GCA CAG GGA AAC 357
Leu Asp Gly Val Asn Gly Ser Leu Asn Asp Leu Ile Ala Gln Gly Asn
855 860 865

TTA AAT ACA GAA TTA TCT AAG GAA ATA TTA AAA ATT GCA AAT GAA CAA 405
Leu Asn Thr Glu Leu Ser Lys Glu Ile Leu Lys Ile Ala Asn Glu Gln
870 875 880 885

AAT CAA GTT TTA AAT GAT GTT AAT AAC AAA CTC GAT GCG ATA AAT ACG 453

Asn Gln Val Leu Asn Asp Val Asn Asn Lys Leu Asp Ala Ile Asn Thr
                890                 895                 900

ATG CTT CGG GTA TAT CTA CCT AAA ATT ACC TCT ATG TTG AGT GAT GTA     501
Met Leu Arg Val Tyr Leu Pro Lys Ile Thr Ser Met Leu Ser Asp Val
            905                 910                 915

ATG AAA CAA AAT TAT GCG CTA AGT CTG CAA ATA GAA TAC TTA AGT AAA     549
Met Lys Gln Asn Tyr Ala Leu Ser Leu Gln Ile Glu Tyr Leu Ser Lys
        920                 925                 930

CAA TTG CAA GAG ATT TCT GAT AAG TTG GAT ATT ATT AAT GTA AAT GTA     597
Gln Leu Gln Glu Ile Ser Asp Lys Leu Asp Ile Ile Asn Val Asn Val
    935                 940                 945

CTT ATT AAC TCT ACA CTT ACT GAA ATT ACA CCT GCG TAT CAA AGG ATT     645
Leu Ile Asn Ser Thr Leu Thr Glu Ile Thr Pro Ala Tyr Gln Arg Ile
950                 955                 960                 965

AAA TAT GTG AAC GAA AAA TTT GAG GAA TTA ACT TTT GCT ACA GAA ACT     693
Lys Tyr Val Asn Glu Lys Phe Glu Glu Leu Thr Phe Ala Thr Glu Thr
                970                 975                 980

AGT TCA AAA GTA AAA AAG GAT GGC TCT CCT GCA GAT ATT CGT GAT GAG     741
Ser Ser Lys Val Lys Lys Asp Gly Ser Pro Ala Asp Ile Arg Asp Glu
            985                 990                 995

TTA ACT GAG TTA ACT GAA CTA GCG AAA AGT GTA ACA AAA AAT GAT GTG     789
Leu Thr Glu Leu Thr Glu Leu Ala Lys Ser Val Thr Lys Asn Asp Val
        1000                1005                1010

GAT GGT TTT GAA TTT TAC CTT AAT ACA TTC CAC GAT GTA ATG GTA GGA     837
Asp Gly Phe Glu Phe Tyr Leu Asn Thr Phe His Asp Val Met Val Gly
    1015                1020                1025

AAT AAT TTA TTC GGG CGT TCA GCT TTA AAA ACT GCA TCG GAA TTA ATT     885
Asn Asn Leu Phe Gly Arg Ser Ala Leu Lys Thr Ala Ser Glu Leu Ile
1030                1035                1040                1045

ACT AAA GAA AAT GTG AAA ACA AGT GGC AGT GAG GTC GGA AAT GTT TAT     933
Thr Lys Glu Asn Val Lys Thr Ser Gly Ser Glu Val Gly Asn Val Tyr
                1050                1055                1060

AAC TTC CTA ATT GTA TTA ACA GCT CTG CAA GCA AAA GCT TTT CTT ACT     981
Asn Phe Leu Ile Val Leu Thr Ala Leu Gln Ala Lys Ala Phe Leu Thr
            1065                1070                1075

TTA ACA CCA TGC CGA AAA TTA TTA GGC TTA GCA GAT ATT GAT TAT ACT     1029
Leu Thr Pro Cys Arg Lys Leu Leu Gly Leu Ala Asp Ile Asp Tyr Thr
        1080                1085                1090

TCT ATT ATG AAT GAA CAT TTA AAT AAG GAA AAA GAG GAA TTT AGA GTA     1077
Ser Ile Met Asn Glu His Leu Asn Lys Glu Lys Glu Glu Phe Arg Val
    1095                1100                1105

AAC ATC CTC CCT ACA CTT TCT AAT ACT TTT TCT AAT CCT AAT TAT GCA 1125
Asn Ile Leu Pro Thr Leu Ser Asn Thr Phe Ser Asn Pro Asn Tyr Ala
1110 1115 1120 1125

AAA GTT AAA GGA AGT GAT GAA GAT GCA AAG ATG ATT GTG GAA GCT AAA 1173
Lys Val Lys Gly Ser Asp Glu Asp Ala Lys Met Ile Val Glu Ala Lys
1130 1135 1140

CCA GGA CAT GCA TTG ATT GGG TTT GAA ATT AGT AAT GAT TCA ATT ACA 1221
Pro Gly His Ala Leu Ile Gly Phe Glu Ile Ser Asn Asp Ser Ile Thr
1145 1150 1155

GTA TTA AAA GTA TAT GAG GCT AAG CTA AAA CAA AAT TAT CAA GTC GAT 1269
Val Leu Lys Val Tyr Glu Ala Lys Leu Lys Gln Asn Tyr Gln Val Asp
1160 1165 1170

AAG GAT TCC TTA TCG GAA GTT ATT TAT GGC GAT ATG GAT AAA TTA TTG 1317
Lys Asp Ser Leu Ser Glu Val Ile Tyr Gly Asp Met Asp Lys Leu Leu
1175 1180 1185

TGC CCA GAT CAA TCT GGA CAA ATC TAT TAT ACA AAT AAC ATA GTA TTT 1365
Cys Pro Asp Gln Ser Gly Gln Ile Tyr Tyr Thr Asn Asn Ile Val Phe
1190 1195 1200 1205

CCA AAT GAA TAT GTA ATT ACT AAA ATT GAT TTC ACT AAA AAA ATG AAA 1413
Pro Asn Glu Tyr Val Ile Thr Lys Ile Asp Phe Thr Lys Lys Met Lys
1210 1215 1220

ACT TTA AGA TAT GAG GTA ACA GCG AAT TTT TAT GAT TCT TCT ACA GGA 1461
Thr Leu Arg Tyr Glu Val Thr Ala Asn Phe Tyr Asp Ser Ser Thr Gly
1225 1230 1235

GAA ATT GAC TTA AAT AAG AAA AAA GTA GAA TCA AGT GAA GCG GAG TAT 1509
Glu Ile Asp Leu Asn Lys Lys Lys Val Glu Ser Ser Glu Ala Glu Tyr
1240 1245 1250

AGA ACG TTA AGT GCT AAT GAT GAT GGG GTG TAT ATG CCG TTA GGT GTC 1557
Arg Thr Leu Ser Ala Asn Asp Asp Gly Val Tyr Met Pro Leu Gly Val
1255 1260 1265

ATC AGT GAA ACA TTT TTG ACT CCG ATT AAT GGG TTT GGC CTC CAA GCT 1605
Ile Ser Glu Thr Phe Leu Thr Pro Ile Asn Gly Phe Gly Leu Gln Ala
1270 1275 1280 1285

GAT GAA AAT TCA AGA TTA ATT ACT TTA ACA TGT AAA TCA TAT TTA AGA 1653
Asp Glu Asn Ser Arg Leu Ile Thr Leu Thr Cys Lys Ser Tyr Leu Arg
1290 1295 1300

GAA CTA CTG CTA GCA ACA GAC TTA AGC AAT AAA GAA ACT AAA TTG ATC 1701
Glu Leu Leu Leu Ala Thr Asp Leu Ser Asn Lys Glu Thr Lys Leu Ile
1305 1310 1315

GTC CCG CCA AGT GGT TTT ATT AGC AAT ATT GTA GAG AAC GGG TCC ATA 1749
Val Pro Pro Ser Gly Phe Ile Ser Asn Ile Val Glu Asn Gly Ser Ile
1320 1325 1330

GAA GAG GAC AAT TTA GAG CCG TGG AAA GCA AAT AAT AAG AAT GCG TAT 1797
Glu Glu Asp Asn Leu Glu Pro Trp Lys Ala Asn Asn Lys Asn Ala Tyr
1335 1340 1345

GTA GAT CAT ACA GGC GGA GTG AAT GGA ACT AAA GCT TTA TAT GTT CAT 1845
Val Asp His Thr Gly Gly Val Asn Gly Thr Lys Ala Leu Tyr Val His
1350 1355 1360 1365

AAG GAC GGA GGA ATT TCA CAA TTT ATT GGA GAT AAG TTA AAA CCG AAA 1893
Lys Asp Gly Gly Ile Ser Gln Phe Ile Gly Asp Lys Leu Lys Pro Lys
1370 1375 1380

ACT GAG TAT GTA ATC CAA TAT ACT GTT AAA GGA AAA CCT TCT ATT CAT 1941
Thr Glu Tyr Val Ile Gln Tyr Thr Val Lys Gly Lys Pro Ser Ile His
1385 1390 1395

TTA AAA GAT GAA AAT ACT GGA TAT ATT CAT TAT GAA GAT ACA AAT AAT 1989
Leu Lys Asp Glu Asn Thr Gly Tyr Ile His Tyr Glu Asp Thr Asn Asn
1400 1405 1410

AAT TTA GAA GAT TAT CAA ACT ATT AAT AAA CGT TTT ACT ACA GGA ACT 2037
Asn Leu Glu Asp Tyr Gln Thr Ile Asn Lys Arg Phe Thr Thr Gly Thr
1415 1420 1425

GAT TTA AAG GGA GTG TAT TTA ATT TTA AAA AGT CAA AAT GGA GAT GAA 2085
Asp Leu Lys Gly Val Tyr Leu Ile Leu Lys Ser Gln Asn Gly Asp Glu
1430 1435 1440 1445

GCT TGG GGA GAT AAC TTT ATT ATT TTG GAA ATT AGT CCT TCT GAA AAG 2133
Ala Trp Gly Asp Asn Phe Ile Ile Leu Glu Ile Ser Pro Ser Glu Lys
1450 1455 1460

TTA TTA AGT CCA GAA TTA ATT AAT ACA AAT AAT TGG ACG AGT ACG GGA 2181
Leu Leu Ser Pro Glu Leu Ile Asn Thr Asn Asn Trp Thr Ser Thr Gly
1465 1470 1475

TCA ACT AAT ATT AGC GGT AAT ACA CTC ACT CTT TAT CAG GGA GGA CGA 2229
Ser Thr Asn Ile Ser Gly Asn Thr Leu Thr Leu Tyr Gln Gly Gly Arg
1480 1485 1490

GGG ATT CTA AAA CAA AAC CTT CAA TTA GAT AGT TTT TCA ACT TAT AGA 2277
Gly Ile Leu Lys Gln Asn Leu Gln Leu Asp Ser Phe Ser Thr Tyr Arg
1495 1500 1505

GTG TAT TTC TCT GTG TCC GGA GAT GCT AAT GTA AGG ATT AGA AAT TCT 2325
Val Tyr Phe Ser Val Ser Gly Asp Ala Asn Val Arg Ile Arg Asn Ser
1510 1515 1520 1525

AGG GAA GTG TTA TTT GAA AAA AGA TAT ATG AGC GGT GCT AAA GAT GTT 2373
Arg Glu Val Leu Phe Glu Lys Arg Tyr Met Ser Gly Ala Lys Asp Val
1530 1535 1540

TCT GAA ATG TTC ACT ACA AAA TTT GAG AAA GAT AAC TTC TAT ATA GAG 2421
Ser Glu Met Phe Thr Thr Lys Phe Glu Lys Asp Asn Phe Tyr Ile Glu

1545                1550                1555

CTT TCT CAA GGG AAT AAT TTA TAT GGT GGT CCT ATT GTA CAT TTT TAC      2469
Leu Ser Gln Gly Asn Asn Leu Tyr Gly Gly Pro Ile Val His Phe Tyr
        1560                1565                1570

GAT GTC TCT ATT AAG TAAGATCGGG ATCTAATATT AACAGTTTTT AGAAGCTAAT      2524
Asp Val Ser Ile Lys
    1575

TCTTGTATAA TGTCCTTGAT TATGGAAAAA CACAATTTTG TTTGCTAAGA TGTATATATA    2584

GCTCACTCAT TAAAAGGCAA TCAAGCTT                                       2612

(2) INFORMATION FOR SEQ ID NO:32:

(i) SEQUENCE CHARACTERISTICS:
(A) LENGTH: 789 amino acids
(B) TYPE: amino acid
(D) TOPOLOGY: linear

(ii) MOLECULE TYPE: protein

(xi) SEQUENCE DESCRIPTION: SEQ ID NO:32:

Met Asn Lys Asn Asn Thr Lys Leu Ser Thr Arg Ala Leu Pro Ser Phe
  1               5                  10                  15

Ile Asp Tyr Phe Asn Gly Ile Tyr Gly Phe Ala Thr Gly Ile Lys Asp
             20                  25                  30

Ile Met Asn Met Ile Phe Lys Thr Asp Thr Gly Gly Asp Leu Thr Leu
         35                  40                  45

Asp Glu Ile Leu Lys Asn Gln Gln Leu Leu Asn Asp Ile Ser Gly Lys
     50                  55                  60

Leu Asp Gly Val Asn Gly Ser Leu Asn Asp Leu Ile Ala Gln Gly Asn
 65                  70                  75                  80

Leu Asn Thr Glu Leu Ser Lys Glu Ile Leu Lys Ile Ala Asn Glu Gln
                 85                  90                  95

Asn Gln Val Leu Asn Asp Val Asn Asn Lys Leu Asp Ala Ile Asn Thr
            100                 105                 110

Met Leu Arg Val Tyr Leu Pro Lys Ile Thr Ser Met Leu Ser Asp Val
        115                 120                 125

Met Lys Gln Asn Tyr Ala Leu Ser Leu Gln Ile Glu Tyr Leu Ser Lys
    130                 135                 140

Gln Leu Gln Glu Ile Ser Asp Lys Leu Asp Ile Ile Asn Val Asn Val
145                 150                 155                 160

Leu Ile Asn Ser Thr Leu Thr Glu Ile Thr Pro Ala Tyr Gln Arg Ile
                165                 170                 175

Lys Tyr Val Asn Glu Lys Phe Glu Glu Leu Thr Phe Ala Thr Glu Thr
            180                 185                 190

Ser Ser Lys Val Lys Lys Asp Gly Ser Pro Ala Asp Ile Arg Asp Glu
        195                 200                 205

Leu Thr Glu Leu Thr Glu Leu Ala Lys Ser Val Thr Lys Asn Asp Val
    210                 215                 220

Asp Gly Phe Glu Phe Tyr Leu Asn Thr Phe His Asp Val Met Val Gly
225                 230                 235                 240

Asn Asn Leu Phe Gly Arg Ser Ala Leu Lys Thr Ala Ser Glu Leu Ile
                245                 250                 255

Thr Lys Glu Asn Val Lys Thr Ser Gly Ser Glu Val Gly Asn Val Tyr
            260                 265                 270

Asn Phe Leu Ile Val Leu Thr Ala Leu Gln Ala Lys Ala Phe Leu Thr
        275                 280                 285

Leu Thr Pro Cys Arg Lys Leu Leu Gly Leu Ala Asp Ile Asp Tyr Thr
    290                 295                 300

Ser Ile Met Asn Glu His Leu Asn Lys Glu Lys Glu Glu Phe Arg Val
305                 310                 315                 320

Asn Ile Leu Pro Thr Leu Ser Asn Thr Phe Ser Asn Pro Asn Tyr Ala
                325                 330                 335

Lys Val Lys Gly Ser Asp Glu Asp Ala Lys Met Ile Val Glu Ala Lys
            340                 345                 350

Pro Gly His Ala Leu Ile Gly Phe Glu Ile Ser Asn Asp Ser Ile Thr
        355                 360                 365

Val Leu Lys Val Tyr Glu Ala Lys Leu Lys Gln Asn Tyr Gln Val Asp
    370                 375                 380

Lys Asp Ser Leu Ser Glu Val Ile Tyr Gly Asp Met Asp Lys Leu Leu
385                 390                 395                 400

Cys Pro Asp Gln Ser Gly Gln Ile Tyr Tyr Thr Asn Asn Ile Val Phe
                405                 410                 415

Pro Asn Glu Tyr Val Ile Thr Lys Ile Asp Phe Thr Lys Lys Met Lys
            420                 425                 430

Thr Leu Arg Tyr Glu Val Thr Ala Asn Phe Tyr Asp Ser Ser Thr Gly
        435                 440                 445

Glu Ile Asp Leu Asn Lys Lys Lys Val Glu Ser Ser Glu Ala Glu Tyr
    450                 455                 460

Arg Thr Leu Ser Ala Asn Asp Asp Gly Val Tyr Met Pro Leu Gly Val
465                 470                 475                 480

Ile Ser Glu Thr Phe Leu Thr Pro Ile Asn Gly Phe Gly Leu Gln Ala
                485                 490                 495

Asp Glu Asn Ser Arg Leu Ile Thr Leu Thr Cys Lys Ser Tyr Leu Arg
            500                 505                 510

Glu Leu Leu Leu Ala Thr Asp Leu Ser Asn Lys Glu Thr Lys Leu Ile
        515                 520                 525

Val Pro Pro Ser Gly Phe Ile Ser Asn Ile Val Glu Asn Gly Ser Ile
    530                 535                 540

Glu Glu Asp Asn Leu Glu Pro Trp Lys Ala Asn Asn Lys Asn Ala Tyr
545                 550                 555                 560

Val Asp His Thr Gly Gly Val Asn Gly Thr Lys Ala Leu Tyr Val His
                565                 570                 575

Lys Asp Gly Gly Ile Ser Gln Phe Ile Gly Asp Lys Leu Lys Pro Lys
            580                 585                 590

Thr Glu Tyr Val Ile Gln Tyr Thr Val Lys Gly Lys Pro Ser Ile His
        595                 600                 605

Leu Lys Asp Glu Asn Thr Gly Tyr Ile His Tyr Glu Asp Thr Asn Asn
    610                 615                 620

Asn Leu Glu Asp Tyr Gln Thr Ile Asn Lys Arg Phe Thr Thr Gly Thr
625                 630                 635                 640

Asp Leu Lys Gly Val Tyr Leu Ile Leu Lys Ser Gln Asn Gly Asp Glu
                645                 650                 655

Ala Trp Gly Asp Asn Phe Ile Ile Leu Glu Ile Ser Pro Ser Glu Lys
            660                 665                 670

Leu Leu Ser Pro Glu Leu Ile Asn Thr Asn Asn Trp Thr Ser Thr Gly
        675                 680                 685

Ser Thr Asn Ile Ser Gly Asn Thr Leu Thr Leu Tyr Gln Gly Gly Arg
    690                 695                 700

Gly Ile Leu Lys Gln Asn Leu Gln Leu Asp Ser Phe Ser Thr Tyr Arg
705                 710                 715                 720

Val Tyr Phe Ser Val Ser Gly Asp Ala Asn Val Arg Ile Arg Asn Ser
                725                 730                 735

Arg Glu Val Leu Phe Glu Lys Arg Tyr Met Ser Gly Ala Lys Asp Val

            740                 745                 750

Ser Glu Met Phe Thr Thr Lys Phe Glu Lys Asp Asn Phe Tyr Ile Glu
        755                 760                 765

Leu Ser Gln Gly Asn Asn Leu Tyr Gly Gly Pro Ile Val His Phe Tyr
    770                 775                 780

Asp Val Ser Ile Lys
785

(2) INFORMATION FOR SEQ ID NO:33:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "forward primer used to make
pCIB5526"

(iii) HYPOTHETICAL: NO

(xi) SEQUENCE DESCRIPTION: SEQ ID NO:33:

GGATCCACCA TGAAGACCAA CCAGATCAGC 30

(2) INFORMATION FOR SEQ ID NO:34:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "reverse primer used to make
pCIB5526"

(iii) HYPOTHETICAL: NO

(xi) SEQUENCE DESCRIPTION: SEQ ID NO:34:

AAGCTTCAGC TCCTT 15

(2) INFORMATION FOR SEQ ID NO:35:

(i) SEQUENCE CHARACTERISTICS:
(A) LENGTH: 2576 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: CDS
(B) LOCATION: 9..2564
(D) OTHER INFORMATION: /note= "Maize optimized sequence
encoding VIP1A(a) with the Bacillus secretion signal removed as
contained in pCIB5526"

(xi) SEQUENCE DESCRIPTION: SEQ ID NO:35:

GATCCACC ATG AAG ACC AAC CAG ATC AGC ACC ACC CAG AAG AAC CAG CAG 50
Met Lys Thr Asn Gln Ile Ser Thr Thr Gln Lys Asn Gln Gln
825 830 835

AAG GAG ATG GAC CGC AAG GGC CTG CTG GGC TAC TAC TTC AAG GGC AAG 98
Lys Glu Met Asp Arg Lys Gly Leu Leu Gly Tyr Tyr Phe Lys Gly Lys
840 845 850

GAC TTC AGC AAC CTG ACC ATG TTC GCC CCC ACG CGT GAC AGC ACC CTG 146
Asp Phe Ser Asn Leu Thr Met Phe Ala Pro Thr Arg Asp Ser Thr Leu
855 860 865

ATC TAC GAC CAG CAG ACC GCC AAC AAG CTG CTG GAC AAG AAG CAG CAG 194
Ile Tyr Asp Gln Gln Thr Ala Asn Lys Leu Leu Asp Lys Lys Gln Gln
870 875 880

GAG TAC CAG AGC ATC CGC TGG ATC GGC CTG ATC CAG AGC AAG GAG ACC 242
Glu Tyr Gln Ser Ile Arg Trp Ile Gly Leu Ile Gln Ser Lys Glu Thr
885 890 895

GGC GAC TTC ACC TTC AAC CTG AGC GAG GAC GAG CAG GCC ATC ATC GAG 290
Gly Asp Phe Thr Phe Asn Leu Ser Glu Asp Glu Gln Ala Ile Ile Glu
900 905 910 915

ATC AAC GGC AAG ATC ATC AGC AAC AAG GGC AAG GAG AAG CAG GTG GTG 338
Ile Asn Gly Lys Ile Ile Ser Asn Lys Gly Lys Glu Lys Gln Val Val
920 925 930

CAC CTG GAG AAG GGC AAG CTG GTG CCC ATC AAG ATC GAG TAC CAG AGC 386
His Leu Glu Lys Gly Lys Leu Val Pro Ile Lys Ile Glu Tyr Gln Ser
935 940 945

GAC ACC AAG TTC AAC ATC GAC AGC AAG ACC TTC AAG GAG CTG AAG CTT 434

Asp Thr Lys Phe Asn Ile Asp Ser Lys Thr Phe Lys Glu Leu Lys Leu
950 955 960

TTC AAG ATC GAC AGC CAG AAC CAG CCC CAG CAG GTG CAG CAG GAC GAG 482
Phe Lys Ile Asp Ser Gln Asn Gln Pro Gln Gln Val Gln Gln Asp Glu
965 970 975

CTG CGC AAC CCC GAG TTC AAC AAG AAG GAG AGC CAG GAG TTC CTG GCC 530
Leu Arg Asn Pro Glu Phe Asn Lys Lys Glu Ser Gln Glu Phe Leu Ala
980 985 990 995

AAG CCC AGC AAG ATC AAC CTG TTC ACC CAG CAG ATG AAG CGC GAG ATC 578
Lys Pro Ser Lys Ile Asn Leu Phe Thr Gln Gln Met Lys Arg Glu Ile
1000 1005 1010

GAC GAG GAC ACC GAC ACC GAC GGC GAC AGC ATC CCC GAC CTG TGG GAG 626
Asp Glu Asp Thr Asp Thr Asp Gly Asp Ser Ile Pro Asp Leu Trp Glu
1015 1020 1025

GAG AAC GGC TAC ACC ATC CAG AAC CGC ATC GCC GTG AAG TGG GAC GAC 674
Glu Asn Gly Tyr Thr Ile Gln Asn Arg Ile Ala Val Lys Trp Asp Asp
1030 1035 1040

AGC CTG GCT AGC AAG GGC TAC ACC AAG TTC GTG AGC AAC CCC CTG GAG 722
Ser Leu Ala Ser Lys Gly Tyr Thr Lys Phe Val Ser Asn Pro Leu Glu
1045 1050 1055

AGC CAC ACC GTG GGC GAC CCC TAC ACC GAC TAC GAG AAG GCC GCC CGC 770
Ser His Thr Val Gly Asp Pro Tyr Thr Asp Tyr Glu Lys Ala Ala Arg
1060 1065 1070 1075

GAC CTG GAC CTG AGC AAC GCC AAG GAG ACC TTC AAC CCC CTG GTG GCC 818
Asp Leu Asp Leu Ser Asn Ala Lys Glu Thr Phe Asn Pro Leu Val Ala
1080 1085 1090

GCC TTC CCC AGC GTG AAC GTG AGC ATG GAG AAG GTG ATC CTG AGC CCC 866
Ala Phe Pro Ser Val Asn Val Ser Met Glu Lys Val Ile Leu Ser Pro
1095 1100 1105

AAC GAG AAC CTG AGC AAC AGC GTG GAG AGC CAC TCG AGC ACC AAC TGG 914
Asn Glu Asn Leu Ser Asn Ser Val Glu Ser His Ser Ser Thr Asn Trp
1110 1115 1120

AGC TAC ACC AAC ACC GAG GGC GCC AGC GTG GAG GCC GGC ATC GGT CCC 962
Ser Tyr Thr Asn Thr Glu Gly Ala Ser Val Glu Ala Gly Ile Gly Pro
1125 1130 1135

AAG GGC ATC AGC TTC GGC GTG AGC GTG AAC TAC CAG CAC AGC GAG ACC 1010
Lys Gly Ile Ser Phe Gly Val Ser Val Asn Tyr Gln His Ser Glu Thr
1140 1145 1150 1155

GTG GCC CAG GAG TGG GGC ACC AGC ACC GGC AAC ACC AGC CAG TTC AAC 1058
Val Ala Gln Glu Trp Gly Thr Ser Thr Gly Asn Thr Ser Gln Phe Asn
1160 1165 1170

ACC GCC AGC GCC GGC TAC CTG AAC GCC AAC GTG CGC TAC AAC AAC GTG 1106
Thr Ala Ser Ala Gly Tyr Leu Asn Ala Asn Val Arg Tyr Asn Asn Val
1175 1180 1185

GGC ACC GGC GCC ATC TAC GAC GTG AAG CCC ACC ACC AGC TTC GTG CTG 1154
Gly Thr Gly Ala Ile Tyr Asp Val Lys Pro Thr Thr Ser Phe Val Leu
1190 1195 1200

AAC AAC GAC ACC ATC GCC ACC ATC ACC GCC AAG TCG AAT TCC ACC GCC 1202
Asn Asn Asp Thr Ile Ala Thr Ile Thr Ala Lys Ser Asn Ser Thr Ala
1205 1210 1215

CTG AAC ATC AGC CCC GGC GAG AGC TAC CCC AAG AAG GGC CAG AAC GGC 1250
Leu Asn Ile Ser Pro Gly Glu Ser Tyr Pro Lys Lys Gly Gln Asn Gly
1220 1225 1230 1235

ATC GCC ATC ACC AGC ATG GAC GAC TTC AAC AGC CAC CCC ATC ACC CTG 1298
Ile Ala Ile Thr Ser Met Asp Asp Phe Asn Ser His Pro Ile Thr Leu
1240 1245 1250

AAC AAG AAG CAG GTG GAC AAC CTG CTG AAC AAC AAG CCC ATG ATG CTG 1346
Asn Lys Lys Gln Val Asp Asn Leu Leu Asn Asn Lys Pro Met Met Leu
1255 1260 1265

GAG ACC AAC CAG ACC GAC GGC GTC TAC AAG ATC AAG GAC ACC CAC GGC 1394
Glu Thr Asn Gln Thr Asp Gly Val Tyr Lys Ile Lys Asp Thr His Gly
1270 1275 1280

AAC ATC GTG ACG GGC GGC GAG TGG AAC GGC GTG ATC CAG CAG ATC AAG 1442
Asn Ile Val Thr Gly Gly Glu Trp Asn Gly Val Ile Gln Gln Ile Lys
1285 1290 1295

GCC AAG ACC GCC AGC ATC ATC GTC GAC GAC GGC GAG CGC GTG GCC GAG 1490
Ala Lys Thr Ala Ser Ile Ile Val Asp Asp Gly Glu Arg Val Ala Glu
1300 1305 1310 1315

AAG CGC GTG GCC GCC AAG GAC TAC GAG AAC CCC GAG GAC AAG ACC CCC 1538
Lys Arg Val Ala Ala Lys Asp Tyr Glu Asn Pro Glu Asp Lys Thr Pro
1320 1325 1330

AGC CTG ACC CTG AAG GAC GCC CTG AAG CTG AGC TAC CCC GAC GAG ATC 1586
Ser Leu Thr Leu Lys Asp Ala Leu Lys Leu Ser Tyr Pro Asp Glu Ile
1335 1340 1345

AAG GAG ATC GAG GGC TTG CTG TAC TAC AAG AAC AAG CCC ATC TAC GAG 1634
Lys Glu Ile Glu Gly Leu Leu Tyr Tyr Lys Asn Lys Pro Ile Tyr Glu
1350 1355 1360

AGC AGC GTG ATG ACC TAT CTA GAC GAG AAC ACC GCC AAG GAG GTG ACC 1682
Ser Ser Val Met Thr Tyr Leu Asp Glu Asn Thr Ala Lys Glu Val Thr
1365 1370 1375

AAG CAG CTG AAC GAC ACC ACC GGC AAG TTC AAG GAC GTG AGC CAC CTG 1730
Lys Gln Leu Asn Asp Thr Thr Gly Lys Phe Lys Asp Val Ser His Leu
1380 1385 1390 1395

TAC GAC GTG AAG CTG ACC CCC AAG ATG AAC GTG ACC ATC AAG CTG AGC 1778
Tyr Asp Val Lys Leu Thr Pro Lys Met Asn Val Thr Ile Lys Leu Ser
1400 1405 1410

ATC CTG TAC GAC AAC GCC GAG AGC AAC GAC AAC AGC ATC GGC AAG TGG 1826
Ile Leu Tyr Asp Asn Ala Glu Ser Asn Asp Asn Ser Ile Gly Lys Trp
1415 1420 1425

ACC AAC ACC AAC ATC GTG AGC GGC GGC AAC AAC GGC AAG AAG CAG TAC 1874
Thr Asn Thr Asn Ile Val Ser Gly Gly Asn Asn Gly Lys Lys Gln Tyr
1430 1435 1440

AGC AGC AAC AAC CCC GAC GCC AAC CTG ACC CTG AAC ACC GAC GCC CAG 1922
Ser Ser Asn Asn Pro Asp Ala Asn Leu Thr Leu Asn Thr Asp Ala Gln
1445 1450 1455

GAG AAG CTG AAC AAG AAC CGC GAC TAC TAC ATC AGC CTG TAC ATG AAG 1970
Glu Lys Leu Asn Lys Asn Arg Asp Tyr Tyr Ile Ser Leu Tyr Met Lys
1460 1465 1470 1475

AGC GAG AAG AAC ACC CAG TGC GAG ATC ACC ATC GAC GGC GAG ATA TAC 2018
Ser Glu Lys Asn Thr Gln Cys Glu Ile Thr Ile Asp Gly Glu Ile Tyr
1480 1485 1490

CCC ATC ACC ACC AAG ACC GTG AAC GTG AAC AAG GAC AAC TAC AAG CGC 2066
Pro Ile Thr Thr Lys Thr Val Asn Val Asn Lys Asp Asn Tyr Lys Arg
1495 1500 1505

CTG GAC ATC ATC GCC CAC AAC ATC AAG AGC AAC CCC ATC AGC AGC CTG 2114
Leu Asp Ile Ile Ala His Asn Ile Lys Ser Asn Pro Ile Ser Ser Leu
1510 1515 1520

CAC ATC AAG ACC AAC GAC GAG ATC ACC CTG TTC TGG GAC GAC ATA TCG 2162
His Ile Lys Thr Asn Asp Glu Ile Thr Leu Phe Trp Asp Asp Ile Ser
1525 1530 1535

ATT ACC GAC GTC GCC AGC ATC AAG CCC GAG AAC CTG ACC GAC AGC GAG 2210
Ile Thr Asp Val Ala Ser Ile Lys Pro Glu Asn Leu Thr Asp Ser Glu
1540 1545 1550 1555

ATC AAG CAG ATA TAC AGT CGC TAC GGC ATC AAG CTG GAG GAC GGC ATC 2258
Ile Lys Gln Ile Tyr Ser Arg Tyr Gly Ile Lys Leu Glu Asp Gly Ile
1560 1565 1570

CTG ATC GAC AAG AAA GGC GGC ATC CAC TAC GGC GAG TTC ATC AAC GAG 2306
Leu Ile Asp Lys Lys Gly Gly Ile His Tyr Gly Glu Phe Ile Asn Glu
1575 1580 1585

GCC AGC TTC AAC ATC GAG CCC CTG CAG AAC TAC GTG ACC AAG TAC GAG 2354
Ala Ser Phe Asn Ile Glu Pro Leu Gln Asn Tyr Val Thr Lys Tyr Glu
1590 1595 1600

GTG ACC TAC AGC AGC GAG CTG GGC CCC AAC GTG AGC GAC ACC CTG GAG 2402
Val Thr Tyr Ser Ser Glu Leu Gly Pro Asn Val Ser Asp Thr Leu Glu

1605                    1610                    1615

AGC GAC AAG ATT TAC AAG GAC GGC ACC ATC AAG TTC GAC TTC ACC AAG        2450
Ser Asp Lys Ile Tyr Lys Asp Gly Thr Ile Lys Phe Asp Phe Thr Lys
1620                    1625                    1630                    1635

TAC AGC AAG AAC GAG CAG GGC CTG TTC TAC GAC AGC GGC CTG AAC TGG        2498
Tyr Ser Lys Asn Glu Gln Gly Leu Phe Tyr Asp Ser Gly Leu Asn Trp
                1640                    1645                    1650

GAC TTC AAG ATC AAC GCC ATC ACC TAC GAC GGC AAG GAG ATG AAC GTG        2546
Asp Phe Lys Ile Asn Ala Ile Thr Tyr Asp Gly Lys Glu Met Asn Val
            1655                    1660                    1665

TTC CAC CGC TAC AAC AAG TAGATCTGAG CT                                  2576
Phe His Arg Tyr Asn Lys
        1670

(2) INFORMATION FOR SEQ ID NO:36:

(i) SEQUENCE CHARACTERISTICS:
(A) LENGTH: 852 amino acids
(B) TYPE: amino acid
(D) TOPOLOGY: linear

(ii) MOLECULE TYPE: protein

(xi) SEQUENCE DESCRIPTION: SEQ ID NO:36:

Met Lys Thr Asn Gln Ile Ser Thr Thr Gln Lys Asn Gln Gln Lys Glu
 1               5                  10                  15

Met Asp Arg Lys Gly Leu Leu Gly Tyr Tyr Phe Lys Gly Lys Asp Phe
            20                  25                  30

Ser Asn Leu Thr Met Phe Ala Pro Thr Arg Asp Ser Thr Leu Ile Tyr
        35                  40                  45

Asp Gln Gln Thr Ala Asn Lys Leu Leu Asp Lys Lys Gln Gln Glu Tyr
    50                  55                  60

Gln Ser Ile Arg Trp Ile Gly Leu Ile Gln Ser Lys Glu Thr Gly Asp
 65                  70                  75                  80

Phe Thr Phe Asn Leu Ser Glu Asp Glu Gln Ala Ile Ile Glu Ile Asn
                85                  90                  95

Gly Lys Ile Ile Ser Asn Lys Gly Lys Glu Lys Gln Val Val His Leu
            100                 105                 110

Glu Lys Gly Lys Leu Val Pro Ile Lys Ile Glu Tyr Gln Ser Asp Thr
        115                 120                 125

Lys Phe Asn Ile Asp Ser Lys Thr Phe Lys Glu Leu Lys Leu Phe Lys

    130                 135                 140

Ile Asp Ser Gln Asn Gln Pro Gln Gln Val Gln Gln Asp Glu Leu Arg
145                 150                 155                 160

Asn Pro Glu Phe Asn Lys Lys Glu Ser Gln Glu Phe Leu Ala Lys Pro
                165                 170                 175

Ser Lys Ile Asn Leu Phe Thr Gln Gln Met Lys Arg Glu Ile Asp Glu
            180                 185                 190

Asp Thr Asp Thr Asp Gly Asp Ser Ile Pro Asp Leu Trp Glu Glu Asn
        195                 200                 205

Gly Tyr Thr Ile Gln Asn Arg Ile Ala Val Lys Trp Asp Asp Ser Leu
    210                 215                 220

Ala Ser Lys Gly Tyr Thr Lys Phe Val Ser Asn Pro Leu Glu Ser His
225                 230                 235                 240

Thr Val Gly Asp Pro Tyr Thr Asp Tyr Glu Lys Ala Ala Arg Asp Leu
                245                 250                 255

Asp Leu Ser Asn Ala Lys Glu Thr Phe Asn Pro Leu Val Ala Ala Phe
            260                 265                 270

Pro Ser Val Asn Val Ser Met Glu Lys Val Ile Leu Ser Pro Asn Glu
        275                 280                 285

Asn Leu Ser Asn Ser Val Glu Ser His Ser Ser Thr Asn Trp Ser Tyr
    290                 295                 300

Thr Asn Thr Glu Gly Ala Ser Val Glu Ala Gly Ile Gly Pro Lys Gly
305                 310                 315                 320

Ile Ser Phe Gly Val Ser Val Asn Tyr Gln His Ser Glu Thr Val Ala
                325                 330                 335

Gln Glu Trp Gly Thr Ser Thr Gly Asn Thr Ser Gln Phe Asn Thr Ala
            340                 345                 350

Ser Ala Gly Tyr Leu Asn Ala Asn Val Arg Tyr Asn Asn Val Gly Thr
        355                 360                 365

Gly Ala Ile Tyr Asp Val Lys Pro Thr Thr Ser Phe Val Leu Asn Asn
    370                 375                 380

Asp Thr Ile Ala Thr Ile Thr Ala Lys Ser Asn Ser Thr Ala Leu Asn
385                 390                 395                 400

Ile Ser Pro Gly Glu Ser Tyr Pro Lys Lys Gly Gln Asn Gly Ile Ala
                405                 410                 415

Ile Thr Ser Met Asp Asp Phe Asn Ser His Pro Ile Thr Leu Asn Lys
            420                 425                 430

Lys Gln Val Asp Asn Leu Leu Asn Asn Lys Pro Met Met Leu Glu Thr
        435                 440                 445

Asn Gln Thr Asp Gly Val Tyr Lys Ile Lys Asp Thr His Gly Asn Ile
    450                 455                 460

Val Thr Gly Gly Glu Trp Asn Gly Val Ile Gln Gln Ile Lys Ala Lys
465                 470                 475                 480

Thr Ala Ser Ile Ile Val Asp Asp Gly Glu Arg Val Ala Glu Lys Arg
                485                 490                 495

Val Ala Ala Lys Asp Tyr Glu Asn Pro Glu Asp Lys Thr Pro Ser Leu
            500                 505                 510

Thr Leu Lys Asp Ala Leu Lys Leu Ser Tyr Pro Asp Glu Ile Lys Glu
        515                 520                 525

Ile Glu Gly Leu Leu Tyr Tyr Lys Asn Lys Pro Ile Tyr Glu Ser Ser
    530                 535                 540

Val Met Thr Tyr Leu Asp Glu Asn Thr Ala Lys Glu Val Thr Lys Gln
545                 550                 555                 560

Leu Asn Asp Thr Thr Gly Lys Phe Lys Asp Val Ser His Leu Tyr Asp
                565                 570                 575

Val Lys Leu Thr Pro Lys Met Asn Val Thr Ile Lys Leu Ser Ile Leu
            580                 585                 590

Tyr Asp Asn Ala Glu Ser Asn Asp Asn Ser Ile Gly Lys Trp Thr Asn
        595                 600                 605

Thr Asn Ile Val Ser Gly Gly Asn Asn Gly Lys Lys Gln Tyr Ser Ser
    610                 615                 620

Asn Asn Pro Asp Ala Asn Leu Thr Leu Asn Thr Asp Ala Gln Glu Lys
625                 630                 635                 640

Leu Asn Lys Asn Arg Asp Tyr Tyr Ile Ser Leu Tyr Met Lys Ser Glu
                645                 650                 655

Lys Asn Thr Gln Cys Glu Ile Thr Ile Asp Gly Glu Ile Tyr Pro Ile
            660                 665                 670

Thr Thr Lys Thr Val Asn Val Asn Lys Asp Asn Tyr Lys Arg Leu Asp
        675                 680                 685

Ile Ile Ala His Asn Ile Lys Ser Asn Pro Ile Ser Ser Leu His Ile
    690                 695                 700

Lys Thr Asn Asp Glu Ile Thr Leu Phe Trp Asp Asp Ile Ser Ile Thr
705                 710                 715                 720

Asp Val Ala Ser Ile Lys Pro Glu Asn Leu Thr Asp Ser Glu Ile Lys
                725                 730                 735

Gln Ile Tyr Ser Arg Tyr Gly Ile Lys Leu Glu Asp Gly Ile Leu Ile
            740                 745                 750

Asp Lys Lys Gly Gly Ile His Tyr Gly Glu Phe Ile Asn Glu Ala Ser
        755                 760                 765

Phe Asn Ile Glu Pro Leu Gln Asn Tyr Val Thr Lys Tyr Glu Val Thr
    770                 775                 780

Tyr Ser Ser Glu Leu Gly Pro Asn Val Ser Asp Thr Leu Glu Ser Asp
785                 790                 795                 800

Lys Ile Tyr Lys Asp Gly Thr Ile Lys Phe Asp Phe Thr Lys Tyr Ser
                805                 810                 815

Lys Asn Glu Gln Gly Leu Phe Tyr Asp Ser Gly Leu Asn Trp Asp Phe
            820                 825                 830

Lys Ile Asn Ala Ile Thr Tyr Asp Gly Lys Glu Met Asn Val Phe His
        835                 840                 845

Arg Tyr Asn Lys
    850

(2) INFORMATION FOR SEQ ID NO:37:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "forward primer used to make
pCIB5527"

(iii) HYPOTHETICAL: NO

(xi) SEQUENCE DESCRIPTION: SEQ ID NO:37:

GGATCCACCA TGCTGCAGAA CCTGAAGATC AC                                32

(2) INFORMATION FOR SEQ ID NO:38:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "reverse primer used to make
pCIB5527"

(iii) HYPOTHETICAL: NO

(xi) SEQUENCE DESCRIPTION: SEQ ID NO:38:

AAGCTTCCAC TCCTTCTC 18

(2) INFORMATION FOR SEQ ID NO:39:

(i) SEQUENCE CHARACTERISTICS:
(A) LENGTH: 1241 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: CDS
(B) LOCATION: 9..1238
(D) OTHER INFORMATION: /note= "Maize optimized DNA
sequence encoding VIP2A(a) with the Bacillus secretion signal
removed as contained in pCIB5527"

(xi) SEQUENCE DESCRIPTION: SEQ ID NO:39:

GATCCACC ATG CTG CAG AAC CTG AAG ATC ACC GAC AAG GTG GAG GAC TTC 50
Met Leu Gln Asn Leu Lys Ile Thr Asp Lys Val Glu Asp Phe
855 860 865

AAG GAG GAC AAG GAG AAG GCC AAG GAG TGG GGC AAG GAG AAG GAG AAG 98
Lys Glu Asp Lys Glu Lys Ala Lys Glu Trp Gly Lys Glu Lys Glu Lys
870 875 880

GAG TGG AAG CTT ACC GCC ACC GAG AAG GGC AAG ATG AAC AAC TTC CTG 146
Glu Trp Lys Leu Thr Ala Thr Glu Lys Gly Lys Met Asn Asn Phe Leu
885 890 895

GAC AAC AAG AAC GAC ATC AAG ACC AAC TAC AAG GAG ATC ACC TTC AGC 194
Asp Asn Lys Asn Asp Ile Lys Thr Asn Tyr Lys Glu Ile Thr Phe Ser
900 905 910

ATA GCC GGC AGC TTC GAG GAC GAG ATC AAG GAC CTG AAG GAG ATC GAC 242

Ile Ala Gly Ser Phe Glu Asp Glu Ile Lys Asp Leu Lys Glu Ile Asp
915                 920                 925                 930

AAG ATG TTC GAC AAG ACC AAC CTG AGC AAC AGC ATC ATC ACC TAC AAG     290
Lys Met Phe Asp Lys Thr Asn Leu Ser Asn Ser Ile Ile Thr Tyr Lys
                935                 940                 945

AAC GTG GAG CCC ACC ACC ATC GGC TTC AAC AAG AGC CTG ACC GAG GGC     338
Asn Val Glu Pro Thr Thr Ile Gly Phe Asn Lys Ser Leu Thr Glu Gly
            950                 955                 960

AAC ACC ATC AAC AGC GAC GCC ATG GCC CAG TTC AAG GAG CAG TTC CTG     386
Asn Thr Ile Asn Ser Asp Ala Met Ala Gln Phe Lys Glu Gln Phe Leu
        965                 970                 975

GAC CGC GAC ATC AAG TTC GAC AGC TAC CTG GAC ACC CAC CTG ACC GCC     434
Asp Arg Asp Ile Lys Phe Asp Ser Tyr Leu Asp Thr His Leu Thr Ala
    980                 985                 990

CAG CAG GTG AGC AGC AAG GAG CGC GTG ATC CTG AAG GTG ACC GTC CCC     482
Gln Gln Val Ser Ser Lys Glu Arg Val Ile Leu Lys Val Thr Val Pro
995                 1000                1005                1010

AGC GGC AAG GGC AGC ACC ACC CCC ACC AAG GCC GGC GTG ATC CTG AAC     530
Ser Gly Lys Gly Ser Thr Thr Pro Thr Lys Ala Gly Val Ile Leu Asn
                1015                1020                1025

AAC AGC GAG TAC AAG ATG CTG ATC GAC AAC GGC TAC ATG GTG CAC GTG     578
Asn Ser Glu Tyr Lys Met Leu Ile Asp Asn Gly Tyr Met Val His Val
            1030                1035                1040

GAC AAG GTG AGC AAG GTG GTG AAG AAG GGC GTG GAG TGC CTC CAG ATC     626
Asp Lys Val Ser Lys Val Val Lys Lys Gly Val Glu Cys Leu Gln Ile
        1045                1050                1055

GAG GGC ACC CTG AAG AAG AGT CTA GAC TTC AAG AAC GAC ATC AAC GCC     674
Glu Gly Thr Leu Lys Lys Ser Leu Asp Phe Lys Asn Asp Ile Asn Ala
    1060                1065                1070

GAG GCC CAC AGC TGG GGC ATG AAG AAC TAC GAG GAG TGG GCC AAG GAC     722
Glu Ala His Ser Trp Gly Met Lys Asn Tyr Glu Glu Trp Ala Lys Asp
1075                1080                1085                1090

CTG ACC GAC AGC CAG CGC GAG GCC CTG GAC GGC TAC GCC CGC CAG GAC     770
Leu Thr Asp Ser Gln Arg Glu Ala Leu Asp Gly Tyr Ala Arg Gln Asp
                1095                1100                1105

TAC AAG GAG ATC AAC AAC TAC CTG CGC AAC CAG GGC GGC AGC GGC AAC     818
Tyr Lys Glu Ile Asn Asn Tyr Leu Arg Asn Gln Gly Gly Ser Gly Asn
            1110                1115                1120

GAG AAG CTG GAC GCC CAG ATC AAG AAC ATC AGC GAC GCC CTG GGC AAG     866
Glu Lys Leu Asp Ala Gln Ile Lys Asn Ile Ser Asp Ala Leu Gly Lys
        1125                1130                1135

AAG CCC ATC CCC GAG AAC ATC ACC GTG TAC CGC TGG TGC GGC ATG CCC     914
Lys Pro Ile Pro Glu Asn Ile Thr Val Tyr Arg Trp Cys Gly Met Pro
    1140                1145                1150

GAG TTC GGC TAC CAG ATC AGC GAC CCC CTG CCC AGC CTG AAG GAC TTC     962
Glu Phe Gly Tyr Gln Ile Ser Asp Pro Leu Pro Ser Leu Lys Asp Phe
1155                1160                1165                1170

GAG GAG CAG TTC CTG AAC ACC ATC AAG GAG GAC AAG GGC TAC ATG AGC     1010
Glu Glu Gln Phe Leu Asn Thr Ile Lys Glu Asp Lys Gly Tyr Met Ser
                1175                1180                1185

ACC AGC CTG AGC AGC GAG CGC CTG GCC GCC TTC GGC AGC CGC AAG ATC     1058
Thr Ser Leu Ser Ser Glu Arg Leu Ala Ala Phe Gly Ser Arg Lys Ile
            1190                1195                1200

ATC CTG CGC CTG CAG GTG CCC AAG GGC AGC ACT GGT GCC TAC CTG AGC     1106
Ile Leu Arg Leu Gln Val Pro Lys Gly Ser Thr Gly Ala Tyr Leu Ser
        1205                1210                1215

GCC ATC GGC GGC TTC GCC AGC GAG AAG GAG ATC CTG CTG GAT AAG GAC     1154
Ala Ile Gly Gly Phe Ala Ser Glu Lys Glu Ile Leu Leu Asp Lys Asp
    1220                1225                1230

AGC AAG TAC CAC ATC GAC AAG GTG ACC GAG GTG ATC ATC AAG GGC GTG     1202
Ser Lys Tyr His Ile Asp Lys Val Thr Glu Val Ile Ile Lys Gly Val
1235                1240                1245                1250

AAG CGC TAC GTG GTG GAC GCC ACC CTG CTG ACC AAC TAG                 1241
Lys Arg Tyr Val Val Asp Ala Thr Leu Leu Thr Asn
                1255                1260

(2) INFORMATION FOR SEQ ID NO:40:

(i) SEQUENCE CHARACTERISTICS:
(A) LENGTH: 410 amino acids
(B) TYPE: amino acid
(D) TOPOLOGY: linear

(ii) MOLECULE TYPE: protein

(xi) SEQUENCE DESCRIPTION: SEQ ID NO:40:

Met Leu Gln Asn Leu Lys Ile Thr Asp Lys Val Glu Asp Phe Lys Glu
1               5                   10                  15

Asp Lys Glu Lys Ala Lys Glu Trp Gly Lys Glu Lys Glu Lys Glu Trp
            20                  25                  30

Lys Leu Thr Ala Thr Glu Lys Gly Lys Met Asn Asn Phe Leu Asp Asn
        35                  40                  45

Lys Asn Asp Ile Lys Thr Asn Tyr Lys Glu Ile Thr Phe Ser Ile Ala
    50                  55                  60

Gly Ser Phe Glu Asp Glu Ile Lys Asp Leu Lys Glu Ile Asp Lys Met
65                  70                  75                  80

Phe Asp Lys Thr Asn Leu Ser Asn Ser Ile Ile Thr Tyr Lys Asn Val
                85                  90                  95

Glu Pro Thr Thr Ile Gly Phe Asn Lys Ser Leu Thr Glu Gly Asn Thr
            100                 105                 110

Ile Asn Ser Asp Ala Met Ala Gln Phe Lys Glu Gln Phe Leu Asp Arg
        115                 120                 125

Asp Ile Lys Phe Asp Ser Tyr Leu Asp Thr His Leu Thr Ala Gln Gln
    130                 135                 140

Val Ser Ser Lys Glu Arg Val Ile Leu Lys Val Thr Val Pro Ser Gly
145                 150                 155                 160

Lys Gly Ser Thr Thr Pro Thr Lys Ala Gly Val Ile Leu Asn Asn Ser
                165                 170                 175

Glu Tyr Lys Met Leu Ile Asp Asn Gly Tyr Met Val His Val Asp Lys
            180                 185                 190

Val Ser Lys Val Val Lys Lys Gly Val Glu Cys Leu Gln Ile Glu Gly
        195                 200                 205

Thr Leu Lys Lys Ser Leu Asp Phe Lys Asn Asp Ile Asn Ala Glu Ala
    210                 215                 220

His Ser Trp Gly Met Lys Asn Tyr Glu Glu Trp Ala Lys Asp Leu Thr
225                 230                 235                 240

Asp Ser Gln Arg Glu Ala Leu Asp Gly Tyr Ala Arg Gln Asp Tyr Lys
                245                 250                 255

Glu Ile Asn Asn Tyr Leu Arg Asn Gln Gly Gly Ser Gly Asn Glu Lys
            260                 265                 270

Leu Asp Ala Gln Ile Lys Asn Ile Ser Asp Ala Leu Gly Lys Lys Pro
        275                 280                 285

Ile Pro Glu Asn Ile Thr Val Tyr Arg Trp Cys Gly Met Pro Glu Phe
    290                 295                 300

Gly Tyr Gln Ile Ser Asp Pro Leu Pro Ser Leu Lys Asp Phe Glu Glu
305                 310                 315                 320

Gln Phe Leu Asn Thr Ile Lys Glu Asp Lys Gly Tyr Met Ser Thr Ser
                325                 330                 335

Leu Ser Ser Glu Arg Leu Ala Ala Phe Gly Ser Arg Lys Ile Ile Leu
            340                 345                 350

Arg Leu Gln Val Pro Lys Gly Ser Thr Gly Ala Tyr Leu Ser Ala Ile
        355                 360                 365

Gly Gly Phe Ala Ser Glu Lys Glu Ile Leu Leu Asp Lys Asp Ser Lys
    370                 375                 380

Tyr His Ile Asp Lys Val Thr Glu Val Ile Ile Lys Gly Val Lys Arg
385                 390                 395                 400

Tyr Val Val Asp Ala Thr Leu Leu Thr Asn
                405                 410

(2) INFORMATION FOR SEQ ID NO:41:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide encoding
eukaryotic secretion signal used to construct pCIB5527"

(iii) HYPOTHETICAL: NO

(xi) SEQUENCE DESCRIPTION: SEQ ID NO:41:

GGATCCACCA TGGGCTGGAG CTGGATCTTC CTGTTCCTGC TGAGCGGCGC CGCGGGCGTG 60

CACTGCCTGC AG 72

(2) INFORMATION FOR SEQ ID NO:42:

(i) SEQUENCE CHARACTERISTICS:
(A) LENGTH: 1241 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: CDS
(B) LOCATION: 9..1238
(D) OTHER INFORMATION: /note= "Maize optimized DNA
sequence encoding VIP2A(a) with the Bacillus secretion signal
removed and the eukaryotic secretion signal inserted as

contained in pCIB5528"

(xi) SEQUENCE DESCRIPTION: SEQ ID NO:42:

GATCCACC ATG CTG CAG AAC CTG AAG ATC ACC GAC AAG GTG GAG GAC TTC 50
Met Leu Gln Asn Leu Lys Ile Thr Asp Lys Val Glu Asp Phe
415 420

AAG GAG GAC AAG GAG AAG GCC AAG GAG TGG GGC AAG GAG AAG GAG AAG 98
Lys Glu Asp Lys Glu Lys Ala Lys Glu Trp Gly Lys Glu Lys Glu Lys
425 430 435 440

GAG TGG AAG CTT ACC GCC ACC GAG AAG GGC AAG ATG AAC AAC TTC CTG 146
Glu Trp Lys Leu Thr Ala Thr Glu Lys Gly Lys Met Asn Asn Phe Leu
445 450 455

GAC AAC AAG AAC GAC ATC AAG ACC AAC TAC AAG GAG ATC ACC TTC AGC 194
Asp Asn Lys Asn Asp Ile Lys Thr Asn Tyr Lys Glu Ile Thr Phe Ser
460 465 470

ATA GCC GGC AGC TTC GAG GAC GAG ATC AAG GAC CTG AAG GAG ATC GAC 242
Ile Ala Gly Ser Phe Glu Asp Glu Ile Lys Asp Leu Lys Glu Ile Asp
475 480 485

AAG ATG TTC GAC AAG ACC AAC CTG AGC AAC AGC ATC ATC ACC TAC AAG 290
Lys Met Phe Asp Lys Thr Asn Leu Ser Asn Ser Ile Ile Thr Tyr Lys
490 495 500

AAC GTG GAG CCC ACC ACC ATC GGC TTC AAC AAG AGC CTG ACC GAG GGC 338
Asn Val Glu Pro Thr Thr Ile Gly Phe Asn Lys Ser Leu Thr Glu Gly
505 510 515 520

AAC ACC ATC AAC AGC GAC GCC ATG GCC CAG TTC AAG GAG CAG TTC CTG 386
Asn Thr Ile Asn Ser Asp Ala Met Ala Gln Phe Lys Glu Gln Phe Leu
525 530 535

GAC CGC GAC ATC AAG TTC GAC AGC TAC CTG GAC ACC CAC CTG ACC GCC 434
Asp Arg Asp Ile Lys Phe Asp Ser Tyr Leu Asp Thr His Leu Thr Ala
540 545 550

CAG CAG GTG AGC AGC AAG GAG CGC GTG ATC CTG AAG GTG ACC GTC CCC 482
Gln Gln Val Ser Ser Lys Glu Arg Val Ile Leu Lys Val Thr Val Pro
555 560 565

AGC GGC AAG GGC AGC ACC ACC CCC ACC AAG GCC GGC GTG ATC CTG AAC 530
Ser Gly Lys Gly Ser Thr Thr Pro Thr Lys Ala Gly Val Ile Leu Asn
570 575 580

AAC AGC GAG TAC AAG ATG CTG ATC GAC AAC GGC TAC ATG GTG CAC GTG 578
Asn Ser Glu Tyr Lys Met Leu Ile Asp Asn Gly Tyr Met Val His Val
585 590 595 600

GAC AAG GTG AGC AAG GTG GTG AAG AAG GGC GTG GAG TGC CTC CAG ATC 626
Asp Lys Val Ser Lys Val Val Lys Lys Gly Val Glu Cys Leu Gln Ile

605 610 615

GAG GGC ACC CTG AAG AAG AGT CTA GAC TTC AAG AAC GAC ATC AAC GCC 674
Glu Gly Thr Leu Lys Lys Ser Leu Asp Phe Lys Asn Asp Ile Asn Ala
620 625 630

GAG GCC CAC AGC TGG GGC ATG AAG AAC TAC GAG GAG TGG GCC AAG GAC 722
Glu Ala His Ser Trp Gly Met Lys Asn Tyr Glu Glu Trp Ala Lys Asp
635 640 645

CTG ACC GAC AGC CAG CGC GAG GCC CTG GAC GGC TAC GCC CGC CAG GAC 770
Leu Thr Asp Ser Gln Arg Glu Ala Leu Asp Gly Tyr Ala Arg Gln Asp
650 655 660

TAC AAG GAG ATC AAC AAC TAC CTG CGC AAC CAG GGC GGC AGC GGC AAC 818
Tyr Lys Glu Ile Asn Asn Tyr Leu Arg Asn Gln Gly Gly Ser Gly Asn
665 670 675 680

GAG AAG CTG GAC GCC CAG ATC AAG AAC ATC AGC GAC GCC CTG GGC AAG 866
Glu Lys Leu Asp Ala Gln Ile Lys Asn Ile Ser Asp Ala Leu Gly Lys
685 690 695

AAG CCC ATC CCC GAG AAC ATC ACC GTG TAC CGC TGG TGC GGC ATG CCC 914
Lys Pro Ile Pro Glu Asn Ile Thr Val Tyr Arg Trp Cys Gly Met Pro
700 705 710

GAG TTC GGC TAC CAG ATC AGC GAC CCC CTG CCC AGC CTG AAG GAC TTC 962
Glu Phe Gly Tyr Gln Ile Ser Asp Pro Leu Pro Ser Leu Lys Asp Phe
715 720 725

GAG GAG CAG TTC CTG AAC ACC ATC AAG GAG GAC AAG GGC TAC ATG AGC 1010
Glu Glu Gln Phe Leu Asn Thr Ile Lys Glu Asp Lys Gly Tyr Met Ser
730 735 740

ACC AGC CTG AGC AGC GAG CGC CTG GCC GCC TTC GGC AGC CGC AAG ATC 1058
Thr Ser Leu Ser Ser Glu Arg Leu Ala Ala Phe Gly Ser Arg Lys Ile
745 750 755 760

ATC CTG CGC CTG CAG GTG CCC AAG GGC AGC ACT GGT GCC TAC CTG AGC 1106
Ile Leu Arg Leu Gln Val Pro Lys Gly Ser Thr Gly Ala Tyr Leu Ser
765 770 775

GCC ATC GGC GGC TTC GCC AGC GAG AAG GAG ATC CTG CTG GAT AAG GAC 1154
Ala Ile Gly Gly Phe Ala Ser Glu Lys Glu Ile Leu Leu Asp Lys Asp
780 785 790

AGC AAG TAC CAC ATC GAC AAG GTG ACC GAG GTG ATC ATC AAG GGC GTG 1202
Ser Lys Tyr His Ile Asp Lys Val Thr Glu Val Ile Ile Lys Gly Val
795 800 805

AAG CGC TAC GTG GTG GAC GCC ACC CTG CTG ACC AAC TAG 1241
Lys Arg Tyr Val Val Asp Ala Thr Leu Leu Thr Asn
810 815 820

(2) INFORMATION FOR SEQ ID NO:43:

(i) SEQUENCE CHARACTERISTICS:
(A) LENGTH: 410 amino acids
(B) TYPE: amino acid
(D) TOPOLOGY: linear

(ii) MOLECULE TYPE: protein

(xi) SEQUENCE DESCRIPTION: SEQ ID NO:43:

Met Leu Gln Asn Leu Lys Ile Thr Asp Lys Val Glu Asp Phe Lys Glu
1               5                   10                  15

Asp Lys Glu Lys Ala Lys Glu Trp Gly Lys Glu Lys Glu Lys Glu Trp
            20                  25                  30

Lys Leu Thr Ala Thr Glu Lys Gly Lys Met Asn Asn Phe Leu Asp Asn
        35                  40                  45

Lys Asn Asp Ile Lys Thr Asn Tyr Lys Glu Ile Thr Phe Ser Ile Ala
    50                  55                  60

Gly Ser Phe Glu Asp Glu Ile Lys Asp Leu Lys Glu Ile Asp Lys Met
65                  70                  75                  80

Phe Asp Lys Thr Asn Leu Ser Asn Ser Ile Ile Thr Tyr Lys Asn Val
                85                  90                  95

Glu Pro Thr Thr Ile Gly Phe Asn Lys Ser Leu Thr Glu Gly Asn Thr
            100                 105                 110

Ile Asn Ser Asp Ala Met Ala Gln Phe Lys Glu Gln Phe Leu Asp Arg
        115                 120                 125

Asp Ile Lys Phe Asp Ser Tyr Leu Asp Thr His Leu Thr Ala Gln Gln
    130                 135                 140

Val Ser Ser Lys Glu Arg Val Ile Leu Lys Val Thr Val Pro Ser Gly
145                 150                 155                 160

Lys Gly Ser Thr Thr Pro Thr Lys Ala Gly Val Ile Leu Asn Asn Ser
                165                 170                 175

Glu Tyr Lys Met Leu Ile Asp Asn Gly Tyr Met Val His Val Asp Lys
            180                 185                 190

Val Ser Lys Val Val Lys Lys Gly Val Glu Cys Leu Gln Ile Glu Gly
        195                 200                 205

Thr Leu Lys Lys Ser Leu Asp Phe Lys Asn Asp Ile Asn Ala Glu Ala
    210                 215                 220

His Ser Trp Gly Met Lys Asn Tyr Glu Glu Trp Ala Lys Asp Leu Thr
225                 230                 235                 240

Asp Ser Gln Arg Glu Ala Leu Asp Gly Tyr Ala Arg Gln Asp Tyr Lys
                245                 250                 255

Glu Ile Asn Asn Tyr Leu Arg Asn Gln Gly Gly Ser Gly Asn Glu Lys
            260                 265                 270

Leu Asp Ala Gln Ile Lys Asn Ile Ser Asp Ala Leu Gly Lys Lys Pro
        275                 280                 285

Ile Pro Glu Asn Ile Thr Val Tyr Arg Trp Cys Gly Met Pro Glu Phe
    290                 295                 300

Gly Tyr Gln Ile Ser Asp Pro Leu Pro Ser Leu Lys Asp Phe Glu Glu
305                 310                 315                 320

Gln Phe Leu Asn Thr Ile Lys Glu Asp Lys Gly Tyr Met Ser Thr Ser
                325                 330                 335

Leu Ser Ser Glu Arg Leu Ala Ala Phe Gly Ser Arg Lys Ile Ile Leu
            340                 345                 350

Arg Leu Gln Val Pro Lys Gly Ser Thr Gly Ala Tyr Leu Ser Ala Ile
        355                 360                 365

Gly Gly Phe Ala Ser Glu Lys Glu Ile Leu Leu Asp Lys Asp Ser Lys
    370                 375                 380

Tyr His Ile Asp Lys Val Thr Glu Val Ile Ile Lys Gly Val Lys Arg
385                 390                 395                 400

Tyr Val Val Asp Ala Thr Leu Leu Thr Asn
                405                 410

(2) INFORMATION FOR SEQ ID NO:44:

     (i) SEQUENCE CHARACTERISTICS:
          (A) LENGTH: 86 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION: /desc = "oligonucleotide encoding
  vacuolar targetting peptide used to construct pCIB5533"

   (iii) HYPOTHETICAL: NO

    (xi) SEQUENCE DESCRIPTION: SEQ ID NO:44:

CCGCGGGCGT GCACTGCCTC AGCAGCAGCA GCTTCGCCGA CAGCAACCCC ATCCGCGTGA        60

CCGACCGCGC CGCCAGCACC CTGCAG                                    86

(2) INFORMATION FOR SEQ ID NO:45:

(i) SEQUENCE CHARACTERISTICS:
(A) LENGTH: 1358 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: CDS
(B) LOCATION: 9..1355
(D) OTHER INFORMATION: /note= "Maize optimized VIP2A(a)
with the Bacillus secretion signal removed and the vacuolar
targetting signal inserted as contained in pCIB5533"

(xi) SEQUENCE DESCRIPTION: SEQ ID NO:45:

GATCCACC ATG GGC TGG AGC TGG ATC TTC CTG TTC CTG CTG AGC GGC GCC     50
Met Gly Trp Ser Trp Ile Phe Leu Phe Leu Leu Ser Gly Ala
415                 420

GCG GGC GTG CAC TGC CTC AGC AGC AGC AGC TTC GCC GAC AGC AAC CCC     98
Ala Gly Val His Cys Leu Ser Ser Ser Ser Phe Ala Asp Ser Asn Pro
425                 430                 435                 440

ATC CGC GTG ACC GAC CGC GCC GCC AGC ACC CTG CAG AAC CTG AAG ATC     146
Ile Arg Val Thr Asp Arg Ala Ala Ser Thr Leu Gln Asn Leu Lys Ile
445                 450                 455

ACC GAC AAG GTG GAG GAC TTC AAG GAG GAC AAG GAG AAG GCC AAG GAG     194
Thr Asp Lys Val Glu Asp Phe Lys Glu Asp Lys Glu Lys Ala Lys Glu
460                 465                 470

TGG GGC AAG GAG AAG GAG AAG GAG TGG AAG CTT ACC GCC ACC GAG AAG     242
Trp Gly Lys Glu Lys Glu Lys Glu Trp Lys Leu Thr Ala Thr Glu Lys
475                 480                 485

GGC AAG ATG AAC AAC TTC CTG GAC AAC AAG AAC GAC ATC AAG ACC AAC     290
Gly Lys Met Asn Asn Phe Leu Asp Asn Lys Asn Asp Ile Lys Thr Asn
490                 495                 500

TAC AAG GAG ATC ACC TTC AGC ATA GCC GGC AGC TTC GAG GAC GAG ATC     338
Tyr Lys Glu Ile Thr Phe Ser Ile Ala Gly Ser Phe Glu Asp Glu Ile
505                 510                 515                 520

AAG GAC CTG AAG GAG ATC GAC AAG ATG TTC GAC AAG ACC AAC CTG AGC     386

Lys Asp Leu Lys Glu Ile Asp Lys Met Phe Asp Lys Thr Asn Leu Ser
                525                 530                 535

AAC AGC ATC ATC ACC TAC AAG AAC GTG GAG CCC ACC ACC ATC GGC TTC     434
Asn Ser Ile Ile Thr Tyr Lys Asn Val Glu Pro Thr Thr Ile Gly Phe
            540                 545                 550

AAC AAG AGC CTG ACC GAG GGC AAC ACC ATC AAC AGC GAC GCC ATG GCC     482
Asn Lys Ser Leu Thr Glu Gly Asn Thr Ile Asn Ser Asp Ala Met Ala
        555                 560                 565

CAG TTC AAG GAG CAG TTC CTG GAC CGC GAC ATC AAG TTC GAC AGC TAC     530
Gln Phe Lys Glu Gln Phe Leu Asp Arg Asp Ile Lys Phe Asp Ser Tyr
    570                 575                 580

CTG GAC ACC CAC CTG ACC GCC CAG CAG GTG AGC AGC AAG GAG CGC GTG     578
Leu Asp Thr His Leu Thr Ala Gln Gln Val Ser Ser Lys Glu Arg Val
585                 590                 595                 600

ATC CTG AAG GTG ACC GTC CCC AGC GGC AAG GGC AGC ACC ACC CCC ACC     626
Ile Leu Lys Val Thr Val Pro Ser Gly Lys Gly Ser Thr Thr Pro Thr
                605                 610                 615

AAG GCC GGC GTG ATC CTG AAC AAC AGC GAG TAC AAG ATG CTG ATC GAC     674
Lys Ala Gly Val Ile Leu Asn Asn Ser Glu Tyr Lys Met Leu Ile Asp
            620                 625                 630

AAC GGC TAC ATG GTG CAC GTG GAC AAG GTG AGC AAG GTG GTG AAG AAG     722
Asn Gly Tyr Met Val His Val Asp Lys Val Ser Lys Val Val Lys Lys
        635                 640                 645

GGC GTG GAG TGC CTC CAG ATC GAG GGC ACC CTG AAG AAG AGT CTA GAC     770
Gly Val Glu Cys Leu Gln Ile Glu Gly Thr Leu Lys Lys Ser Leu Asp
    650                 655                 660

TTC AAG AAC GAC ATC AAC GCC GAG GCC CAC AGC TGG GGC ATG AAG AAC     818
Phe Lys Asn Asp Ile Asn Ala Glu Ala His Ser Trp Gly Met Lys Asn
665                 670                 675                 680

TAC GAG GAG TGG GCC AAG GAC CTG ACC GAC AGC CAG CGC GAG GCC CTG     866
Tyr Glu Glu Trp Ala Lys Asp Leu Thr Asp Ser Gln Arg Glu Ala Leu
                685                 690                 695

GAC GGC TAC GCC CGC CAG GAC TAC AAG GAG ATC AAC AAC TAC CTG CGC     914
Asp Gly Tyr Ala Arg Gln Asp Tyr Lys Glu Ile Asn Asn Tyr Leu Arg
            700                 705                 710

AAC CAG GGC GGC AGC GGC AAC GAG AAG CTG GAC GCC CAG ATC AAG AAC     962
Asn Gln Gly Gly Ser Gly Asn Glu Lys Leu Asp Ala Gln Ile Lys Asn
        715                 720                 725

ATC AGC GAC GCC CTG GGC AAG AAG CCC ATC CCC GAG AAC ATC ACC GTG     1010
Ile Ser Asp Ala Leu Gly Lys Lys Pro Ile Pro Glu Asn Ile Thr Val
    730                 735                 740

TAC CGC TGG TGC GGC ATG CCC GAG TTC GGC TAC CAG ATC AGC GAC CCC 1058
Tyr Arg Trp Cys Gly Met Pro Glu Phe Gly Tyr Gln Ile Ser Asp Pro
745 750 755 760

CTG CCC AGC CTG AAG GAC TTC GAG GAG CAG TTC CTG AAC ACC ATC AAG 1106
Leu Pro Ser Leu Lys Asp Phe Glu Glu Gln Phe Leu Asn Thr Ile Lys
765 770 775

GAG GAC AAG GGC TAC ATG AGC ACC AGC CTG AGC AGC GAG CGC CTG GCC 1154
Glu Asp Lys Gly Tyr Met Ser Thr Ser Leu Ser Ser Glu Arg Leu Ala
780 785 790

GCC TTC GGC AGC CGC AAG ATC ATC CTG CGC CTG CAG GTG CCC AAG GGC 1202
Ala Phe Gly Ser Arg Lys Ile Ile Leu Arg Leu Gln Val Pro Lys Gly
795 800 805

AGC ACT GGT GCC TAC CTG AGC GCC ATC GGC GGC TTC GCC AGC GAG AAG 1250
Ser Thr Gly Ala Tyr Leu Ser Ala Ile Gly Gly Phe Ala Ser Glu Lys
810 815 820

GAG ATC CTG CTG GAT AAG GAC AGC AAG TAC CAC ATC GAC AAG GTG ACC 1298
Glu Ile Leu Leu Asp Lys Asp Ser Lys Tyr His Ile Asp Lys Val Thr
825 830 835 840

GAG GTG ATC ATC AAG GGC GTG AAG CGC TAC GTG GTG GAC GCC ACC CTG 1346
Glu Val Ile Ile Lys Gly Val Lys Arg Tyr Val Val Asp Ala Thr Leu
845 850 855

CTG ACC AAC TAG 1358
Leu Thr Asn

(2) INFORMATION FOR SEQ ID NO:46:

(i) SEQUENCE CHARACTERISTICS:
(A) LENGTH: 449 amino acids
(B) TYPE: amino acid
(D) TOPOLOGY: linear

(ii) MOLECULE TYPE: protein

(xi) SEQUENCE DESCRIPTION: SEQ ID NO:46:

Met Gly Trp Ser Trp Ile Phe Leu Phe Leu Leu Ser Gly Ala Ala Gly
1               5                   10                  15

Val His Cys Leu Ser Ser Ser Ser Phe Ala Asp Ser Asn Pro Ile Arg
            20                  25                  30

Val Thr Asp Arg Ala Ala Ser Thr Leu Gln Asn Leu Lys Ile Thr Asp
        35                  40                  45

Lys Val Glu Asp Phe Lys Glu Asp Lys Glu Lys Ala Lys Glu Trp Gly
    50                  55                  60

Lys Glu Lys Glu Lys Glu Trp Lys Leu Thr Ala Thr Glu Lys Gly Lys
65                  70                  75                  80

Met Asn Asn Phe Leu Asp Asn Lys Asn Asp Ile Lys Thr Asn Tyr Lys
                85                  90                  95

Glu Ile Thr Phe Ser Ile Ala Gly Ser Phe Glu Asp Glu Ile Lys Asp
            100                 105                 110

Leu Lys Glu Ile Asp Lys Met Phe Asp Lys Thr Asn Leu Ser Asn Ser
        115                 120                 125

Ile Ile Thr Tyr Lys Asn Val Glu Pro Thr Thr Ile Gly Phe Asn Lys
    130                 135                 140

Ser Leu Thr Glu Gly Asn Thr Ile Asn Ser Asp Ala Met Ala Gln Phe
145                 150                 155                 160

Lys Glu Gln Phe Leu Asp Arg Asp Ile Lys Phe Asp Ser Tyr Leu Asp
                165                 170                 175

Thr His Leu Thr Ala Gln Gln Val Ser Ser Lys Glu Arg Val Ile Leu
            180                 185                 190

Lys Val Thr Val Pro Ser Gly Lys Gly Ser Thr Thr Pro Thr Lys Ala
        195                 200                 205

Gly Val Ile Leu Asn Asn Ser Glu Tyr Lys Met Leu Ile Asp Asn Gly
    210                 215                 220

Tyr Met Val His Val Asp Lys Val Ser Lys Val Val Lys Lys Gly Val
225                 230                 235                 240

Glu Cys Leu Gln Ile Glu Gly Thr Leu Lys Lys Ser Leu Asp Phe Lys
                245                 250                 255

Asn Asp Ile Asn Ala Glu Ala His Ser Trp Gly Met Lys Asn Tyr Glu
            260                 265                 270

Glu Trp Ala Lys Asp Leu Thr Asp Ser Gln Arg Glu Ala Leu Asp Gly
        275                 280                 285

Tyr Ala Arg Gln Asp Tyr Lys Glu Ile Asn Asn Tyr Leu Arg Asn Gln
    290                 295                 300

Gly Gly Ser Gly Asn Glu Lys Leu Asp Ala Gln Ile Lys Asn Ile Ser
305                 310                 315                 320

Asp Ala Leu Gly Lys Lys Pro Ile Pro Glu Asn Ile Thr Val Tyr Arg
                325                 330                 335

Trp Cys Gly Met Pro Glu Phe Gly Tyr Gln Ile Ser Asp Pro Leu Pro
            340                 345                 350

Ser Leu Lys Asp Phe Glu Glu Gln Phe Leu Asn Thr Ile Lys Glu Asp
        355                 360                 365

Lys Gly Tyr Met Ser Thr Ser Leu Ser Ser Glu Arg Leu Ala Ala Phe
    370                 375                 380

Gly Ser Arg Lys Ile Ile Leu Arg Leu Gln Val Pro Lys Gly Ser Thr
385                 390                 395                 400

Gly Ala Tyr Leu Ser Ala Ile Gly Gly Phe Ala Ser Glu Lys Glu Ile
                405                 410                 415

Leu Leu Asp Lys Asp Ser Lys Tyr His Ile Asp Lys Val Thr Glu Val
            420                 425                 430

Ile Ile Lys Gly Val Lys Arg Tyr Val Val Asp Ala Thr Leu Leu Thr
        435                 440                 445

Asn

(2) INFORMATION FOR SEQ ID NO:47:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(ix) FEATURE:
(A) NAME/KEY: Peptide
(B) LOCATION: 1..16
(D) OTHER INFORMATION: /note= "linker peptide for fusion
of VIP1A(a) and VIP2A(a) used to construct pCIB5533"

(xi) SEQUENCE DESCRIPTION: SEQ ID NO:47:

Pro Ser Thr Pro Pro Thr Pro Ser Pro Ser Thr Pro Pro Thr Pro Ser
1               5                   10                  15

(2) INFORMATION FOR SEQ ID NO:48:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "DNA encoding linker peptide
used to construct pCIB5533"

(iii) HYPOTHETICAL: NO

(xi) SEQUENCE DESCRIPTION: SEQ ID NO:48:

CCCGGGCCTT CTACTCCCCC AACTCCCTCT CCTAGCACGC CTCCGACACC TAGCGATATC 60

GGATCC 66

(2) INFORMATION FOR SEQ ID NO:49:

(i) SEQUENCE CHARACTERISTICS:
(A) LENGTH: 4031 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic DNA"

(iii) HYPOTHETICAL: NO

(ix) FEATURE:
(A) NAME/KEY: CDS
(B) LOCATION: 6..4019
(D) OTHER INFORMATION: /note= "Maize optimized DNA
sequence encoding a VIP2A(a) - VIP1A(a) fusion protein as
contained in pCIB5531"

(xi) SEQUENCE DESCRIPTION: SEQ ID NO:49:

GATCC ATG AAG CGC ATG GAG GGC AAG CTG TTC ATG GTG AGC AAG AAG 47
Met Lys Arg Met Glu Gly Lys Leu Phe Met Val Ser Lys Lys
450 455 460

CTC CAG GTG GTG ACC AAG ACC GTG CTG CTG AGC ACC GTG TTC AGC ATC 95
Leu Gln Val Val Thr Lys Thr Val Leu Leu Ser Thr Val Phe Ser Ile
465 470 475

AGC CTG CTG AAC AAC GAG GTG ATC AAG GCC GAG CAG CTG AAC ATC AAC 143
Ser Leu Leu Asn Asn Glu Val Ile Lys Ala Glu Gln Leu Asn Ile Asn
480 485 490 495

AGC CAG AGC AAG TAC ACC AAC CTC CAG AAC CTG AAG ATC ACC GAC AAG 191
Ser Gln Ser Lys Tyr Thr Asn Leu Gln Asn Leu Lys Ile Thr Asp Lys
500 505 510

GTG GAG GAC TTC AAG GAG GAC AAG GAG AAG GCC AAG GAG TGG GGC AAG 239

Val Glu Asp Phe Lys Glu Asp Lys Glu Lys Ala Lys Glu Trp Gly Lys
515 520 525

GAG AAG GAG AAG GAG TGG AAG CTT ACC GCC ACC GAG AAG GGC AAG ATG 287
Glu Lys Glu Lys Glu Trp Lys Leu Thr Ala Thr Glu Lys Gly Lys Met
530 535 540

AAC AAC TTC CTG GAC AAC AAG AAC GAC ATC AAG ACC AAC TAC AAG GAG 335
Asn Asn Phe Leu Asp Asn Lys Asn Asp Ile Lys Thr Asn Tyr Lys Glu
545 550 555

ATC ACC TTC AGC ATA GCC GGC AGC TTC GAG GAC GAG ATC AAG GAC CTG 383
Ile Thr Phe Ser Ile Ala Gly Ser Phe Glu Asp Glu Ile Lys Asp Leu
560 565 570 575

AAG GAG ATC GAC AAG ATG TTC GAC AAG ACC AAC CTG AGC AAC AGC ATC 431
Lys Glu Ile Asp Lys Met Phe Asp Lys Thr Asn Leu Ser Asn Ser Ile
580 585 590

ATC ACC TAC AAG AAC GTG GAG CCC ACC ACC ATC GGC TTC AAC AAG AGC 479
Ile Thr Tyr Lys Asn Val Glu Pro Thr Thr Ile Gly Phe Asn Lys Ser
595 600 605

CTG ACC GAG GGC AAC ACC ATC AAC AGC GAC GCC ATG GCC CAG TTC AAG 527
Leu Thr Glu Gly Asn Thr Ile Asn Ser Asp Ala Met Ala Gln Phe Lys
610 615 620

GAG CAG TTC CTG GAC CGC GAC ATC AAG TTC GAC AGC TAC CTG GAC ACC 575
Glu Gln Phe Leu Asp Arg Asp Ile Lys Phe Asp Ser Tyr Leu Asp Thr
625 630 635

CAC CTG ACC GCC CAG CAG GTG AGC AGC AAG GAG CGC GTG ATC CTG AAG 623
His Leu Thr Ala Gln Gln Val Ser Ser Lys Glu Arg Val Ile Leu Lys
640 645 650 655

GTG ACC GTC CCC AGC GGC AAG GGC AGC ACC ACC CCC ACC AAG GCC GGC 671
Val Thr Val Pro Ser Gly Lys Gly Ser Thr Thr Pro Thr Lys Ala Gly
660 665 670

GTG ATC CTG AAC AAC AGC GAG TAC AAG ATG CTG ATC GAC AAC GGC TAC 719
Val Ile Leu Asn Asn Ser Glu Tyr Lys Met Leu Ile Asp Asn Gly Tyr
675 680 685

ATG GTG CAC GTG GAC AAG GTG AGC AAG GTG GTG AAG AAG GGC GTG GAG 767
Met Val His Val Asp Lys Val Ser Lys Val Val Lys Lys Gly Val Glu
690 695 700

TGC CTC CAG ATC GAG GGC ACC CTG AAG AAG AGT CTA GAC TTC AAG AAC 815
Cys Leu Gln Ile Glu Gly Thr Leu Lys Lys Ser Leu Asp Phe Lys Asn
705 710 715

GAC ATC AAC GCC GAG GCC CAC AGC TGG GGC ATG AAG AAC TAC GAG GAG 863
Asp Ile Asn Ala Glu Ala His Ser Trp Gly Met Lys Asn Tyr Glu Glu
720 725 730 735

TGG GCC AAG GAC CTG ACC GAC AGC CAG CGC GAG GCC CTG GAC GGC TAC          911
Trp Ala Lys Asp Leu Thr Asp Ser Gln Arg Glu Ala Leu Asp Gly Tyr
                740                 745                 750

GCC CGC CAG GAC TAC AAG GAG ATC AAC AAC TAC CTG CGC AAC CAG GGC          959
Ala Arg Gln Asp Tyr Lys Glu Ile Asn Asn Tyr Leu Arg Asn Gln Gly
            755                 760                 765

GGC AGC GGC AAC GAG AAG CTG GAC GCC CAG ATC AAG AAC ATC AGC GAC          1007
Gly Ser Gly Asn Glu Lys Leu Asp Ala Gln Ile Lys Asn Ile Ser Asp
        770                 775                 780

GCC CTG GGC AAG AAG CCC ATC CCC GAG AAC ATC ACC GTG TAC CGC TGG          1055
Ala Leu Gly Lys Lys Pro Ile Pro Glu Asn Ile Thr Val Tyr Arg Trp
    785                 790                 795

TGC GGC ATG CCC GAG TTC GGC TAC CAG ATC AGC GAC CCC CTG CCC AGC          1103
Cys Gly Met Pro Glu Phe Gly Tyr Gln Ile Ser Asp Pro Leu Pro Ser
800                 805                 810                 815

CTG AAG GAC TTC GAG GAG CAG TTC CTG AAC ACC ATC AAG GAG GAC AAG          1151
Leu Lys Asp Phe Glu Glu Gln Phe Leu Asn Thr Ile Lys Glu Asp Lys
                820                 825                 830

GGC TAC ATG AGC ACC AGC CTG AGC AGC GAG CGC CTG GCC GCC TTC GGC          1199
Gly Tyr Met Ser Thr Ser Leu Ser Ser Glu Arg Leu Ala Ala Phe Gly
            835                 840                 845

AGC CGC AAG ATC ATC CTG CGC CTG CAG GTG CCC AAG GGC AGC ACT GGT          1247
Ser Arg Lys Ile Ile Leu Arg Leu Gln Val Pro Lys Gly Ser Thr Gly
        850                 855                 860

GCC TAC CTG AGC GCC ATC GGC GGC TTC GCC AGC GAG AAG GAG ATC CTG          1295
Ala Tyr Leu Ser Ala Ile Gly Gly Phe Ala Ser Glu Lys Glu Ile Leu
    865                 870                 875

CTG GAT AAG GAC AGC AAG TAC CAC ATC GAC AAG GTG ACC GAG GTG ATC          1343
Leu Asp Lys Asp Ser Lys Tyr His Ile Asp Lys Val Thr Glu Val Ile
880                 885                 890                 895

ATC AAG GGC GTG AAG CGC TAC GTG GTG GAC GCC ACC CTG CTG ACC AAC          1391
Ile Lys Gly Val Lys Arg Tyr Val Val Asp Ala Thr Leu Leu Thr Asn
                900                 905                 910

TCC CGG GGG CCT TCT ACT CCC CCA ACT CCC TCT CCT AGC ACG CCT CCG          1439
Ser Arg Gly Pro Ser Thr Pro Pro Thr Pro Ser Pro Ser Thr Pro Pro
            915                 920                 925

ACA CCT AGC GAT ATC GGA TCC ACC ATG AAG ACC AAC CAG ATC AGC ACC          1487
Thr Pro Ser Asp Ile Gly Ser Thr Met Lys Thr Asn Gln Ile Ser Thr
        930                 935                 940

ACC CAG AAG AAC CAG CAG AAG GAG ATG GAC CGC AAG GGC CTG CTG GGC          1535
Thr Gln Lys Asn Gln Gln Lys Glu Met Asp Arg Lys Gly Leu Leu Gly
    945                 950                 955

TAC TAC TTC AAG GGC AAG GAC TTC AGC AAC CTG ACC ATG TTC GCC CCC 1583
Tyr Tyr Phe Lys Gly Lys Asp Phe Ser Asn Leu Thr Met Phe Ala Pro
960 965 970 975

ACG CGT GAC AGC ACC CTG ATC TAC GAC CAG CAG ACC GCC AAC AAG CTG 1631
Thr Arg Asp Ser Thr Leu Ile Tyr Asp Gln Gln Thr Ala Asn Lys Leu
980 985 990

CTG GAC AAG AAG CAG CAG GAG TAC CAG AGC ATC CGC TGG ATC GGC CTG 1679
Leu Asp Lys Lys Gln Gln Glu Tyr Gln Ser Ile Arg Trp Ile Gly Leu
995 1000 1005

ATC CAG AGC AAG GAG ACC GGC GAC TTC ACC TTC AAC CTG AGC GAG GAC 1727
Ile Gln Ser Lys Glu Thr Gly Asp Phe Thr Phe Asn Leu Ser Glu Asp
1010 1015 1020

GAG CAG GCC ATC ATC GAG ATC AAC GGC AAG ATC ATC AGC AAC AAG GGC 1775
Glu Gln Ala Ile Ile Glu Ile Asn Gly Lys Ile Ile Ser Asn Lys Gly
1025 1030 1035

AAG GAG AAG CAG GTG GTG CAC CTG GAG AAG GGC AAG CTG GTG CCC ATC 1823
Lys Glu Lys Gln Val Val His Leu Glu Lys Gly Lys Leu Val Pro Ile
1040 1045 1050 1055

AAG ATC GAG TAC CAG AGC GAC ACC AAG TTC AAC ATC GAC AGC AAG ACC 1871
Lys Ile Glu Tyr Gln Ser Asp Thr Lys Phe Asn Ile Asp Ser Lys Thr
1060 1065 1070

TTC AAG GAG CTG AAG CTT TTC AAG ATC GAC AGC CAG AAC CAG CCC CAG 1919
Phe Lys Glu Leu Lys Leu Phe Lys Ile Asp Ser Gln Asn Gln Pro Gln
1075 1080 1085

CAG GTG CAG CAG GAC GAG CTG CGC AAC CCC GAG TTC AAC AAG AAG GAG 1967
Gln Val Gln Gln Asp Glu Leu Arg Asn Pro Glu Phe Asn Lys Lys Glu
1090 1095 1100

AGC CAG GAG TTC CTG GCC AAG CCC AGC AAG ATC AAC CTG TTC ACC CAG 2015
Ser Gln Glu Phe Leu Ala Lys Pro Ser Lys Ile Asn Leu Phe Thr Gln
1105 1110 1115

CAG ATG AAG CGC GAG ATC GAC GAG GAC ACC GAC ACC GAC GGC GAC AGC 2063
Gln Met Lys Arg Glu Ile Asp Glu Asp Thr Asp Thr Asp Gly Asp Ser
1120 1125 1130 1135

ATC CCC GAC CTG TGG GAG GAG AAC GGC TAC ACC ATC CAG AAC CGC ATC 2111
Ile Pro Asp Leu Trp Glu Glu Asn Gly Tyr Thr Ile Gln Asn Arg Ile
1140 1145 1150

GCC GTG AAG TGG GAC GAC AGC CTG GCT AGC AAG GGC TAC ACC AAG TTC 2159
Ala Val Lys Trp Asp Asp Ser Leu Ala Ser Lys Gly Tyr Thr Lys Phe
1155 1160 1165

GTG AGC AAC CCC CTG GAG AGC CAC ACC GTG GGC GAC CCC TAC ACC GAC 2207
Val Ser Asn Pro Leu Glu Ser His Thr Val Gly Asp Pro Tyr Thr Asp

1170 1175 1180

TAC GAG AAG GCC GCC CGC GAC CTG GAC CTG AGC AAC GCC AAG GAG ACC 2255
Tyr Glu Lys Ala Ala Arg Asp Leu Asp Leu Ser Asn Ala Lys Glu Thr
1185 1190 1195

TTC AAC CCC CTG GTG GCC GCC TTC CCC AGC GTG AAC GTG AGC ATG GAG 2303
Phe Asn Pro Leu Val Ala Ala Phe Pro Ser Val Asn Val Ser Met Glu
1200 1205 1210 1215

AAG GTG ATC CTG AGC CCC AAC GAG AAC CTG AGC AAC AGC GTG GAG AGC 2351
Lys Val Ile Leu Ser Pro Asn Glu Asn Leu Ser Asn Ser Val Glu Ser
1220 1225 1230

CAC TCG AGC ACC AAC TGG AGC TAC ACC AAC ACC GAG GGC GCC AGC GTG 2399
His Ser Ser Thr Asn Trp Ser Tyr Thr Asn Thr Glu Gly Ala Ser Val
1235 1240 1245

GAG GCC GGC ATC GGT CCC AAG GGC ATC AGC TTC GGC GTG AGC GTG AAC 2447
Glu Ala Gly Ile Gly Pro Lys Gly Ile Ser Phe Gly Val Ser Val Asn
1250 1255 1260

TAC CAG CAC AGC GAG ACC GTG GCC CAG GAG TGG GGC ACC AGC ACC GGC 2495
Tyr Gln His Ser Glu Thr Val Ala Gln Glu Trp Gly Thr Ser Thr Gly
1265 1270 1275

AAC ACC AGC CAG TTC AAC ACC GCC AGC GCC GGC TAC CTG AAC GCC AAC 2543
Asn Thr Ser Gln Phe Asn Thr Ala Ser Ala Gly Tyr Leu Asn Ala Asn
1280 1285 1290 1295

GTG CGC TAC AAC AAC GTG GGC ACC GGC GCC ATC TAC GAC GTG AAG CCC 2591
Val Arg Tyr Asn Asn Val Gly Thr Gly Ala Ile Tyr Asp Val Lys Pro
1300 1305 1310

ACC ACC AGC TTC GTG CTG AAC AAC GAC ACC ATC GCC ACC ATC ACC GCC 2639
Thr Thr Ser Phe Val Leu Asn Asn Asp Thr Ile Ala Thr Ile Thr Ala
1315 1320 1325

AAG TCG AAT TCC ACC GCC CTG AAC ATC AGC CCC GGC GAG AGC TAC CCC 2687
Lys Ser Asn Ser Thr Ala Leu Asn Ile Ser Pro Gly Glu Ser Tyr Pro
1330 1335 1340

AAG AAG GGC CAG AAC GGC ATC GCC ATC ACC AGC ATG GAC GAC TTC AAC 2735
Lys Lys Gly Gln Asn Gly Ile Ala Ile Thr Ser Met Asp Asp Phe Asn
1345 1350 1355

AGC CAC CCC ATC ACC CTG AAC AAG AAG CAG GTG GAC AAC CTG CTG AAC 2783
Ser His Pro Ile Thr Leu Asn Lys Lys Gln Val Asp Asn Leu Leu Asn
1360 1365 1370 1375

AAC AAG CCC ATG ATG CTG GAG ACC AAC CAG ACC GAC GGC GTC TAC AAG 2831
Asn Lys Pro Met Met Leu Glu Thr Asn Gln Thr Asp Gly Val Tyr Lys
1380 1385 1390

ATC AAG GAC ACC CAC GGC AAC ATC GTG ACG GGC GGC GAG TGG AAC GGC 2879

Ile Lys Asp Thr His Gly Asn Ile Val Thr Gly Gly Glu Trp Asn Gly
1395 1400 1405

GTG ATC CAG CAG ATC AAG GCC AAG ACC GCC AGC ATC ATC GTC GAC GAC 2927
Val Ile Gln Gln Ile Lys Ala Lys Thr Ala Ser Ile Ile Val Asp Asp
1410 1415 1420

GGC GAG CGC GTG GCC GAG AAG CGC GTG GCC GCC AAG GAC TAC GAG AAC 2975
Gly Glu Arg Val Ala Glu Lys Arg Val Ala Ala Lys Asp Tyr Glu Asn
1425 1430 1435

CCC GAG GAC AAG ACC CCC AGC CTG ACC CTG AAG GAC GCC CTG AAG CTG 3023
Pro Glu Asp Lys Thr Pro Ser Leu Thr Leu Lys Asp Ala Leu Lys Leu
1440 1445 1450 1455

AGC TAC CCC GAC GAG ATC AAG GAG ATC GAG GGC TTG CTG TAC TAC AAG 3071
Ser Tyr Pro Asp Glu Ile Lys Glu Ile Glu Gly Leu Leu Tyr Tyr Lys
1460 1465 1470

AAC AAG CCC ATC TAC GAG AGC AGC GTG ATG ACC TAT CTA GAC GAG AAC 3119
Asn Lys Pro Ile Tyr Glu Ser Ser Val Met Thr Tyr Leu Asp Glu Asn
1475 1480 1485

ACC GCC AAG GAG GTG ACC AAG CAG CTG AAC GAC ACC ACC GGC AAG TTC 3167
Thr Ala Lys Glu Val Thr Lys Gln Leu Asn Asp Thr Thr Gly Lys Phe
1490 1495 1500

AAG GAC GTG AGC CAC CTG TAC GAC GTG AAG CTG ACC CCC AAG ATG AAC 3215
Lys Asp Val Ser His Leu Tyr Asp Val Lys Leu Thr Pro Lys Met Asn
1505 1510 1515

GTG ACC ATC AAG CTG AGC ATC CTG TAC GAC AAC GCC GAG AGC AAC GAC 3263
Val Thr Ile Lys Leu Ser Ile Leu Tyr Asp Asn Ala Glu Ser Asn Asp
1520 1525 1530 1535

AAC AGC ATC GGC AAG TGG ACC AAC ACC AAC ATC GTG AGC GGC GGC AAC 3311
Asn Ser Ile Gly Lys Trp Thr Asn Thr Asn Ile Val Ser Gly Gly Asn
1540 1545 1550

AAC GGC AAG AAG CAG TAC AGC AGC AAC AAC CCC GAC GCC AAC CTG ACC 3359
Asn Gly Lys Lys Gln Tyr Ser Ser Asn Asn Pro Asp Ala Asn Leu Thr
1555 1560 1565

CTG AAC ACC GAC GCC CAG GAG AAG CTG AAC AAG AAC CGC GAC TAC TAC 3407
Leu Asn Thr Asp Ala Gln Glu Lys Leu Asn Lys Asn Arg Asp Tyr Tyr
1570 1575 1580

ATC AGC CTG TAC ATG AAG AGC GAG AAG AAC ACC CAG TGC GAG ATC ACC 3455
Ile Ser Leu Tyr Met Lys Ser Glu Lys Asn Thr Gln Cys Glu Ile Thr
1585 1590 1595

ATC GAC GGC GAG ATA TAC CCC ATC ACC ACC AAG ACC GTG AAC GTG AAC 3503
Ile Asp Gly Glu Ile Tyr Pro Ile Thr Thr Lys Thr Val Asn Val Asn
1600 1605 1610 1615

AAG GAC AAC TAC AAG CGC CTG GAC ATC ATC GCC CAC AAC ATC AAG AGC 3551
Lys Asp Asn Tyr Lys Arg Leu Asp Ile Ile Ala His Asn Ile Lys Ser
1620 1625 1630

AAC CCC ATC AGC AGC CTG CAC ATC AAG ACC AAC GAC GAG ATC ACC CTG 3599
Asn Pro Ile Ser Ser Leu His Ile Lys Thr Asn Asp Glu Ile Thr Leu
1635 1640 1645

TTC TGG GAC GAC ATA TCG ATT ACC GAC GTC GCC AGC ATC AAG CCC GAG 3647
Phe Trp Asp Asp Ile Ser Ile Thr Asp Val Ala Ser Ile Lys Pro Glu
1650 1655 1660

AAC CTG ACC GAC AGC GAG ATC AAG CAG ATA TAC AGT CGC TAC GGC ATC 3695
Asn Leu Thr Asp Ser Glu Ile Lys Gln Ile Tyr Ser Arg Tyr Gly Ile
1665 1670 1675

AAG CTG GAG GAC GGC ATC CTG ATC GAC AAG AAA GGC GGC ATC CAC TAC 3743
Lys Leu Glu Asp Gly Ile Leu Ile Asp Lys Lys Gly Gly Ile His Tyr
1680 1685 1690 1695

GGC GAG TTC ATC AAC GAG GCC AGC TTC AAC ATC GAG CCC CTG CAG AAC 3791
Gly Glu Phe Ile Asn Glu Ala Ser Phe Asn Ile Glu Pro Leu Gln Asn
1700 1705 1710

TAC GTG ACC AAG TAC GAG GTG ACC TAC AGC AGC GAG CTG GGC CCC AAC 3839
Tyr Val Thr Lys Tyr Glu Val Thr Tyr Ser Ser Glu Leu Gly Pro Asn
1715 1720 1725

GTG AGC GAC ACC CTG GAG AGC GAC AAG ATT TAC AAG GAC GGC ACC ATC 3887
Val Ser Asp Thr Leu Glu Ser Asp Lys Ile Tyr Lys Asp Gly Thr Ile
1730 1735 1740

AAG TTC GAC TTC ACC AAG TAC AGC AAG AAC GAG CAG GGC CTG TTC TAC 3935
Lys Phe Asp Phe Thr Lys Tyr Ser Lys Asn Glu Gln Gly Leu Phe Tyr
1745 1750 1755

GAC AGC GGC CTG AAC TGG GAC TTC AAG ATC AAC GCC ATC ACC TAC GAC 3983
Asp Ser Gly Leu Asn Trp Asp Phe Lys Ile Asn Ala Ile Thr Tyr Asp
1760 1765 1770 1775

GGC AAG GAG ATG AAC GTG TTC CAC CGC TAC AAC AAG TAGATCTGAG 4029
Gly Lys Glu Met Asn Val Phe His Arg Tyr Asn Lys
1780 1785

CT 4031

(2) INFORMATION FOR SEQ ID NO:50:

(i) SEQUENCE CHARACTERISTICS:
(A) LENGTH: 1338 amino acids
(B) TYPE: amino acid
(D) TOPOLOGY: linear

(ii) MOLECULE TYPE: protein

(xi) SEQUENCE DESCRIPTION: SEQ ID NO:50:

Met Lys Arg Met Glu Gly Lys Leu Phe Met Val Ser Lys Lys Leu Gln
1               5                   10                  15

Val Val Thr Lys Thr Val Leu Leu Ser Thr Val Phe Ser Ile Ser Leu
            20                  25                  30

Leu Asn Asn Glu Val Ile Lys Ala Glu Gln Leu Asn Ile Asn Ser Gln
        35                  40                  45

Ser Lys Tyr Thr Asn Leu Gln Asn Leu Lys Ile Thr Asp Lys Val Glu
    50                  55                  60

Asp Phe Lys Glu Asp Lys Glu Lys Ala Lys Glu Trp Gly Lys Glu Lys
65                  70                  75                  80

Glu Lys Glu Trp Lys Leu Thr Ala Thr Glu Lys Gly Lys Met Asn Asn
                85                  90                  95

Phe Leu Asp Asn Lys Asn Asp Ile Lys Thr Asn Tyr Lys Glu Ile Thr
            100                 105                 110

Phe Ser Ile Ala Gly Ser Phe Glu Asp Glu Ile Lys Asp Leu Lys Glu
        115                 120                 125

Ile Asp Lys Met Phe Asp Lys Thr Asn Leu Ser Asn Ser Ile Ile Thr
    130                 135                 140

Tyr Lys Asn Val Glu Pro Thr Thr Ile Gly Phe Asn Lys Ser Leu Thr
145                 150                 155                 160

Glu Gly Asn Thr Ile Asn Ser Asp Ala Met Ala Gln Phe Lys Glu Gln
                165                 170                 175

Phe Leu Asp Arg Asp Ile Lys Phe Asp Ser Tyr Leu Asp Thr His Leu
            180                 185                 190

Thr Ala Gln Gln Val Ser Ser Lys Glu Arg Val Ile Leu Lys Val Thr
        195                 200                 205

Val Pro Ser Gly Lys Gly Ser Thr Thr Pro Thr Lys Ala Gly Val Ile
    210                 215                 220

Leu Asn Asn Ser Glu Tyr Lys Met Leu Ile Asp Asn Gly Tyr Met Val
225                 230                 235                 240

His Val Asp Lys Val Ser Lys Val Val Lys Lys Gly Val Glu Cys Leu
                245                 250                 255

Gln Ile Glu Gly Thr Leu Lys Lys Ser Leu Asp Phe Lys Asn Asp Ile
            260                 265                 270

Asn Ala Glu Ala His Ser Trp Gly Met Lys Asn Tyr Glu Glu Trp Ala

275                 280                 285

Lys Asp Leu Thr Asp Ser Gln Arg Glu Ala Leu Asp Gly Tyr Ala Arg
    290                 295                 300

Gln Asp Tyr Lys Glu Ile Asn Asn Tyr Leu Arg Asn Gln Gly Gly Ser
305                 310                 315                 320

Gly Asn Glu Lys Leu Asp Ala Gln Ile Lys Asn Ile Ser Asp Ala Leu
                325                 330                 335

Gly Lys Lys Pro Ile Pro Glu Asn Ile Thr Val Tyr Arg Trp Cys Gly
            340                 345                 350

Met Pro Glu Phe Gly Tyr Gln Ile Ser Asp Pro Leu Pro Ser Leu Lys
        355                 360                 365

Asp Phe Glu Glu Gln Phe Leu Asn Thr Ile Lys Glu Asp Lys Gly Tyr
    370                 375                 380

Met Ser Thr Ser Leu Ser Ser Glu Arg Leu Ala Ala Phe Gly Ser Arg
385                 390                 395                 400

Lys Ile Ile Leu Arg Leu Gln Val Pro Lys Gly Ser Thr Gly Ala Tyr
                405                 410                 415

Leu Ser Ala Ile Gly Gly Phe Ala Ser Glu Lys Glu Ile Leu Leu Asp
            420                 425                 430

Lys Asp Ser Lys Tyr His Ile Asp Lys Val Thr Glu Val Ile Ile Lys
        435                 440                 445

Gly Val Lys Arg Tyr Val Val Asp Ala Thr Leu Leu Thr Asn Ser Arg
    450                 455                 460

Gly Pro Ser Thr Pro Pro Thr Pro Ser Pro Ser Thr Pro Pro Thr Pro
465                 470                 475                 480

Ser Asp Ile Gly Ser Thr Met Lys Thr Asn Gln Ile Ser Thr Thr Gln
                485                 490                 495

Lys Asn Gln Gln Lys Glu Met Asp Arg Lys Gly Leu Leu Gly Tyr Tyr
            500                 505                 510

Phe Lys Gly Lys Asp Phe Ser Asn Leu Thr Met Phe Ala Pro Thr Arg
        515                 520                 525

Asp Ser Thr Leu Ile Tyr Asp Gln Gln Thr Ala Asn Lys Leu Leu Asp
    530                 535                 540

Lys Lys Gln Gln Glu Tyr Gln Ser Ile Arg Trp Ile Gly Leu Ile Gln
545                 550                 555                 560

Ser Lys Glu Thr Gly Asp Phe Thr Phe Asn Leu Ser Glu Asp Glu Gln
                565                 570                 575

Ala Ile Ile Glu Ile Asn Gly Lys Ile Ile Ser Asn Lys Gly Lys Glu
            580                 585                 590

Lys Gln Val Val His Leu Glu Lys Gly Lys Leu Val Pro Ile Lys Ile
        595                 600                 605

Glu Tyr Gln Ser Asp Thr Lys Phe Asn Ile Asp Ser Lys Thr Phe Lys
    610                 615                 620

Glu Leu Lys Leu Phe Lys Ile Asp Ser Gln Asn Gln Pro Gln Gln Val
625                 630                 635                 640

Gln Gln Asp Glu Leu Arg Asn Pro Glu Phe Asn Lys Lys Glu Ser Gln
                645                 650                 655

Glu Phe Leu Ala Lys Pro Ser Lys Ile Asn Leu Phe Thr Gln Gln Met
            660                 665                 670

Lys Arg Glu Ile Asp Glu Asp Thr Asp Thr Asp Gly Asp Ser Ile Pro
        675                 680                 685

Asp Leu Trp Glu Glu Asn Gly Tyr Thr Ile Gln Asn Arg Ile Ala Val
    690                 695                 700

Lys Trp Asp Asp Ser Leu Ala Ser Lys Gly Tyr Thr Lys Phe Val Ser
705                 710                 715                 720

Asn Pro Leu Glu Ser His Thr Val Gly Asp Pro Tyr Thr Asp Tyr Glu
                725                 730                 735

Lys Ala Ala Arg Asp Leu Asp Leu Ser Asn Ala Lys Glu Thr Phe Asn
            740                 745                 750

Pro Leu Val Ala Ala Phe Pro Ser Val Asn Val Ser Met Glu Lys Val
        755                 760                 765

Ile Leu Ser Pro Asn Glu Asn Leu Ser Asn Ser Val Glu Ser His Ser
    770                 775                 780

Ser Thr Asn Trp Ser Tyr Thr Asn Thr Glu Gly Ala Ser Val Glu Ala
785                 790                 795                 800

Gly Ile Gly Pro Lys Gly Ile Ser Phe Gly Val Ser Val Asn Tyr Gln
                805                 810                 815

His Ser Glu Thr Val Ala Gln Glu Trp Gly Thr Ser Thr Gly Asn Thr
            820                 825                 830

Ser Gln Phe Asn Thr Ala Ser Ala Gly Tyr Leu Asn Ala Asn Val Arg
        835                 840                 845

Tyr Asn Asn Val Gly Thr Gly Ala Ile Tyr Asp Val Lys Pro Thr Thr
    850                 855                 860

Ser Phe Val Leu Asn Asn Asp Thr Ile Ala Thr Ile Thr Ala Lys Ser
865                 870                 875                 880

Asn Ser Thr Ala Leu Asn Ile Ser Pro Gly Glu Ser Tyr Pro Lys Lys
                885                 890                 895

Gly Gln Asn Gly Ile Ala Ile Thr Ser Met Asp Asp Phe Asn Ser His
            900                 905                 910

Pro Ile Thr Leu Asn Lys Lys Gln Val Asp Asn Leu Leu Asn Asn Lys
        915                 920                 925

Pro Met Met Leu Glu Thr Asn Gln Thr Asp Gly Val Tyr Lys Ile Lys
    930                 935                 940

Asp Thr His Gly Asn Ile Val Thr Gly Gly Glu Trp Asn Gly Val Ile
945                 950                 955                 960

Gln Gln Ile Lys Ala Lys Thr Ala Ser Ile Ile Val Asp Asp Gly Glu
                965                 970                 975

Arg Val Ala Glu Lys Arg Val Ala Ala Lys Asp Tyr Glu Asn Pro Glu
            980                 985                 990

Asp Lys Thr Pro Ser Leu Thr Leu Lys Asp Ala Leu Lys Leu Ser Tyr
        995                 1000                1005

Pro Asp Glu Ile Lys Glu Ile Glu Gly Leu Leu Tyr Tyr Lys Asn Lys
    1010                1015                1020

Pro Ile Tyr Glu Ser Ser Val Met Thr Tyr Leu Asp Glu Asn Thr Ala
1025                1030                1035                1040

Lys Glu Val Thr Lys Gln Leu Asn Asp Thr Thr Gly Lys Phe Lys Asp
                1045                1050                1055

Val Ser His Leu Tyr Asp Val Lys Leu Thr Pro Lys Met Asn Val Thr
            1060                1065                1070

Ile Lys Leu Ser Ile Leu Tyr Asp Asn Ala Glu Ser Asn Asp Asn Ser
        1075                1080                1085

Ile Gly Lys Trp Thr Asn Thr Asn Ile Val Ser Gly Gly Asn Asn Gly
    1090                1095                1100

Lys Lys Gln Tyr Ser Ser Asn Asn Pro Asp Ala Asn Leu Thr Leu Asn
1105                1110                1115                1120

Thr Asp Ala Gln Glu Lys Leu Asn Lys Asn Arg Asp Tyr Tyr Ile Ser
                1125                1130                1135

Leu Tyr Met Lys Ser Glu Lys Asn Thr Gln Cys Glu Ile Thr Ile Asp
            1140                1145                1150

Gly Glu Ile Tyr Pro Ile Thr Thr Lys Thr Val Asn Val Asn Lys Asp

1155                 1160                 1165

Asn Tyr Lys Arg Leu Asp Ile Ile Ala His Asn Ile Lys Ser Asn Pro
    1170                 1175                 1180

Ile Ser Ser Leu His Ile Lys Thr Asn Asp Glu Ile Thr Leu Phe Trp
1185                 1190                 1195                 1200

Asp Asp Ile Ser Ile Thr Asp Val Ala Ser Ile Lys Pro Glu Asn Leu
                1205                 1210                 1215

Thr Asp Ser Glu Ile Lys Gln Ile Tyr Ser Arg Tyr Gly Ile Lys Leu
            1220                 1225                 1230

Glu Asp Gly Ile Leu Ile Asp Lys Lys Gly Gly Ile His Tyr Gly Glu
        1235                 1240                 1245

Phe Ile Asn Glu Ala Ser Phe Asn Ile Glu Pro Leu Gln Asn Tyr Val
    1250                 1255                 1260

Thr Lys Tyr Glu Val Thr Tyr Ser Ser Glu Leu Gly Pro Asn Val Ser
1265                 1270                 1275                 1280

Asp Thr Leu Glu Ser Asp Lys Ile Tyr Lys Asp Gly Thr Ile Lys Phe
                1285                 1290                 1295

Asp Phe Thr Lys Tyr Ser Lys Asn Glu Gln Gly Leu Phe Tyr Asp Ser
            1300                 1305                 1310

Gly Leu Asn Trp Asp Phe Lys Ile Asn Ala Ile Thr Tyr Asp Gly Lys
        1315                 1320                 1325

Glu Met Asn Val Phe His Arg Tyr Asn Lys
    1330                 1335

(2) INFORMATION FOR SEQ ID NO:51:

    (i) SEQUENCE CHARACTERISTICS:
        (A) LENGTH: 2444 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (ix) FEATURE:
        (A) NAME/KEY: CDS
        (B) LOCATION: 17..2444
        (D) OTHER INFORMATION: /product= "3A(a) synthetic:native
  fusion"

(xi) SEQUENCE DESCRIPTION: SEQ ID NO:51:

GGATCCACCA ATGAAC ATG AAC AAG AAC AAC ACC AAG CTG AGC ACC CGC 49
Met Asn Lys Asn Asn Thr Lys Leu Ser Thr Arg
1               5                   10

GCC CTG CCG AGC TTC ATC GAC TAC TTC AAC GGC ATC TAC GGC TTC GCC 97
Ala Leu Pro Ser Phe Ile Asp Tyr Phe Asn Gly Ile Tyr Gly Phe Ala
            15                  20                  25

ACC GGC ATC AAG GAC ATC ATG AAC ATG ATC TTC AAG ACC GAC ACC GGC 145
Thr Gly Ile Lys Asp Ile Met Asn Met Ile Phe Lys Thr Asp Thr Gly
        30                  35                  40

GGC GAC CTG ACC CTG GAC GAG ATC CTG AAG AAC CAG CAG CTG CTG AAC 193
Gly Asp Leu Thr Leu Asp Glu Ile Leu Lys Asn Gln Gln Leu Leu Asn
    45                  50                  55

GAC ATC AGC GGC AAG CTG GAC GGC GTG AAC GGC AGC CTG AAC GAC CTG 241
Asp Ile Ser Gly Lys Leu Asp Gly Val Asn Gly Ser Leu Asn Asp Leu
60                  65                  70                  75

ATC GCC CAG GGC AAC CTG AAC ACC GAG CTG AGC AAG GAG ATC CTT AAG 289
Ile Ala Gln Gly Asn Leu Asn Thr Glu Leu Ser Lys Glu Ile Leu Lys
                80                  85                  90

ATC GCC AAC GAG CAG AAC CAG GTG CTG AAC GAC GTG AAC AAC AAG CTG 337
Ile Ala Asn Glu Gln Asn Gln Val Leu Asn Asp Val Asn Asn Lys Leu
            95                  100                 105

GAC GCC ATC AAC ACC ATG CTG CGC GTG TAC CTG CCG AAG ATC ACC AGC 385
Asp Ala Ile Asn Thr Met Leu Arg Val Tyr Leu Pro Lys Ile Thr Ser
        110                 115                 120

ATG CTG AGC GAC GTG ATG AAG CAG AAC TAC GCC CTG AGC CTG CAG ATC 433
Met Leu Ser Asp Val Met Lys Gln Asn Tyr Ala Leu Ser Leu Gln Ile
    125                 130                 135

GAG TAC CTG AGC AAG CAG CTG CAG GAG ATC AGC GAC AAG CTG GAC ATC 481
Glu Tyr Leu Ser Lys Gln Leu Gln Glu Ile Ser Asp Lys Leu Asp Ile
140                 145                 150                 155

ATC AAC GTG AAC GTC CTG ATC AAC AGC ACC CTG ACC GAG ATC ACC CCG 529
Ile Asn Val Asn Val Leu Ile Asn Ser Thr Leu Thr Glu Ile Thr Pro
                160                 165                 170

GCC TAC CAG CGC ATC AAG TAC GTG AAC GAG AAG TTC GAA GAG CTG ACC 577
Ala Tyr Gln Arg Ile Lys Tyr Val Asn Glu Lys Phe Glu Glu Leu Thr
            175                 180                 185

TTC GCC ACC GAG ACC AGC AGC AAG GTG AAG AAG GAC GGC AGC CCG GCC 625
Phe Ala Thr Glu Thr Ser Ser Lys Val Lys Lys Asp Gly Ser Pro Ala
        190                 195                 200

GAC ATC CTG GAC GAG CTG ACC GAG CTG ACC GAG CTG GCC AAG AGC GTG 673

Asp Ile Leu Asp Glu Leu Thr Glu Leu Thr Glu Leu Ala Lys Ser Val
    205                 210                 215

ACC AAG AAC GAC GTG GAC GGC TTC GAG TTC TAC CTG AAC ACC TTC CAC     721
Thr Lys Asn Asp Val Asp Gly Phe Glu Phe Tyr Leu Asn Thr Phe His
220                 225                 230                 235

GAC GTG ATG GTG GGC AAC AAC CTG TTC GGC CGC AGC GCC CTG AAG ACC     769
Asp Val Met Val Gly Asn Asn Leu Phe Gly Arg Ser Ala Leu Lys Thr
                240                 245                 250

GCC AGC GAG CTG ATC ACC AAG GAG AAC GTG AAG ACC AGC GGC AGC GAG     817
Ala Ser Glu Leu Ile Thr Lys Glu Asn Val Lys Thr Ser Gly Ser Glu
            255                 260                 265

GTG GGC AAC GTG TAC AAC TTC CTG ATC GTG CTG ACC GCC CTG CAG GCC     865
Val Gly Asn Val Tyr Asn Phe Leu Ile Val Leu Thr Ala Leu Gln Ala
        270                 275                 280

CAG GCC TTC CTG ACC CTG ACC ACC TGT CGC AAG CTG CTG GGC CTG GCC     913
Gln Ala Phe Leu Thr Leu Thr Thr Cys Arg Lys Leu Leu Gly Leu Ala
    285                 290                 295

GAC ATC GAC TAC ACC AGC ATC ATG AAC GAG CAC TTG AAC AAG GAG AAG     961
Asp Ile Asp Tyr Thr Ser Ile Met Asn Glu His Leu Asn Lys Glu Lys
300                 305                 310                 315

GAG GAG TTC CGC GTG AAC ATC CTG CCG ACC CTG AGC AAC ACC TTC AGC     1009
Glu Glu Phe Arg Val Asn Ile Leu Pro Thr Leu Ser Asn Thr Phe Ser
                320                 325                 330

AAC CCG AAC TAC GCC AAG GTG AAG GGC AGC GAC GAG GAC GCC AAG ATG     1057
Asn Pro Asn Tyr Ala Lys Val Lys Gly Ser Asp Glu Asp Ala Lys Met
            335                 340                 345

ATC GTG GAG GCT AAG CCG GGC CAC GCG TTG ATC GGC TTC GAG ATC AGC     1105
Ile Val Glu Ala Lys Pro Gly His Ala Leu Ile Gly Phe Glu Ile Ser
        350                 355                 360

AAC GAC AGC ATC ACC GTG CTG AAG GTG TAC GAG GCC AAG CTG AAG CAG     1153
Asn Asp Ser Ile Thr Val Leu Lys Val Tyr Glu Ala Lys Leu Lys Gln
    365                 370                 375

AAC TAC CAG GTG GAC AAG GAC AGC TTG AGC GAG GTG ATC TAC GGC GAC     1201
Asn Tyr Gln Val Asp Lys Asp Ser Leu Ser Glu Val Ile Tyr Gly Asp
380                 385                 390                 395

ATG GAC AAG CTG CTG TGT CCG GAC CAG AGC GAG CAA ATC TAC TAC ACC     1249
Met Asp Lys Leu Leu Cys Pro Asp Gln Ser Glu Gln Ile Tyr Tyr Thr
                400                 405                 410

AAC AAC ATC GTG TTC CCG AAC GAG TAC GTG ATC ACC AAG ATC GAC TTC     1297
Asn Asn Ile Val Phe Pro Asn Glu Tyr Val Ile Thr Lys Ile Asp Phe
            415                 420                 425

ACC AAG AAG ATG AAG ACC CTG CGC TAC GAG GTG ACC GCC AAC TTC TAC 1345
Thr Lys Lys Met Lys Thr Leu Arg Tyr Glu Val Thr Ala Asn Phe Tyr
        430                 435                 440

GAC AGC AGC ACC GGC GAG ATC GAC CTG AAC AAG AAG AAG GTG GAG AGC 1393
Asp Ser Ser Thr Gly Glu Ile Asp Leu Asn Lys Lys Lys Val Glu Ser
    445                 450                 455

AGC GAG GCC GAG TAC CGC ACC CTG AGC GCG AAC GAC GAC GGC GTC TAC 1441
Ser Glu Ala Glu Tyr Arg Thr Leu Ser Ala Asn Asp Asp Gly Val Tyr
460                 465                 470                 475

ATG CCA CTG GGC GTG ATC AGC GAG ACC TTC CTG ACC CCG ATC AAC GGC 1489
Met Pro Leu Gly Val Ile Ser Glu Thr Phe Leu Thr Pro Ile Asn Gly
                480                 485                 490

TTT GGC CTG CAG GCC GAC GAG AAC AGC CGC CTG ATC ACC CTG ACC TGT 1537
Phe Gly Leu Gln Ala Asp Glu Asn Ser Arg Leu Ile Thr Leu Thr Cys
            495                 500                 505

AAG AGC TAC CTG CGC GAG CTG CTG CTA GCC ACC GAC CTG AGC AAC AAG 1585
Lys Ser Tyr Leu Arg Glu Leu Leu Leu Ala Thr Asp Leu Ser Asn Lys
        510                 515                 520

GAG ACC AAG CTG ATC GTG CCA CCG AGC GGC TTC ATC AGC AAC ATC GTG 1633
Glu Thr Lys Leu Ile Val Pro Pro Ser Gly Phe Ile Ser Asn Ile Val
    525                 530                 535

GAG AAC GGC AGC ATC GAG GAG GAC AAC CTG GAG CCG TGG AAG GCC AAC 1681
Glu Asn Gly Ser Ile Glu Glu Asp Asn Leu Glu Pro Trp Lys Ala Asn
540                 545                 550                 555

AAC AAG AAC GCC TAC GTG GAC CAC ACC GGC GGC GTG AAC GGC ACC AAG 1729
Asn Lys Asn Ala Tyr Val Asp His Thr Gly Gly Val Asn Gly Thr Lys
                560                 565                 570

GCC CTG TAC GTG CAC AAG GAC GGC GGC ATC AGC CAG TTC ATC GGC GAC 1777
Ala Leu Tyr Val His Lys Asp Gly Gly Ile Ser Gln Phe Ile Gly Asp
            575                 580                 585

AAG CTG AAG CCG AAG ACC GAG TAC GTG ATC CAG TAC ACC GTG AAG GGC 1825
Lys Leu Lys Pro Lys Thr Glu Tyr Val Ile Gln Tyr Thr Val Lys Gly
        590                 595                 600

AAG CCA TCG ATT CAC CTG AAG GAC GAG AAC ACC GGC TAC ATC CAC TAC 1873
Lys Pro Ser Ile His Leu Lys Asp Glu Asn Thr Gly Tyr Ile His Tyr
    605                 610                 615

GAG GAC ACC AAC AAC AAC CTG GAG GAC TAC CAG ACC ATC AAC AAG CGC 1921
Glu Asp Thr Asn Asn Asn Leu Glu Asp Tyr Gln Thr Ile Asn Lys Arg
620                 625                 630                 635

TTC ACC ACC GGC ACC GAC CTG AAG GGC GTG TAC CTG ATC CTG AAG AGC 1969
Phe Thr Thr Gly Thr Asp Leu Lys Gly Val Tyr Leu Ile Leu Lys Ser
                640                 645                 650

CAG AAC GGC GAC GAG GCC TGG GGC GAC AAC TTC ATC ATC CTG GAG ATC     2017
Gln Asn Gly Asp Glu Ala Trp Gly Asp Asn Phe Ile Ile Leu Glu Ile
            655                 660                 665

AGC CCG AGC GAG AAG CTG CTG AGC CCG GAG CTG ATC AAC ACC AAC AAC     2065
Ser Pro Ser Glu Lys Leu Leu Ser Pro Glu Leu Ile Asn Thr Asn Asn
        670                 675                 680

TGG ACC AGC ACC GGC AGC ACC AAC ATC AGC GGC AAC ACC CTG ACC CTG     2113
Trp Thr Ser Thr Gly Ser Thr Asn Ile Ser Gly Asn Thr Leu Thr Leu
    685                 690                 695

TAC CAG GGC GGC CGG GGG ATT CTA AAA CAA AAC CTT CAA TTA GAT AGT     2161
Tyr Gln Gly Gly Arg Gly Ile Leu Lys Gln Asn Leu Gln Leu Asp Ser
700                 705                 710                 715

TTT TCA ACT TAT AGA GTG TAT TTT TCT GTG TCC GGA GAT GCT AAT GTA     2209
Phe Ser Thr Tyr Arg Val Tyr Phe Ser Val Ser Gly Asp Ala Asn Val
                720                 725                 730

AGG ATT AGA AAT TCT AGG GAA GTG TTA TTT GAA AAA AGA TAT ATG AGC     2257
Arg Ile Arg Asn Ser Arg Glu Val Leu Phe Glu Lys Arg Tyr Met Ser
            735                 740                 745

GGT GCT AAA GAT GTT TCT GAA ATG TTC ACT ACA AAA TTT GAG AAA GAT     2305
Gly Ala Lys Asp Val Ser Glu Met Phe Thr Thr Lys Phe Glu Lys Asp
        750                 755                 760

AAC TTT TAT ATA GAG CTT TCT CAA GGG AAT AAT TTA TAT GGT GGT CCT     2353
Asn Phe Tyr Ile Glu Leu Ser Gln Gly Asn Asn Leu Tyr Gly Gly Pro
    765                 770                 775

ATT GTA CAT TTT TAC GAT GTC TCT ATT AAG NAA GAT CGG GAT CTA ATA     2401
Ile Val His Phe Tyr Asp Val Ser Ile Lys Xaa Asp Arg Asp Leu Ile
780                 785                 790                 795

TTA ACA GTT TTT AAA AGC NAA TTC TTG TAT AAT GTC CTT GAT  T          2444
Leu Thr Val Phe Lys Ser Xaa Phe Leu Tyr Asn Val Leu Asp
                800                 805

(2) INFORMATION FOR SEQ ID NO:52:

(i) SEQUENCE CHARACTERISTICS:
(A) LENGTH: 809 amino acids
(B) TYPE: amino acid
(D) TOPOLOGY: linear

(ii) MOLECULE TYPE: protein

(xi) SEQUENCE DESCRIPTION: SEQ ID NO:52:

Met Asn Lys Asn Asn Thr Lys Leu Ser Thr Arg Ala Leu Pro Ser Phe
 1               5                  10                  15

Ile Asp Tyr Phe Asn Gly Ile Tyr Gly Phe Ala Thr Gly Ile Lys Asp
            20                  25                  30

Ile Met Asn Met Ile Phe Lys Thr Asp Thr Gly Gly Asp Leu Thr Leu
        35                  40                  45

Asp Glu Ile Leu Lys Asn Gln Gln Leu Leu Asn Asp Ile Ser Gly Lys
    50                  55                  60

Leu Asp Gly Val Asn Gly Ser Leu Asn Asp Leu Ile Ala Gln Gly Asn
65                  70                  75                  80

Leu Asn Thr Glu Leu Ser Lys Glu Ile Leu Lys Ile Ala Asn Glu Gln
                85                  90                  95

Asn Gln Val Leu Asn Asp Val Asn Asn Lys Leu Asp Ala Ile Asn Thr
            100                 105                 110

Met Leu Arg Val Tyr Leu Pro Lys Ile Thr Ser Met Leu Ser Asp Val
        115                 120                 125

Met Lys Gln Asn Tyr Ala Leu Ser Leu Gln Ile Glu Tyr Leu Ser Lys
    130                 135                 140

Gln Leu Gln Glu Ile Ser Asp Lys Leu Asp Ile Ile Asn Val Asn Val
145                 150                 155                 160

Leu Ile Asn Ser Thr Leu Thr Glu Ile Thr Pro Ala Tyr Gln Arg Ile
                165                 170                 175

Lys Tyr Val Asn Glu Lys Phe Glu Glu Leu Thr Phe Ala Thr Glu Thr
            180                 185                 190

Ser Ser Lys Val Lys Lys Asp Gly Ser Pro Ala Asp Ile Leu Asp Glu
        195                 200                 205

Leu Thr Glu Leu Thr Glu Leu Ala Lys Ser Val Thr Lys Asn Asp Val
    210                 215                 220

Asp Gly Phe Glu Phe Tyr Leu Asn Thr Phe His Asp Val Met Val Gly
225                 230                 235                 240

Asn Asn Leu Phe Gly Arg Ser Ala Leu Lys Thr Ala Ser Glu Leu Ile
                245                 250                 255

Thr Lys Glu Asn Val Lys Thr Ser Gly Ser Glu Val Gly Asn Val Tyr
            260                 265                 270

Asn Phe Leu Ile Val Leu Thr Ala Leu Gln Ala Gln Ala Phe Leu Thr
        275                 280                 285

Leu Thr Thr Cys Arg Lys Leu Leu Gly Leu Ala Asp Ile Asp Tyr Thr
    290                 295                 300

Ser Ile Met Asn Glu His Leu Asn Lys Glu Lys Glu Glu Phe Arg Val
305                 310                 315                 320

Asn Ile Leu Pro Thr Leu Ser Asn Thr Phe Ser Asn Pro Asn Tyr Ala
                325                 330                 335

Lys Val Lys Gly Ser Asp Glu Asp Ala Lys Met Ile Val Glu Ala Lys
            340                 345                 350

Pro Gly His Ala Leu Ile Gly Phe Glu Ile Ser Asn Asp Ser Ile Thr
        355                 360                 365

Val Leu Lys Val Tyr Glu Ala Lys Leu Lys Gln Asn Tyr Gln Val Asp
    370                 375                 380

Lys Asp Ser Leu Ser Glu Val Ile Tyr Gly Asp Met Asp Lys Leu Leu
385                 390                 395                 400

Cys Pro Asp Gln Ser Glu Gln Ile Tyr Tyr Thr Asn Asn Ile Val Phe
                405                 410                 415

Pro Asn Glu Tyr Val Ile Thr Lys Ile Asp Phe Thr Lys Lys Met Lys
            420                 425                 430

Thr Leu Arg Tyr Glu Val Thr Ala Asn Phe Tyr Asp Ser Ser Thr Gly
        435                 440                 445

Glu Ile Asp Leu Asn Lys Lys Lys Val Glu Ser Ser Glu Ala Glu Tyr
    450                 455                 460

Arg Thr Leu Ser Ala Asn Asp Asp Gly Val Tyr Met Pro Leu Gly Val
465                 470                 475                 480

Ile Ser Glu Thr Phe Leu Thr Pro Ile Asn Gly Phe Gly Leu Gln Ala
                485                 490                 495

Asp Glu Asn Ser Arg Leu Ile Thr Leu Thr Cys Lys Ser Tyr Leu Arg
            500                 505                 510

Glu Leu Leu Leu Ala Thr Asp Leu Ser Asn Lys Glu Thr Lys Leu Ile
        515                 520                 525

Val Pro Pro Ser Gly Phe Ile Ser Asn Ile Val Glu Asn Gly Ser Ile
    530                 535                 540

Glu Glu Asp Asn Leu Glu Pro Trp Lys Ala Asn Asn Lys Asn Ala Tyr
545                 550                 555                 560

Val Asp His Thr Gly Gly Val Asn Gly Thr Lys Ala Leu Tyr Val His
                565                 570                 575

Lys Asp Gly Gly Ile Ser Gln Phe Ile Gly Asp Lys Leu Lys Pro Lys
            580                 585                 590

Thr Glu Tyr Val Ile Gln Tyr Thr Val Lys Gly Lys Pro Ser Ile His

        595                 600                 605

Leu Lys Asp Glu Asn Thr Gly Tyr Ile His Tyr Glu Asp Thr Asn Asn
    610                 615                 620

Asn Leu Glu Asp Tyr Gln Thr Ile Asn Lys Arg Phe Thr Thr Gly Thr
625                 630                 635                 640

Asp Leu Lys Gly Val Tyr Leu Ile Leu Lys Ser Gln Asn Gly Asp Glu
                645                 650                 655

Ala Trp Gly Asp Asn Phe Ile Ile Leu Glu Ile Ser Pro Ser Glu Lys
            660                 665                 670

Leu Leu Ser Pro Glu Leu Ile Asn Thr Asn Asn Trp Thr Ser Thr Gly
        675                 680                 685

Ser Thr Asn Ile Ser Gly Asn Thr Leu Thr Leu Tyr Gln Gly Gly Arg
    690                 695                 700

Gly Ile Leu Lys Gln Asn Leu Gln Leu Asp Ser Phe Ser Thr Tyr Arg
705                 710                 715                 720

Val Tyr Phe Ser Val Ser Gly Asp Ala Asn Val Arg Ile Arg Asn Ser
                725                 730                 735

Arg Glu Val Leu Phe Glu Lys Arg Tyr Met Ser Gly Ala Lys Asp Val
            740                 745                 750

Ser Glu Met Phe Thr Thr Lys Phe Glu Lys Asp Asn Phe Tyr Ile Glu
        755                 760                 765

Leu Ser Gln Gly Asn Asn Leu Tyr Gly Gly Pro Ile Val His Phe Tyr
    770                 775                 780

Asp Val Ser Ile Lys Xaa Asp Arg Asp Leu Ile Leu Thr Val Phe Lys
785                 790                 795                 800

Ser Xaa Phe Leu Tyr Asn Val Leu Asp
                805

## Description

The present invention is drawn to methods and compositions for controlling plant and non-plant pests. Particularly, new pesticidal proteins are disclosed which are isolatable from the vegetative growth stage of *Bacillus. Bacillus* strains, proteins, and genes encoding the proteins are provided. The methods and compositions of the invention may be used in a variety of systems for controlling plant and non-plant pests.

Insect pests are a major factor in the loss of the world's commercially important agricultural crops. Broad spectrum chemical pesticides have been used extensively to control or eradicate pests of agricultural importance. There is, however, substantial interest in developing effective alternative pesticides.

Microbial pesticides have played an important role as alternatives to chemical pest control. The most extensively used microbial product is based on the bacterium *Bacillus thuringiensis* (Bt). Bt is a gram-positive spore forming *Bacillus* which produces an insecticidal crystal protein (lCP) during sporulation.

Numerous varieties of Bt are known that produce more than 25 different but related lCP's. The majority of lCP's made by Bt are toxic to larvae of certain insects in the orders Lepidoptera, *Diptera* and *Coleoptera*. ln general, when an ICP is ingested by a susceptible insect the crystal is solubilized and transformed into a toxic moiety by the insect gut proteases. None of the lCP's active against coleopteran larvae such as Colorado potato beetle (*Leptinotarsa decemlineata)* or Yellow mealworm (*Tenebrio molitor)* have demonstrated significant effects on members of the genus *Diabrotica* particularly *Diabrotica virgifera virgifera*, the western corn rootworm (WCRW) or *Diabrotica longicornis barberi,* the northern corn rootworm.

*Bacillus* cereus (Bc) is closely related to Bt. A major distinguishing characteristic is the absence of a parasporal crystal in Bc. Bc is a widely distributed bacterium that is commonly found in soil and has been isolated from a variety of foods and drugs. The organism has been implicated in the spoilage of food.

Although Bt has been very useful in controlling insect pests, there is a need to expand the number of potential biological control agents. during vegetative growth of *Bacillus* strains. The proteins are useful as pesticidal agents.

More specifically, the present invention relates to a substantially purified *Bacillus* strain which produces a pesticidal protein during vegetative growth wherein said Bacillus is not *B. sphaericus* SSII-1. Preferred are a *Bacillus* cereus strain having Accession No. NRRL B-21058 and *Bacillus thuringiensis* strain having Accession No. NRRL B-21060. Also preferred is a Bacillus strain selected from Accession Numbers NRRL B-21224, NRRL B-21225, NRRL B-21226, NRRL B-21227, NRRL B-21228, NRRL B-21229, NRRL B-21230, and NRRL B-21439.

The invention further relates to an insect-specific protein isolatable during the vegetative growth phase of *Bacillus* spp, but preferably of a *Bacillus thuringiensis* and B. cereus strain, and components thereof, wherein said protein is not the mosquitocidal toxin from *B. sphaericus* SSII-1. The insect-specific protein of the invention is preferably toxic to Coleoptera or Lepidoptera insects and has a molecular weight of about 30 kDa or greater, preferably of about 60 to about 100 kDa, and more preferably of about 80 kDa.

More particularly, the insect-specific protein of the invention has a spectrum of insecticidal activity that includes an activity against *Agrotis* and/or *Spodoptera* species, but preferably a black cutworm [*Agrotis ipsilon;* BCW] and/or fall armyworm [*Spodoptera frugiperda*] and/or beet armyworm [*Spodoptera exigua* ] and/or tobacco budworm and/or corn earworm [*Helicoverpa zea*] activity.

The insect-specific protein of the invention can preferably be isolated, for example, from Bacillus cereus having Accession No. NRRL B-21058, or from *Bacillus thuringiensis* having Accession No. NRRL B-21060.

The insect-specific protein of the invention can also preferably be isolated from a *Bacillus* spp strain selected from Accession Numbers NRRL B-21224, NRRL B-21225, NRRL B-21226, NRRL B-21227, NRRL B-21228, NRRL B-21229, NRRL B-21230, and NRRL B-21439.

The present invention especially encompasses an insect-specific protein that has the amino acid sequence selected from the group consisting of SEQ ID NO:5 and SEQ ID NO:7, including any proteins that are structurally and/or functionally homologous thereto.

Further preferred is an insect-specific protein, wherein said protein has the sequence selected from the group consisting of SEQ lD NO:20, SEQ lD NO:21, SEQ ID NO:29 SEQ ID NO:32 and SEQ ID NO:2, including any proteins that are structurally and/or functionally homologous thereto.

Especially preferred is an insect-specific protein, wherein said protein has the sequence selected from the group consisting of SEQ ID NO:29 and SEQ ID NO:32, including any proteins that are structurally and/or functionally homologous thereto.

A further preferred embodiment of the invention comprises an insect-specific protein of the invention, wherein the sequences representing the secretion signal have been removed or inactivated.

The present invention further encompasses auxiliary proteins which enhance the insect-specific activity of an insect-specific protein. The said auxiliary proteins preferably have a molecular weight of about 50 kDa and can be isolated, for example, from the vegetative growth phase of a Bacillus cereus strain, but especially of Bacillus cereus strain AB78.

A preferred embodiment of the invention relates to an auxiliary protein, wherein the sequences representing the secretion signal have been removed or inactivated.

The present invention further relates to multimeric pesticidal proteins, which comprise more than one polypeptide chain and wherein at least one of the said polypeptide chains represents an insect-specific protein of the invention and at least one of the said polypeptide chains represents an auxiliary protein of the invention, which activates or enhances the pesticidal activity of the said insect-specific protein.

The multimeric pesticidal proteins according to the invention preferably have a molecular weight of about 50 kDa to about 200 kDa.

The invention especially encompasses a multimeric pesticidal protein, which comprises an insect-specific protein of the invention and an auxiliary protein according to the invention, which activates or enhances the pesticidal activity of the said insect-specific protein.

The present invention further relates to fusion proteins comprising several protein domains including at least an insect-specific protein of the invention and/or an auxiliary protein according to the invention produced by in frame genetic fusions, which, when translated by ribosomes, produce a fusion protein with at least the combined attributes of the insect-specific protein of the invention and/or an auxiliary protein according to the invention and, optionally, of the other components used in the fusion.

A specific embodiment of the invention relates to a fusion protein comprising a ribonuclease S-protein, an insect-specific protein of the invention and an auxiliary protein according to the invention.

A further specific embodiment of the invention relates to a fusion protein comprising an insect-specific protein according to the invention and an auxiliary protein according to the invention having either the insect-specific protein or the auxiliary protein at the N-terminal end of the said fusion protein.

Preferred is a fusion protein, which comprises an insect-specific protein as given in SEQ ID NO:5 and an auxiliary protein as given in SEQ ID NO: 2 resulting in the protein given in SEO ID NO: 23, including any proteins that are structurally and/or functionally homologous thereto.

Also preferred is a fusion protein, which comprises an insect-specific protein as given in SEQ ID NO:35 and an auxiliary protein as given in SEQ ID NO: 27 resulting in the protein given in SEQ ID NO: 50, including any proteins that are structurally and/or functionally homologous thereto.

The invention further relates to a fusion protein comprising an insect-specific protein of the invention and/or an auxiliary protein according to the invention fused to a signal sequence, preferably a secretion signal sequence or a targeting sequence that directs the transgene product to a specific organelle or cell compartment, which signal sequence is of herterologous origin with respect to the recipient protein.

Especially preferred within this invention is a fusion protein wherein the said protein has a sequence as given in SEQ ID NO: 43, or in SEQ ID NO: 46, including any proteins that are structurally and/or functionally homologous thereto.

As used in the present application, substantial sequence homology means close structural relationship between sequences of amino acids. For example, substantially homologous proteins may be 40% homologous, preferably 50% and most preferably 60% or 80% homologous, or more. Homology also includes a relationship wherein one or several subsequences of amino acids are missing, or subsequences with additional amino acids are interdispersed.

A further aspect of the invention relates to a DNA molecule comprising a nucleotide sequence which encodes an insect-specific protein isolatable during the vegetative growth phase of Bacillus spp. and components thereof, wherein said protein is not the mosquitocidal toxin from B. sphaericus SSII-1. In particular, the present invention relates to a DNA molecule comprising a nucleotide sequence which encodes an insect-specific protein wherein the spectrum of insecticidal activity includes an activity against *Agrotis* and/or *Spodoptera* species, but preferably a black cutworm [*Agrotis ipsilon* BCW] and/or fall armyworm [*Spodoptera frugiperda*] and/or beet armyworm [*Spodoptera exigua* ] and/or tobacco budworm and/or corn earworm [*Helicoverpa zea*] activity.

Preferred is a DNA molecule, wherein the said molecule comprises a nucleotide sequence as given in SEQ, ID NO: 4, or SEQ ID NO: 6, including any DNA molecules that are structurally and/or functionally homologous thereto.

Also preferred is a DNA molecule, wherein the said molecule comprises a nucleotide sequence as given SEQ ID NO:19, SEQ ID NO:28, SEQ ID NO:31, or SEQ ID NO:1, including any DNA molecules that are structurally and/or functionally homologous thereto.

The invention further relates to a DNA molecule comprising a nucleotide sequence which encodes an auxiliary protein according to the invention which enhances the insect-specific activity of an insect-specific protein.

Preferred is a DNA molecule, wherein the said molecule comprises a nucleotide sequence as given SEQ lD NO:19, including any DNA molecules that are structurally and/or functionally homologous thereto.

A further embodiment of the invention relates to a DNA molecule comprising a nucleotide sequence which encodes an insect-specific protein isolatable during the vegetative growth phase of *Bacillus* spp. and components thereof, wherein said protein is not the mosquitocidal toxin from *B. sphaericus* SSII-1, which nucleotide sequence has been optimized for expression in a microorganism or a plant.

Preferred is a DNA molecule, wherein the said molecule comprises a nucleotide sequence as given in SEQ ID NO:17 or SEQ ID NO:18, including any DNA molecules that are structurally and/or functionally homologous thereto.

Also preferred is a DNA molecule, wherein the said molecule comprises a nucleotide sequence as given in SEQ ID NO:24, SEQ ID NO:26, SEQ ID NO:27, or SEQ ID NO:30, including any DNA molecules that are structurally and/or functionally homologous thereto.

The invention further relates to a DNA molecule which comprises a nucleotide sequence encoding a multimeric pesticidal protein, which comprises more than one polypeptide chains and wherein at least one of the said polypeptide chains represents an insect-specific protein of the invention and at least one of the said polypeptide chains represents an auxiliary protein according to the invention, which activates or enhances the pesticidal activity of the said insect-specific protein.

Preferred is a DNA molecule comprising a nucleotide sequence encoding an insect-specific protein of the invention and an auxiliary protein according to the invention, which activates or enhances the pesticidal activity of the said insect-specific protein.

Especially preferred is a DNA molecule, wherein said molecule comprises a nucleotide sequence as given in SEQ ID NO:1 or SEQ ID NO:19, including any nucleotide sequences that are structurally and/or functionally homologous thereto.

A further embodiment of the invention relates to a DNA molecule which comprises a nucleotide sequence encoding a fusion protein comprising several protein domains including at least an insect-specific protein of the invention and/or an auxiliary protein according to the invention produced by in frame genetic fusions, which, when translated by ribosomes, produce a fusion protein with at least the combined attributes of the insect-specific protein of the invention and/or an auxiliary protein according to the invention and, optionally, of the other components used in the fusion.

Preferred within the invention is a DNA molecule which comprises a nucleotide sequence encoding a fusion protein comprising an insect-specific protein according to the invention and an auxiliary protein according to the invention having either the insect-specific protein or the auxiliary protein at the N-terminal end of the said fusion protein. Especially preferred is a DNA molecule, wherein the said molecule comprises a nucleotide sequence as given in SEQ ID NO:22, including any DNA molecules that are structurally and/or functionally homologous thereto.

The invention further relates to a DNA molecule which comprises a nucleotide sequence encoding a fusion protein comprising an insect-specific protein of the invention and/or an auxiliary protein of the invention fused to a signal sequence, preferably a secretion signal sequence or a targeting sequence that directs the transgene product to a specific organelle or cell compartment, which signal sequence is of herterologous origin with respect to the recipient DNA.

The present invention further encompasses a DNA molecule comprising a nucleotide sequence encoding a fusion protein or a mulitmeric protein according to the invention that has been optimized for expression in a microorganism or plant.

Preferred is an optimized DNA molecule, wherein the said molecule comprises a nucleotide sequence as given in SEQ ID NO:42, SEQ ID NO:45, or SEQ ID NO:49, including any DNA molecules that are structurally and/or functionally homologous thereto.

The invention further relates to an optimized DNA molecule, wherein the sequences encoding the secretion signal have been removed from its 5' end, but especially to an optimized DNA molecule, wherein the said molecule comprises a nucleotide sequence as given in SEQ ID NO: 35 or SEQ ID NO:39, including any DNA molecules that are structurally and/or functionally homologous thereto.
As used in the present application, substantial sequence homology means close structural relationship between sequences of nucleotides. For example, substantially homologous DNA molecules may be 60% homologous, preferably 80% and most preferably 90% or 95% homologous, or more. Homology also includes a relationship wherein one or several subsequences of nucleotides or amino acids are missing, or subsequences with additional nucleotides or amino acids are interdispersed.

Also comprised by the present invention are DNA molecules which hybridizes to a DNA molecule according to the invention as defined hereinbefore, but preferably to an oligonucleotide probe obtainable from said DNA molecule comprising a contiguous portion of the coding sequence for the said insect-specific protein at least 10 nucleotides in length, under moderately stringent conditions and which molecules have insect-specific activity and also the insect-specific proteins being encoded by the said DNA molecules.

Preferred are DNA molecules, wherein hybridization occurs at 65°C in a buffer comprising 7% SDS and 0.5 M sodium phosphate.
Especially preferred is a DNA molecule comprising a nucleotide sequence which encodes an insect-specific protein according to the invention obtainable by a process comprising
(a) obtaining a DNA molecule comprising a nucleotide sequence encoding an insect-specific protein; and
(b) hybridizing said DNA molecule with an oligonucleotide probe acording to claim 107 obtained from a DNA molecule comprising a nucleotide sequence as given in SEQ ID NO: 28, SEQ ID NO: 30, or SEQ ID NO: 31; and
(c) isolating said hybridized DNA.

The invention further relates to an insect-specific protein, wherein the said protein is encoded by a DNA molecule according to the invention.

Also encompassed by the invention is an expression cassette comprising a DNA molecule according to the invention operably linked to expression sequences including the transcriptional and translational regulatory signals necessary for expression of the associated DNA constructs in a host organism, preferably a microorganism or a plant, and optionally further regulatory sequences.

The invention further relates to a vector molecule comprising an expression cassette according to the invention.

The expression cassette and/or the vector molecule according to the invention are preferably part of the plant genome.

A further embodiment of the invention relates to a host organism, preferably a host organism selected from the group consisting of plant and insect cells, bacteria, yeast, baculoviruses, protozoa, nematodes and algae, comprising a DNA molecule according to the invention, an expression cassette comprising the said DNA molecule or a vector molecule comprising the said expression cassette, preferably stably incorporated into the genome of the host organism.

The invention further relates to a transgenic plant, but preferably a maize plant, including parts as well as progeny and seed thereof comprising a DNA molecule according to the invention, an expression cassette comprising the said DNA molecule or a vector molecule comprising the said expression cassette, preferably stably incorporated into the plant genome.

Preferred is a transgenic plant including parts as well as progeny and seed thereof which has been stably transformed with a DNA molecule according to the invention, an expression cassette comprising the said DNA molecule or a vector molecule comprising the said expression cassette.

Also preferred is a transgenic plant including parts as well as progeny and seed thereof which expresses an insect-specific protein according to the invention.

The invention further relates to a transgenic plant, preferably a maize plant, according to the invention as defined hereinbefore, which further expresses a second distinct insect control principle, but preferably a *Bt* δ-endotoxin. The said plant is preferably a hybrid plant.

Parts of transgenic plants are to be understood within the scope of the invention to comprise, for example, plant cells, protoplasts, tissues, callus, embryos as well as flowers, stems, fruits, leaves, roots originating in transgenic plants or their progeny previously transformed with a DNA molecule according to the invention and therefore consisting at least in part of transgenic cells, are also an object of the present invention.

The invention further relates to plant propagating material of a plant according to the invention, which is treated with a seed protectant coating.

The invention further encompasses a microorganism transformed with a DNA molecule according to the invention, an expression cassette comprising the said DNA molecule or a vector molecule comprising the said expression cassette, wherein the said microorganism is preferably a microorganism that multiply on plants and more preferably a root colonizing bacterium.

A further embodiment of the invention relates to an encapsulated insect-specific protein which comprises a microorganism comprising an insect specific protein according to the invention.

The invention also relates to an entomocidal composition comprising a host organism of the invention, but preferably a purified *Bacillus* strain, in an insecticidally-effective amount together with a suitable carrier.

Further comprised by the invention is an entomocidal composition comprising an isolated protein molecule according to the invention, alone or in combination with a host organism of the invention and/or an encapsulated insect-specific protein according to the invention, in an insecticidally-effective amount, together with a suitable carrier.

A further embodiment of the invention relates to a method of obtaining a purified insect-specific protein according to the invention, said method comprising applying a solution comprising said insect-specific protein to a NAD column and eluting bound protein.

Also comprised is a method for identifying insect activity of an insect-specific protein according to the invention, said method comprising:
growing a *Bacillus* strain in a culture;
obtaining supernatant from said culture;
allowing insect larvae to feed on diet with said supernatant; and,
determining mortality.

Another aspect of the invention relates to a method for isolating an insect-specific protein according to the invention, said method comprising:
growing a *Bacillus* strain in a culture;
obtaining supernatant from said culture; and,
isolating said insect-specific protein from said supernatant.

The invention also encompasses a method for isolating a DNA molecule comprising a nucleotide sequence encoding an insect-specific protein exhibiting the insecticidal activity of the proteins according to the invention, said method comprising:
obtaining a DNA molecule comprising a nucleotide sequence encoding an insect-specific protein; and
hybridizing said DNA molecule with DNA obtained from a *Bacillus* species; and
isolating said hybridized DNA.

The invention further relates to a method of increasing insect target range by using an insect specific protein according to the invention in combination with at least one second insecticidal protein that is different from the insect specific protein according to the invention, but preferably with an insecticidal protein selected from the group consisting of *Bt* δ-endotoxins, protease inhibitors, lectins, α-amylases and peroxidases.

Preferred is a method for increasing insect target range within a plant by expressing within the said plant a insect specific protein according to the invention in combination with at least one second insecticidal protein that is different from the insect specific protein according to the invention, but preferably with an insecticidal protein selected from the group consisting of *Bt* δ-endotoxins, protease inhibitors, lectins, α-amylases and peroxidases.

Also comprised is a method of protecting plants against damage caused by an insect pest, but preferably by *Spodoptera* and/or *Agrotis* species, and more preferably by an insect pest selected from the group consisting of black cutworm [*Agrotis ipsilon*; BCW], fall armyworm [*Spodoptera frugiperda*], beet armyworm [*Spodoptera exigua* ], tobacco budworm and corn earworm [*Helicoverpa zea*] comprising applying to the plant or the growing area of the said plant an entomocidal composition or a toxin protein according to the invention.

The invention further relates to method of protecting plants against damage caused by an insect pest, but preferably by *Spodoptera* and/or *Agrotis* species, and more preferably by an insect pest selected from the group consisting of black cutworm [*Agrotis ipsilon;* BCW], fall armyworm [*Spodoptera frugiperda*], beet armyworm [*Spodoptera exigua*], tobacco budworm and corn earworm [*Helicoverpa zea]* comprising planting a transgenic plant expressing a insect-specific protein according to the invention within an area where the said insect pest may occur.

The invention also encompasses a method of producing a host organism which comprises stably integrated into its genome a DNA molecule according to the invention and preferably expresses an insect-specific protein according to the invention comprising transforming the said host organism with a DNA molecule according to the invention, an expression cassette comprising the said DNA molecule or a vector molecule comprising the said expression cassette.

A further embodiment of the invention relates to a method of producing a transgenic plant or plant cell which comprises stably integrated into the plant genome a DNA molecule according to the invention and preferably expresses an insect-specific protein according to the invention comprising transforming the said plant and plant cell, respectively, with a DNA molecule according to the invention, an expression cassette comprising the said DNA molecule or a vector molecule comprising the said expression cassette.

The invention also relates to a method of producing an entomocidal composition comprising mixing an isolated *Bacillus* strain and/or a host organism and/or an isolated protein molecule, and/or an encapsulated protein according to the invention in an insecticidally-effective amount with a suitable carrier.

The invention also encompasses a method of producing transgenic progeny of a transgenic parent plant comprising stably incorporated into the plant genome a DNA molecule comprising a nucleotide sequence encoding an insect-specific protein according to the invention comprising transforming the said parent plant with a DNA molecule according to the invention, an expression cassette comprising the said DNA molecule or a vector molecule comprising the said expression cassette and transferring the pesticidal trait to the progeny of the said transgenic parent plant involving known plant breeding techniques.

Also encompassed by the invention is oligonucleotide probe capable of specifically hybridizing to a nucleotide sequence encoding a insect-specific protein isolatable during the vegetative growth phase of *Bacillus* spp. and components thereof, wherein said protein is not the mosquitocidal toxin from *B. sphaericus* SSII-1, wherein said probe comprises a contiguous portion of the coding sequence for the said insect-specific protein at least 10 nucleotides in length and the use of the said oligonucleotide probe for screening of any *Bacillus* strain or other organisms to determine whether the insect-specific protein is naturally present or whether a particular transformed organism includes the said gene

The present invention recognizes that pesticidal proteins are produced during vegetative growth of *Bacillus* strains. Having recognized that such a class exists, the present invention embraces all vegetative insecticidal proteins, hereinafter referred to as VIPs, except for the mosquitocidal toxin from *B*. *sphaericus._*

The present VIPs are not abundant after sporulation and are particularly expressed during log phase growth before stationary phase. For the purpose of the present invention vegetative growth is defined as that period of time before the onset of sporulation. Genes encoding such VlPs can be isolated, cloned and transformed into various delivery vehicles for use in pest management programs.

For purposes of the present invention, pests include but are not limited to insects, fungi, bacteria, nematodes, mites, ticks, protozoan pathogens, animal-parasitic liver flukes, and the like. Insect pests include insects selected from the orders Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera, etc., particularly Coleoptera and Lepidoptera.

Tables 1 - 10 gives a list of pests associated with major crop plants and pests of human and veterinary importance. Such pests are included within the scope of the present invention.

**TABLE 1**

| Lepidoptera (Butterflies and Moth) |
|---|
| Maize |
| *Ostrinia nubilalis,* European corn borer |
| *Agrotis ipsilon,* black cutworm |
| *Helicoverpa zea,* corn earworm |
| *Spodoptera frugiperda,* fall armyworm |
| *Diatraea grandiosella*, southwestern corn borer |
| *Elasmopalpus lignosellus,* lesser cornstalk borer |
| *Diatraea saccharalis*, sugarcane borer |

| Sorghum |
|---|
| *Chilo partellus,* sorghum borer |
| *Spodoptera frugiperda,* fall armyworm |
| *Helicoverpa zea,* corn earworm |
| *Elasmopalpus lignosellus*, lesser cornstalk borer |
| *Feltia subterranea*, granulate cutworm |

| Wheat |
|---|
| *Pseudaletia unipunctata,* army worm |
| *Spodoptera frugiperda,* fall armyworm |
| *Elasmopalpus lignosellus,* lesser cornstalk borer |
| *Agrotis orthogonia,* pale western cutworm |
| *Elasmopalpus lignosellus,* lesser cornstalk borer |

| Sunflower |
|---|
| *Suleima helianthana,* sunflower bud moth |
| *Homoeosoma electellum,* sunflower moth |

| Cotton |
|---|
| *Heliothis virescens,* cotton boll worm |
| *Helicoverpa zea,* cotton bollworm |
| *Spodoptera exigua*, beet armyworm |
| *Pectinophora gossypiella,* pink bollworm |

| Rice |
|---|
| *Diatraea saccharalis*, sugarcane borer |
| *Spodoptera frugiperda,* fall armyworm |
| *Helicoverpa zea,* corn earworm |

| Soybean |
|---|
| *Pseudoplusia includens,* soybean looper |
| *Anticarsia gemmatalis,* velvetbean caterpillar |
| *Plathypena scabra*, green cloverworm |
| *Ostrinia nubilalis,* European corn borer |
| *Agrotis ipsilon,* black cutworm |
| *Spodoptera exigua*, beet armyworm |
| *Heliothis virescens,* cotton boll worm |
| *Helicoverpa zea*, cotton bollworm |

| Barley |
|---|
| *Ostrinia nubilalis,* European corn borer |
| *Agrotis ipsilon,* black cutworm |

**TABLE 2**

| Coleoptera (Beetles) |
|---|
| Maize |
| *Diabrotica virgifera virgifera,* western corn rootworm |
| *Diabrotica longicornis barberi,* northern corn rootworm |
| *Diabrotica undecimpunctata howardi,* southern corn rootworm |
| *Melanotus spp.,* wireworms |
| *Cyclocephala borealis,* northern masked chafer (white grub) |
| *Cyclocephala immaculata,* southern masked chafer (white grub) |
| *Popillia japonica,* Japanese beetle |
| *Chaetocnema pulicaria,* corn flea beetle |
| *Sphenophorus maidis,* maize billbug |

| Sorghum |
|---|
| *Phyllophaga crinita,* white grub |
| *Eleodes, Conoderus,* and *Aeolus spp.*, wireworms |
| *Oulema melanopus,* cereal leaf beetle |
| *Chaetocnema pulicaria,* corn flea beetle |
| *Sphenophorus maidis*, maize billbug |

| Wheat |
|---|
| *Oulema melanopus,* cereal leaf beetle |
| *Hypera punctata,* clover leaf weevil |
| *Diabrotica undecimpunctata howardi*, southern corn rootworm |

| Sunflower |
|---|
| *Zygogramma exclamationis,* sunflower beetle |
| *Bothyrus gibbosus*, carrot beetle |

| Cotton |
|---|
| *Anthonomus grandis,* boll weevil |

| Rice |
|---|
| *Colaspis brunnea,* grape colaspis |
| *Lissorhoptrus oryzophilus,* rice water weevil |
| *Sitophilus oryzae,* rice weevil |

| Soybean |
|---|
| *Epilachna varivestis,* Mexican bean beetle |

**TABLE 3**

| Homoptera (Whiteflies, Aphids etc..) |
|---|
| Maize |
| *Rhopalosiphum maidis,* corn leaf aphid |
| *Anuraphis maidiradicis,* corn root aphid |

| Sorghum |
|---|
| *Rhopalosiphum maidis,* corn leaf aphid |
| *Sipha flava,* yellow sugarcane aphid |

| Wheat |
|---|
| Russian wheat aphid |
| *Schizaphis graminum,* greenbug |
| *Macrosiphum avenae,* English grain aphid |

| Cotton |
|---|
| *Aphis gossypii,* cotton aphid |
| *Pseudatomoscelis seriatus*, cotton fleahopper |
| Trialeurodes abutilonea, bandedwinged whitefly |

| Rice |
|---|
| Nephotettix nigropictus, rice leafhopper |

| Soybean |
|---|
| Myzus persicae, green peach aphid |
| *Empoasca fabae,* potato leafhopper |

| Barley |
|---|
| *Schizaphis graminum,* greenbug |

| Oil Seed Rape |
|---|
| *Brevicoryne brassicae*, cabbage aphid |

**TABLE 4**

| Hemiptera (Bugs) |
|---|
| Maize |
| *Blissus leucopterus leucopterus,* chinch bug |

| Sorghum |
|---|
| Blissus leucopterus leucopterus, chinch bug |

| Cotton |
|---|
| *Lygus lineolaris,* tarnished plant bug |

| Rice |
|---|
| *Blissus leucopterus leucopterus,* chinch bug |
| *Acrosternum hilare,* green stink bug |

| Soybean |
|---|
| *Acrosternum hilare,* green stink bug |

| Barley |
|---|
| *Blissus leucopterus* leucopterus, chinch bug |
| *Acrostemum hilare,* green stink bug |
| *Euschistus servus,* brown stink bug |

**TABLE 5**

| Orthoptera (Grasshoppers, Crickets, and Cockroaches) |
|---|
| Maize |
| *Melanoplus femurrubrum,* redlegged grasshopper |
| *Melanoplus sanguinipes,* migratory grasshopper |

| Wheat |
|---|
| *Melanoplus femurrubrum,* redlegged grasshopper |
| *Melanoplus differentialis,* differential grasshopper |
| *Melanoplus sanguinipes,* migratory grasshopper |

| Cotton |
|---|
| *Melanoplus femurrubrum,* redlegged grasshopper |
| *Melanoplus differentialis,* differential grasshopper |

| Soybean |
|---|
| *Melanoplus femurrubrum,* redlegged grasshopper |
| *Melanoplus differentialis,* differential grasshopper |

| Structural/Household |
|---|
| Periplaneta americana, American cockroach |
| *Blattella germanica,* German cockroach |
| *Blatta orientalis,* oriental cockroach |

**TABLE 6**

| Diptera (Flies and Mosquitoes) |
|---|
| Maize |
| *Hylemya platura,* seedcorn maggot |
| *Agromyza parvicornis,* corn blotch leafminer |

| Sorghum |
|---|
| *Contarinia sorghicola,* sorghum midge |
| *Melanoplus sanguinipes,* migratory grasshopper |

| Wheat |
|---|
| *Mayetiola destructor,* Hessian fly |
| *Sitodiplosis mosellana,* wheat midge |
| *Meromyza americana,* wheat stem maggot |
| *Hylemya coarctata,* wheat bulb fly |

| Sunflower |
|---|
| *Neolasioptera murtfeldtiana,* sunflower seed midge |

| Soybean |
|---|
| Hylemya platura, seedcorn maggot |

| Barley |
|---|
| Hylemya platura, seedcorn maggot |
| Mayetiola destructor, Hessian fly |

| Insects attacking humans and animals and disease carriers |
|---|
| *Aedes aegypti,* yellowfever mosquito |
| *Aedes albopictus,* forest day mosquito |
| *Phlebotomus papatasii,* sand fly |
| *Musca domestica,* house fly |
| *Tabanus atratus,* black horse fly |
| *Cochliomyia hominivorax,* screwworm fly |

**TABLE 7**

| Thysanoptera (Thrips) |
|---|
| Maize |
| *Anaphothrips obscurus,* grass thrips |

| Wheat |
|---|
| *Frankliniella fusca,* tobacco thrips |

| Cotton |
|---|
| *Thrips tabaci,* onion thrips |
| *Frankliniella fusca,* tobacco thrips |

| Soybean |
|---|
| *Sericothrips variabilis*, soybean thrips |
| *Thrips tabaci,* onion thrips |

**TABLE 8**

| Hymenoptera (Sawflies, Ants, Wasps, etc.) |
|---|
| Maize |
| *Solenopsis milesta*, thief ant |

| Wheat |
|---|
| *Cephus cinctus,* wheat stem sawfly |

**TABLE 9**

| Other Orders and Representative Species |
|---|
| *Dermaptera* (Earwigs) |
| *Forficula auricularia,* European earwig |

| *Isoptera* (Termites) |
|---|
| *Reticulitermes flavipes,* eastern subterranean termite |

| *Mallophaga* (Chewing Lice) |
|---|
| *Cuclotogaster heterographa,* chicken head louse |
| *Bovicola bovis*, cattle biting |

| *Anoplura* (Sucking Lice) |
|---|
| *Pediculus humanus,* head and body louse |

| *Siphonaptera* (Fleas) |
|---|
| *Ctenocephalides felis*, cat flea |

**TABLE 10**

| Acari (Mites and Ticks) |
|---|
| Maize |
| *Tetranychus urticae,* twospotted spider mite |

| Sorghum |
|---|
| *Tetranychus cinnabarinus,* carmine spider mite |
| *Tetranychus urticae,* twospotted spider mite |

| Wheat |
|---|
| *Aceria tulipae,* wheat curl mite |

| Cotton |
|---|
| *Tetranychus cinnabarinus,* carmine spider mite |
| *Tetranychus urticae,* twospotted spider mite |

| Soybean |
|---|
| *Tetranychus turkestani,* strawberry spider mite |
| *Tetranychus urticae,* twospotted spider mite |

| Barley |
|---|
| *Petrobia latens,* brown wheat mite |

| Important human and animal Acari |
|---|
| *Demacentor variabilis,* American dog tick |
| *Argas persicus,* fowl tick |
| *Dermatophagoides farinae,* American house dust mite |
| *Dermatophagoides pteronyssinus,* European house dust mite |

Now that it has been recognized that pesticidal proteins can be isolated from the vegetative growth phase of *Bacillus*, other strains can be isolated by standard techniques and tested for activity against particular plant and non-plant pests. Generally Bacillus strains can be isolated from any environmental sample, including soil, plant, insect, grain elevator dust, and other sample material, etc., by methods known in the art. See, for example, Travers *et al.* (1987) Appl. Environ. Microbiol. 53:1263-1266; Saleh *et al.* (1969) Can J. Microbiol. 15:1101-1104; DeLucca *et al.* (1981) Can. J. Microbiol. 27:865-870; and Norris, *et al.* (1981) "The genera *Bacillus* and *Sporolactobacillus,"* In Starr *et al.* (eds.), The Prokaryotes: A Handbook on Habitats, Isolation, and Identification of Bacteria, Vol. II, Springer-Verlog Berlin Heidelberg. After isolation, strains can be tested for pesticidal activity during vegetative growth. In this manner, new pesticidal proteins and strains can be identified.

Such *Bacillus* microorganisms which find use in the invention include Bacillus cereus and *Bacillus thuringiensis,* as well as those *Bacillus* species listed in Table 11.

**TABLE 11**

| List of Bacillus species |
|---|
| Morphological Group 1 |
| *B. megaterium* |
| *B. cereus** |
| *B. cereus var. mycoides* |
| *B. thuringiensis** |
| *B. licheniformis* |
| *B. subtilis** |
| *B. pumilus* |
| *B. firmus** |
| *B. coagulans* |

| Morphological Group 2 |
|---|
| *B. polymyxa* |
| *B. macerans* |
| *B. circulans* |
| *B. stearothermophilus* |
| *B. alvei** |
| *B. laterosporus** |
| *B. brevis* |
| *B. pulvifaciens* |
| *B. popilliae** |
| *B. lentimorbus** |
| *B. larvae** |

| Morphological Group 3 |
|---|
| *B. sphaericus** |
| *B. pasteurii* |

| Unassigned Strains |
|---|
| Subgroup A |
| *B. apiarus** |
| *B. filicolonicus* |
| *B. thiaminolyticus* |
| *B. alcalophilus* |

| Subgroup B |
|---|
| *B. cirroflagellosus* |
| *B. chitinosporus* |
| *B. lentus* |

| Subgroup C |
|---|
| *B. badius* |
| *B. aneurinolyticus* |
| *B. macroides* |
| *B. freundenreichii* |

| Subgroup D |
|---|
| *B. pantothenticus* |
| *B. epiphytus* |

| Subgroup E1 |
|---|
| *B. aminovorans* |
| *B. globisporus* |
| *B. insolitus* |
| *B. psychrophilus* |

| Subgroup E2 |
|---|
| *B. psychrosaccharolyticus* |
| *B. macquariensis* |

| |
|---|
| *=Those *Bacillus* strains that have been previously found associated with insects Grouping according to Parry, J.M. et al. (1983) Color Atlas of *Bacillus* species, Wolfe Medical Publications, London. |

In accordance with the present invention, the pesticidal proteins produced during vegetative growth can be isolated from *Bacillus*. In one embodiment, insecticidal proteins produced during vegetative growth, can be isolated. Methods for protein isolation are known in the art. Generally, proteins can be purified by conventional chromatography, including gel-filtration, ion-exchange, and immunoaffinity chromatography, by high-performance liquid chromatography, such as reversed-phase high-performance liquid chromatography, ion-exchange high-performance liquid chromatography, size-exclusion high-performance liquid chromatography, high-performance chromatofocusing and hydrophobic interaction chromatography, etc., by electrophoretic separation, such as one-dimensional gel electrophoresis, two-dimensional gel electrophoresis, etc. Such methods are known in the art. See for example Current Protocols in Molecular Biology, Vols. 1 and 2, Ausubel et al. (eds.), John Wiley & Sons, NY (1988). Additionally, antibodies can be prepared against substantially pure preparations of the protein. See, for example, Radka et al. (1983) J. Immunol. 128:2804; and Radka et al. (1984) Immunogenetics 19:63. Any combination of methods may be utilized to purify protein having pesticidal properties. As the protocol is being formulated, pesticidal activity is determined after each purification step.

Such purification steps will result in a substantially purified protein fraction. By "substantially purified" or "substantially pure" is intended protein which is substantially free of any compound normally associated with the protein in its natural state. "Substantially pure" preparations of protein can be assessed by the absence of other detectable protein bands following SDS-PAGE as determined visually or by densitometry scanning. Alternatively, the absence of other amino-terminal sequences or N-terminal residues in a purified preparation can indicate the level of purity. Purity can be verified by rechromatography of "pure" preparations showing the absence of other peaks by ion exchange, reverse phase or capillary electrophoresis. The terms "substantially pure" or "substantially purified" are not meant to exclude artificial or synthetic mixtures of the proteins with other compounds. The terms are also not meant to exclude the presence of minor impurities which do not interfere with the biological activity of the protein, and which may be present, for example, due to incomplete purification.

Once purified protein is isolated, the protein, or the polypeptides of which it is comprised, can be characterized and sequenced by standard methods known in the art. For example, the purified protein, or the polypeptides of which it is comprised, may be fragmented as with cyanogen bromide, or with proteases such as papain, chymotrypsin, trypsin, lysyl-C endopeptidase, etc. (Oike et al. (1982) J. Biol. Chem. 257:9751-9758; Liu *et al.* (1983) Int. J. Pept. Protein Res. 21:209-215). The resulting peptides are separated, preferably by HPLC, or by resolution of gels and electroblotting onto PVDF membranes, and subjected to amino acid sequencing. To accomplish this task, the peptides are preferably analyzed by automated sequenators. It is recognized that N-terminal, C-terminal, or internal amino acid sequences can be determined. From the amino acid sequence of the purified protein, a nucleotide sequence can be synthesized which can be used as a probe to aid in the isolation of the gene encoding the pesticidal protein.

It is recognized that the pesticidal proteins may be oligomeric and will vary in molecular weight, number of protomers, component peptides, activity against particular pests, and in other characteristics. However, by the methods set forth herein, proteins active against a variety of pests may be isolated and characterized.

Once the purified protein has been isolated and characterized it is recognized that it may be altered in various ways including amino acid substitutions, deletions, truncations, and insertions. Methods for such manipulations are generally known in the art. For example, amino acid sequence variants of the pesticidal proteins can be prepared by mutations in the DNA. Such variants will possess the desired pesticidal activity. Obviously, the mutations that will be made in the DNA encoding the variant must not place the sequence out of reading frame and preferably will not create complementary regions that could produce secondary mRNA structure. See, EP Patent Application Publication No. 75,444.

In this manner, the present invention encompasses the pesticidal proteins as well as components and fragments thereof. That is, it is recognized that component protomers, polypeptides or fragments of the proteins may be produced which retain pesticidal activity. These fragments include truncated sequences, as well as N-terminal, C-terminal, internal and internally deleted amino acid sequences of the proteins.

Most deletions, insertions, and substitutions of the protein sequence are not expected to produce radical changes in the characteristics of the pesticidal protein. However, when it is difficult to predict the exact effect of the substitution, deletion, or insertion in advance of doing so, one skilled in the art will appreciate that the effect will be evaluated by routine screening assays.

The proteins or other component polypeptides described herein may be used alone or in combination. That is, several proteins may be used to control different insect pests.

Some proteins are single polypeptide chains while many proteins consist of more than one polypeptide chain, i.e., they are oligomeric. Additionally, some VIPs are pesticidally active as oligomers. In these instances, additional protomers are utilized to enhance the pesticidal activity or to activate pesticidal proteins. Those protomers which enhance or activate are referred to as auxiliary proteins. Auxiliary proteins activate or enhance a pesticidal protein by interacting with the pesticidal protein to form an oligomeric protein having increased pesticidal activity compared to that observed in the absence of the auxiliary protein.

Auxiliary proteins activate or increase the activity of pesticidal proteins such as the VIP1 protein from AB78. Such auxiliary proteins are exemplified by, but not limited to, the VIP2 protein from AB78. As demonstrated in the Experimental section of the application, auxiliary proteins can activate a number of pesticidal proteins. Thus, in one embodiment of the invention, a plant, Parent 1, can be transformed with an auxiliary protein. This Parent 1 can be crossed with a number of Parent 2 plants transformed with one or more pesticidal proteins whose pesticidal activities are activated by the auxiliary protein.

Amongst the pesticidal proteins of the invention a new class of insect-specific proteins could be surprisingly identified within the scope of the present invention. The said proteins, which are designated throughout this application as VIP3, can be obtained from *Bacillus* spp strains, but preferably from *Bacillus thuringiensis* strains and most preferably from *Bacillus thuringiensis* strains AB88 and AB424. The said VIPs are present mostly in the supernatants of *Bacillus* cultures amounting to at least 75% of the total in strain AB88. The VlP3 proteins are further characterized by their unique spectrum of insectical acitivity, which includes an activity against *Agrotis* and/or *Spodoptera* species, but especially a black cutworm [BCW] and/or fall armyworm and/or beet armyworm and/or tobacco budworm and/or corn earworm activity.

Black cutworm is an agronomically important insect quite resistant to δ-endotoxins. MacIntosh et al (1990) J lnvertebr Pathol 56, 258-266 report that the δ-endotoxins CrylA(b) and CrylA(c) possesses insecticidal properties against BCW with LC₅₀ of more than 80 µg and 18 µg/ml of diet respectively. The vip3A insecticidal proteins according to the invenition provide >50% mortality when added in an amount of protein at least 10 to 500, preferably 50 to 350, and more preferably 200 to 300 fold lower than the amount of CrylA proteins needed to achieve just 50% mortality. Especially preferred within the invention are vip3A insecticidal proteins which provide 100% mortality when added in an amount of protein at least 260 fold lower than the amount of CrylA proteins needed to achieve just 50% mortality.

The vip3 insecticidal proteins according to the invention are present mostly in the supernatants of the cultures and are therefore are to be classified as secreted proteins. They preferably contain in the N-terminal sequence a number of positively charged residues followed by a hydrophobic core region and are not N-terminally processed during export.

As the other pesticidal proteins reported hereto within the scope of the invention, the VIP3 proteins can be detected in growth stages prior to sporulation establishing a further clear distinction from other proteins that belong to the δ-endotoxin family. Preferably, expression of the insect-specific protein starts during mid-log phase and continues during sporulation. Owing to the specific expression pattern in combination with the high stability of the VIP3 proteins, large amounts of the VIP3 proteins can be found in supernatants of sporulating cultures. Especially preferred are the VIP3 proteins identified in SEQ ID NO:29 and SEQ ID NO:32 and the corresponding DNA molecules comprising nucleotide sequences encoding the said proteins, but especially those DNA molecules comprising the nucleotide sequences given in SEQ lD NO:28, SEQ ID NO:30 and SEQ ID NO:31.

The pesticidal proteins of the invention can be used in combination with Bt endotoxins or other insecticidal proteins to increase insect target range. Furthermore, the use of the VIPs of the present invention in combination with Bt δ-endotoxins or other insecticidal principles of a distinct nature has particular utility for the prevention and/or management of insect resistance. Other insecticidal principles include protease inhibitors (both serine and cysteine types), lectins, α-amylase and peroxidase. In one preferred embodiment, expression of VIPs in a transgenic plant is accompanied by the expression of one or more Bt δ-endotoxins. This co-expression of more than one insecticidal principle in the same transgenic plant can be achieved by genetically engineering a plant to contain and express all the genes necessary. Alternatively, a plant, Parent 1, can be genetically engineered for the expression of VIPs. A second plant, Parent 2, can be genetically engineered for the expression of Bt δ-endotoxin. By crossing Parent 1 with Parent 2, progeny plants are obtained which express all the genes introduced into Parents 1 and 2. Particularly preferred Bt δ-endotoxins are those disclosed in EP-A 0618976, herein incorporated by reference.

A substantial number of cytotoxic proteins, though not all, are binary in action. Binary toxins typically consist of two protein domains, one called the A domain and the other called the B domain (see Sourcebook of Bacterial Protein Toxins, J. E. Alouf and J. H. Freer eds.(1991) Academic Press). The A domain possesses a potent cytotoxic activity. The B domain binds an external cell surface receptor before being internalized. Typically, the cytotoxic A domain must be escorted to the cytoplasm by a translocation domain. Often the A and B domains are separate polypeptides or protomers, which are associated by a protein-protein interaction or a di-sulfide bond. However, the toxin can be a single polypeptide which is proteolytically processed within the cell into two domains as in the case for *Pseudomonas* exotoxin A. In summary binary toxins typically have three important domains, a cytotoxic A domain, a receptor binding B domain and a translocation domain. The A and B domain are often associated by protein-protein interacting domains.

The receptor binding domains of the present invention are useful for delivering any protein, toxin, enzyme, transcription factor, nucleic acid, chemical or any other factor into target insects having a receptor recognized by the receptor binding domain of the binary toxins described in this patent. Similarly, since binary toxins have translocation domains which penetrate phosopholipid bilayer membranes and escort cytotoxins across those membranes, such translocation domains may be useful in escorting any protein, toxin, enzyme, transcription factor, nucleic acid, chemical or any other factor across a phospholipid bilayer such as the plasma membrane or a vesicle membrane. The translocation domain may itself perforate membranes, thus having toxic or insecticidal properties. Further, all binary toxins have cytotoxic domains; such a cytotoxic domain may be useful as a lethal protein, either alone or when delivered into any target cell(s) by any means.

Finally, since binary toxins comprised of two polypeptides often form a complex, it is likely that there are protein-protein interacting regions within the components of the binary toxins of the invention. These protein-protein interacting domains may be useful in forming associations between any combination of toxins, enzymes, transcription factors, nucleic acids, antibodies, cell binding moieties, or any other chemicals, factors, proteins or protein domains.

Toxins, enzymes, transcription factors, antibodies, cell binding moieties or other protein domains can be fused to pesticidal or auxiliary proteins by producing in frame genetic fusions which, when translated by ribosomes, would produce a fusion protein with the combined attributes of the VIP and the other component used in the fusion. Furthermore, if the protein domain fused to the VIP has an affinity for another protein, nucleic acid, carbohydrate, lipid, or other chemical or factor, then a three-component complex can be formed. This complex will have the attributes of all of its components. A similar rationale can be used for producing four or more component complexes. These complexes are useful as insecticidal toxins, pharmaceuticals, laboratory reagents, and diagnostic reagents, etc. Examples where such complexes are currently used are fusion toxins for potential cancer therapies, reagents in ELISA assays and immunoblot analysis.

One strategy of altering pesticidal or auxiliary proteins is to fuse a 15-amino-acid "S-tag" to the protein without destroying the insect cell binding domain(s), translocation domains or protein-protein interacting domains of the proteins. The S-tag has a high affinity (K_{d} = 10⁻⁹ M) for a ribonuclease S-protein, which, when bound to the S-tag, forms an active ribonuclease (See F. M. Richards and H. W. Wyckoff (1971) in "The Enzymes", Vol. IV (Boyer, P.D. ed.). pp. 647-806. Academic Press, New York). The fusion can be made in such a way as to destroy or remove the cytotoxic activity of the pesticidal or auxiliary protein, thereby replacing the VIP cytotoxic activity with a new cytotoxic ribonuclease activity. The final toxin would be comprised of the S-protein, a pesticidal protein and an auxiliary protein, where either the pesticidal protein or the auxiliary protein is produced as translational fusions with the S-tag. Similar strategies can be used to fuse other potential cytotoxins to pesticidal or auxiliary proteins including (but not limited to) ribosome inactivating proteins, insect hormones, hormone receptors, transcription factors, proteases, phosphatases, *Pseudomonas* exotoxin A, or any other protein or chemical factor that is lethal when delivered into cells. Similarly, proteins can be delivered into cells which are not lethal, but might alter cellular biochemistry or physiology.

The spectrum of toxicity toward different species can be altered by fusing domains to pesticidal or auxiliary proteins which recognize cell surface receptors from other species. Such domains might include (but are not limited to) antibodies, transferrin, hormones, or peptide sequences isolated from phage displayed affinity selectable libraries. Also, peptide sequences which are bound to nutrients, vitamins, hormones, or other chemicals that are transported into cells could be used to alter the spectrum of toxicity. Similarly, any other protein or chemical which binds a cell surface receptor or the membrane and could be internalized might be used to alter the spectrum of activity of VIP1 and VIP2.

The pesticidal proteins of the present invention are those proteins which confer a specific pesticidal property. Such proteins may vary in molecular weight, having component polypeptides at least a molecular weight of 30 kDa or greater, preferably about 50 kDa or greater.

The auxiliary proteins of the invention may vary in molecular weight, having at least a molecular weight of about 15 kDa or greater, preferably about 20 kDa or greater; more preferably, about 30 kDa or greater. The auxiliary proteins themselves may have component polypeptides.

It is possible that the pesticidal protein and the auxiliary protein may be components of a multimeric, pesticidal protein. Such a pesticidal protein which includes the auxiliary proteins as one or more of its component polypeptides may vary in molecular weight, having at least a molecular weight of 50 kDa up to at least 200 kDa, preferably about 100 kDa to 150 kDa.

An auxiliary protein may be used in combination with the pesticidal proteins of the invention to enhance activity or to activate the pesticidal protein. To determine whether the auxiliary protein will affect activity, the pesticidal protein can be expressed alone and in combination with the auxiliary protein and the respective activities compared in feeding assays for pesticidal activity.

It may be beneficial to screen strains for potential pesticidal activity by testing activity of the strain alone and in combination with the auxiliary protein. In some instances an auxiliary protein in combination with the native proteins of the strains yields pesticidal activity where none is seen in the absence of an auxiliary protein.

The auxiliary protein can be modified, as described above, by various methods known in the art. Therefore, for purposes of the invention, the term "Vegetative Insecticidal Protein" (VIP) encompasses those proteins produced during vegetative growth which alone or in combination can be used for pesticidal activity. This includes pesticidal proteins, auxiliary proteins and those proteins which demonstrate activity only in the presence of the auxiliary protein or the polypeptide components of these proteins.

It is recognized that there are alternative methods available to obtain the nucleotide and amino acid sequences of the present proteins. For example, to obtain the nucleotide sequence encoding the pesticidal protein, cosmid clones, which express the pesticidal protein, can be isolated from a genomic library. From larger active cosmid clones, smaller subclones can be made and tested for activity. In this manner, clones which express an active pesticidal protein can be sequenced to determine the nucleotide sequence of the gene. Then, an amino acid sequence can be deduced for the protein. For general molecular methods, see, for example, Molecular Cloning, A Laboratory Manual, Second Edition, Vols. 1-3, Sambrook *et al.* (eds.) Cold Spring Harbor Laboratory Press, Cold Spring Harbor, NY (1989), and the references cited therein.

The present invention also encompasses nucleotide sequences from organisms other than Bacillus, where the nucleotide sequences are isolatable by hybridization with the Bacillus nucleotide sequences of the invention. Proteins encoded by such nucleotide sequences can be tested for pesticidal activity. The invention also encompasses the proteins encoded by the nucleotide sequences. Furthermore, the invention encompasses proteins obtained from organisms other than *Bacillus* wherein the protein cross-reacts with antibodies raised against the proteins of the invention. Again the isolated proteins can be assayed for pesticidal activity by the methods disclosed herein or others well-known in the art.

Once the nucleotide sequences encoding the pesticidal proteins of the invention have been isolated, they can be manipulated and used to express the protein in a variety of hosts including other organisms, including microorganisms and plants.

The pesticidal genes of the invention can be optimized for enhanced expression in plants. See, for example EP-A 0618976; EP-A 0359472; EP-A 0385962; WO 91/16432; Perlak *et al.* (1991) Proc. Natl. Acad. Sci. USA 88:3324-3328; and Murray et *al.* (1989) Nucleic Acids Research 17: 477-498. In this manner, the genes can be synthesized utilizing plant preferred codons. That is the preferred codon for a particular host is the single codon which most frequently encodes that amino acid in that host. The maize preferred codon, for example, for a particular amino acid may be derived from known gene sequences from maize. Maize codon usage for 28 genes from maize plants is found in Murray *et al*. (1989), Nucleic Acids Research 17:477-498, the disclosure of which is incorporated herein by reference. Synthetic genes can also be made based on the distribution of codons a particular host uses for a particular amino acid.

In this manner, the nucleotide sequences can be optimized for expression in any plant. It is recognized that all or any part of the gene sequence may be optimized or synthetic. That is, synthetic or partially optimized sequences may also be used.

In like manner, the nucleotide sequences can be optimized for expression in any microorganism. For *Bacillus* preferred codon usage, see, for example US Patent No. 5,024,837 and Johansen *et al.* (1988) Gene 65:293-304.

Methodologies for the construction of plant expression cassettes as well as the introduction of foreign DNA into plants are described in the art. Such expression cassettes may include promoters, terminators, enhancers, leader sequences, introns and other regulatory sequences operably linked to the pesticidal protein coding sequence. It is further recognized that promoters or terminators of the VIP genes can be used in expression cassettes.

Generally, for the introduction of foreign DNA into plants Ti plasmid vectors have been utilized for the delivery of foreign DNA as well as direct DNA uptake, liposomes, electroporation, micro-injection, and the use of microprojectiles. Such methods had been published in the art. See, for example, Guerche et al., (1987) Plant Science 52:111-116; Neuhause *et al.,* (1987) Theor. Appl. Genet. 75:30-36; Klein *et al.*, (1987) Nature 327: 70-73; Howell *et al.*, (1980) Science 208:1265; Horsch et al., (1985) Science 227: 1229-1231; DeBlock et *al.,* (1989) Plant Physiology 91:694-701; Methods for Plant Molecular Biology (Weissbach and Weissbach, eds.) Academic Press, Inc. (1988); and Methods in Plant Molecular Biology (Schuler and Zielinski, eds.) Academic Press, Inc. (1989). See also US patent application serial no. 08/008,374 herein incorporated by reference. See also, EP-A 0193259 and EP-A 0451878. It is understood that the method of transformation will depend upon the plant cell to be transformed.

It is further recognized that the components of the expression cassette may be modified to increase expression. For example, truncated sequences, nucleotide substitutions or other modifications may be employed. See, for example Perlak et al. (1991) Proc. Natl. Acad. Sci. USA 88:3324-3328; Murray et al., (1989) Nucleic Acids Research 17:477-498; and WO 91/16432.

The construct may also include any other necessary regulators such as terminators, (Guerineau et al., (1991), Mol. Gen. Genet., 226:141-144; Proudfoot, (1991), Cell, 64:671-674; Sanfacon *et al.*, (1991), Genes Dev., 5:141-149; Mogen *et al.*, (1990), Plant Cell, 2:1261-1272; Munroe *et al.*, (1990), Gene, 91:151-158; Ballas *et al et al.*, (1989), Nucleic Acids Res., 17:7891-7903; Joshi *et al.*, (1987), Nucleic Acid Res., 15:9627-9639); plant translational consensus sequences (Joshi, C.P., (1987), Nucleic Acids Research, 15:6643-6653), introns (Luehrsen and Walbot, (1991), Mol. Gen. Genet., 225:81-93) and the like, operably linked to the nucleotide sequence. It may be beneficial to include 5' leader sequences in the expression cassette construct. Such leader sequences can act to enhance translation. Translational leaders are known in the art and include:
Picornavirus leaders, for example, EMCV leader (encephalomyocarditis 5' noncoding region) (Elroy-Stein, O., Fuerst, T.R., and Moss, B. (1989) PNAS USA 86:6126-6130);
Potyvirus leaders, for example, TEV leader (Tobacco Etch Virus) (Allison *et al.*, (1986); MDMV leader (Maize Dwarf Mosaic Virus); Virology, 154:9-20), and
Human immunoglobulin heavy-chain binding protein (BiP), (Macejak, D.G., and Sarnow, P., (1991), Nature, 353:90-94;
Untranslated leader from the coat protein mRNA of alfalfa mosaic virus (AMV RNA 4), (Jobling, S.A., and Gehrke, L., (1987), Nature, 325:622-625;
Tobacco mosaic virus leader (TMV), (Gallie, D.R. *et al.,* (1989), Molecular Biology of RNA, pages 237-256; and
Maize Chlorotic Mottle Virus leader (MCMV) (Lommel, S.A. *et al.,* (1991), Virology, 81:382-385. See also, Della-Cioppa *et al.*, (1987), Plant Physiology, 84:965-968.

A plant terminator may be utilized in the expression cassette. See, Rosenberg *et al.*, (1987), Gene, 56:125; Guerineau *et al.,* (1991), Mol. Gen. Genet., 226:141-144; Proudfoot, (1991), Cell, 64:671-674; Sanfacon *et al.,* (1991), Genes Dev., 5:141-149; Mogen *et al.,* (1990), Plant Cell, 2:1261-1272; Munroe *et al.,* (1990), Gene, 91:151-158; Ballas *et al*., (1989), Nucleic Acids Res., 17:7891-7903; Joshi *et al.,* (1987), Nucleic Acid Res., 15:9627-9639.

For tissue specific expression, the nucleotide sequences of the invention can be operably linked to tissue specific promoters. See, for example, EP-A 0618976, herein incorporated by reference.

Further comprised within the scope of the present invention are transgenic plants, in particular transgenic fertile plants transformed by means of the aforedescribed processes and their asexual and/or sexual progeny, which comprise and preferably also express the pesticidal protein according to the invention. Especially preferred are hybrid plants.

The transgenic plant according to the invention may be a dicotyledonous or a monocotyledonous plant. Preferred are monocotyledonous plants of the *Graminaceae* family involving *Lolium, Zea, Triticum, Triticale, Sorghum, Saccharum, Bromus, Oryzae, Avena, Hordeum, Secale* and *Setaria* plants.

Especially preferred are transgenic maize, wheat, barley, sorghum, rye, oats, turf grasses and rice.

Among the dicotyledonous plants soybean, cotton, tobacco, sugar beet, oilseed rape, and sunflower are especially preferred herein.

The expression 'progeny' is understood to embrace both, "asexually" and "sexually" generated progeny of transgenic plants. This definition is also meant to include all mutants and variants obtainable by means of known processes, such as for example cell fusion or mutant selection and which still exhibit the characteristic properties of the initially transformed parent plant, together with all crossing and fusion products of the transformed plant material.

Another object of the invention concerns the proliferation material of transgenic plants.

The proliferation material of transgenic plants is defined relative to the invention as any plant material that may be propagated sexually or asexually *in vivo* or *in vitro.* Particularly preferred within the scope of the present invention are protoplasts, cells, calli, tissues, organs, seeds, embryos, pollen, egg cells, zygotes, together with any other propagating material obtained from transgenic plants.

Parts of plants, such as for example flowers, stems, fruits, leaves, roots originating in transgenic plants or their progeny previously transformed by means of the process of the invention and therefore consisting at least in part of transgenic cells, are also an object of the present invention.

Before the plant propagation material [fruit, tuber, grains, seed], but expecially seed is sold as a commerical product, it is customarily treated with a protectant coating comprising herbicides, insecticides, fungicides, bactericides, nematicides, molluscicides or mixtures of several of these preparations, if desired together with further carriers, surfactants or application-promoting adjuvants customarily employed in the art of formulation to provide protection against damage caused by bacterial, fungal or animal pests.

In order to treat the seed, the protectant coating may be applied to the seeds either by impregnating the tubers or grains with a liquid formulation or by coating them with a combined wet or dry formulation. In addition, in special cases, other methods of application to plants are possible, eg treatment directed at the buds or the fruit.

The plant seed according to the invention comprising a DNA molecule comprising a nucleotide sequence encoding a pesticidal protein according to the invention may be treated with a seed protectant coating comprising a seed treatment compound, such as, for example, captan, carboxin, thiram (TMTD® ), methalaxyl (Apron® ) and pirimiphos-methyl (Actellic® ) and others that are commonly used in seed treatment. Preferred within the scope of the invention are seed protectant coatings comprising an entomocidal composition according to the invention alone or in combination with one of the a seed protectant coating customarily used in seed treatment.

It is thus a further object of the present invention to provide plant propagation material for cultivated plants, but especially plant seed that is treated with a seed protectant coating as defined hereinbefore.

It is recognized that the genes encoding the pesticidal proteins can be used to transform insect pathogenic organisms. Such organisms include Baculoviruses, fungi, protozoa, bacteria and nematodes.

The *Bacillus* strains of the invention may be used for protecting agricultural crops and products from pests. Alternatively, a gene encoding the pesticide may be introduced via a suitable vector into a microbial host, and said host applied to the environment or plants or animals. Microorganism hosts may be selected which are known to occupy the "phytosphere" (phylloplane, phyllosphere, rhizosphere, and/or rhizoplana) of one or more crops of interest. These microorganisms are selected so as to be capable of successfully competing in the particular environment with the wild-type microorganisms, provide for stable maintenance and expression of the gene expressing the polypeptide pesticide, and, desirably, provide for improved protection of the pesticide from environmental degradation and inactivation.

Such microorganisms include bacteria, algae, and fungi. Of particular interest are microorganisms, such as bacteria, e.g., *Pseudomonas, Erwinia, Serratia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc,* and *Alcaligenes;* fungi, particularly yeast, e.g., *Saccharomyces, Cryptococcus, Kluyveromyces, Sporobolomyces, Rhodotorula,* and *Aureobasidium.* Of particular interest are such phytosphere bacterial species as *Pseudomonas syringae, Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacter xyli and Azotobacter vinlandii;* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces rosues, S. odorus, Kluyveromyces veronae,* and *Aureobasidium pollulans.* Of particular interest are the pigmented microorganisms.

A number of ways are available for introducing a gene expressing the pesticidal protein into the microorganism host under conditions which allow for stable maintenance and expression of the gene. For example, expression cassettes can be constructed which include the DNA constructs of interest operably linked with the transcriptional and translational regulatory signals for expression of the DNA constructs, and a DNA sequence homologous with a sequence in the host organism, whereby integration will occur, and/or a replication system which is functional in the host, whereby integration or stable maintenance will occur.

Transcriptional and translational regulatory signals include but are not limited to promoter, transcriptional initiation start site, operators, activators, enhancers, other regulatory elements, ribosomal binding sites, an initiation codon, termination signals, and the like. See, for example, US Patent 5,039,523; US Patent No. 4,853,331; EPO 0480762A2; Sambrook *et al.* supra; Molecular Cloning, a Laboratory Manual, Maniatis *et al*. (eds) Cold Spring Harbor Laboratory, Cold Spring Harbor, NY (1982); Advanced Bacterial Genetics, Davis *et al*. (eds.) Cold Spring Harbor Laboratory, Cold Spring Harbor, NY (1980); and the references cited therein.

Suitable host cells, where the pesticide-containing cells will be treated to prolong the activity of the toxin in the cell when the then treated cell is applied to the environment of the target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include *Enterobacteriaceae,* such as *Escherichia, Erwinia, Shigella, Salmonella, and Proteus; Bacillaceae; Rhizobiceae,* such as *Rhizobium; Spirillaceae,* such as *photobacterium, Zymomonas, Serratia, Aeromonas, Vibrio, Desulfovibrio, Spirillum; Lactobacillaceae; Pseudomonadaceae,* such as *Pseudomonas* and *Acetobacter, Azotobacteraceae* and *Nitrobacteraceae.* Among eukaryotes are fungi, such as *Phycomycetes* and *Ascomycetes,* which includes yeast, such a *Saccharomyces* and *Schizosaccharromyces*; and *Basidiomycetes* yeast, such as *Rhodotorula, Aureobasidium, Sporobolomyces,* and the like.

Characteristics of particular interest in selecting a host cell for purposes of production include ease of introducing the protein gene into the host, availability of expression systems, efficiency of expression, stability of the protein in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as *Rhodotorula sp., Aureobasidium sp., Saccharomyces sp., and Sporobolomyces sp.; phylloplane* organisms such as *Pseudomonas sp., Erwinia sp.* and *Flavobacterium sp.;* or such other organisms as *Escherichia, LactoBacillus sp., Bacillus sp.,* and the like. Specific organisms include *Pseudomonas aeurginosa, Pseudomonas fluorescens, Saccharomyces cerevisiae, Bacillus thuringiensis, Escherichia coli, Bacillus subtilis,* and the like.

VIP genes can be introduced into micro-organisms that multiply on plants (epiphytes) to deliver VIP proteins to potential target pests. Epiphytes can be gram-positive or gram-negative bacteria for example.

Root colonizing bacteria, for example, can be isolated from the plant of interest by methods known in the art. Specifically, a *Bacillus cereus* strain which colonizes roots could be isolated from roots of a plant ( for example see J. Handelsman, S. Raffel, E. Mester, L. Wunderlich and C. Grau, Appl. Environ. Microbiol. 56:713-718, (1990)). VIP1 and/or VIP2 and/or VIP3 could be introduced into a root colonizing *Bacillus cereus* by standard methods known in the art.

Specifically, VIP1 and/or VIP2 derived from *Bacillus cereus* strain AB78 can be introduced into a root colonizing *Bacillus* cereus by means of conjugation using standard methods (J. Gonzalez, B. Brown and B. Carlton, Proc. Natl. Acad. Sci. 79:6951-6955, (1982)).

Also, VIP1 and/or VIP2 and/or VIP3 or other VIPs of the invention can be introduced into the root colonizing *Bacillus* by means of electro-transformation. Specifically, VIPs can be cloned into a shuttle vector, for example, pHT3101 (D. Lereclus *et al.,* FEMS Microbiol. Letts., 60:211-218 (1989)) as described in Example 10. The shuttle vector pHT3101 containing the coding sequence for the particular VIP can then be transformed into the root colonizing *Bacillus* by means of electroporation (D. Lereclus *et* al. 1989, FEMS Microbiol. Letts. 60:211-218).

Expression systems can be designed so that VIP proteins are secreted outside the cytoplasm of gram negative bacteria, *E. coli*, for example. Advantages of having VIP proteins secreted are (1) it avoids potential toxic effects of VIP proteins expressed within the cytoplasm and (2) it can increase the level of VIP protein expressed and (3) can aid in efficient purification of VIP protein.

VIP proteins can be made to be secreted in *E. coli*, for example, by fusing an appropriate E. *coli* signal peptide to the amino-terminal end of the VIP signal peptide or replacing the VIP signal peptide with the *E. coli* signal peptide. Signal peptides recognized by *E. coli* can be found in proteins already known to be secreted in *E. coli,* for example the OmpA protein (J. Ghrayeb, H. Kimura, M. Takahara, Y. Masui and M. Inouye, EMBO J., 3:2437-2442 (1984)). OmpA is a major protein of the *E. coli* outer membrane and thus its signal peptide is thought to be efficient in the translocation process. Also, the OmpA signal peptide does not need to be modified before processing as may be the case for other signal peptides, for example lipoprotein signal peptide ( G. Duffaud, P. March and M. Inouye, Methods in Enzymology, 153:492 (1987)).

Specifically, unique BamHl restriction sites can be introduced at the amino-terminal and carboxy-terminal ends of the VIP coding sequences using standard methods known in the art. These BamHl fragments can be cloned, in frame, into the vector plN-III-ompA1, A2 or A3 (J. Ghrayeb, H. Kimura, M. Takahara, H. Hsiung, Y. Masui and M. lnouye, EMBO J., 3:2437-2442 (1984)) thereby creating ompA:VIP fusion gene which is secreted into the periplasmic space. The other restriction sites in the polylinker of pIN-III-ompA can be eliminated by standard methods known in the art so that the VIP amino-terminal amino acid coding sequence is directly after the ompA signal peptide cleavage site. Thus, the secreted VIP sequence in *E. coli* would then be identical to the native VIP sequence.

When the VIP native signal peptide is not needed for proper folding of the mature protein, such signal sequences can be removed and replaced with the ompA signal sequence. Unique BamHl restriction sites can be introduced at the amino-termini of the proprotein coding sequences directly after the signal peptide coding sequences of VIP and at the carboxy-termini of VIP coding sequence. These BamHl fragments can then be cloned into the pIN-III-ompA vectors as described above.

General methods for employing the strains of the invention in pesticide control or in engineering other organisms as pesticidal agents are known in the art. See, for example US Patent No. 5,039,523 and EP 0480762A2.

VIPs can be fermented in a bacterial host and the resulting bacteria processed and used as a microbial spray in the same manner that *Bacillus thuringiensis* strains have been used as insecticidal sprays. In the case of a VIP(s) which is secreted from *Bacillus,* the secretion signal is removed or mutated using procedures known in the art. Such mutations and/or deletions prevent secretion of the VIP protein(s) into the growth medium during the fermentation process. The VIPs are retained within the cell and the cells are then processed to yield the encapsulated VIPs. Any suitable microorganism can be used for this purpose. *Psuedomonas* has been used to express *Bacillus thuringiensis* endotoxins as encapsulated proteins and the resulting cells processed and sprayed as an insecticide. (H. Gaertner *et al*. 1993, In Advanced Engineered Pesticides, L. Kim ed.)

Various strains of *Bacillus thuringiensis* are used in this manner. Such Bt strains produce endotoxin protein(s) as well as VIPs. Alternatively, such strains can produce only VIPs. A sporulation deficient strain of *Bacillus subtilis* has been shown to produce high levels of the CryIIIA endotoxin from *Bacillus thuringiensis* (Agaisse, H. and Lereclus, D., "Expression in *Bacillus subtilis* of the *Bacillus thuringiensis CrylllA* toxin gene is not dependent on a sporulation-specific sigma factor and is increased in a spoOA mutant", J. Bacteriol., 176:4734-4741 (1994)). A similar *spoOA* mutant can be prepared in *Bacillus thuringiensis* and used to produce encapsulated VIPs which are not secreted into the medium but are retained within the cell.

To have VIPs maintained within the *Bacillus* cell the signal peptide can be disarmed so that it no longer functions as a secretion signal. Specifically, the putative signal peptide for VIP1 encompasses the first 31 amino acids of the protein with the putative consensus cleavage site, Ala-X-Ala, at the C-terminal portion of this sequence (G. von Heijne , J. Mol. Biol. 184:99-105 (1989)) and the putative signal peptide for VIP2 encompasses the first 40 amino acids of the protein with the putative cleavage site after Ala40. The cleavage sites in either VIP1 or VIP2 can be mutated with methods known in the art to replace the cleavage site consensus sequence with alternative amino acids that are not recognized by the signal peptidases.

Alternatively, the signal peptides of VIP1, VIP2 and/or other VIPs of the invention can be eliminated from the sequence thereby making them unrecognizable as secretion proteins in *Bacillus.* Specifically, a methionine start site can be engineered in front of the proprotein sequence in VIP1, starting at Asp32, or the proprotein sequence in VIP2, starting at Glu41 using methods known in the art.

VIP genes can be introduced into micro-organisms that mutiply on plants (epiphytes) to deliver VIP proteins to potential target pests. Epiphytes can be gram-positive or gram-negative bacteria for example.

The *Bacillus* strains of the invention or the microorganisms which have been genetically altered to contain the pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticides are produced by introducing a heterologous gene into a cellular host. Expression of the heterologous gene results, directly or indirectly, in the intracellular production and maintenance of the pesticide. These cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein.

The active ingredients of the present invention are normally applied in the form of compositions and can be applied to the crop area or plant to be treated, simultaneously or in succession, with other compounds. These compounds can be both fertilizers or micronutrient donors or other preparations that influence plant growth. They can also be selective herbicides, insecticides, fungicides, bactericides, nematicides, mollusicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

Preferred methods of applying an active ingredient of the present invention or an agrochemical composition of the present invention which contains at least one of the insect-specific proteins produced by the bacterial strains of the present invention are leaf application, seed coating and soil application. The number of applications and the rate of application depend on the intensity of infestation by the corresponding pest.

The present invention thus further provides an entomocidal composition comprising as an active ingrdient at least one of the novel insect-specific proteins according to the invention and/or a recombinant microorganism containing at least one DNA molecule comprising a nucleotide sequence encoding the novel insect-specific proteins in recombinant form, but especially a recombinant *Bacillus spp* strain, such as *Bacillus cereus* or *Bacillus thuringiensis*, containing at least one one DNA molecule comprising a nucleotide sequence encoding the novel insect-specific proteins in recombinant form, or a derivative or mutant thereof, together with an agricultural adjuvant such as a carrier, diluent, surfactant or application-promoting adjuvant. The composition may also contain a further biologically active compound. The said compound can be both a fertilizer or micronutrient donor or other preparations that influence plant growth. It can also be a selective herbicide, insecticide, fungicide, bactericide, nematicide, molluscide or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers

The composition may comprise from 0.1 to 99% by weight of the active ingredient, from 1 to 99.9% by weight of a solid or liquid adjuvant, and from 0 to 25% by weight of a surfactant. The acitve ingredient comprising at least one of the novel insect-specific proteins according to the invention or a recombinant microorganism containing at least one DNA molecule comprising a nucleotide sequence encoding the novel insect-specific proteins in recombinant form, but especially a recombinant *Bacillus spp strain,* such as *Bacillus cereus* or *Bacillus thuringiensis* strain containing at least one DNA molecule comprising a nucleotide sequence encoding the novel insect-specific proteins in recombinant form, or a derivative or mutant thereof, or the composition containing the said acitve ingredient, may be administered to the plants or crops to be protected together with certain other insecticides or chemicals (1993 Crop Protection Chemicals Reference, Chemical and Pharmaceutical Press, Canada) without loss of potency. It is compatible with most other commonly used agricultural spray materials but should not be used in extremely alkaline spray solutions. It may be administered as a dust, a suspension, a wettable powder or in any other material form suitable for agricultural application.

The invention further provides methods for for controlling or inhibiting of insect pests by applying an active ingredient comprising at least one of the novel insect-specific proteins according to the invention or a recombinant microorganism containing at least one DNA molecule comprising a nucleotide sequence encoding the novel insect-specific proteins in recombinant form or a composition comprising the said active ingredient to (a) an environment in which the insect pest may occur, (b) a plant or plant part in order to protect said plant or plant part from damage caused by an insect pest, or (c) seed in order to protect a plant which develops from said seed from damage caused by an insect pest.

A preferred method of application in the area of plant protection is application to the foliage of the plants (foliar application), with the number of applications and the rate of application depending on the plant to be protected and the risk of infestation by the pest in question. However, the active ingredient may also penetrate the plants through the roots (systemic action) if the locus of the plants is impregnated with a liquid formulation or if the active ingredient is incorporated in solid form into the locus of the plants, for example into the soil, e.g. in granular form (soil application). In paddy rice crops, such granules may be applied in metered amounts to the flooded rice field.

The compositions according to the invention are also suitable for protecting plant propagating material, e.g. seed, such as fruit, tubers or grains, or plant cuttings, from insect pests. The propagation material can be treated with the formulation before planting: seed, for example, can be dressed before being sown. The acitve ingredient of the invention can also be applied to grains (coating), either by impregnating the grains with a liquid formulation or by coating them with a solid formulation. The formulation can also be applied to the planting site when the propagating material is being planted, for example to the seed furrow during sowing. The invention relates also to those methods of treating plant propagation material and to the plant propagation material thus treated.

The compositions according to the invention comprising as an active ingredient a recombinant microorganism containing at least one of the novel toxin genes in recombinant form, but especially a recombinant *Bacillus spp strain*, such as *Bacillus cereus* or *Bacillus thuringiensis* strain containing at least one DNA molecule comprising a nucleotide sequence encoding the novel insect-specific proteins in recombinant form, or a derivative or mutant thereof may be applied in any method known for treatment of seed or soil with bacterial strains. For example, see US Patent No.4,863,866. The strains are effective for biocontrol even if the microorganism is not living. Preferred is, however, the application of the living microorganism.

Target crops to be protected within the scope of the present invention comprise, e.g., the following species of plants:
cereals (wheat, barley, rye, oats, rice, sorghum and related crops), beet (sugar beet and fodder beet), forage grasses (orchardgrass, fescue, and the like), drupes, pomes and soft fruit (apples, pears, plums, peaches, almonds, cherries, strawberries, raspberries and blackberries), leguminous plants (beans, lentils, peas, soybeans), oil plants (rape, mustard, poppy, olives, sunflowers, coconuts, castor oil plants, cocoa beans, groundnuts), cucumber plants (cucumber, marrows, melons) fiber plants (cotton, flax, hemp, jute), citrus fruit (oranges, lemons, grapefruit, mandarins), vegetables (spinach, lettuce, asparagus, cabbages and other Brassicae, onions, tomatoes, potatoes, paprika), lauraceae (avocados, carrots, cinnamon, camphor), deciduous trees and conifers (e.g. linden-trees, yew-trees, oak-trees, alders, poplars, birch-trees, firs, larches, pines), or plants such as maize, tobacco, nuts, coffee, sugar cane, tea, vines, hops, bananas and natural rubber plants, as well as ornamentals (including composites).

A recombinant *Bacillus spp strain,* such as *Bacillus cereus* or *Bacillus thuringiensis* strain, containing at least one DNA molecule comprising a nucleotide sequence encoding the novel insect-specific proteins in recombinant form is normally applied in the form of entomocidal compositions and can be applied to the crop area or plant to be treated, simultaneously or in succession, with further biologically active compounds. These compounds may be both fertilizers or micronutrient donors or other preparations that influence plant growth. They may also be selective herbicides, insecticides, fungicides, bactericides, nematicides, molluscicides or mixtures of several of these preparations, if desired together with further carriers, surfactants or application-promoting adjuvants customarily employed in the art of formulation.

The active ingredient according to the invention may be used in unmodified form or together with any suitable agriculturally acceptable carrier. Such carriers are adjuvants conventionally employed in the art of agricultural formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objective and the prevailing circumstances. Advantageous rates of application are normally from about 50 g to about 5 kg of active ingredient (a.i.) per hectare ("ha", approximately 2.471 acres), preferably from about 100 g to about 2kg a.i./ha. Important rates of application are about 200 g to about 1 kg a.i./ha and 200g to 500g a.i./ha.
For seed dressing advantageous application rates are 0.5 g to 1000 g a.i.per 100 kg seed, preferably 3 g to 100 g a.i. per 100 kg seed or 10 g to 50 g a.i.per 100 kg seed.

Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. The formulations, i.e. the entomocidal compositions, preparations or mixtures containing the recombinant *Bacillus spp strain*, such as *Bacillus cereus* or *Bacillus thuringiensis* strain containing at least one DNA molecule comprising a nucleotide sequence encoding the novel insect-specific proteins in recombinant form as an active ingredient or combinations thereof with other active ingredients, and, where appropriate, a solid or liquid adjuvant, are prepared in known manner, e.g., by homogeneously mixing and/or grinding the active ingredients with extenders, e.g., solvents, solid carriers, and in some cases surface-active compounds (surfactants).

Suitable solvents are: aromatic hydrocarbons, preferably the fractions containing 8 to 12 carbon atoms, e.g. xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol monomethyl or monoethyl ether, ketones such as cyclohexanone, strongly polar solvents such as N-methyl-2-pyrrolidone, dimethylsulfoxide or dimethylformamide, as well as vegetable oils or epoxidised vegetable oils such as epoxidised coconut oil or soybean oil; or water.

The solid carriers used, e.g., for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredients to be formulated, suitable surface-active compounds are non-ionic, cationic and/or anionic surfactants having good emulsifying, dispersing and wetting properties. The term "surfactants" will also be understood as comprising mixtures of surfactants. Suitable anionic surfactants can be both water-soluble soaps and
water-soluble synthetic surface-active compounds. Suitable soaps are the alkali metal salts, alkaline earth metal salts or unsubstituted or substituted ammonium salts of higher fatty acids (C₁₀ -C₂₂), e.g. the sodium or potassium salts of oleic or stearic acid, or of natural fatty acid mixtures which can be obtained, e.g. from coconut oil or tallow oil. Further suitable surfactants are also the fatty acid methyltaurin salts as well as modified and unmodified phospholipids.

More frequently, however, so-called synthetic surfactants are used, especially fatty sulfonates, fatty sulfates, sulfonated benzimidazole derivatives or alkylarylsulfonates. The fatty sulfonates or sulfates are usually in the forms of alkali metal salts, alkaline earth metal salts or unsubstituted or substituted ammonium salts and generally contain a C₈ -C₂₂ alkyl radical which also includes the alkyl moiety of acyl radicals, e.g. the sodium or calcium salt of lignosulfonic acid, of dodecylsulfate, or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing about 8 to 22 carbon atoms. Examples of alkylarylsulfonates are the sodium, calcium or triethanolamine salts of dodecylbenzenesulfonic acid, dibutylnaphthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide.
Non-ionic surfactant are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit. Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxypolyethoxyethanol. Fatty acid esters of polyoxyethylene sorbitan, such as polyoxyethylene sorbitan trioleate, are also suitable non-ionic surfactants.

Cationic surfactants are preferably quaternary ammonium salts which contain, as N-substituent, at least one C₈ -C₂₂ alkyl radical and, as further substituents, lower unsubstituted or halogenated alkyl, benzyl or hydroxyl-lower alkyl radicals. The salts are preferably in the form of halides, methylsulfates or ethylsulfates, e.g., stearyltrimethylammonium chloride or benzyldi-(2-chloroethyl)ethylammonium bromide.

The surfactants customarily employed in the art of formulation are described, e.g., in "McCutcheon's Detergents and Emulsifiers Annual", MC Publishing Corp. Ridgewood, N.J., 1979; Dr. Helmut Stache, "Tensid Taschenbuch" (Handbook of Surfactants), Carl Hanser Verlag, MunichVienna.

Another particularly preferred characteristic of an entomocidal composition of the present invention is the persistence of the active ingredient when applied to plants and soil. Possible causes for loss of activity include inactivation by ultra-violet light, heat, leaf exudates and pH. For example, at high pH, particularly in the presence of reductant, δ-endotoxin crystals are solubilized and thus become more accessible to proteolytic inactivation. High leaf pH might also be important, particularly where the leaf surface can be in the range of pH 8-10. Formulation of an entomocidal composition of the present invention can address these problems by either including additives to help prevent loss of the active ingredient or encapsulating the material in such a way that the active ingredient is protected from inactivation. Encapsulation can be accomplished chemically (McGuire and Shasha, J Econ Entomol 85: 1425-1433, 1992) or biologically (Barnes and Cummings, 1986; EP-A 0 192 319). Chemical encapsulation involves a process in which the active ingredient is coated with a polymer while biological encapsulation involves the expression of the δ-endotoxin genes in a microbe. For biological encapsulation, the intact microbe containing at least one DNA molecule comprising a nucleotide sequence encoding the novel insect-specific proteins in recombinant form is used as the active ingredient in the formulation. The addition of UV protectants might effectively reduce irradiation damage. lnactivation due to heat could also be controlled by including an appropriate additive.

Preferred within the present application are formulations comprising living microorganisms as active ingredient either in form of the vegetative cell or more preferable in form of spores, if available. Suitable formulations may consist, for example, of polymer gels which are crosslinked with polyvalent cations and comprise these microorganisms. This is described, for example, by D.R. Fravel et al. in Phytopathology, Vol. 75, No. 7, 774-777, 1985 for alginate as the polymer material. It is also known from this publication that carrier materials can be co-used. These formulations are as a rule prepared by mixing solutions of naturally occurring or synthetic gel-forming polymers, for example alginates, and aqueous salt solutions of polyvalent metal ions such that individual droplets form, it being possible for the microorganisms to be suspended in one of the two or in both reaction solutions. Gel formation starts with the mixing in drop form. Subsequent drying of these gel particles is possible. This process is called ionotropic gelling. Depending on the degree of drying, compact and hard particles of polymers which are structurally crosslinked via polyvalent cations and comprise the microorganisms and a carrier present predominantly uniformly distributed are formed. The size of the particles can be up to 5 mm.

Compositions based on partly crosslinked polysaccharides which, in addition to a microorganism, for example, can also comprise finely divided silicic acid as the carrier material, crosslinking taking place, for example, via Ca ⁺⁺ ions, are described in EP-A1-0 097 571. The compositions have a water activity of not more than 0.3. W.J. Cornick et al. describe in a review article [New Directions in Biological Control: Alternatives for Suppressing Agricultural Pests and Diseases, pages 345-372, Alan R. Liss, Inc. (1990)] various formulation systems, granules with vermiculite as the carrier and compact alginate beads prepared by the ionotropic gelling process being mentioned. Such compositions are also disclosed by D.R.Fravel in Pesticide Formulations and Application Systems: 11 th Volume, ASTM STP 1112 American Society for Testing and Materials, Philadelphia, 1992, pages 173 to 179 and can be used to formulate the recombinant microorganisms according to the invention.

The entomocidal compositions of the invention usually contain from about 0.1 to about 99%, preferably about 0.1 to about 95%, and most preferably from about 3 to about 90% of the active ingredient, from about 1 to about 99.9%, preferably from about 1 to about 99%, and most preferably from about 5 to about 95% of a solid or liquid adjuvant, and from about 0 to about 25%, preferably about 0.1 to about 25%, and most preferably from about 0.1 to about 20% of a surfactant.

In a preferred embodiment of the invention the entomocidal compositions usually contain 0.1 to 99%, preferably 0.1 to 95%, of a recombinant *Bacillus spp strain,* such as *Bacillus cereus or Bacillus thuringiensis* strain containing at least one DNA molecule comprising a nucleotide sequence encoding the novel insect-specific proteins in recombinant form, or combination thereof with other active ingredients, 1 to 99.9% of a solid or liquid adjuvant, and 0 to 25%, preferably 0.1 to 20%, of a surfactant.

Whereas commercial products are preferably formulated as concentrates, the end user will normally employ dilute formulations of substantially lower concentration. The entomocidal compositions may also contain further ingredients, such as stabilizers, antifoams, viscosity regulators, binders, tackifiers as well as fertilizers or other active ingredients in order to obtain special effects.

In one embodiment of the invention a *Bacillus cereus* microorganism has been isolated which is capable of killing *Diabrotica virgifera virgifera,* and *Diabrotica longicornis barberi.* The novel *B. cereus* strain AB78 has been deposited in the Agricultural Research Service, Patent Culture Collection (NRRL), Northern Regional Research Center, 1815 North University Street, Peoria, IL 61604, USA and given Accession No. NRRL B-21058.

A fraction protein has been substantially purified from the *B. cereus* strain. This purification of the protein has been verified by SDS-PAGE and biological activity. The protein has a molecular weight of about 60 to about 100 kDa, particularly about 70 to about 90 kDa, more particularly about 80 kDa, hereinafter VIP.

Amino-terminal sequencing has revealed the N-terminal amino-acid sequence to be:
NH₂-Lys-Arg-Glu-Ile-Asp-Glu-Asp-Thr-Asp-Thr-Asx-Gly-Asp-Ser-Ile-Pro-(SEQ ID NO:8) where Asx represents either Asp or Asn. The entire amino acid sequence is given in SEQ ID NO:7. The DNA sequence which encodes the amino acid sequence of SEQ ID NO:7 is disclosed in SEQ ID NO:6.

An oligonuleotide probe for the region of the gene encoding amino acids 3-9 of the NH₂-terminus has been generated. The probe was synthesized based on the codon usage of a *Bacillus thuringiensis* (Bt) δ-endotoxin gene. The nucleotide sequence of the oligonucleotide probe used for Southern hybridizations was as follows:
5'- GAA ATT GAT CAA GAT ACN GAT -3' (SEQ ID NO:9) where N represents any base.

In addition, the DNA probe for the Bc AB78 VIP1 gene described herein, permits the screening of any *Bacillus* strain or other organisms to determine whether the VIP1 gene (or related gene) is naturally present or whether a particular transformed organism includes the VIP1 gene.

The invention now being generally described, the same will be better understood by reference to the following detailed examples that are provided for the purpose of illustration and are not to be considered limiting of the invention unless so specified.

A standard nomenclature has been developed based on the sequence identity of the proteins encompassed by the present invention. The gene and protein names for the detailed examples which follow and their relationship to the names used in the parent application [US application serial no 314594/08] are shown below.

| **Gene / Protein Name under Standard Nomenclature** | **Gene / Protein Name in Parent** | **Description of Protein** |
|---|---|---|
| VIP1A(a) | VIP1 | VIP1 from strain AB78 as disclosed in SEQ ID NO:5. |
| VIP2A(a) | VIP2 | VIP2 from strain AB78 as disclosed in SEQ ID NO:2. |
| VIP1A(b) | VIP1 homolog | VIP1 from *Bacillus thuringiensis* var. *tenebrionis* as disclosed in SEQ ID NO:21. |
| VIP2A(b) | VIP2 homolog | VIP2 from *Bacillus thuringiensis* var. *tenebrionis* as disclosed in SEQ ID NO:20. |
| VIP3A(a) | -- | VIP from strain AB88 as disclosed in SEQ ID NO:28 of the present application |
| VIP3A(b) | -- | VIP from strain AB424 as disclosed in SEQ ID NO:31 of the present application |

### EXPERIMENTAL

### Formulation Examples

The active ingredient used in the following formulation examples are *Bacillus cereus* strain AB78 having Accession No. NRRL B-21058; *Bacillus thuringiensis* strains having Accession Nos. NRRL B-21060, NRRL B-21224, NRRL B-21225, NRRL B-21226, NRRL B-21227, and NRRL B-21439; and *Bacillus spp* strains having Accession Nos NRRL B-21228, NRRL B-21229, and NRRL B-21230. All the mentioned strains are natural isolates comprising the insect-specific proteins according to the invention. Alternatively, the isolated insect-specific proteins are used as the active ingredient alone or in combination with the above-mentioned *Bacillus strains.*

### A1. Wettable powders

| | a) | b) | c) |
|---|---|---|---|
| *Bacillus thuringiensis* spores | 25% | 50% | 75% |
| sodium lignosufonate | 5% | 5% | -- |
| sodium laurylsulfate | 3% | -- | 5% |
| sodium diisobutylnaphthalenesulfonate | -- | 6% | 10% |
| octylphenol polyethylene glycol ether (7-8 moles of ethylene oxid) | -- | 2% | -- |
| highly dispersed silicid acid | 5% | 10% | 10% |
| kaolin | 62% | 27% | -- |

The spores are thoroughly mixed with the adjuvants and the mixture is thoroughly ground in a suitable mill, affording wettable powders which can be diluted with water to give suspensions of the desired concentrations.

### A2. Emulsifiable concentrate

| | |
|---|---|
| *Bacillus thuringiensis* spores | 10% |
| octylphenol polyethylene glycol ether (4-5 moles ethylene oxide) | 3% |
| clacium dodecylbenzensulfonate | 3% |
| castor oil polyglycol ether (36 moles of ethylene oxide) | 4% |
| cyclohexanone | 30% |
| xylene mixture | 50% |

Emulsions of any required concentration can be obtained from this concentrate by dilution with water.

### A3. Dusts

| | a) | b) |
|---|---|---|
| *Bacillus thuringiensis* spores | 5% | 8% |
| talcum | 95% | -- |
| kaolin | -- | 92% |

Ready for use dusts are obtained by mixing the active ingredient with the carriers and grinding the mixture in a suitable mill.

### A4. Extruder Granulate

| | |
|---|---|
| *Bacillus thuringiensis* spores | 10% |
| sodium lignosulfonate | 2% |
| carboxymethylcellulose | 1% |
| kaolin | 87% |

The active ingredient or combination is mixed and ground with the adjuvants and the mixture is subsequently moistened with water. The mixture is extruded, granulated and the dried in a stream of air.

### A5. Coated Granule

| | |
|---|---|
| *Bacillus thuringiensis* spores | 3% |
| polyethylene glycol (mol wt 200) | 3% |
| kaolin | 94% |

The active ingredient or combination is uniformly applied in a mixer to the kaolin moistened with polyethylene glycol. Non-dusty coated granulates are obtained in this manner.

### A6. Suspension Concentrate

| | |
|---|---|
| *Bacillus thuringiensis* spores | 40% |
| ethylene glycol | 10% |
| nonylphenol polyethylene glycol ether (15 moles of ethylene oxide) | 6% |
| sodium lignosulfonate | 10% |
| carboxymethylcellulose | 1 % |
| 37% aqueous formaldehyde solution | 0.2% |
| silicone oil in the form of a 75% aqueous solution | 0.8% |
| water | 32% |

The active ingredient or combination is intimately mixed with the adjuvants giving a suspension concentrate from which suspensions of any desired concentration can be obtained by dilution with water.

### EXAMPLE 1. AB78 ISOLATION AND CHARACTERIZATION

*Bacillus cereus* strain AB78 was isolated as a plate contaminant in the laboratory on T3 media (per liter: 3 g tryptone, 2 g tryptose, 1.5 g yeast extract, 0.05 M sodium phosphate (pH 6.8), and 0.005 g MnCl₂; Travers, R.S. 1983). During log phase growth, AB78 gave significant activity against western corn rootworm. Antibiotic activity against gram-positive *Bacillus spp.* was also demonstrated (Table 12).

**TABLE 12**

| **Antibiotic activity of AB78 culture supernatant** | | |
|---|---|---|
| | Zone of inhibition(cm) | |
| Bacteria tested | AB78 | Streptomycin |
| *E. coli* | 0.0 | 3.0 |
| *B. megaterium* | 1.1 | 2.2 |
| *B. mycoides* | 1.3 | 2.1 |
| *B. cereus* CB | 1.0 | 2.0 |
| *B. cereus* 11950 | 1.3 | 2.1 |
| *B. cereus* 14579 | 1.0 | 2.4 |
| *B. cereus* AB78 | 0.0 | 2.2 |
| *Bt var. israelensis* | 1.1 | 2.2 |
| *Bt var. tenebrionis* | 0.9 | 2.3 |

Morphological characteristics of AB78 are as follows:
Vegetative rods straight, 3.1-5.0 mm long and 0.5-2.0 mm wide. Cells with rounded ends, single in short chains. Single subterminal, cylindrical-oval, endospore formed per cell. No parasporal crystal formed. Colonies opaque, erose, lobate and flat. No pigments produced. Cells motile. Flagella present.
Growth characteristics of AB78 are as follows:
Facultative anaerobe with optimum growth temperature of 21-30°C. Will grow at 15, 20, 25, 30 and 37°C. Will not grow above 40°C. Grows in 5-7% NaCl.

Table 13 provides the biochemical profile of AB78.

**TABLE 13**

| **Biochemical characteristics of B. cereus strain AB78.** | | | |
|---|---|---|---|
| Acid from L-arabinose | - | Methylene blue reoxidized | + |
| Gas from L-arabinose | | - Nitrate reduced | + |
| Acid from D-xylose | - | NO₃ reduced to NO₂ | + |
| Gas from D-xylose - | - | VP | + |
| Acid from D-glucose | + | H₂O₂ decomposed | + |
| Gas from D-glucose- | Indole | | - |
| Acid from lactose | - | Tyrosine decomposed | + |
| Gas from lactose | - | Dihydroxiacetone | - |
| Acid from sucrose | - | Litmus milk acid | - |
| Gas from sucrose | - | Litmus milk coagulated- | |
| Acid from D-mannitol | - | Litmus milk alkaline | - |
| Gas from D-mannitol | - | Litmus milk peptonized- | |
| Proprionate utilization | + | Litmus milk reduced | - |
| Citrate utilization | + | Casein hydrolyzed | + |
| Hippurate hydrolysis | w | Starch hydrolyzed | + |
| Methylene blue reduced | + | Gelatin liquidified | + |
| Lecithinase produced | w | | |
| w= weak reaction | | | |

### EXAMPLE 2. BACTERIAL CULTURE

A subculture of Bc strain AB78 was used to inoculate the following medium, known as TB broth:

| | |
|---|---|
| Tryptone | 12 g/l |
| Yeast Extract | 24 g/l |
| Glycerol | 4 ml/l |
| KH₂PO₄ | 2.1 g/l |
| K₂HPO₄ | 14.7 g/l |
| pH 7.4 | |

The potassium phosphate was added to the autoclaved broth after cooling. Flasks were incubated at 30°C on a rotary shaker at 250 rpm for 24 h-36 h, which represents an early to mid-log growth phase.

The above procedure can be readily scaled up to large fermentors by procedures well known in the art.

During vegetative growth, usually 24-36 h. after starting the culture, which represents an early to mid-log growth phase, AB78 bacteria were centrifuged from the culture supernatant. The culture supernatant containing the active protein was used in bioassays.

### EXAMPLE 3. INSECT BIOASSAYS

B. cereus strain AB78 was tested against various insects as described below.

Western, Northern and Southern corn rootworm, *Diabrotica virgifera virgifera,* D. *longcornis barberi* and *D. undecempunctata howardi*, respectively: dilutions were made of AB78 culture supernatant grown 24-36 h., mixed with molten artificial diet (Marrone *et al.* (1985) J. of Economic Entomology 78:290-293) and allowed to solidify. Solidified diet was cut and placed in dishes. Neonate larvae were placed on the diet and held at 30°C. Mortality was recorded after 6 days.

*E. coli* clone bioassay: *E. coli* cells were grown overnight in broth containing 100 µg/ml ampicillin at 37°C. Ten ml culture was sonicated 3X for 20 sec each. 500 µl of sonicated culture was added to molten western corn rootworm diet.

Colorado potato beetle, *Leptinotarsa decemlineata:* dilutions in Triton X-100 (to give final concentration of 0.1 % TX-100) were made of AB78 culture supernatant grown 24-36 h. Five cm² potato leaf pieces were dipped into these dilutions, air dried, and placed on moistened filter paper in plastic dishes. Neonate larvae were placed on the leaf pieces and held at 30°C. Mortality was recorded after 3-5 days.

Yellow mealworm, *Tenebrio molitor.* dilutions were made of AB78 culture supernatant grown 24-36 h., mixed with molten artificial diet (Bioserv #F9240) and allowed to solidify. Solidified diet was cut and placed in plastic dishes. Neonate larvae were placed on the diet and held at 30°C. Mortality was recorded after 6-8 days.

European corn borer, black cutworm, tobacco budworm, tobacco hornworm and beet armyworm; *Ostrinia nubilalis, Agrotis ipsilon, Heliothis virescens, Manduca sexta* and *Spodoptera exigua*, respectively: dilutions, in TX-100 (to give final concentration of 0.1 % TX-100), were made of AB78 culture supernatant grown 24-36 hrs. 100 µl was pipetted onto the surface of 18 cm of solidified artificial diet (Bioserv #F9240) and allowed to air dry. Neonate larvae were then placed onto the surface of the diet and held at 30°C. Mortality was recorded after 3-6 days.

Northern house mosquito, *Culex pipiens*:-dilutions were made of AB78 culture supernatant grown 24-36 h. 100 µl was pipetted into 10 ml water in a 30 ml plastic cup. Third instar larvae were added to the water and held at room temperature. Mortality was recorded after 24-48 hours. The spectrum of entomocidal activity of AB78 is given in Table 14.

**TABLE 14**

| Activity of AB78 culture supernatant against various insect species | | |
|---|---|---|
| Insect species tested to date | Order | Activity |
| Western corn rootworm (Diabrotica *virgifera virgifera)* | Col | +++ |
| Northern corn rootworm *(Diabrotica longicornis barberi)* | Col | +++ |
| Southern corn rootworm *(Diabrotica undecimpunctata* howardi) | Col | - |
| Colorado potato beetle (*Leptinotarsa decemlineata*) Yellow mealworm | Col | - |
| (Tenebrio molitor) | Col | - |
| European corn borer *(Ostrinia nubilalis)* | Lep | - |
| Tobacco budworm *(Heliothis virescens)* | Lep | - |
| Tobacco hornworm | | |
| (*Manduca sexta*) | Lep | - |
| Beet armyworm (*Spodoptera exigua*) | Lep | - |
| Black cutworm (*Agrotis ipsilon*) | Lep | - |
| Northern house mosquito (*Culex pipiens*) | Dip | - |

The newly discovered *B. cereus* strain AB78 showed a significantly different spectrum of insecticidal activity as compared to known coleopteran active δ-endotoxins from Bt. In particular, AB78 showed more selective activity against beetles than known coleopteran-active Bt strains in that it was specifically active against *Diabrotica* spp. More specifically, it was most active against *D. virgifera virgifera* and *D. longicornis barberi* but not *D. undecimpunctata howardi.*

A number of *Bacillus* strains were bioassayed for activity during vegetative growth (Table 15) against western corn rootworm. The results demonstrate that AB78 is unique in that activity against western corn rootworm is not a general phenomenon.

**TABLE 15**

| **Activity of culture supernatants from various Bacillus spp. against western corn rootworm** | |
|---|---|
| *Bacillus strain* | Percent WCRW mortality |
| *B. cereus* AB78 (Bat.1 ) | 100 |
| *B. cereus* AB78 (Bat.2) | 100 |
| *B. cereus* (Carolina Bio.) | 12 |
| *B. cereus* ATCC 11950 | 12 |
| *B. cereus* ATCC 14579 | 8 |
| *B. mycoides* (Carolina Bio.) | 30 |
| *B. popilliae* | 28 |
| *B. thuringiensis* HD135 | 41 |
| *B. thuringiensis* HD191 | 9 |
| *B. thuringiensis* GC91 | 4 |
| *B. thuringiensis isrealensis* | 24 |
| Water Control | 4 |

Specific activity of AB78 against western corn rootworm is provided in Table 16.

**TABLE 16**

| **Activity of AB78 culture supernatant against neonate western corn rootworm** | |
|---|---|
| Culture supernatant concentration (µl/ml) | Percent WCRW mortality |
| 100 | 100 |
| 25 | 87 |
| 10 | 80 |
| 5 | 40 |
| 2.5 | 20 |
| 1 | 6 |
| 0 | 0 |

The LC₅₀ was calculated to be 6.2 µl of culture supernatant per ml of western corn rootworm diet.

The cell pellet was also bioassayed and had no activity against WCRW. Thus, the presence of activity only in the supernatant indicates that this VIP is an exotoxin.

### EXAMPLE 4. ISOLATION AND PURIFICATION OF CORN ROOTWORM ACTIVE PROTEINS FROM AB78.

Culture media free of cells and debris was made to 70% saturation by the addition of solid ammonium sulfate (472 g/L). Dissolution was at room temperature followed by cooling in an ice bath and centrifugation at 10,000 X g for thirty minutes to pellet the precipitated proteins. The supernatant was discarded and the pellet was dissolved in 1/10 the original volume of 20 mM TRIS-HCl at pH 7.5. The dissolved pellet was desalted either by dialysis in 20 mM TRIS-HCl pH 7.5, or passing through a desalting column.

The desalted material was titrated to pH 3.5 using 20 mM sodium citrate pH 2.5. Following a thirty minute room temperature incubation the solution was centrifuged at 3000 X g for ten minutes. The supernatant at this stage contained the greatest amount of active protein.

Following neutralization of the pH to 7.0 the supernatant was applied to a Mono-Q, anion exchange, column equilibrated with 20 mM TRIS pH 7.5 at a flow rate of 300 mL/min. The column was developed with a stepwise and linear gradient employing 400 mM NaCl in 20 mM TRIS pH 7.5.

Bioassay of the column fractions and SDS-PAGE analysis were used to confirm the active fractions. SDS-PAGE analysis identified the biologically active protein as having components of a molecular weight in the range of about 80 kDa and 50 kDa.

### EXAMPLE 5. SEQUENCE ANALYSIS OF THE CORN ROOTWORM ACTIVE PROTEIN

The 80 kDa component isolated by SDS-PAGE was transferred to PVDF membrane and was subjected to amino-terminal sequencing as performed by repetitive Edman cycles on an ABI 470 pulsed-liquid sequencer. Transfer was carried out in 10 mM CAPS buffer with 10% methanol pH 11.0 as follows:
Incubation of the gel following electrophoresis was done in transfer buffer for five minutes. ProBlott PVDF membrane was wetted with 100% MeOH briefly then equilibrated in transfer buffer. The sandwich was arranged between foam sponges and filter paper squares with the configuration of cathode-gel-membrane-anode.
Transfer was performed at 70 V constant voltage for 1 hour.
Following transfer, the membrane was rinsed with water and stained for two minutes with 0.25% Coomassie Blue R-250 in 50% MeOH.
Destaining was done with several rinses with 50% MeOH 40% water 10% acetic acid.
Following destaining the membrane was air dried prior to excision of the bands for sequence analysis. A BlottCartridge and appropriate cycles were utilized to achieve maximum efficiency and yield. Data analysis was performed using model 610 Sequence Analysis software for identifying and quantifying the PTH-amino acid derivatives for each sequential cycle.
The N-terminal sequence was determined to be: NH2-Lys-Arg-Glu-Ile-Asp-Glu-Asp-Thr-Asp-Thr-Asx-Gly-Asp-Ser-Ile-Pro- (SEQ ID NO:8) where Asx represents Asp or Asn. The complete amino acid sequence for the 80 kDa component is disclosed in SEQ ID NO:7. The DNA sequence which encodes SEQ ID NO:7 is disclosed in SEQ ID NO:6.

### EXAMPLE 6. CONSTRUCTION OF DNA PROBE

An oligonucleotide probe for the region of the gene encoding amino acids 3-9 of the N-terminal sequence (Example 5) was generated. The probe was synthesized based on the codon usage of a *Bacillus thuringiensis* (Bt) δ-endotoxin gene. The nucleotide sequence 5'- GAA ATT GAT CAA GAT  ACN GAT -3'     (SEQ ID NO:9) was used as a probe in Southern hybridizations. The oligonucleotide was synthesized using standard procedures and equipment.

### EXAMPLE 7. ISOELECTRIC POINT DETERMINATION OF THE CORN ROOTWORM ACTIVE PROTEIN

Purified protein from step 5 of the purification process was analyzed on a 3-9 pl isoelectric focusing gel using the Phastgel electrophoresis system (Pharmacia). Standard operating procedures for the unit were followed for both the separation and silver staining development procedures. The pl was approximated at about 4.9.

### EXAMPLE 8. PCR DATA ON AB78

PCR analysis (See, for example US patent application serial no. 08/008,006; and, Carozzi et al. (1991) Appl. Environ. Microbiol. 57(11 ):3057-3061, herein incorporated by reference.) was used to verify that the *B. cereus* strain AB78 did not contain any insecticidal crystal protein genes of *B. thuringiensis* or *B. sphaericus* (Table 17).

**TABLE 17**

| **Bacillus insecticidal crystal protein gene primers tested by PCR against AB78 DNA.** | |
|---|---|
| Primers Tested | Product Produced |
| 2 sets specific for CrylllA | Negative |
| CrylllB | Negative |
| 2 sets specific for CrylA | Negative |
| CrylA(a) | Negative |
| CrylA(b) specific | Negative |
| CrylB | Negative |
| CrylC specific | Negative |
| CrylE specific | Negative |
| 2 sets specific for B. sphaericus | Negative |
| 2 sets specific for CrylV | Negative |
| *Bacillus* control (PI-PLC) | Positive |

### EXAMPLE 9. COSMID CLONING OF TOTAL DNA FROM B. CEREUS STRAIN AB78

The VIP1A(a) gene was cloned from total DNA prepared from strain AB78 as follows:

### Isolation of AB78 DNA was as follows:

1. Grow bacteria in 10 ml L-broth overnight. (Use 50 ml sterile centrifuge tube)
2. Add 25 ml of fresh L-broth and ampicillin (30 µg/ml).
3. Grow cells 2-6 h. at 30°C with shaking.
4. Spin cells in a 50 ml polypropylene orange cap tube in IEC benchtop clinical centrifuge at 3/4 speed.
5. Resuspend cell pellet in 10 ml TES (TES = 50 mM TRIS pH 8.0, 100 mM EDTA, 15 mM NaCl).
6. Add 30 mg lysozyme and incubate 2 hrs at 37°C.
7. Add 200 µl 20% SDS and 400 µl Proteinase K stock (20 mg/ml). Incubate at 37°C.
8. Add 200 µl fresh Proteinase K. Incubate 1 hr. at 55°C. Add 5 ml TES to make 15 ml final volume.
9. Phenol extract twice (10 ml phenol, spin at room temperature at 3/4 speed in an IEC benchtop clinical centrifuge). Transfer supernatant (upper phase) to a clean tube using a wide bore pipette.
10. Extract once with 1:1 vol. phenol:chloroform/isoamyl alcohol (24:1 ratio).
11. Precipitate DNA with an equal volume of cold isopropanol; Centrifuge to pellet DNA.
12. Resuspend pellet in 5 ml TE.
13. Precipitate DNA with 0.5 ml 3M NaOAc pH 5.2 and 11 ml 95% ethanol. Place at -20°C for 2 h.
14. "Hook" DNA from tube with a plastic loop, transfer to a microfuge tube, spin, pipette off excess ethanol, dry in vacuo.
15. Resuspend in 0.5 ml TE. Incubate 90 min. at 65°C to help get DNA back into solution.
16. Determine concentration using standard procedures.

### Cosmid Cloning of AB78

All procedures, unless indicated otherwise, were performed according to Stratagene Protocol, Supercos 1 Instruction Manual, Cat. No. 251301.

Generally, the steps were as follows:
A. Sau 3A partial digestion of the AB78 DNA.
B. Preparation of vector DNA
C. Ligation and packaging of DNA
D. Tittering the cosmid library
   1. Start a culture of HB101 cells by placing 50 ml of an overnight culture in 5 mls of TB with 0.2% maltose. Incubate 3.5 hrs. at 37°C.
   2. Spin out cells and resuspend in 0.5 ml 10 mM MgSO₄.
   3. Add together:
      100 µl cells
      100 µl diluted packaging mixture
      100 µl 10 mM MgS04
      30 µl TB
   4. Adsorb at room temperature for 30 minutes with no shaking.
   5. Add 1 ml TB and mix gently. Incubate 30 minutes at 37°C.
   6. Plate 200 µl onto L-amp plates. Incubate at 37°C overnight.

At least 400 cosmid clones were selected at random and screened for activity against western corn rootworm as described in Example 3. DNA from 5 active clones and 5 non-active clones were used in Southern hybridizations. Results demonstrated that hybridization using the above described oligonucleotide probe correlated with western corn rootworm activity (Table 18).

Cosmid clones P3-12 and P5-4 have been deposited with the Agricultural Research Service Patent Culture Collection (NRRL) and given Accession Nos. NRRL B-21061 and NRRL B-21059 respectively.

**TABLE 18**

| **Activity of AB78 cosmid clones against western corn rootworm.** | |
|---|---|
| Clone | Mean percent mortality (N=4) |
| Clones which hybridize with probe | |
| P1-73 | 47 |
| P1-83 | 64 |
| P2-2 | 69 |
| P3-12 | 85 |
| P5-4 | 97 |

| Clones which do not hybridize with probe | |
|---|---|
| P1-2 | 5 |
| P3-8 | 4 |
| P3-9 | 12 |
| P3-18 | 0 |
| P4-6 | 9 |

### EXAMPLE 10. IDENTIFICATION OF A 6 KB REGION ACTIVE AGAINST WESTERN CORN ROOTWORM.

DNA from P3-12 was partially digested with restriction enzyme Sau 3A, and ligated into the *E. coli* vector pUC19 and transformed into *E. coli*. A DNA probe specific for the 80 kDa VIP1A(a) protein was synthesized by PCR amplification of a portion of P3-12 DNA. Oligonucleotides MK113 and MK117, which hybridize to portions of VIP1A(a), were synthesized using the partial amino acid sequence of the 80 kDa protein. Plasmid subclones were identified by colony hybridization to the PCR-generated probe, and tested for activity against western corn rootworm. One such clone, PL2, hybridized to the PCR-generated fragment, and was active against western corn rootworm in the assay previously described.

A 6 kb CIa I restriction fragment from pL2 was cloned into the Sma I site of the *E. coli-*_{*-*}*Bacillus* shuttle vector pHT 3101 (Lereclus, D. et al., FEMS Microbiology Letters 60:211-218 (1989)) to yield pCIB6201. This construct confers anti-western corn rootworm activity upon both Bacillus and *E.coli* strains, in either orientation. pCIB6022 contains this same 6 kb *CIa* I fragment in pBluescript SK(+) (Stratagene), produces equivalent VIP1A(a) protein (by western blot), and is also active against western corn rootworm.

The nucleotide sequence of pCIB6022 was determined by the dideoxy termination method of Sanger et al., Proc. Natl. Acad. Sci. USA, 74:5463-5467 (1977), using PRISM Ready Reaction Dye Deoxy Terminator Cycle Sequencing Kits and PRISM Sequenase® Terminator Double-Stranded DNA Sequencing Kit and analyzed on an ABI 373 automatic sequencer. The sequence is given in SEQ ID NO:1. The 6 kb fragment encodes both VIP1A(a) and VIP2A(a), as indicated by the open reading frames described in SEQ ID NO:1. The sequence encoding VIP2A(a) is further disclosed in SEQ ID NO:4. The relationship between VIP1A(a) and VIP2A(a) within the 6 kb fragment found in pCIB6022 is depicted in Table 19. pCIB6022 was deposited with the Agricultural Research Service, Patent Culture Collection, (NRRL), Northern Regional Research Center, 1815 North University Street, Peoria, lllinois 61604, USA, and given the Accession No. NRRL B-21222.

### EXAMPLE 11. FUNCTIONAL DISSECTION OF THE VIP1A(a) DNA REGION.

To confirm that the VIP1A(a) open reading frame (ORF) is necessary for insecticidal activity a translational frameshift mutation was created in the gene. The restriction enzyme Bgl II recognizes a unique site located 857 bp into the coding region of VIP1A(a). pCIB6201 was digested with Bgl II, and the single-stranded ends filled-in with DNA polymerase (Klenow fragment) and dNTPS. The plasmid was re-ligated and transformed into *E. coli.* The resulting plasmid, pCIB6203, contains a four nucleotide insertion in the coding region of VIP1A(a). pCIB6203 does not confer WCRW insecticidal activity, confirming that VIP1A(a) is an essential component of western corn rootworm activity.

To further define the region necessary to encode VIP1A(a), subclones of the VIP1A(a) and VIP2A(a) (auxiliary protein) region were constructed and tested for their ability to complement the mutation in pCIB6203. pCIB6023 contains the 3.7kb Xba I-EcoRV fragment in pBluescript SK(+) (Stratagene). Western blot analysis indicates that pCIB6023 produces VIP1A(a) protein of equal size and quantity as clones PL2 and pCIB6022. pCIB6023 contains the entire gene encoding the 80 kD protein. pCIB6023 was deposited with the Agricultural Research Service, Patent Culture Collection, (NRRL), Northern Regional Research Center, 1815 North University Street, Peoria, lllinois 61604, USA, and given the Accession No. NRRL B-21223N. pCIB6206 contains the 4.3 kb Xba I-Cla fragment from pCIB6022 in pBluescript SK(+) (Stratagene). pCIB6206 was also deposited with the Agricultural Research Service, Patent Culture Collection, (NRRL), Northern Regional Research Center, 1815 North University Street, Peoria, lllinois 61604, USA, and given the Accession No. NRRL B-21321.

pCIB6023, pCIB6206, and pCIB6203 do not produce detectable western corn rootworm activity when tested individually. However, a mixture of cells containing pCIB6203 (VIP1A(a)-mutated, plus VIP2A(a)) and cells containing pCIB6023 (only VIP1A(a)) shows high activity against western corn rootworm. Similarly, a mixture of cells containing pCIB6206 and cells containing pCIB6203 shows high activity against western corn rootworm.

To further define the limits of VIP2A(a), we constructed pCIB6024, which contains the entirety of VIP2A(a), but lacks most of the VIP1A(a) coding region. pCIB6024 was constructed by gel purifying the 2.2 kb Cla I-Sca I restriction fragment from pCIB6022, filling in the single-stranded ends with DNA polymerase (Klenow fragment) and dNTPs, and ligating this fragment into pBluescript SK(+) vector (Stratagene) digested with the enzyme Eco RV. Cells containing pCIB6024 exhibit no activity against western corn rootworm. However, a mixture of cells containing pCIB6024 and cells containing pCIB6023 shows high activity against western corn rootworm .(See Table 19).

Thus, pCIB6023 and pCIB6206 must produce a functional VIP1A(a) gene product, while pCIB6203 and pCIB6024 must produce a functional VIP2A(a) gene product. These results suggest a requirement for a gene product(s) from the VIP2A(a) region, in combination with VIP1A(a), to confer maximal western corn rootworm activity. (See Table 19.)

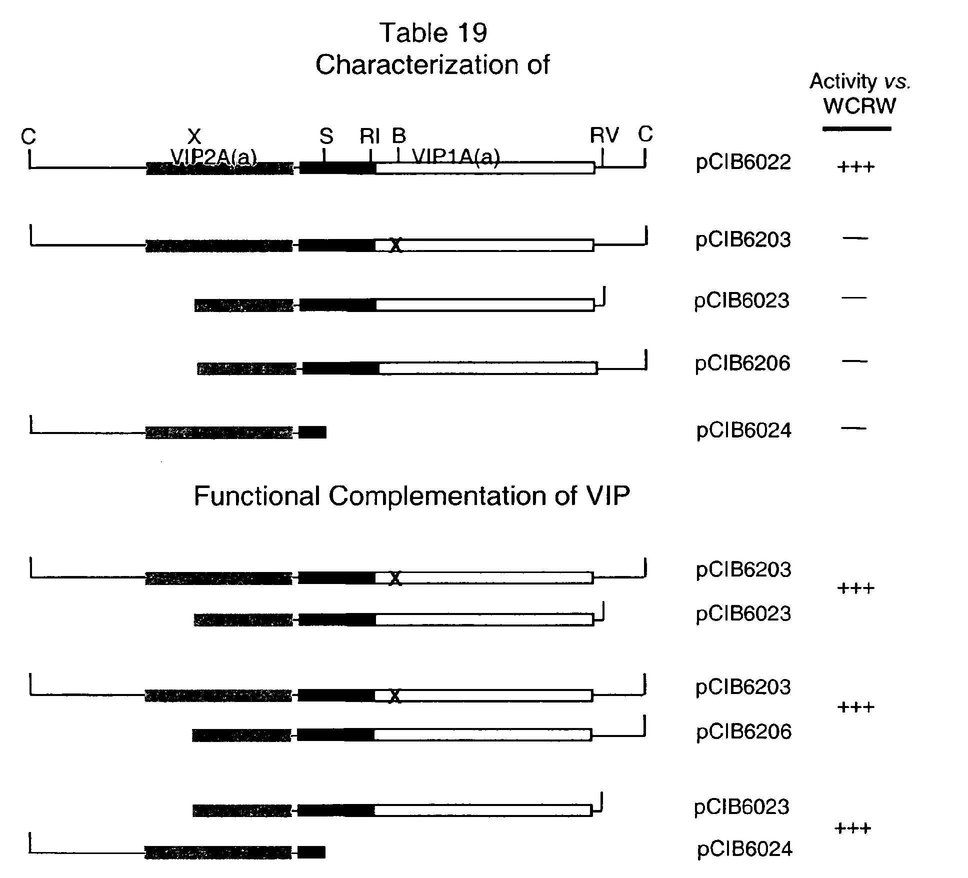

Boxed regions represent the extent of VlP1A(a) and VlP2A(a). White box represents the portion of VlP1 encoding the 80 kDa peptide observed in *Bacillus*. Dark box represents the N-terminal 'propeptide' of VlP1A(a) predicted by DNA sequence analysis. Stippled box represents the VlP2A(a) coding region. Large 'X' represents the location of the frameshift mutation introduced into VlP1A(a). Arrows represent constructs transcribed by the beta-galactosidase promoter.

### EXAMPLE 12. AB78 ANTIBODY PRODUCTION

Antibody production was initiated in 2 Lewis rats to allow for both the possibility of moving to production of hybridoma cell lines and also to produce enough serum for limited screening of genomic DNA library. Another factor was the very limited amount of antigen available and the fact that it could only be produced to purity by PAGE and subsequent electrotransfer to nitrocellulose.

Due to the limited availability of antigen on nitrocellulose, the nitrocellulose was emulsified in DMSO and injected into the hind footpads of the animals to elicit B-cell production in the popliteal lymph nodes just upstream. A strong reacting serum was produced as judged by western blot analysis with the first production bleed. Several subsequent injections and bleeds produced enough serum to accomplish all of the screening required.

Hybridoma production with one of the rats was then initiated. The popliteal lymph node was excised, macerated, and the resulting cells fused with mouse myeloma P3x63Ag8.653. Subsequent cell screening was accomplished as described below. Four initial wells were selected which gave the highest emulsified antigen reaction to be moved to limited dilution cloning. An additional 10 wells were chosen for expansion and cryoperservation.

### Procedure to Emulsify AB78 on nitrocellulose in DMSO for ELISA screening:

After electrotransfer of AB78 samples run on PAGE to nitrocellulose, the reversible strain Ponceau S is used to visualize all protein transferred. The band corresponding to AB78 toxin, previously identified and N-terminal sequenced, was identified and excised from nitrocellulose. Each band is approximately 1 mm x 5 mm in size to minimize the amount of nitrocellulose emulsified. A single band is placed in a microfuge tube with 250 µl of DMSO and macerated using a plastic pestle (Kontes, Vineland, NJ). To aid in emulsification, the DMSO mixture is heated for 2-3 minutes at 37°C-45°C. Some further maceration might be necessary following heating; however, all of the nitrocellulose should be emulsified. Once the AB78 sample is emulsified, it is placed on ice. In preparation for microtiter plate coating with the emulsified antigen, the sample must be diluted in borate buffered saline as follows: 1:5, 1:10, 1:15, 1:20, 1:30, 1:50, 1:100, and 0. The coating antigen must be prepared fresh immediately prior to use.

### ELISA protocol:

1. Coat with AB78/DMSO in BBS. Incubate overnight at 4°C.
2. Wash plate 3X with 1 X ELISA wash buffer.
3. Block (1 % BSA & 0.05% Tween 20 in PBS) for 30 minutes at Room Temperature.
4. Wash plate 3X with 1X ELISA wash buffer.
5. Add rat serum. Incubate 1.5 hours at 37°C.
6. Wash plate 3X with 1 X ELISA wash buffer.
7. Add goat anti-rat at a concentration of 2 µg/ml in ELISA diluent. Incubate 1 hr. at 37°C.
8. Wash plate 3X with 1X ELISA wash buffer.
9. Add rabbit anti-goat alkaline phosphatase at 2 µg/ml in ELISA diluent. Incubate 1 hr. at 37°C.
10. Wash 3X with 1X ELISA wash buffer.
11. Add Substrate. Incubate 30 minutes at room temperature.
12. Stop with 3N NaOH after 30 minutes.

### Preparation of VIP2A(a) Antisera

A partially purified AB78 culture supernatant was separated by discontinuous SDS PAGE (Novex) following manufacturer's instructions. Separated proteins were electrophoresed to nitrocellulose (S&S #21640) as described by Towbin et al., (1979). The nitrocellulose was stained with Ponceau S and the VIP2A(a) band identified. The VIP2A(a) band was excised and emulsified in DMSO immediately prior to injection. A rabbit was initially immunized with emulsified VIP2A(a) mixed approximately 1:1 with Freund's Complete adjuvant by intramuscular injection at four different sites. Subsequent immunizations occurred at four week intervals and were identical to the first, except for the use of Freund' Incomplete adjuvant. The first serum harvested following immunization reacted with VIP2A(a) protein. Western blot analysis of AB78 culture supernatant using this antisera identifies predominately full length VIP2A(a) protein.

### EXAMPLE 13. ACTIVATION OF INSECTICIDAL ACTIVITY OF NON-ACTIVE BT STRAINS WITH AB78 VIP CLONES.

Adding pCIB6203 together with a 24 h culture (early to mid-log phase) supernatant from Bt strain GC91 produces 100% mortality in Diabrotica *virgifera virgifera.* Neither pCIB6203 nor GC91 is active on *Diabrotica virgifera virgifera* by itself. Data are shown below:

| Test material | Percent Diabrotica mortality |
|---|---|
| pCIB6203 | 0 |
| GC91 | 16 |
| pClB6203 + GC91 | 100 |
| Control | 0 |

### EXAMPLE 14. ISOLATION AND BIOLOGICAL ACTIVITY OF B. CEREUS AB81.

A second B. cereus strain, designated AB81, was isolated from grain bin dust samples by standard methodologies. A subculture of AB81 was grown and prepared for bioassay as described in Example 2. Biological activity was evaluated as described in Example 3. The results are as follows:

| Insect species tested | Percent Mortality |
|---|---|
| *Ostrinia nubilalis* | 0 |
| *Agrotis ipsilon* | 0 |
| *Diabrotica virgifera virgifera* | 55 |

### EXAMPLE 15. ISOLATION AND BIOLOGICAL ACTIVITY OF B. THURINGIENSIS AB6.

A *B. thuringiensis* strain, designated AB6, was isolated from grain bin dust samples by standard methods known in the art. A subculture of AB6 was grown and prepared for bioassay as described in Example 2. Half of the sample was autoclaved 15 minutes to test for the presence of β-exotoxin.

Biological activity was evaluated as described in Example 3. The results are as follows:

| Insect species tested | Percent Mortality |
|---|---|
| *Ostrinia nubilalis* | 0 |
| *Agrotis ipsilon* | 100 |
| *Agrotis ipsilon* (autoclaved sample) | 0 |
| *Diabrotica virgifera virgifera* | 0 |

The reduction of insecticidal acitivity of the culture supernatant to insignificant levels by autoclaving indicates that the active principle is not β-exotoxin.

Strain AB6 has been deposited in the Agricultural Research Service, Patent Culture Collection (NRRL), Northern Regional Research Center, 1815 North University Street, Peoria, lllinois 61604, USA, and given Accession No. NRRL B-21060.

### EXAMPLE 16. ISOLATION AND BIOLOGICAL CHARACTERIZATION OF B. THURINGIENSIS AB88.

A Bt strain, designated AB88, was isolated from grain bin dust samples by standard methodologies. A subculture of AB88 was grown and prepared for bioassay as described in Example 2. Half of the sample was autoclaved 15 minutes to test for the presence of β-exotoxin. Biological activity was evaluated against a number of insect species as described in Example 3. The results are as follows:

| | | Percent mortality of culture supernatant | |
|---|---|---|---|
| Insect species tested | Order | Non-autoclaved | Autoclave d |
| *Agrotis ipsilon* | *Lepidoptera* | 100 | 5 |
| *Ostrinia nubilalis* | *Lepidoptera* | 100 | 0 |
| *Spodoptera frugiperda* | *Lepidoptera* | 100 | 4 |
| *Helicoverpa zea* | *Lepidoptera* | 100 | 12 |
| *Heliothis virescens* | *Lepidoptera* | 100 | 12 |
| *Leptinotarsa decemlineata* | *Coleoptera* | 0 | 0 |
| *Diabrotica virgifera virgifera* | *Coleoptera* | 0 | 5 |

The reduction of insecticidal acitivity of the culture supernatant to insignificant levels by autoclaving indicates that the active principle is not β-exotoxin.

Delta-endotoxin crystals were purified from strain AB88 by standard methodologies. No activity from pure crystals was observed when bioassayed against Agrotis ipsilon.

### EXAMPLE 17. PURIFICATION OF VIPS FROM STRAIN AB88:

Bacterial liquid culture was grown overnight [for 12h] at 30°C in TB media. Cells were centrifuged at 5000 x g for 20 minutes and the supernatant retained. Proteins present in the supernatant were precipitated with ammonium sulfate (70% saturation), centrifuged [at 5000 x g for 15 minutes] and the pellet retained. The pellet was resuspended in the original volume of 20 mM Tris pH 7.5 and dialyzed overnight against the same buffer at 4°C. AB88 dialysate was more turbid than comparable material from AB78. The dialysate was titrated to pH 4.5 using 20 mM sodium citrate (pH 2.5) and, after 30 min incubation at room temperature, the solution was centrifuged at 3000 x g for 10 min. The protein pellet was redissolved in 20 mM Bis-Tris-Propane pH 9.0.

AB88 proteins have been separated by several different methods following clarification including isoelectric focusing (Rotofor, BioRad, Hercules, CA), precipitation at pH 4.5, ion-exchange chromotography, size exclusion chromatography and ultrafiltration.
Proteins were separated on a Poros HQ/N anion exchange column (PerSeptive Biosystems, Cambridge, MA) using a linear gradient from 0 to 500 mM NaCl in 20 mM Bis-Tris-Propane pH 9.0 at a flow rate of 4 ml/min. The insecticidal protein eluted at 250 mM NaCl.

European corn borer (ECB)-active protein remained in the pellet obtained by pH 4.5 precipitation of dialysate. When preparative IEF was done on the dialysate using pH 3-10 ampholytes, ECB insecticidal activity was found in all fractions with pH of 7 or greater. SDS-PAGE analysis of these fractions showed protein bands of MW ∼60 kDa and -80 kDa. The 60 kDa and 80 kDa bands were separated by anion exchange HPLC on a Poros-Q column (PerSeptive Biosystems, Cambridge, MA). N-terminal sequence was obtained from two fractions containing proteins of slightly differing MW, but both of approximately 60 kDa in size. The sequences obtained were similar to each other and to some δ-endotoxins.
anion exchange fraction 23 (smaller): xEPFVSAxxxQxxx (SEQ)DNO:10) anion exchange fraction 28 (larger): xEYENVEPFVSAx (SEQ ID NO:11)

When the ECB-active pH 4.5 pellet was further separated by anion exchange on a Poros-Q column, activity was found only in fractions containing a major band of -60 kDa.

Black cutworm-active protein also remained in the pellet when AB88 dialysate was brought down to pH 4.5. In preparative IEF using pH 3-10 ampholytes, activity was not found in the ECB-active IEF fractions; instead, it was highest in a fraction of pH 4.5-5.0. Its major components have molecular weights of ∼35 and ∼80 kDa.

The pH 4.5 pellet was separated by anion exchange HPLC to yield fractions containing only the 35 kDa material and fractions containing both 35 kDa and 80 kDa bands.

### EXAMPLE 18. CHARACTERIZATION OF AB88 VIP.

Fractions containing the various lepidopteran active vegetative proteins were generated as described in Example 17. Fractions with insecticidal acitivity were separated in 8 to 16% SDS-polyacrylamide gels and transferred to PVDF membranes [LeGendre et al, (1989) in: A Practical Guide to Protein and Peptide Purification for Microsequencing, ed Matsudaria PT (Academic Press Inc, New Yorkl]. Biological analysis of fractions demonstrated that different VIPs were responsible for the different lepidopteran species activity.

The *Agrotis ipsilon* activity is due to an 80 kDa and/or a 35 kDa protein, either delivered singly or in combination. These proteins are not related to any δ-endotoxins from Bt as evidenced by the lack of sequence homology of known Bt δ-endotoxin sequences. The vip3A(a) insecticidal protein from strain AB88 is present mostly (at least 75% of the total) in supernatants of AB88 cultures.

Also, these proteins are not found in the AB88 δ-endotoxin crystal. N-terminal sequences of the major δ-endotoxin proteins were compared with the N-terminal sequences of the 80 kDa and 35 kDa VIP and revealed no sequence homology. The N-terminal sequence of the vip3A(a) insecticidal protein posses a number of positively charged residues (from Asn2 to Asn7) followed by a hydrophobic core region (from Thr8 to Ile34). Unlike most of the known secretion proteins, the vip3A(a) insecticidal protein from strain AB88 is not N-terminally processed during export.

A summary of the results follows:

| Agrotis VI P N-terminal sequences | N-terminal sequence of major δ-endotoxin proteins |
|---|---|
| | 130 kDa MDNNPNINE (SEQ ID NO:14) |
| 80 kDa MNKNNTKLPTRALP (SEQ ID NO:12) | 80 kDa MDNNPNINE (SEQ ID NO:15) |
| | 60 kDa MNVLNSGRTTI (SEQ ID NO:16) |
| 35 kDa ALSENTGKDGGYIVP (SEQ ID NO:13) | |

The *Ostrinia nubilalis* activity is due to a 60 kDa VIP and the Spodoptera *frugiperda* activity is due to a VIP of unknown size.

Bacillus *thuringiensis* strain AB88 has been deposited in the Agricultural Research Service, Patent Culture Collection (NRRL), Northern Regional Research Center, 1815 North University Street, Peoria, Illinois 61604, USA and given the Accession No. NRRL B-21225.

### EXAMPLE 18A. ISOLATION AND BIOLOGICAL ACTIVITY OF B. THURINGIENSIS AB424

A B. *thuringiensis* strain, designated AB424, was isolated from a moss covered pine cone sample by standard methods known in the art. A subculture of AB424 was grown and prepared for bioassay as described in Example 2.

Biological activity was evaluated as described in Example 3. The results are as follows:

| Insect species tested | Percent mortality |
|---|---|
| *Ostrinia* nubilalis | 100 |
| *Agrotis ipsilon* | 100 |
| *Diabrotica virgifera virgifera* | 0 |

Strain AB424 has been deposited in the Agricultural Research Service, Patent Culture Collection (NRRL), Northern Regional Research Center, 1815 North University Street, Peoria, lllinois 61604, USA, and given Accession No. NRRL B-21439.

### EXAMPLE 18B. CLONING OF THE VIP3A(a) and VIP3A(b) GENES WHICH ENCODE PROTEINS ACTIVE AGAINST BLACK CUTWORM.

Total DNA from isolates AB88 and AB424 was isolated [Ausubel et al (1988), in: Current Protocols in Molecular Biology (John Wiley & Sons, NY)] and digested with the restriction enzymes *Xba*l [library of 4.0 to 5.0 Kb size-fractionated Xbal fragments of B thuringiensis AB88 DNA] and *EcoR*l [library of 4.5 to 6.0 Kb size-fractionated *EcoR*l fragments B thuringiensis AB424 DNA] respectively, ligated into pBluescript vector previously linearized with the same enzymes and dephosphorylated, and transformed into *E. coli* DH5α strain. Recombinant clones were blotted onto nitrocellulose filters which were subsequently probed with a ³² P labeled 33-bases long oligonucleotide corresponding to the 11-N terminal amino acids of the 80 kDa protein active against Agrotis ipsilon (black cutworm). Hybridization was carried out at 42°C in 2 x SSC/0.1 % SDS (1 x SSC = 0.15 m NaCI/0.015 M sodium citrate, pH 7.4) for 5 min and twice at 50°C in 1 x SSC/0.1 SDS for 10 min. Four out of 400 recombinant clones were positive. Insect bioassays of the positive recombinants exhibited toxicity to black cutworm larvae comparable to that of AB88 or AB424 supernantants.

Plasmid pCIB7104 contains a 4.5 Kb *Xba*l fragment of AB88 DNA. Subclones were constructed to define the coding region of the insecticidal protein.

*E coli* pCIB7105 was constructed by cloning the 3.5 Kb *Xbal-Accl* fragment of pCIB7104 into pBluescript.

Plasmid pCIB7106 contained a 5.0 Kb *EcoR*l fragment of AB424 DNA. This fragment was further digested with *Hincll* to render a 2.8 kb *EcoRl-Hincll* insert (pCIB7107), which still encoded a functional insecticidal protein.

The nucleotide sequence of pCIB7104, a positive recombinant clone from AB88, and of pCIB7107, a positive recombinant clone from AB424, was determined by the dideoxy termination method of Sanger *et al.,* Proc. Natl. Acad. Sci. USA, 74: 5463-5467 (1977), using PRISM Ready Reaction Dye Deoxy Terminator Cycle Sequencing Kits and PRISM Sequenase® Terminator Double-Stranded DNA Sequencing Kit and analysed on an ABI 373 automatic sequencer.

The clone pCIB7104 contains the VIP3A(a) gene whose coding region is disclosed in SEQ ID NO:28 and the encoded protein sequence is disclosed in SEQ ID NO:29. A synthetic version of the coding region designed to be highly expressed in maize is given in SEQ ID NO:30. Any number of synthetic genes can be designed based on the amino acid sequence given in SEQ ID NO:29.

The clone pCIB7107 contains the VIP3A(b) gene whose coding region is disclosed in SEQ ID NO:31 and the encoded protein is disclosed in SEQ ID NO:32. Both pCIB7104 and pCIB7107 have been deposited with the Agricultural Research Service Patent Culture Collection (NRRL) and given Accession Nos. NRRL B-21422 and B-21423, respectively.

The VIP3A(a) gene contains an open reading frame (ORF) that extends form nucleotide 732 to 3105. This ORF encodes a peptide of 791 amino acids corresponding to a molecular mass of 88,500 daltons. A Shine-Dalgarno (SD) sequence is located 6 bases before the first methionine and its sequence identifies a strong SD for *Bacillus*.

The VIP3A(b) gene is 98% identical to VIP3A(a).

When blost of total DNA isolated from AB88 *B thuringiensis* cells were probed with a 33.base fragment that spans the N-terminal region of the VIP3A-insecticidal protein, single bands could be observed in different restriction digests. This result was confirmed by using larger probes spanning the coding region of the gene. A search of the GenBank data base revealed no homology to known proteins.

### EXAMPLE 18C. EXPRESSION OF THE VIP3A INSECTICIDAL PROTEINS

The time course for expression of the VIP3A(a) insecticidal protein was analyzed by western blot. Samples from Bacillus *thuringiensis* Ab88 clutures were taken throughout ist growth curve and sporulation. The VIP3A(a) insecticidal protein can be detected in the supernatants of AB88 cultures during logarithmic phase, as early as 15 h after initiating the culture. It reached its maximum level during early stages of stationary phase and remained at high levels during and after sporulation. Similar results were obtained when supernatants of AB424 Bacillus cereus cultures were used. The levels of VIP3A(a) insecticidal protein reflected the expression of the VIP3A(a) gene as determined by Northern blot. The initiation of the sporulation was determined by direct microscopic observations and by analyzing the presence of δ-endotoxins in cell pellets. Cry-I type prtoeins could be detected late in the stationary phase , during and after sporulation.

### EXAMPLE 18D. IDENTIFICATION OF NOVEL VIP3-LIKE GENES BY HYBRIDIZATION

To identify *Bacillus* containing genes related to the VIP3A(a) from isolate AB88, a collection of *Bacillus* isolates was screened by hybridization. Cultures of 463 *Bacillus* strains were grown in microtiter wells until sporulation. A 96-pin colony stampel was used to transfer the cultures to 150 mm plates containing L-agar. Inoculated plates were kept at 30°C for 10 hours, then at 4°C overnight. Colonies were blotted onto nylon filters and probed with a 1.2Kb *Hind*lll VIP3A(a) derived fragment. Hybridization was performed overnight at 62°C using hybridization conditions of Maniatis et *al.* Molecular Cloning: A Laboratory Manual (1982). Filters were washed with 2xSSC/0.1 % SDS at 62°C and exposed to X-ray film.

Of the 463 *Bacillus* strains screened, 60 contain VIP3-like genes that could detected by hybridization. Further characterization of some of them (AB6 and AB426) showed that their supernatants contain a BCW insecticidal protein similar to the Vip3 protein that are active against black cutworm.

### EXAMPLE 18E. CHARACTERIZATION OF A B. thuringiensis STRAIN M2194 CONTAINING A CRYPTIC VIP3-LIKE GENE

A B. thuringiensis strain, designated M2194, was shown to contain VIP3-like gene(s) by colony hybridization as described in Example 18C. The M2194 VIP3 like gene is considered cryptic since no expression can be detected throughout the bacterial growth phases either by immunoblot analysis using polyclonal antibodies raised against the VIP3A(a) protein isolated from AB88 or by bioassay as described in Example 3.
Antiserum against purified VIP3A(a) insecticidal protein was produced in rabbits. Nictrocellulose-bound protein (50 µg) was dissolved in DMSO and emulsified with Freund's complete adjuvant (Difco). Two rabbits were given subcutaneous injections each month for three month. They were bled 10 days after the second and third injection and the serum was recovered from the blood sample [Harlow et at (1988) in: Antibodies: A Laboratory Manual (Cold Spring Harbor Lab Press, Plainview, NY)].

The M2194 VIP3-like gene was cloned into pKS by following the protocol described in Example 9, which created pCIB7108. *E. coli* containing pCIB7108 which comprises the M2194 VIP3 gene were active against black cutworm demonstrating that the gene encodes a functional protein with insecticidal activity. The plasmid pCIB7108 has been deposited with the Agricultural Research Service Patent Culture Collection (NRRL) and given Accession No. NRRL B-21438.

### EXAMPLE 18F. INSECTICIDAL ACITIVITY OF VIP3A PROTEINS

The activity spectrum of VIP3A insecticidal proteins was qualitatively determined in insect bioassays in which recombinant *E* coli carrying the VIP*A genes were fed to larvae. In these assays, cells carrying the VIP3A(a) and VIP3A(b) genes were insecticidal to *Agrotis ipsilon, Spodoptera frugiperda, Spodoptera exigua, Heliothis virescens* and *Helicoverpa zea.* Under the same expermimental conditions, bacterial extracts containing VIP3A proteins did not show any activity against *Ostrinia nubilalis.*

Effect of VIP*A insecticidal proteins on *Agrotis ipsilon* larvae

| Treatment | (%) Mortality |
|---|---|
| TB medium | 5 |
| AB88 Supernatant | 100 |
| Ab424 Supernatant | 100 |
| Buffer | 7 |
| *E coli* pKS | 10 |
| *E coli* pCIB7104 (AB88) | 100 |
| *E coli* pCIB7105 (AB88) | 100 |
| *E coli* pCIB7106 (AB424) | 100 |
| *E coli* pCIB7107 (AB424) | 100 |
| Effect of VIP3A insecticidal proteins on lepidopteran insect larvae | |

| Treatment | Insect | (%) Mortality |
|---|---|---|
| *E coli* pKS | BCW | 10 |
| | FAW | 5 |
| | BAW | 10 |
| | TBW | 8 |
| | CEW | 10 |
| | ECB | 5 |
| *E coli* pCIB7105 | | |
| *E coli* pCIB7107 | BCW | 100 |
| | FAW | 100 |
| | BAW | 100 |
| | TBW | 100 |
| | CEW | 50 |
| | ECB | 10 |
| BCW = Black Cut Worm; FAW = Fall Army Worm; BAW = Beet Army Worm; TBW = Tobacco Bud Worm; | | |
| CEW = Corn Ear Worm; ECB = European Corn Borer | | |

### EXAMPLE 19. ISOLATION AND BIOLOGICAL ACTIVITY OF OTHER BACILLUS SP.

Other *Bacillus* species have been isolated which produce proteins with insecticidal activity during vegetative growth. These strains were isolated from environmental samples by standard methodologies. Isolates were prepared for bioassay and assayed as described in Examples 2 and 3 respectively. Isolates which produced insecticidal proteins during vegetative growth with activity against Agrotis ipsilon in the bioassay are tabulated below. No correlation was observed between the presence of a δ-endotoxin crystal and vegetative insecticidal protein production.

| Bacillus isolate | Presence of δ-endotoxin crystal | Percent mortality |
|---|---|---|
| AB6 | + | 100 |
| AB53 | - | 80 |
| AB88 | + | 100 |
| AB195 | - | 60 |
| AB211 | - | 70 |
| AB217 | - | 83 |
| AB272 | - | 80 |
| AB279 | - | 70 |
| AB289 | + | 100 |
| AB292 | + | 80 |
| AB294 | - | 100 |
| AB300 | - | 80 |
| AB359 | - | 100 |

Isolates AB289, AB294 and AB359 have been deposited in the Agricultural Research Service, Patent Culture Collection (NRRL), Northern Regional Research Center, 1815 North University Street, Peoria II 61604, USA and given the Accession Numbers NRRL B-21227, NRRL B-21229, and NRRL B-21226 respectively.

*Bacillus* isolates which produce insecticidal proteins during vegetative growth with activity against *Diabrotica virgifera virgifera* are tabulated below.

| *Bacillus* isolate | Presence of δ-endotoxin crystal | Percent mortality |
|---|---|---|
| AB52 | - | 50 |
| AB59 | - | 71 |
| AB68 | + | 60 |
| AB78 | - | 100 |
| AB122 | - | 57 |
| AB218 | - | 64 |
| AB256 | - | 64 |

Isolates AB59 and AB256 have been deposited in the Agricultural Research Service, Patent Culture Collection (NRRL), Northern Regional Research Center, 1815 North University Street, Peoria lllinois 61604, USA, and given the Accession Numbers NRRL B-21228 and NRRL B-21230, respectively.

### EXAMPLE 20. IDENTIFICATION OF NOVEL VIP1/VIP2 LIKE GENES BY HYBRIDIZATION

To identify strains containing genes related to those found in the VIP1A(a)/VIP2A(a) region of AB78, a collection of Bacillus strains was screened by hybridization. Independent cultures of 463 Bacillus strains were grown in wells of 96 well microtiter dishes (five plates total) until the cultures sporulated. Of the strains tested, 288 were categorized as Bacillus thuringiensis, and 175 were categorized as other Bacillus species based on the presence or absence of δ-endotoxin crystals. For each microtiter dish, a 96-pin colony stamper was used to transfer approximately 10 µl of spore culture to two 150 mm plates containing L-agar. Inoculated plates were grown 4-8 hours at 30 °C, then chilled to 4 °C. Colonies were transferred to nylon filters, and the cells lysed by standard methods known in the art. The filters were hybridized to a DNA probe generated from DNA fragments containing both VIP1A(a) and VIP2A(a) DNA sequences. Hybridization was performed overnight at 65 °C using the hybridization conditions of Church and Gilbert (Church, G.M., and W. Gilbert, PNAS, 81:1991-1995 (1984)). Filters were washed with 2x SSC containing 0.1% SDS at 65 °C and exposed to X-Ray film.

Of the 463 *Bacillus* strains screened, 55 strains were identified that hybridized to the VIP1A(a)/VIP2A(a) probe. DNA was isolated from 22 of these strains, and analyzed using a Southern blot with VIP1A(a)/VIP2A(a) DNA as probes. These strains were grouped into 8 classes based on their Southern blot pattern. Each class differed in Southern blot pattern from AB78. One class had a pattern identical to that of the VIP1A(a)/VIP2A(a) homologs from *Bacillus thuringiensis* var *tenebrionis* (see below). Each of the 22 strains was tested for activity against western corn rootworm (WCRW). Three strains, AB433, AB434, and AB435 were found to be active on WCRW. Western blot analysis using VIP2A(a) antisera revealed that strains AB6, AB433, AB434, AB435, AB444, and AB445 produce a protein(s) of equivalent size to VIP2A(a).

Notable among the strains identified was *Bacillus thuringiensis* strain AB6, (NRRL B-21060) which produced a VIP active against black cutworm (*Agrotis ipsilon*) as described in Example 15. Western blot analysis with polyclonal antisera to VIP2A(a) and polyclonal antisera to VIP1A(a) suggests that AB6 produces proteins similar to VIP2A(a) and VIP1A(a). Thus, AB6 may contain VIPs similar to VIP1A(a) and VIP2A(a), but with a different spectrum of insecticidal activity.

### EXAMPLE 21. CLONING OF A VIP1A(a)/VIP2A(a) HOMOLOG FROM BACILLUS THURINGIENSIS VAR. TENEBRIONIS.

Several previously characterized *Bacillus* strains were tested for presence of DNA similar to VIP1A(a)/VIP2A(a) by Southern blot analysis. DNA from *Bacillus* strains AB78, AB88, GC91, HD-1 and ATCC 10876 was analyzed for presence of VIP1A(a)/VIP2A(a) like sequences. DNA from Bt strains GC91 and HD-1, and the Bc strain ATCC 10876 did not hybridize to VlP2A(a)/VlP1A(a) DNA, indicating they lack DNA sequences similar to VlP1A(a)/VlP2A(a) genes. Similarly, DNA from the insecticidal strain AB88 (Example 16) did not hybridize to VIP1A(a)/VIP2A(a) DNA region, suggesting that the VlP activity produced by this strain does not result from VlP1A(a)/VlP2A(a) homologs. In contrast, *Bacillus thuringiensis* var. *tenebrionis* (Btt) contained sequences that hybridized to the VIP1A(a)/VIP2A(a) region. Further analysis confirmed that Btt contains VlP1A(a)/VlP2A(a) like sequences.

To characterize the Btt homologs of VIP2A(a) and VIP1A(a), the genes encoding these proteins were cloned. Southern blot analysis identified a 9.5 kb Eco RI restriction fragment likely to contain the coding regions for the homologs. Genomic DNA was digested with Eco RI, and DNA fragments of approximately 9.5 kb in length were gel-purified. This DNA was ligated into pBluescript SK(+) digested with Eco RI, and transformed into E. coli to generate a plasmid library. Approximately 10,000 colonies were screened by colony hybridization for the presence of VIP2A(a) homologous sequences. Twenty eight positive colonies were identified. All twenty eight clones are identical, and contain VIP1A(a)/VIP2A(a) homologs. Clone pCIB7100 has been deposited in the Agricultural Research Service, Patent Culture Collection (NRRL), Northern Regional Research Center, 1815 North University Street, Peoria lllinois 61604, USA, and given the Accession Number B-21322. Several subclones were constructed from pCIB7100. A 3.8 kb Xba I fragment from pCIB7100 was cloned into pBluescript SK(+) to yield pCIB7101. A 1.8 kb Hind lll fragment and a 1.4 kb Hind lll fragment from pCIB7100 were cloned into pBluescript SK(+) to yield pCIB7102 and pCIB7103, respectively. Subclones pCIB7101, pCIB7102 and pCIB7103 have been deposited in the Agricultural Research Service, Patent Culture Collection (NRRL), Northern Regional Research Center, 1815 North University Street, Peoria lllinois 61604, USA, and given the Accession Numbers B-21323, B-21324 and B-21325 respectively.

The DNA sequence of the region of pCIB7100 containing the VIP2A(a)/VIP1A(a) homologs was determined by the dideoxy chain termination method (Sanger et *al.,* 1977, Proc. Natl. Acad. Sci. USA 74:5463-5467). Reactions were performed using PRISM Ready Reaction Dye Deoxy Terminator Cycle Sequencing Kits and PRISM Sequenase® Terminator Double-Stranded DNA Sequencing Kits, and analyzed on an ABI model 373 automated sequencer. Custom oligonucleotides were used as primers to determine the DNA sequence in certain regions. The DNA sequence of this region is shown in SEQ lD NO:19.

The 4 kb region shown in SEQ ID NO:19 contains two open readings frames (ORFs), which encode proteins with a high degree of similarity to VIP1A(a) and VIP2A(a) proteins from strain AB78. The amino acid sequence of the VIP2A(a) homolog, designated as VIP2A(b) using the standardized nomenclature, is found at SEQ ID NO:20 and the amino acid sequence of the VIP1A(a) homolog, designated as VIP1A(b) using the standardized nomenclature, is disclosed at SEQ ID NO:21. The VIP2A(b) protein exhibits 91% amino acid identity to VIP2A(a) from AB78. An alignment of the amino acid sequences of the two VIP2 proteins is provided in Table 20. The VIP1A(b) protein exhibits 77 % amino acid identity to VIP1A(a) from AB78. An alignment of these two VIP1 proteins is provided in Table 21. The alignment shown in Table 21 discloses the similarity between VIP1A(b) and VIP1A(a) from AB78. This alignment reveals that the amino terminal regions of the two VIP1 proteins share higher amino acid identity in the amino-terminal region than in the carboxy terminal region. In fact, the amino terminal two thirds (up to aa 618 of the VIP1A(b) sequence shown in Table 21 ) of the two proteins exhibit 91 % identity, while the carboxy-terminal third (from aa 619-833 of VIP1A(b)) exhibit only 35% identity.

Western blot analysis indicated that *Bacillus* thuringiensis var. tenebrionis (Btt) produces both VIP1A(a) like and VIP2A(a) like proteins. However, these proteins do not appear to have activity against western corn rootworm. Bioassay for activity against western corn rootworm was performed using either a 24 h culture supernatant from Btt or E. *coli* clone pCIB7100 (which contains the entire region of the VIP1A(a)/VIP2A(a) homologs). No activity against western corn rootworm was detected in either case.

Given the similarity between the VIP2 proteins from Btt and AB78, the ability of VIP2A(b) from Btt to substitute for VIP2A(a) from AB78 was tested. Cells containing pCIB6206 (which produces AB78 VIP1A(a) but not VIP2A(a) protein) were mixed with Btt culture supernatant, and tested for activity against western corn rootworm. While neither Btt culture supernatant nor cells containing pCIB6206 had activity on WCRW, the mixture of Btt and pCIB6206 gave high activity against WCRW. Furthermore, additional bioassay showed that the Btt clone pCIB7100, which contains the Btt VIP1A(b)/VIP2A(b) genes in *E. coli,* also confers activity against WCRW when mixed with pCIB6206. Thus, the VIP2A(b) protein produced by Btt is functionally equivalent to the VIP2A(a) protein produced by AB78.

Thus, the ability to identify new strains with insecticidal activity by using VIP DNA as hybridization probes has been demonstrated. Furthermore, *Bacillus* strains that contain VIP1A(a)/VIP2A(a) like sequences, produce VIP1A(a)/VIP2A(a) like protein, yet demonstrate toxicity toward different insect pests. Similar methods can identify many more members of the VIP1/VIP2 family. Furthermore, use of similar methods can identify homologs of other varieties of VIPs (for example, the VIPs from AB88).

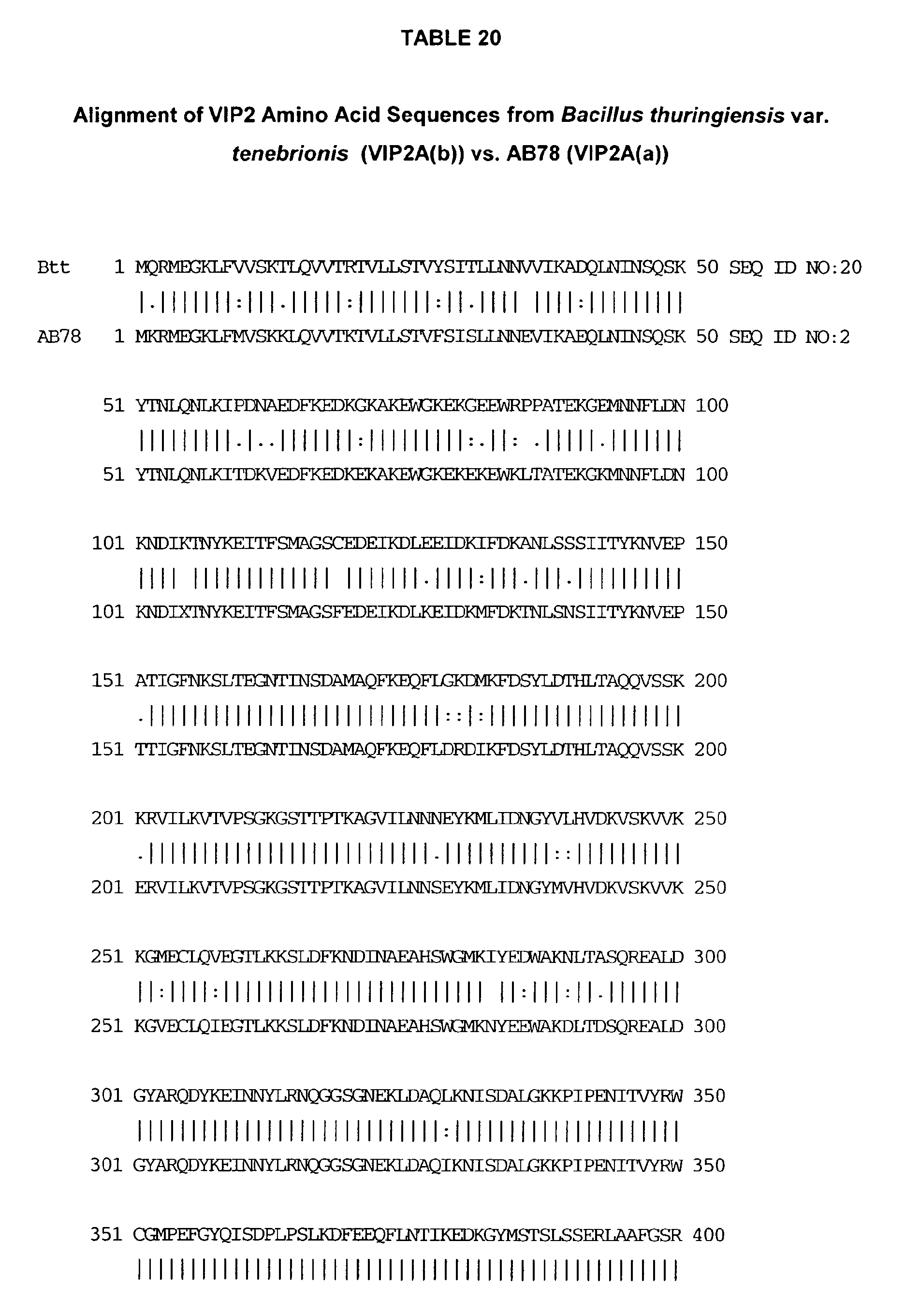

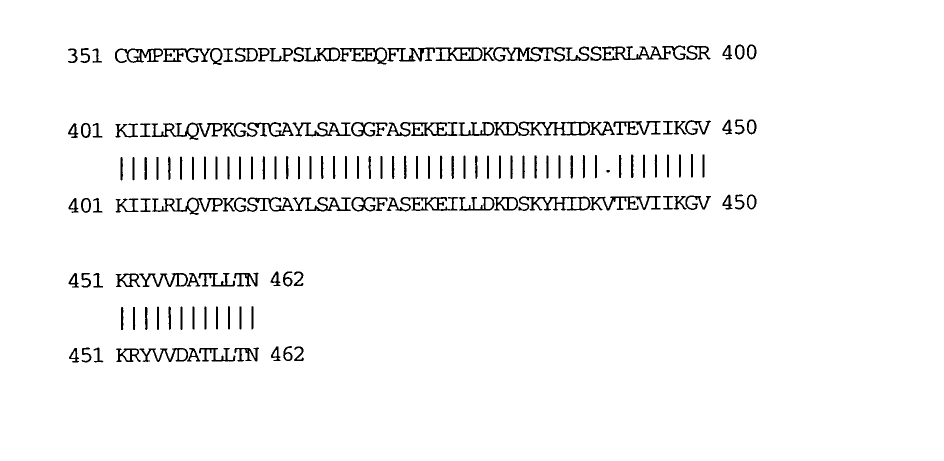

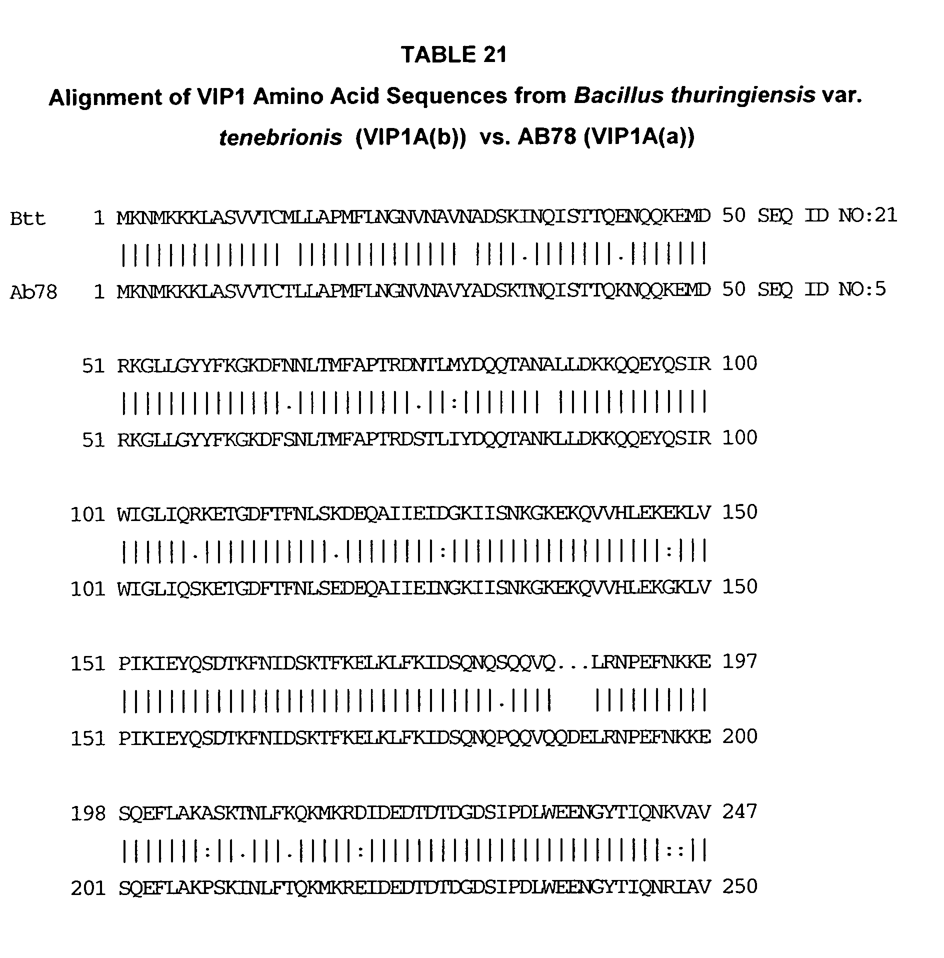

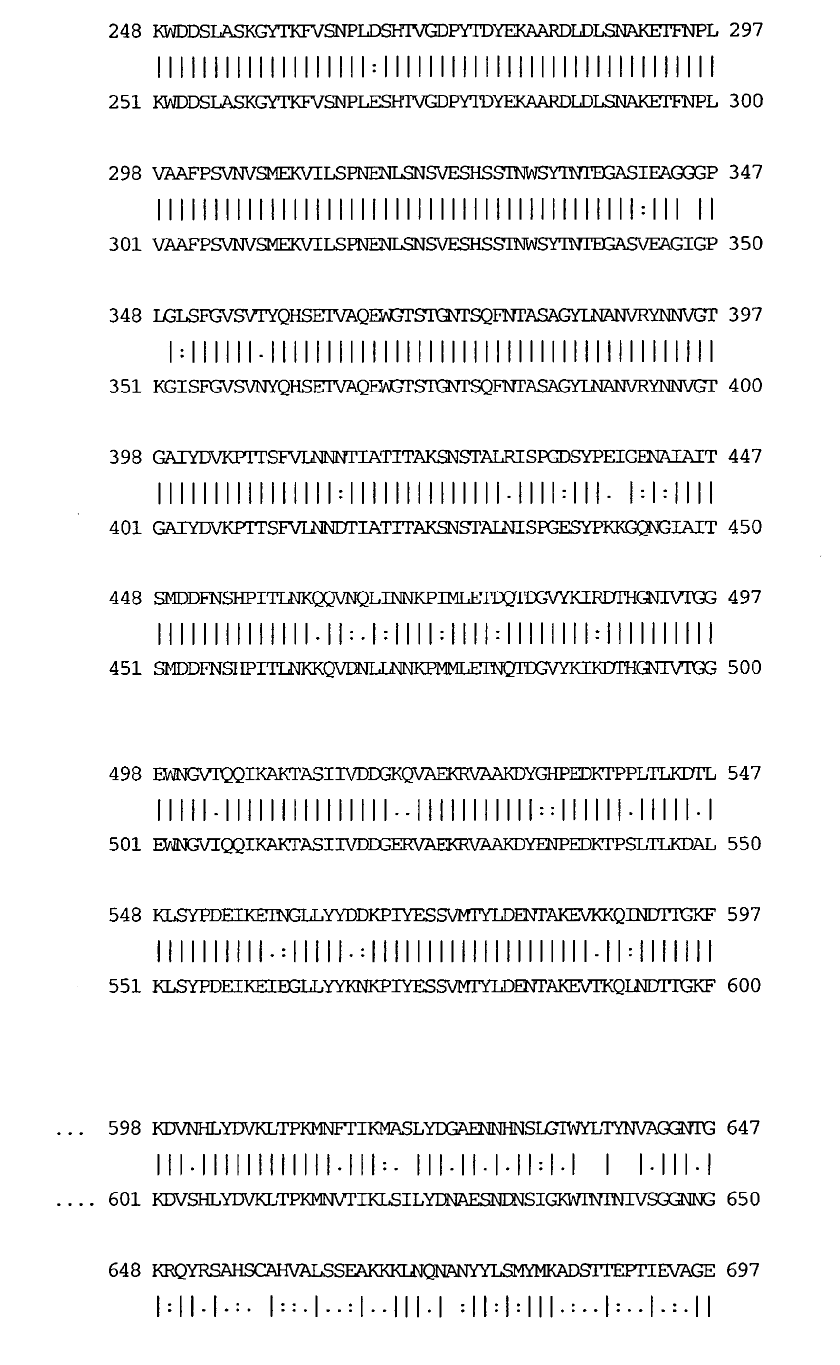

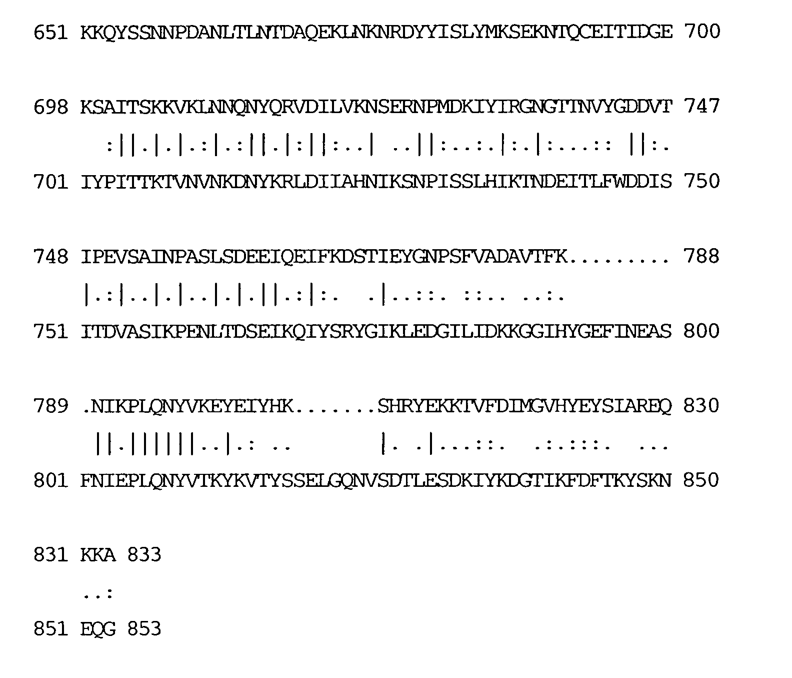

### EXAMPLE 22. FUSION OF VIP PROTEINS TO MAKE A SINGLE POLYPEPTIDE

VIP proteins may occur in nature as single polypeptides, or as two or more interacting polypeptides. When an active VIP is comprised of two or more interacting protein chains, these protein chains can be produced as a single polypeptide chain from a gene resulting from the fusion of the two (or more) VIP coding regions. The genes encoding the two chains are fused by merging the coding regions of the genes to produce a single open reading frame encoding both VIP polypeptides. The composite polypeptides can be fused to produce the smaller polypeptide as the NH₂ terminus of the fusion protein, or they can be fused to produce the larger of the polypeptides as the NH₂ terminus of the fusion protein. A linker region can optionally be used between the two polypeptide domains. Such linkers are known in the art. This linker can optionally be designed to contain protease cleavage sites such that once the single fused polypeptide is ingested by the target insect it is cleaved in the linker region to liberate the two polypeptide components of the active VIP molecule.

VIP1A(a) and VIP2A(a) from *B. cereus* strain AB78 are fused to make a single polypeptide by fusing their coding regions. The resulting DNA comprises a sequence given in SEQ ID NO:22 with the encoded protein given in SEQ ID NO:23. In like manner, other fusion proteins may be produced.

The fusion of the genes encoding VIP1A(a) and VIP2A(a) is accomplished using standard techniques of molecular biology. The nucleotides deleted between the VIP1A(a) and VIP2A(a) coding regions are deleted using known mutagenesis techniques or, alternatively, the coding regions are fused using PCR techniques.

The fused VIP polypeptides can be expressed in other organisms using a synthetic gene, or partially synthetic gene, optimized for expression in the alternative host. For instance, to express the fused VIP polypeptide from above in maize, one makes a synthetic gene using the maize preferred codons for each amino acid, see for example EP-A 0618976, herein incorporated by reference. Synthetic DNA sequences created according to these methods are disclosed in SEQ ID NO:17 (maize optimized version of the 100 kDa VIP1A(a) coding sequence), SEQ ID NO:18 (maize optimized version of the 80 kDa VIP1A(a) coding sequence) and SEQ ID NO:24 (maize optimized version of the VIP2A(a) coding sequence).

Synthetic VIP1 and VIP2 genes optimized for expression in maize can be fused using PCR techniques, or the synthetic genes can be designed to be fused at a common restriction site. Alternatively, the synthetic fusion gene can be designed to encode a single polypeptide comprised of both VIP1 and VIP2 domains.

Addition of a peptide linker between the VIP1 and VIP2 domains of the fusion protein can be accomplished by PCR mutagenesis, use of a synthetic DNA linker encoding the linker peptide, or other methods known in the art.

The fused VIP polypeptides can be comprised of one or more binding domains. If more than one binding domain is used in the fusion, multiple target pests are controlled using such a fusion. The other binding domains can be obtained by using all or part of other VIPs; *Bacillus thuringiensis* endotoxins, or parts thereof; or other proteins capable of binding to the target pest or appropriate biding domains derived from such binding proteins.

One example of a fusion construction comprising a maize optimized DNA sequence encoding a single polypeptide chain fusion having VIP2A(a) at the N-terminal end and VIP1A(a) at the C-terminal end is provided by pCIB5531. A DNA sequence encoding a linker with the peptide sequence PSTPPTPSPSTPPTPS (SEQ ID NO:47) has been inserted between the two coding regions. The sequence encoding this linker and relevant cloning sites is 5'- CCC GGG CCT TCT ACT CCC CCA ACT CCC TCT CCT AGC ACG CCT CCG ACA CCT AGC GAT ATC GGA TC C - 3' (SEQ ID NO:48). Oligonucleotides were synthesized to represent both the upper and lower strands and cloned into a pUC vector following hybridization and phosphorylation using standard procedures. The stop codon in VIP2A(a) was removed using PCR and replaced by the Bglll restriction site with a Smal site. A translation fusion was made by ligating the Bam HI / Pstl fragment of the VIP2A(a) gene from pCIB5522 (see Example 24), a PCR fragment containing the Pstl-end fragment of the VIP2A(a) gene (identical to that used to construct pCIB5522), a synthetic linker having ends that would ligate with a blunt site at the 5' end and with BamHl at the 3' end and the modified synthetic VIP1A(a) gene from pCIB5526 described below (See SEQ ID NO:35). The fusion was obtained by a four way ligation that resulted in a plasmid containing the VIP2A(a) gene without a translation stop codon, with a linker and the VIP1 A(a) coding region without the *Bacillus* secretion signal. The DNA sequence for this construction is disclosed in SEQ ID NO:49, which encodes the fusion protein disclosed in SEQ ID NO:50. A single polypeptide fusion where VIP1A(a) is at the N-terminal end and VIP2A(a) is at the C-terminal end can be made in a similar fashion. Furthermore, either one or both genes can be linked in a translation fusion with or without a linker at either the 5' or the 3' end to other molecules like toxin encoding genes or reporter genes.

### EXAMPLE 23. TARGETING OF VIP2 TO PLANT ORGANELLES

Various mechanisms for targeting gene products are known to exist in plants and the sequences controlling the functioning of these mechanisms have been characterized in some detail. For example, the targeting of gene products to the chloroplast is controlled by a signal sequence found at the amino-terminal end of various proteins. This signal is cleaved during chloroplast import, yielding the mature protein (e.g. Comai *et al.* J. Biol. Chem. 263: 15104-15109 (1988)). These signal sequences can be fused to heterologous gene products such as VIP2 to effect the import of those products into the chloroplast (van den Broeck et *al.* Nature 313: 358-363 (1985)). DNA encoding for appropriate signal sequences can be isolated from the 5' end of the cDNAs encoding the RUBISCO protein, the CAB protein, the EPSP synthase enzyme, the GS2 protein and many other proteins which are known to be chloroplast localized.

Other gene products are localized to other organelles such as the mitochondrion and the peroxisome (e.g. Unger *et al.* Plant Molec. Biol. 13: 411-418 (1989)). The cDNAs encoding these products can also be manipulated to effect the targeting of heterologous gene products such as VIP2 to these organelles. Examples of such sequences are the nuclear-encoded ATPases and specific aspartate amino transferase isoforms for mitochondria. Similarly, targeting to cellular protein bodies has been described by Rogers *et al.* (Proc. Natl. Acad. Sci. USA 82: 6512-6516 (1985)).

By the fusion of the appropriate targeting sequences described above to coding sequences of interest such as VIP2 it is possible to direct the transgene product to any organelle or cell compartment. For chloroplast targeting, for example, the chloroplast signal sequence from the RUBISCO gene, the CAB gene, the EPSP synthase gene, or the GS2 gene is fused in frame to the amino-terminal ATG of the transgene. The signal sequence selected should include the known cleavage site and the fusion constructed should take into account any amino acids after the cleavage site which are required for cleavage. In some cases this requirement may be fulfilled by the addition of a small number of amino acids between the cleavage site and the start codon ATG, or alternatively replacement of some amino acids within the coding sequence. Fusions constructed for chloroplast import can be tested for efficacy of chloroplast uptake by *in vitro* translation of *in vitro* transcribed constructions followed by in vitro chloroplast uptake using techniques described by (Bartlett *et al.* In: Edelmann *et al.* (Eds.) Methods in Chloroplast Molecular Biology, Elsevier. pp 1081-1091 (1982); Wasmann *et al.* Mol. Gen. Genet. 205: 446-453 (1986)). These construction techniques are well known in the art and are equally applicable to mitochondria and peroxisomes.

The above described mechanisms for cellular targeting can be utilized not only in conjunction with their cognate promoters, but also in conjunction with heterologous promoters so as to effect a specific cell targeting goal under the transcriptional regulation of a promoter which has an expression pattern different to that of the promoter from which the targeting signal derives.

A DNA sequence encoding a secretion signal is present in the native *Bacillus* VlP2 gene. This signal is not present in the mature protein which has the N-terminal sequence of LKITDKVEDF (amino acid residues 57 to 66 of SEQ ID NO:2). It is possible to engineer VIP2 to be secreted out of the plant cell or to be targeted to subcellular organelles such as the endoplasmic reticulum, vacuole, mitochondria or plastids including chloroplasts. Hybrid proteins made by fusion of a secretion signal peptide to a marker gene have been successfully targeted into the secretion pathway. (Itirriaga G. *et al.,* The Plant Cell, 1: 381-390 (1989) , Denecke *et al.*, The Plant Cell, 2:51-59 (1990). Amino-terminal sequences have been identified that are responsible for targeting to the ER, the apoplast, and extracellular secretion from aleurone cells (Koehler & Ho, Plant Cell 2: 769-783 (1990)).

The presence of additional signals are required for the protein to be retained in the endoplasmic reticulum or the vacuole. The peptide sequence KDEUHDEL at the carboxy-terminal of a protein is required for its retention in the endoplasmic reticulum (reviewed by Pelham, Annual Review Cell Biol., 5:1-23 (1989). The signals for retention of proteins in the vacuole have also been characterized. Vacuolar targeting signals may be present either at the amino-terminal portion, (Holwerda *et al.,* The Plant Cell, 4:307-318 (1992), Nakamura *et al.,* Plant Physiol., 101:1-5 (1993)), carboxy-terminal portion, or in the internal sequence of the targeted protein. (Tague *et al.*, The Plant Cell, 4:307-318 (1992), Saalbach *et al.,* The Plant Cell, 3:695-708 (1991)). Additionally, amino-terminal sequences in conjunction with carboxy-terminal sequences are responsible for vacuolar targeting of gene products (Shinshi *et al.* Plant Molec. Biol. 14: 357-368 (1990)). Similarly, proteins may be targeted to the mitochondria or plastids using specific carboxy terminal signal peptide fusions (Heijne *et al.,* Eur. J. Biochem., 180:535-545 (1989), Archer and Keegstra, Plant Molecular Biology, 23:1105-1115 (1993)).

In order to target VIP2, either for secretion or to the various subcellular organelles, a maize optimized DNA sequence encoding a known signal peptide(s) may be designed to be at the 5' or the 3' end of the gene as required. To secrete VIP2 out of the cell, a DNA sequence encoding the eukaryotic secretion signal peptide MGWSWIFLFLLSGAAGVHCL (SEQ ID NO:25) from PCT application No. IB95/00497 or any other described in the literature (Itirriaga *et al*., The Plant Cell,-1:381-390 (1989) , Denecke, et al., The Plant Cell, 2:51-59 (1990)) may be added to the 5' end of either the complete VIP2 gene sequence or to the sequence truncated to encode the mature protein or the gene truncated to nucleotide 286 or encoding a protein to start at amino acid residue 94 (methionine). To target VIP2 to be retained in the endoplasmic reticulum, a DNA sequence encoding the ER signal peptide KDEL /HDEL, in addition to the secretion signal, can be added to the 3' end of the gene. For vacuolar targeting a DNA sequence encoding the signal peptide SSSSFADSNPIRVTDRAAST (SEQ ID NO:3; Holwerda *et al.,* The Plant Cell, 4:307-318 (1992)) can be designed to be adjacent to the secretion signal or a sequence encoding a carboxyl signal peptide as described by Dombrowski *et al.,* The Plant Cell, 5:587-596 (1993) or a functional variation may be inserted at the 3' end of the gene. Similarly, VIP2 can be designed to be targeted to either the mitochondria or the plastids, including the chloroplasts, by inserting sequences in the VIP2 sequence described that would encode the required targeting signals. The bacterial secretion signal present in VIP2 may be retained or removed from the final construction.

One example of a construction which incorporates a eukaryotic secretion signal fused to a coding sequence for a VIP is provided by pCIB5528. Oligonucleotides corresponding to both the upper and lower strand of sequences encoding the secretion signal peptide of SEQ ID NO:25 was synthesized and has the sequence 5'-GGATCCACC ATG GGC TGG AGC TGG ATC TTC CTG TTC CTG CTG AGC GGC GCC GCG GGC GTG CAC TGC CTGCAG-3' (SEQ ID NO:41). When hybridized, the 5' end of the secretion signal resembled "sticky-ends" corresponding to restriction sites BamHl and Pstl. The oligonucleotide was hybridized and phosphorylated and ligated into pCIB5527 (construction described in Example 23A) which had been digested with BamHl/ Pstl using standard procedures. The resulting maize optimized coding sequence is disclosed in SEQ ID NO:42 which encodes the protein disclosed in SEQ ID NO:43. This encoded protein comprises the eukaryotic secretion signal in place of the Bacillus secretion signal.

One example of a construction which incorporates a vacuolar targetting signal fused to a coding sequence for a VIP is provided by pCIB5533. Oligonucleotides corresponding to both the upper and lower strand of sequences encoding the vacuolar targetting peptide of SEQ ID NO:3 was synthesized and has the sequence 5'-CCG CGG GCG TGC ACT GCC TCA GCA GCA GCA GCT TCG CCG ACA GCA ACC CCA TCC GCG TGA CCG ACC GCG CCG CCA GCA CCC TGC AG-3' (SEQ ID NO:44). When hybridized, the 5' end of the vacuolar targetting signal resembled "sticky-ends" corresponding to restriction sites Sacll and Pstl. The oligonucleotide was hybridized and phosphorylated and ligated into pCIB5528 (construction described above) which had been digested with Sacll / Pstl using standard procedures. The resulting maize optimized coding sequence is disclosed in SEQ ID NO:45 which encodes the protein disclosed in SEQ ID NO:46. This encoded protein comprises the vacuolar targetting peptide in addition to the eukaryotic secretion signal.

The VIP1 gene can also be designed to be secreted or targeted to subcellular organelles by similar procedures.

### EXAMPLE 23A. REMOVAL OF BACILLUS SECRETION SIGNAL FROM VIP1A(a) AND VIP2A(a)

VIP1A(a) and VIP2A(a) are secreted during the growth of strain AB78. The nature of peptide sequences that act as secretion signals has been described in the literature (Simonen and Palva, Microbiological reviews, pg. 109-137 (1993)). Following the information in the above publication, the putative secretion signal was identified in both genes. In VIP1A(a) this signal is composed of amino acids 1-33 (*See* SEQ ID NO:5). Processing of the secretion signal probably occurs after the serine at amino acid 33. The secretion signal in VIP2A(a) was identified as amino acids 1-49 (*See* SEQ ID NO:2). N-terminal peptide analysis of the secreted mature VIP2A(a) protein revealed the N-terminal sequence LKITDKVEDFKEDK. This sequence is found beginning at amino acid 57 in SEQ ID NO:2. The genes encoding these proteins have been modified by removal of the *Bacillus* secretion signals.

A maize optimized VIP1A(a) coding region was constructed which had the sequences encoding the first 33 amino acids, i.e., the secretion signal, removed from its 5' end. This modification was obtained by PCR using an forward primer that contained the sequence 5'-GGA TCC ACC ATG AAG ACC AAC CAG ATC AGC-3' (SEQ ID NO:33), which hybridizes with the maize optimized gene (SEQ ID NO:26) at nucleotide position 100, and added a BamHl restriction site and a eukaryotic translation start site consensus including a start codon. The reverse primer that contained the sequence 5'-AAG CTT CAG CTC CTT G-3' (SEQ ID NO:34) hybridizes on the complementary strand at nucelotide position 507. A 527 bp amplification product was obtained containing the restriction sites BamHl at the 5' end and Hindlll site at the 3' end. The amplification product was cloned into a T- vector (described in Example 24, below) and sequenced to ensure the correct DNA sequence. The BamHl / HindIII fragment was then obtained by restriction digest and used to replace the BamHl/Hindlll fragment of the maize optimized VIP1A(a) gene cloned in the root-preferred promoter cassette. The construct obtained was designated pCIB5526. The maize optimized coding region for VIP1 A(a) with the *Bacillus* secretion signal removed is disclosed as SEQ ID NO:35 and the encoded protein is disclosed as SEQ ID NO:36.

The gene encoding the processed form of VIP2A(a), i.e., a coding region with the secretion signal removed, was constructed by a procedure similar to that described for that used to construct the processed form of VIP1A(a), above. The modification was obtained by PCR using the forward primer 5'-GGA TCC ACC ATG CTG CAG AAC CTG AAG ATC AC -3' (SEQ ID NO:37). This primer hybridizes at nucleotide position 150 of the maize optimized VIP2A(a) gene (SEQ ID NO:27). A silent mutation has been inserted at nucleotide position 15 of this primer to obtain a Pstl restriction site. The reverse primer has the sequence 5'-AAG CTT CCA CTC CTT CTC-3' (SEQ ID NO:38). A 259 bp product was obtained with Hindlll restriction site at the 3' end. The amplification product was cloned into a T- vector, sequenced and ligated to a BamHl /Hindlll digested root-preferred promoter cassette containing the maize optimized VIP2A(a). The construct obtained was designated pCIB5527. The maize optimized coding region for VIP2A(a) with the *Bacillus* secretion signal removed is disclosed as SEQ ID NO:39 and the encoded protein is disclosed as SEQ ID NO:40.

### EXAMPLE 24. CONSTRUCTION AND CLONING OF THE VIP1A(a) AND VIP2A(a) MAIZE OPTIMIZED GENES

*Design*: The maize optimized genes were designed by reverse translation of the native VIP1A(a) and VIP2A(a) protein sequences using codons that are used most often in maize (Murray *et al.,* Nucleic Acid Research, 17:477-498 (1989)). To facilitate cloning, the DNA sequence was further modified to incorporate unique restriction sites at intervals of every 200-360 nucleotides. VIP1 A(a) was designed to be cloned in 11 such fragments and VIP2A(a) was cloned in 5 fragments. Following cloning of the individual fragments, adjacent fragments were joined using the restriction sites common to both fragments, to obtain the complete gene. To clone each fragment, oligonucleotides (50-85 nucleotides) were designed to represent both the upper and the lower strand of the DNA. The upper oligo of the first oligo pair was designed to have a 15 bp single stranded region at the 3' end which was homologous to a similar single stranded region of the lower strand of the next oligo pair to direct the orientation and sequence of the various oligo pairs within a given fragment. The oligos are also designed such that when the all the oligos representing a fragment are hybridized, the ends have single stranded regions corresponding to the particular restriction site to be formed. The structure of each oligomer was examined for stable secondary structures such as hairpin loops using the OLIGO program from NBI Inc. Whenever neccesary, nucleotides were changed to decrease the stability of the secondary structure without changing the amino acid sequence of the protein. A plant ribosomal binding site consensus sequence, TAAACAATG (Joshi et al., Nucleic Acid Res., 15:6643-6653 (1987)) or eukaryotic ribosomal binding site concensus sequence CCACCATG (Kozak, Nucleic Acid Research, 12:857-872 (1984)) was inserted at the translational start codon of the gene.

**Cloning:** Oligos were synthesized by IDT Inc., and were supplied as lyophilized powders. They were resuspended at a concentration of 200 µM. To 30 µl of each oligo formamide was added a final concentration of 25-50% and the sample was boiled for two minutes before separation on a premade 10% polyacryamide / urea gel obtained from Novex. After electrophoresis, the oligo was detected by UV shadowing by placing the gel on a TLC plate containing a fluorescent indicator and exposing it to UV light. The region containing DNA of the correct size was excised and extracted from the polyacryamide by an overnight incubation of the minced gel fragment in a buffer containing 0.4 M LiCl, 0.1 mM EDTA. The DNA was separated from the gel residue by centrifugation through a Millipore UFMC filter. The extracted DNA was ethanol precipitated by the addition of 2 volumes of absolute alcohol. After centrifugation, the precipitate was resuspended in dH₂O at a concentration of 2.5 µM. Fragments were cloned either by hybridization of the oligos and ligation with the appropriate vector or by amplification of the hybridized fragment using a equimolar mixture of all the oligos for a particular fragment as a template and end-specific PCR primers.

**Cloning by hybridization and ligation:** Homologous double stranded oligo pairs were obtained by mixing 5 µl of the upper and of the lower oligo for each oligo pair with buffer containing 1X polynucleotide kinase (PNK) buffer (70 mM Tris-HCl (pH 7.6), 10 mM MgCl₂,5 mM dithiothreitol (DTT)), 50 mM KCl, and 5 % formamide in a final volume of 50 µl. The oligos were boiled for 10 minutes and slow cooled to 37° C or room temperature. 10 µl was removed for analysis on a 4% agarose in a TAE buffer system (Metaphore®; FMC). Each hybridized oligo pair was kinased by the addition of ATP at a final concentration of 1 mM, BSA at a final concentration of 100 µg per ml and 200 units of polynucleotide kinase and 1 µl of 10X PNK buffer in a volume of 10 µl. Following hybridization and phosphorylation, the reaction was incubated at 37° C for 2 hours to overnight. 10 µl of each of the oligo pairs for a particular fragment, were mixed in a final volume of 50 µl. The oligo pairs were hybridized by heating at 80° C for 10 minutes and slow cooling to 37° C. 2 µl of oligos was mixed with about 100 ng of an appropriate vector and ligated using a buffer containing 50 mM Tris-HCl (pH 7.8), 10 mM MgCl₂, 10 mM DTT, 1 mM ATP. The reaction was incubated at room temp. for 2 hours to overnight and transformed into DH5α strain of *E.coli* plated on L- plates containing ampicillin at a concentration of 100 µg/ml using standard procedures. Positive clones were further characterized and confirmed by PCR miniscreen described in detail in EP-A 0618976 using the universal primers "Reverse" and M13 "-20 " as primers. Positive clones were identified by digestion of DNA with appropriate enzymes followed by sequencing. Recombinants that had the expected DNA sequence were then selected for further work.

### PCR Amplification and cloning into T- vector:

PCR amplification was carried out by using a mixture of all the oligomers that represented the upper and the lower strand of a particular fragment ( final concentration 5 mM each) as template, specific end primers for the particular fragment ( final concentration 2 µM) 200 µM of each dATP, dTTP, dCTP and dGTP, 10 mM Tris-HCl (pH 8.3), 50 mM KCl, 1.5 mM MgCl₂,0.01 % gelatin and 5 units of Taq polymerase in a final reaction volume of 50 µl. The amplification reaction was carried out in a Perkin Elmer thermocycler 9600 by incubation at 95° C for 1 min (1 cycle ), followed by 20 cycles of 95 °C for 45 sec., 50 °C for 45 sec., 72 °C for 30 sec. Finally the reaction was incubated for 5 min at 72°C before analyzing the product. 10 µl of the reaction was analyzed on a 2.5% Nusieve (FMC) agarose gel in a TAE buffer system. The correct size fragment was gel purified and used for cloning into a PCR cloning vector or T-vector. T-vector construction was as described by Marchuk *et al.,* Nucleic Acid Research, 19:1154 (1991). pBluescriptsk+ (Stratagene®, Ca.) was used as the parent vector. Transformation and identification of the correct clone was carried out as described above.

Fragments 1, 3, 4, 5, 6, 8, and 9 of VIP1A(a) and fragments 2 and 4 of VIP2A(a) were obtained by cloning of PCR amplification products; whereas, fragments 2, 7, 10 and 11 of VIP1A(a) and fragments 1, 3, and 5 of VIP2A(a) were obtained by hybridization/ ligation.

Once fragments with the desired sequence were obtained, the complete gene was assembled by cloning together adjacent fragments. The complete gene was resequenced and tested for activity against WCRW before moving it into plant expression vectors containing the root preferred promoter (disclosed in U.S. patent application serial no. 08/017,209, herein incorporated by reference) and the rice actin promoter.

One such plant expression vector is pCIB5521. The maize optimized VIP1A(a) coding region (SEQ ID NO:26) was cloned in a plant expression vector containing the root preferred promoter at the 5' of the gene with the PEP Carboxylase intron #9 followed by the 35S terminator at the 3' end. The plasmid also contains sequences for ampicillin resistance from the plasmid pUC19. Another plant expression vector is pCIB5522, which contains the maize optimized VIP2A(a) coding region (SEQ ID NO:27) fused to the root preferred promoter at the 5' of the gene with the PEP Carboxylase intron #9 followed by the 35S terminator at the 3' end.

### EXAMPLE 25. NAD AFFINITY CHROMATOGRAPHY

A purification strategy was used based on the affinity of VIP2 for the substrate NAD. The supernatant from the pH 3.5 sodium citrate buffer treatment described in Example 4 was dialyzed in 20 mM TRIS pH 7.5 overnight. The neutralized supernatant was added to an equal volume of washed NAD agarose and incubated with gentle rocking at 4° C overnight. The resin and protein solution were added to a 10 ml disposable polypropylene column and the protein solution allowed to flow out. The column was washed with 5 column volumes of 20 mM TRIS pH 7.5 then washed with 2-5 column volumes of 20 mM TRIS pH 7.5, 100 mM NaCl, followed by 2-5 column volumes of 20 mM TRIS 7.5. The VIP proteins were eluted in 20 mM TRIS pH 7.5 supplemented with 5 mM NAD. Approximately 3 column volumes of the effluent were collected and concentrated in a Centricon -10. Yield is typically about 7-15 µg of protein per ml of resin.

When the purified proteins were analyzed by SDS-PAGE followed by silver staining, two polypeptides were visible, one with Mr of approximately 80,000 and one with Mr of approximately 45,000. N-terminal sequencing revealed that the Mr 80,000 protein corresponded to a proteolytically processed form of VIP1A(A) and the Mr 45,000 form corresponded to a proteolytically processed form of VIP2A(a). The co-purification of VIP1A(a) with VIP2A(a) indicates that the two proteins probably form a complex and have protein-protein interacting regions. VIP1A(a) and VIP2A(a) proteins purified in this manner were biologically active against western corn rootworm.

### EXAMPLE 26. EXPRESSION OF MAIZE OPTIMIZED VIP1A(a) AND VIP2A(a)

*E.* coli strains containing different plasmids comprising VIP genes were assayed for expression of VIPs. *E. coli* strains harboring the individual plasmids were grown overnight in L-broth and expressed protein was extracted from the culture as described in Example 3, above. Protein expression was assayed by Western Blot analysis using antibodies developed using standard methods known in the art, similar to those described in Example 12, above. Also, insecticidal activity of the expressed proteins were tested against Western corn rootworm according to the method in Example 3, above. The results of the *E. coli* expression assays are described below.

| Expression of VIPs in *E. coli* | | | |
|---|---|---|---|
| **Extract of E. coli Strain Harboring Indicated Plasmid** | **Assay No. *1*** | **Assay No. 2** | **Protein Detected** |
| | % Mortality | | |
| Control | 0 | 0 | no |
| pCIB5521 (maize optimized VIP1A(a)) | 47 | 27 | yes |
| pCIB5522 (maize optimized VIP2A(a)) | 7 | 7 | yes |
| pCIB6024 (native VIP2A(a)) | 13 | 13 | yes |
| pCIB6206 (native VIP1A(a)) | 27 | 40 | yes |
| Extracts pCIB5521 + pCIB5522 combined | 87 | 47 | |
| Extracts pCIB5521 + pCIB6024 combined | 93 | 100 | |
| Extracts pCIB5522 + pCIB6206 combined | 100 | 100 | |
| Extracts pCIB6024 + pCIB6206 combined | 100 | 100 | |

The DNA from these plasmids was used to transiently express the VIPs in a maize protoplast expression system. Protoplasts were isolated from maize 2717 Line 6 suspension cultures by digestion of the cell walls using Cellulase RS and Macerase R10 in appropriate buffer. Protoplasts were recovered by sieving and centrifugation. Protoplasts were transformed by a standard direct gene transfer method using approximately 75 µg plasmid DNA and PEG-40. Treated protoplasts were incubated overnight in the dark at room temperature. Analysis of VIP expression was accomplished on protoplast explants by Western blot analysis and insecticidal activity against Western corn rootworm as described above for the expression in *E. coli.* The results of the maize protoplast expression assays are described below.

| **Expression of VIPs in Plant Protoplasts** | | | |
|---|---|---|---|
| ***Extract Tested*** | **Assay No. *1*** | ***Assay No. 2*** | ***Protein Detected*** |
| | % Mortality | | |
| No DNA control | 27 | 10 | no |
| pCIB5521 (p) (maize optimized VIP1A(a)) | 20 (0) | 30 | yes |
| pCIB5522 (p) (maize optmizied VIP2A(a)) | 20 (0) | 20 | yes |
| Extracts pCIB5521 (p) + pCIB5522 (p) combined | 87 (82) | 90 | |
| Extracts pCIB5521 (p) + pCIB5522 (e) combined | 100 | - | |
| Extracts pCIB5522 (p) + pCIB5521 (e) combined | 53 (36) | - | |
| Extracts pCIB5521 (p)+ | 100 | - | |
| pCIB6024 (e) combined | | | |
| Extracts pCIB5522 (p) + pCIB6206 (e) combined | 100 | - | |
| pCIB6024(e) (native VIP2A(a)) | 0 | - | yes |
| pCIB6206(e) (native VIP1A(a)) | 20 | - | yes |
| pCIB5521 + pCIB 5522 (plasmids delivered by cotransformation) | 100 | 100 | yes |

| | | | |
|---|---|---|---|
| (p) = extract of protoplast culture transformed with indicated plasmid | | | |
| (e) = extract of *E. coli* strain harboring indicated plasmid | | | |

The expression data obtained with both *E. coli* and maize protoplasts show that the maize optimized VIP1A(a) and VIP2A(a) genes make the same protein as the native VIP1A(a) and VIP2A(a) genes, respectively, and that the proteins encoded by the maize optimized genes are functionally equivalent to the proteins encoded by the native genes.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The following deposits have been made at Agricultural Research Service, Patent Culture Collection (NRRL), Northern Regional Research Center, 1815 North University Street, Peoria, lllinois 61604, USA:

| Strain designation Deposition Number Deposition Date | | | |
|---|---|---|---|
| - *1.* | *E. coli* PL2 | NRRL B-21221 | March 09, 1994 |
| 2. | *E. coli* PL2 | NRRL B-21221 N | September 02, 1994 |
| 3. | *E. coli* pCIB6022 | NRRL B-21222 | March 09, 1994 |
| 4. | *E. coli* pClB6023 | NRRL B-21223 | March 09, 1994 |
| 5. | *E. coli* pClB6023 | NRRL B-21223N | September 02, 1994 |
| 6. | *Bacillus thuringiensis* HD73-78VlP | NRRL B-21224 | March 09, 1994 |
| 7. | *Bacillus thuringiensis* AB88 | NRRL B-21225 | March 09, 1994 |
| 8. | *Bacillus thuringiensis* AB359 | NRRL B-21226 | March 09, 1994 |
| 9. | *Bacillus thuringiensis* AB289 | NRRL B-21227 | March 09, 1994 |
| 10. | *Bacillus* sp. AB59 | NRRL B-21228 | March 09, 1994 |
| 11. | *Bacillus* sp. AB294 | NRRL B-21229 | March 09, 1994 |
| 12. | *Bacillus* sp. AB256 | NRRL B-21230 | March 09, 1994 |
| 13. | *E. coli* P5-4 | NRRL B-21059 | March 18, 1993 |
| 14. | *E. coli* P3-12 | NRRL B-21061 | March 18, 1993 |
| 15. | *Bacillus cereus* AB78 | NRRL B-21058 | March 18, 1993 |
| 16. | *Bacillus thuringiensis* AB6 | NRRL B-21060 | March 18, 1993 |
| 17. | *E. coli* pCIB6202 | NRRL B-21321 | September 02, 1994 |
| 18. | *E. coli* pCIB7100 | NRRL B-21322 | September 02, 1994 |
| 19. | *E. coli* pCIB7101 | NRRL B-21323 | September 02, 1994 |
| 20. | *E. coli* pCIB7102 | NRRL B-21324 | September 02, 1994 |
| 21. | *E. coli* pCIB7103 | NRRL B-21325 | September 02, 1994 |
| 22. | *E. coli* pCIB7104 | NRRL B-21422 | March 24, 1995 |
| 23. | *E. coli* pCI B7107 | NRRL B-21423 | March 24, 1995 |
| 24. | *E. coli* pCIB7108 | NRRL B-21438 | May 05, 1995 |
| 25. | *Bacillus thuringiensis* AB424 | NRRL B-21439 | May 05, 1995 |

Although the foregoing invention has been described in some detail by way of illustration and example for purposes of clarity of understanding, it will be obvious that certain changes and modifications may be practiced within the scope of the appended claims.

### Embodiments of the Invention (Summary)

The present invention comprises the following embodiments:
1. A substantially purified *Bacillus* strain which produces a pesticidal protein during vegetative growth wherein said Bacillus is not *B. sphaericus* SSII-1.
2. A *Bacillus* strain which produces a pesticidal protein during vegetative growth, wherein said *Bacillus* is *Bacillus cereus* having Accession No. NRRL B-21058.
3. A *Bacillus* strain which produces a pesticidal protein during vegetative growth, wherein said *Bacillus* is *Bacillus thuringiensis* having Accession No. NRRL B-21060
4. A Bacillus strain which produces a pesticidal protein during vegetative growth, wherein said Bacillus is a Bacillus selected from Accession Numbers NRRL B-21224, NRRL B-21225, NRRL B-21226, NRRL B-21227, NRRL B-21228, NRRL B-21229, NRRL B-21230, and NRRL B-21439.
5. An insect-specific protein isolatable during the vegetative growth phase of *Bacillus* spp. and components thereof, wherein said protein is not the mosquitocidal toxin from B. *sphaericus* SSII-1.
6. The insect-specific protein of embodiment 5 wherein said *Bacillus* is selected from a *Bacillus*_{*-*}*thuringiensis* and *B. cereus.*
7. The insect-specific protein of embodiment 5 wherein said protein is toxic to Coleoptera or Lepidoptera.
8. The insect-specific protein of embodiment 5 wherein the spectrum of insecticidal activity includes an activity against Agrotis and/or *Spodoptera* species, but preferably a black cutworm [Agrotis *ipsilon;* BCW] and/or fall armyworm [*Spodoptera frugiperda]* and/or beet armyworm [*Spodoptera exigua* ] and/or tobacco budworm and/or corn earworm [*Helicoverpa zea]* activity.
9. The insect-specific protein of embodiment 5, wherein said *Bacillus* is *Bacillus cereus* having Accession No. NRRL B-21058.
10. The insect-specific protein of embodiment 5, wherein said *Bacillus* is *Bacillus thuringiensis* having Accession No. NRRL B-21060.
11. The insect-specific protein of embodiment 5, wherein said Bacillus is a Bacillus selected from Accession Numbers NRRL B-21224, NRRL B-21225, NRRL B-21226, NRRL B-21227, NRRL B-21228, NRRL B-21229, NRRL B-21230, and NRRL B-21439.
12. The insect-specific protein of embodiment 5 wherein said protein has a molecular weight of about 30 kDa or greater.
13. The insect-specific protein of embodiment 12 wherein said protein has a molecular weight of about 60 to about 100 kDa.
14. The insect-specific protein of embodiment 13, wherein said protein has a molecular weight of about 80 kDa.
15. The insect-specific protein of embodiment 5, wherein said protein comprises a sequence selected from the group consisting of SEQ ID NO:2, SEO ID NO:5, SEQ ID NO:7, including homologues thereof.
16. The insect-specific protein of embodiment 5, wherein said protein has the sequence selected from the group consisting of SEQ ID NO:20, SEQ ID NO:21, SEQ ID NO:29 SEQ ID NO:32 and SEQ ID NO:2 including homologues thereof.
17. The insect-specific protein of embodiment 8, wherein said protein has the sequence selected from the group consisting of SEQ ID NO:29 and SEQ ID NO:32 including homologues thereof.
18. An insect-specific protein according to any one of embodiments 5 to 15, wherein the sequences representing the secretion signal have been removed or inactivated.
19. An auxiliary protein which enhances the insect-specific activity of an insect-specific protein.
20. The auxiliary protein of embodiment 19 wherein said auxiliary protein has a molecular weight of about 50 kDa.
21. The auxiliary protein of embodiment 19 wherein said auxiliary protein is from *Bacillus cereus.*
22. The auxiliary protein of any one of embodiments 19 to 21 wherein both the said auxiliary protein as well as said insect-specific protein is from strain AB78.
23. An auxiliary protein according to any one embodiments 19 to 22, wherein the sequences representing the secretion signal have been removed or inactivated.
24. A multimeric pesticidal protein, which comprises more than one polypeptide chain and wherein at least one of the said polypeptide chains represents an insect-specific protein of any one of embodiments 5 to 18 and at least one of the said polypeptide chains represents an auxiliary protein according to any one of embodiments 19 to 23, which activates or enhances the pesticidal activity of the said insect-specific protein.
25. The multimeric pesticidal protein according to embodiment 24 having a molecular weight of about 50 kDa to about 200 kDa.
26. The multimeric pesticidal protein of embodiment 25 comprising an insect-specific protein of any one of embodiments 5 to 18 and an auxiliary protein according to any one of embodiments 19 to 23, which activates or enhances the pesticidal activity of the said insect-specific protein.
27. A fusion protein comprising several protein domains including at least an insect-specific protein of any one of embodiments 5 to 18 and/or an auxiliary protein according to any one of embodiments 19 to 23 produced by in frame genetic fusions, which, when translated by ribosomes, produce a fusion protein with at least the combined attributes of the insect-specific protein of any one of embodiments 5 to 18 and/or an auxiliary protein according to any one of embodiments 19 to 23 and, optionally, of the other components used in the fusion.
28. A fusion protein according to embodiment 27, comprising a ribonuclease S-protein, an insect-specific protein of any one of embodiments 5 to 18 and an auxiliary protein according to any one of embodiments 19 to 23.
29. A fusion protein according to embodiment 27 comprising an insect-specific protein according to embodiment 5 and an auxiliary protein according to embodiment 19 having either the insect-specific protein or the auxiliary protein at the N-terminal end of the said fusion protein.
30. A fusion protein according to embodiment 29, comprising an insect-specific protein as given in SEQ ID NO:5 and an auxiliary protein as given in SEQ ID NO: 2 resulting in the protein given in SEO ID NO: 23 including homologues thereof.
31. A fusion protein according to embodiment 29, comprising an insect-specific protein as given in SEQ ID NO:35 and an auxiliary protein as given in SEQ ID NO: 27 resulting in the protein given in SEQ ID NO: 50 including homologues thereof.
32. A fusion protein according to embodiment 28 comprising an insect-specific protein of any one of embodiments 5 to 18 and/or an auxiliary protein according to any one of embodiments 19 to 23 fused to a signal sequence, which is of herterologous origin with respect to the recipient protein.
33. A fusion protein according to embodiment 32, wherein the said signal sequence is a secretion signal.
34. A fusion protein according to embodiment 32, wherein the said signal sequence is a targeting sequence that directs the transgene product to a specific organelle or cell compartment.
35. A fusion protein according to embodiment 33 wherein the said protein has a sequence as given in SEQ ID NO: 43 including homologues thereof.
36. A fusion protein according to embodiment 34 wherein the said protein has a sequence as given in SEQ ID NO: 46 including homologues thereof.
37. A DNA molecule comprising a nucleotide sequence which encodes the protein of any one of embodiments 5-7, 9,10, 12-15, and 19-22.
38. A DNA molecule comprising a nucleotide sequence which encodes the protein of any one of embodiments 8, 11, 16-18 and 23 to 36.
39. A DNA molecule comprising a nucleotide sequence which encodes an insect-specific protein isolatable during the vegetative growth phase of *Bacillus* spp. and components thereof, wherein said protein is not the mosquitocidal toxin from *B. sphaericus* SSII-1.
40. The DNA molecule of embodiment 39, wherein the said molecule comprises a nucleotide sequence as given in SEQ ID NO: 4, or SEQ ID NO: 6 including homologues thereof.
41. The DNA molecule of embodiment 39, wherein the said molecule comprises a nucleotide sequence as given SEQ ID NO:19, SEQ ID NO:28, SEQ ID NO:31, or SEQ ID NO:1 including homologues thereof.
42. A DNA molecule comprising a nucleotide sequence which encodes an auxiliary protein which enhances the insect-specific activity of an insect-specific protein.
43. The DNA molecule of embodiment 42 wherein the said molecule comprises a nucleotide sequence as given SEQ ID NO:19 including homologues thereof.
44. The DNA molecule according to any one of embodiments 37, 39, 40 or 42 which comprises a nucleotide sequence that has been optimized for expression in a microorganism.
45. The DNA molecule according to embodiment 37, 39, 40 or 42 which comprises a nucleotide sequence that has been optimized for expression in a plant.
46. The DNA molecule according to any one of embodiments 38, 41, or 43 which comprises a nucleotide sequence that has been wholly or partially optimized for expression in a microorganism.
47. The DNA molecule according to embodiment 38, 41 or 43 which comprises a nucleotide sequence that has been optimized for expression in a plant.
48. The DNA molecule of embodiment 45, wherein the said molecule comprises a nucleotide sequence as given in SEQ ID NO:17 or SEQ ID NO:18 including homologues thereof.
49. The DNA molecule of embodiment 47, wherein the said molecule comprises a nucleotide sequence as given in SEQ ID NO:24, SEQ ID NO:26, SEQ ID NO:27, or SEQ ID NO:30 including homologues thereof.
50. A DNA molecule which comprises a nucleotide sequence encoding a multimeric pesticidal protein, which comprises more than one polypeptide chains and wherein at least one of the said polypeptide chains represents an insect-specific protein of any one of embodiments 5 to 18 and at least one of the said polypeptide chains represents an auxiliary protein according to any one of embodiments 19 to 23, which activates or enhances the pesticidal activity of the said insect-specific protein.
51. The DNA molecule of embodiment 50 comprising a nucleotide sequence encoding an insect-specific protein of any one of embodiments 5 to 18 and an auxiliary protein according to any one of embodiments 19 to 23, which activates or enhances the pesticidal activity of the said insect-specific protein.
52. The DNA molecule of embodiment 51, wherein said molecule comprises a nucleotide sequence as given in SEQ ID NO:1 or SEQ ID NO:19 including homologues thereof.
53. A DNA molecule which encodes a fusion protein comprising several protein domains including at least an insect-specific protein of any one of embodiments 5 to 18 and/or an auxiliary protein according to any one of embodiments 19 to 23 produced by in frame genetic fusions, which, when translated by ribosomes, produce a fusion protein with at least the combined attributes of the insect-specific protein of any one of embodiments 5 to 18 and/or an auxiliary protein according to any one of embodiments 19 to 23 and, optionally, of the other components used in the fusion.
54. The DNA molecule of embodiment 53 which encodes a fusion protein comprising an insect-specific protein according to embodiment 5 and an auxiliary protein according to embodiment 19 having either the insect-specific protein or the auxiliary protein at the N-terminal end of the said fusion protein.
55. The DNA molecule of embodiment 53, wherein the said molecule comprises a nucleotide sequence as given in SEQ ID NO:22 including homologues thereof.
56. The DNA molecule of embodiment 53 which encodes a fusion protein comprising an insect-specific protein of any one of embodiments 5 to 18 and/or an auxiliary protein according to any one of embodiments 19 to 23 fused to a signal sequence, which is of herterologous origin respective to the recipient DNA.
57. The DNA molecule of embodiment 56, wherein the said signal sequence is a secretion signal.
58. The DNA molecule of embodiment 56, wherein the said signal sequence is a targeting sequence that directs the transgene product to a specific organelle or cell compartment.
59. The DNA molecule according to any one of embodiments 53 to 58, wherein at least one of its component sequences comprises a nucleotide sequence that has been optimized for expression in a microorganism.
60. The DNA molecule according to any one of embodiments 53 to 58, wherein at least one of its component sequences comprises a nucleotide sequence that has been optimized for expression in a plant.
61. The DNA molecule of embodiment 60, wherein the said molecule comprises a nucleotide sequence as given in SEQ ID NO:42, SEO ID NO:45, or SEQ ID NO:49 including homologues thereof.
62. The DNA molecule of embodiment 45, wherein the sequences encoding the secretion signal have been removed from its 5' end.
63. The DNA molecule of embodiment 62, wherein the said molecule comprises a nucleotide sequence as given in SEQ ID NO: 35 or SEQ ID NO:39 including homologues thereof.
64. A DNA molecule which hybridizes to a DNA molecule according to any one of embodiments 37-63 under moderately stringent conditions and which molecule has insect-specific activity.
65. The DNA molecule of embodiment 64, wherein hybridization occurs at 65°C in a buffer comprising 7% SDS and 0.5 M sodium phosphate.
66. An insect specific protein wherein the said protein is encoded by a DNA molecule according to embodiments 64 or 65.
67. An expression cassette comprising a DNA molecule according to any one of embodiments 37, 39, 40, 42, 44, 45 or 48 operably linked to plant expression sequences including the transcriptional and translational regulatory signals necessary for expression of the associated DNA constructs in a host organism and optionally further regulatory sequences.
68. An expression cassette comprising a DNA molecule according to any one of embodiments 38, 41, 43, 46, 47 or 49-65 operably linked to plant expression sequences including the transcriptional and translational regulatory signals necessary for expression of the associated DNA constructs in a host organism and optionally further regulatory sequences.
69. An expression cassette according to embodiment 67, wherein the said host organism is a plant.
70. An expression cassette according to embodiment 68, wherein the said host organism is a plant.
71. A vector molecule comprising an expression cassette according to embodiment 67 or 69.
72. A vector molecule comprising an expression cassette according to embodiment 68 or 70.
73. An expression cassette according to embodiments 69 or 70 or a vector molecule according to embodiments 71 or 73 which is part of the plant genome.
74. A host organism comprising a DNA molecule according to any one of embodiments 37, 39, 40, 42, 44, 45 or 48, an expression cassette comprising the said DNA molecule or a vector molecule comprising the said expression cassette, preferably stably incorporated into the genome of the host organism..
75. A host organism comprising a DNA molecule according to any one of embodiments 38, 41, 43, 46, 47 or 49-65, an expression cassette comprising the said DNA molecule or a vector molecule comprising the said expression cassette, preferably stably incorporated into the genome of the host organism..
76. A host organism according to embodiment 74 or 75, selected from the group consisting of plant and insect cells, bacteria, yeast, baculoviruses, protozoa, nematodes and algae.
77. A transgenic plant including parts as well as progeny and seed thereof comprising a DNA molecule according to any one of embodiments 37, 39, 40, 42, 44, 45 or 48, an expression cassette comprising the said DNA molecule or a vector molecule comprising the said expression cassette, preferably stably incorporated into the plant genome.
78. A transgenic plant including parts as well as progeny and seed thereof comprising a DNA molecule according to any one of embodiments 38, 41, 43, 46, 47 or 49-65, an expression cassette comprising the said DNA molecule or a vector molecule comprising the said expression cassette, preferably stably incorporated into the plant genome.
79. A transgenic plant including parts as well as progeny and seed thereof which has been stably transformed with a DNA molecule according to any one of embodiments 38, 41, 43, 46, 47 or 49-65.
80. A transgenic plant including parts as well as progeny and seed thereof which expresses an insect-specific protein according to any one of embodiments 5, 7, 9, 10, 12-15, or 19-22.
81. A transgenic plant including parts as well as progeny and seed thereof which expresses an insect-specific protein according to any one of embodiments 8, 11, 16-18, 23-36 or 66.
82. The transgenic plant according to embodiment 80 or 81, which further expresses a second distinct insect control principle.
83. The transgenic plant of embodiment 82, wherein said second insect control principle is a *Bt* δ-endotoxin.
84. A transgenic plant according to any one of embodiments 77-83, which is a maize plant.
85. A transgenic plant according to any one of embodiments 77 to 84, which is a hybrid plant.
86. Plant propagating material of a plant according to any one of embodiments 77 to 84 treated with a seed protectant coating.
87. A microorganism transformed with an expression cassette according to any one of embodiments 67 to 70 and/or a vector molecule according to any one of embodiments 71 or 72, wherein the said microorganism is preferably a microorganism that multiply on plants.
88. The microorganism of embodiments 87, which is a root colonizing bacterium.
89. An encapsulated insect-specific protein which comprises a microorganism of any one of embodiments 87 or 88 comprising an insect specific protein according to embodiments 18 or 23.
90. An entomocidal composition comprising a host organism of any one of embodiments 74-76 in an insecticidally-effective amount together with a suitable carrier.
91. An entomocidal composition comprising a purified *Bacillus strain according to any one of embodiments 1 to 4* in an insecticidally-effective amount together with a suitable carrier.
92. An entomocidal composition comprising an isolated protein molecule according to any one of embodiments 5 to 36 and 66, alone or in combination with a host organism of any one of embodiments 74-76 and/or an encapsulated insect-specific protein according to embodiment 89 in an insecticidally-effective amount, together with a suitable carrier.
93. A method of obtaining a purified insect-specific protein according to any one of embodiments 5 to 36 said method comprising applying a solution comprising said insect-specific protein to a NAD column and eluting bound protein.
94. A method for identifying insect activity of an insect-specific protein according to any one of embodiments 5 to 36, said method comprising:
   (a) growing a *Bacillus* strain in a culture;
   (b) obtaining supernatant from said culture;
   (c) allowing insect larvae to feed on diet with said supernatant; and,
   (d) determining mortality.
95. A method for isolating an insect-specific protein according to any one of embodiments 5 to 36, said method comprising:
   (a) growing a *Bacillus* strain in a culture;
   (b) obtaining supernatant from said culture; and,
   (c) isolating said insect-specific protein from said supernatant.
96. A method for isolating a DNA molecule comprising a nucleotide sequence encoding an insect-specific protein exhibiting the insecticidal activity of the proteins according to any one of embodiments 5 to 36, said method comprising:
   (a) obtaining a DNA molecule comprising a nucleotide sequence encoding an insect-specific protein; and
   (b) hybridizing said DNA molecule with DNA obtained from a *Bacillus* species; and
   (c) isolating said hybridized DNA.
97. A method of increasing insect target range by using an insect specific protein according to any one of embodiments 5 to 36 in combination with at least one second insecticidal protein that is different from the insect specific protein according to any one of embodiments 5 to 36.
98. A method of increasing insect target range wherein an insect specific protein according to any one of embodiments 5 to 36 is expressed in a plant together with a at least one second insecticidal protein that is different from the insect specific protein according to any one of embodiments 5 to 36.
99. A method according to embodiment 97 or 98 wherein the second insecticidal protein is selected from the group consisting of Bt δ-endotoxins, protease inhibitors, lectins, α-amylases and peroxidases.
100. A method of protecting plants against damage caused by an insect pest comprising applying to the plant or the growing area of the said plant an entomocidal composition according to any one of embodiments 90 to 92.
101. A method of protecting plants against damage caused by an insect pest comprising applying to the plant a toxin protein according to any one of embodiments 5 to 36.
102. A method of protecting plants against damage caused by an insect pest comprising planting a transgenic plant expressing a insect-specific protein according to any one of embodiments 5 to 36 within an area where the said insect pest may occur.
103. A method of producing a host organism according to embodiment 74 to 76 comprising transforming the said host organism with a DNA molecule according to any one of embodiments 67 to 70 and 73 or a vector molecule according to embodiment 71 and 72.
104. A method of producing a transgenic plant or plant cell according to any one of embodiments 77 to 85 comprising transforming the said plant and plant cell, respectively, with an expression cassette according to any one of embodiments 70 or 73 or a vector molecule according to embodiment 72.
105. A method of producing an entomocidal composition according to any one of embodiments 90 to 92 comprising mixing a *Bacillus* strain according to any one of embodiments 1 to 4 and/or a host organism according to embodiment 74 to 76 and/or an isolated protein molecule according to any one of embodiments 5 to 36 and 66, and/or an encapsulated protein according to embodiment 89 in an insecticidally-effective amount with a suitable carrier.
106. A method of producing transgenic progeny of a transgenic parent plant comprising stably incorporated into the plant genome a DNA molecule comprising a nucleotide sequence encoding an insect-specific protein according to any one of embodiments 5 to 36 and 66 comprising transforming the said parent plant with an expression cassette according to any one of embodiments 70 or 73 or a vector molecule according to embodiment 72, and transferring the pesticidal trait to the progeny of the said transgenic parent plant involving known plant breeding techniques.
107. A oligonucleotide probe capable of specifically hybridizing to a nucleotide sequence encoding an insect-specific protein isolatable during the vegetative growth phase of *Bacillus* spp. and components thereof, wherein said protein is not the mosquitocidal toxin from *B. sphaericus* SSII-1 , wherein said probe comprises a contiguous portion of the coding sequence for the said insect-specific protein at least 10 nucleotides in length.
108. Use of a oligonucleotide probe for screening of any *Bacillus* strain or other organisms to determine whether the insect-specific protein is naturally present or whether a particular transformed organism includes the said gene.
109. A DNA molecule comprising a nucleotide sequence which encodes the protein of any one of embodiments 8, 11, 16-18 and 23 to 36 obtainable by a process comprising
   (a) obtaining a DNA molecule comprising a nucleotide sequence encoding an insect-specific protein; and
   (b) hybridizing said DNA molecule with an oligonucleotide probe acording to embodiment 107 obtained from a DNA molecule comprising a nucleotide sequence as given in SEQ lD NO: 28, SEQ lD NO: 30, or SEQ lD NO: 31; and
   (c) isolating said hybridized DNA.

## Claims

1. A DNA molecule encoding a pesticidal protein isolatable during the vegetative growth phase of Bacillus spp. or variants and fragments of the protein retaining pesticidal activity, wherein said protein is isolatable from liquid culture media, and wherein said protein is encoded by a nucleotide sequence that hybridizes to a nucleotide sequence of SEQ ID NO: 19, SEQ ID NO:22 or SEQ ID NO:24 at 65°C in a buffer comprising 7% SDS and 0.5 M sodium phosphate.

2. A DNA molecule according to claim 1 encoding a protein as defined by SEQ ID NO: 20, SEQ ID NO: 21 or SEQ ID NO: 23, or having the nucleotide sequence given in SEQ ID NO: 19 or SEQ ID NO: 22.

3. A DNA molecule according to claim 1 having the nucleotide sequence given in SEQ ID NO: 24, SEQ ID NO:26, SEQ ID NO: 27.

4. A pesticidal protein encoded by a DNA molecule according to claim 1.

5. A pesticidal protein according to claim 4 having the amino acid sequence given in SEQ ID NO: 20, 21 or 23.

6. An auxiliary protein which enhances the pesticidal activity of a pesticidal protein according to any one of claims 4 or 5.

7. A multimeric pesticidal protein, which comprises more than one polypeptide chain and wherein at least one of the said polypeptide chains represents an pesticidal protein according to any one of claims 4 or 5 and wherein at least one of the said polypeptide chains represents an auxiliary protein according to claim 6, which activates or enhances the pesticidal activity of the said pesticidal protein.

8. A fusion protein comprising several protein domains including at least a pesticidal protein according to any one of claims 4 or 5 and / or an auxiliary protein according to claim 6.

9. A DNA molecule encoding a protein according to any one of claims 7 to 8.

10. A host organism comprising a DNA molecule according to any one of claims 1 to 3 or 9, an expression cassette comprising said DNA molecule, or a vector molecule comprising said expression cassette, preferably stably incorporated into the genome of the host organism.

11. A transgenic plant including parts as well as progeny and seed thereof stably transformed with a DNA molecule according to any one of claims 1 to 3 or 9.

12. A transgenic plant according to claim 11 expressing a protein according to any one of claims 4 to 8.

13. A transgenic plant according to claim 12 further expressing a distinct insect control principle such as a Bt δ-endotoxin.

14. A transgenic plant according to any one of claims 11 to 13 which is selected from the group consisting of maize, sorghum, wheat, sunflower, cotton, rice, soybean, barley and oil seed rape.
